# EUROPEAN PATENT APPLICATION

(11) **EP 4 445 911 A1**
(43) Date of publication of application: **16.10.2024**
(21) Application number: 22903211.5
(22) Date of filing: 24.11.2022
(51) Int. Cl.: A61K 39/02, A61K 39/108, A61K 39/395, A61K 39/40

(54) **BISPECIFIC ANTIBODY THAT SPECIFICALLY BINDS TO KLEBSIELLA PNEUMONIAE O2 AND O1 ANTIGENS, AND COMPOSITION**

(30) Priority: 06.12.2021 CN 202111476395
(71) Applicant: Beijing SoloBio Genetechnology Co., Ltd., Beijing 100176 (CN)
(72) Inventor: NIU, Yuqiang, Beijing 100176 (CN); LI, Ping, Beijing 100176 (CN); LI, Zhong, Beijing 100176 (CN)
(74) Representative: Barker Brettell LLP
(86) International application number: PCT/CN2022/133880
(87) International publication number: WO 2023/103788

(57) **Abstract**

The present application pertains to antibodies or antigen-binding fragments that specifically bind to *Klebsiella pneumoniae (K. pneumoniae)* O2 antigen, antibodies or antigen-binding fragments that specifically bind to *K. pneumoniae* O1 antigen, and bispecific antibodies that specifically bind to *K. pneumoniae* O2 antigen and *K. pneumoniae* O1 antigen, and pharmaceutical compositions comprising the antibodies or antigen-binding fragments and/or bispecific antibodies, as well as methods of manufacture and uses thereof.

## Description

The content of the following sequence listing is incorporated herein by reference in its entirety: file name: bispecific antibody specifically binding to*Klebsiella pneumoniae* O2 antigen and O1 antigen and compositions thereof, date recorded: November 18th, 2022, size: 161 KB).

### FIELD OF THE APPLICATION

This application pertains to antibodies that specifically bind to *Klebsiella pneumoniae (K. pneumoniae)* O2 antigen, bispecific antibodies that specifically bind to *K. pneumoniae* O2 antigen and *K. pneumoniae* O1 antigen, pharmaceutical compositions comprising antibodies that specifically bind to *K. pneumoniae* O2 antigen and antibodiesthat specifically bind to *K. pneumoniae* O1 antigen and/or bispecific antibodies that specifically bind to *K. pneumoniae* O1 antigen and O2 antigen, as well as methods of manufacture and uses thereof, including methods of treating and preventing *Klebsiellainfections.*

### BACKGROUND OF THE APPLICATION

The *Klebsiella bacteria* belongs to gram-negative bacteria, and is a hospital opportunistic pathogenwhich causes diseases such as pneumonia, meningitis, liver abscess, urinary system inflammation, woundinfection or septicemia. *Klebsiella* infection is mainly caused by *Klebsiella pneumoniae,* which is the medically most important *Klebsiella*species(Podschun, R, and U Ullmann. Clinical microbiology reviews vol. 11,4 (1998): 589-603.). In recent years, the resistance of *Klebsiella pneumoniae* strain has increased and the rate of resistant strain has increased year by year, which makesthe bacterial infections difficult totreat.

*Klebsiella* virulence factors include capsular polysaccharides (CPS), lipopolysaccharides (LPS), adhesion factors, siderophore systems, and other virulence factors. These virulence factors play an important role in the process of adhesion of bacteria to host cells, the evasion of hostimmune responses/immune killing, etc. Wherein lipopolysaccharide (LPS) plays a very important role in bacterialpathogenesis and is an important molecule for causing systemic inflammatory response of cells. LPS is a majorand essential component of all gram-negative bacterial cell membrane outer leaves. Although structural diversity is greater in the regions of LPS between bacteria, it is generally composed of lipid A, coreoligosaccharide and O antigen ((Paczosa, Michelle K, and Joan Mecsas. Microbiology and molecular biology reviews: MMBR vol. 80,3 629-61. 15 Jun. 2016). *Klebsiella*LPSserotypes can be distinguished by unique O antigen structure, of which the most common are the O1serotypeand O2serotype (Follador, R. et al. The diversity of Klebsiella pneumoniae surface polysaccharides. Microb. Genom. 2, e000073 (2016)).

The prior art has demonstrated the promotion of bacterial killing in vitro by antibodies that specifically bind to LPS O antigen is effective. Thereby, developing new antibodies that bind to different LPS O antigens or bispecific antibodies that simultaneously bind to different LPS O antigens, as well as pharmaceutical compositions comprising antibodies that bind to different LPS O antigens, is crucial for supplementing antibiotic therapy.

The disclosures of all publications, patents, patent applications and published patent applications referred to herein are hereby incorporated herein by reference in their entirety.

### BRIEF SUMMARY OF THE APPLICATION

In some embodiments, the present application providesan isolated antibody or antigen-binding fragment specifically binding to *K. pneumoniae* O2 antigen, comprising: (i)a V_{H} comprising an HC-CDR1, an HC-CDR2, and an HC-CDR3 of a V_{H} comprising the amino acid sequence of SEQ ID NO: 17; and a V_{L} comprising an LC-CDR1, an LC-CDR2, and an LC-CDR3 of a V_{L} comprising the amino acid sequence of SEQ ID NO: 21; (ii) a V_{H} comprising an HC-CDR1, an HC-CDR2, and an HC-CDR3 of a V_{H} comprising the amino acid sequence of SEQ ID NO: 18; and a V_{L} comprising an LC-CDR1, an LC-CDR2, and an LC-CDR3 of a V_{L} comprising the amino acid sequence of SEQ ID NO: 22; (iii) a V_{H} comprising an HC-CDR1, an HC-CDR2, and an HC-CDR3 of a V_{H} comprising the amino acid sequence of SEQ ID NO: 19; and a V_{L} comprising an LC-CDR1, an LC-CDR2, and an LC-CDR3 of a V_{L} comprising the amino acid sequence of SEQ ID NO: 23; (iv) a V_{H} comprising an HC-CDR1, an HC-CDR2, and an HC-CDR3 of a V_{H} comprising the amino acid sequence of SEQ ID NO: 19; and a V_{L} comprising an LC-CDR1, an LC-CDR2, and an LC-CDR3 of a V_{L} comprising the amino acid sequence of SEQ ID NO: 24; or(v) a V_{H} comprising an HC-CDR1, an HC-CDR2, and an HC-CDR3 of a V_{H} comprising the amino acid sequence of SEQ ID NO: 20; and a V_{L} comprising an LC-CDR1, an LC-CDR2, and an LC-CDR3 of a V_{L} comprising the amino acid sequence of SEQ ID NO: 25.

In some embodiments, there is provided anisolated antibody or antigen-binding fragment specifically binding to *K. pneumoniae* O2 antigen, comprising: (i) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 12, and an LC-CDR3 comprising the amino acid sequence of SEQ ID NO: 15, or a variant thereof comprising up to about 5 amino acid substitutions in the LC-CDRs; (ii) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 9, an LC-CDR2 comprising the amino acid sequence of SEQ ID NO: 12, and an LC-CDR3 comprising the amino acid sequence of SEQ ID NO: 16, or a variant thereof comprising up to about 5 amino acid substitutions in the LC-CDRs; (iii) a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 10, an LC-CDR2 comprising the amino acid sequence of SEQ ID NO: 13, and an LC-CDR3 comprising the amino acid sequence of SEQ ID NO: 16, or a variant thereof comprising up to about 5 amino acid substitutions in the LC-CDRs; (iv) a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16, or a variant thereof comprising up to about 5 amino acid substitutions in the LC-CDRs; or(v) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7, or a variant thereof comprising up to about 5 amino acid substitutions in the HC-CDRs; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15, or a variant thereof comprising up to about 5 amino acid substitutions in the LC-CDRs.

In some embodiments, there is provided anisolated antibody or antigen-binding fragment specifically binding to *K. pneumoniae* O2 antigen, comprising: (i) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 21, or a variant thereof having at least about 80% sequence identity to the amino acid sequence of SEQ ID NO: 21; (ii) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 22, or a variant thereof having at least about 80% sequence identity to the amino acid sequence of SEQ ID NO: 22;(iii) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 23, or a variant thereof having at least about 80% sequence identity to the amino acid sequence of SEQ ID NO: 23; (iv) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 24, or a variant thereof having at least about 80% sequence identity to the amino acid sequence of SEQ ID NO: 24; or(v) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 25, or a variant thereof having at least about 80% sequence identity to the amino acid sequence of SEQ ID NO: 25.

In some embodiments, there is provided anisolated antibody or antigen-binding fragment specifically binding to *K. pneumoniae*O2 antigen, which is the antibody or antigen-binding fragment specifically binding to the D-galactan I domain within LPS side chain of *K. pneumoniae.In* some embodiments, there is provided anisolated antibody specifically binding to *K. pneumoniae* O2 antigen, which binds to *K. pneumoniae* with D-galactan I domain competitively with any one of the isolated antibodies described above. In some embodiments, there is provided anisolated antibody specifically binding to *K. pneumoniae* O2 antigen, which binds to the same epitope as any one ofthe isolated antibodies specifically binding to *K. pneumoniae* O2 antigen described above.

In some embodiments, according to any one of the isolated antibodies specifically binding to *K. pneumoniae* O2 antigen described above, comprising an Fc region. In some embodiments, the isolated antibody specifically binding to *K. pneumoniae* O2 antigen is a full-length IgGantibody. In some embodiments, the isolated antibody specifically binding to *K. pneumoniae* O2 antigen is a full-length IgG1 or IgG4 antibody. In some embodiments, the isolated antibody specifically binding to *K. pneumoniae* O2 antigen is chimeric, human, or humanized.In some embodiments, the isolated antibody specifically binding to *K. pneumoniae* O2 antigen is an antigen-binding fragment, wherein the antigen-binding fragment is selected from Fab, Fab', F(ab)'2, Fab'-SH, single-chain Fv (scFv), Fv fragment, dAb, Fd, nanobody, diabodyor linear antibody.

In some embodiments, there is provided an isolated nucleic acid moleculethat encodes any one of the antibodies specifically binding to *K. pneumoniae* O2 antigen described above. In some embodiments, there is provided a vector comprising any one of the nucleic acid molecules described above.In some embodiments, there is provided a host cell comprising any one of the antibodies specifically binding to *K. pneumoniae* O2 antigen described above, any one of the nucleic acid molecules described above, or any one of the vectors described above. In some embodiments, there is provided a method of producing an antibody specifically binding to *K. pneumoniae* O2 antigen, comprising: a) culturing any one of the host cells described above under conditions effective to express the antibody specifically binding to *K. pneumoniae* O2 antigen; and b) obtaining the expressed antibody from the host cell.

In some embodiments, there are provided pharmaceutical compositions, kits and articles of manufacture comprising any one of the antibodies specifically binding to *K. pneumoniae* O2 antigen described above.

In some embodiments, there is provided a method of treating and/or preventing a disease or condition in an individual in need thereof, comprising administering to the individual an effective amount ofany one of the antibodies specifically binding to *K. pneumoniae O2* antigen described aboveora pharmaceutical composition containing thereof. In some embodiments, there is providedthe use of any one of the antibodies specifically binding to *K. pneumoniae* O2 antigen described above for the preparation of pharmaceutical compositions for treating a disease or condition in an individual in need. In some embodiments, there is providedthe use of any one of the antibodies specifically binding to *K. pneumoniae O2* antigen described above ora pharmaceutical composition containing thereof for the preparation of a medicament for treating a disease or condition in an individual in need. In some embodiments, the disease or conditioncomprises the condition related to*Klebsiella* infection. In some embodiments, the disease or conditioncomprises pneumonia, urinary tract infection, septicaemia/bacteremia/sepsis, neonatal septicaemia/bacteremia/sepsis, diarrhea, soft tissue infection, infection after organ transplantation, surgical infection, wound infection, lung infection, purulent liver abscess, lung abscess, cellulitis, necrotizing myositis, myositis, endophthalmitis, peritonitis, meningitis, necrotizing meningitis, ankylosing spondylitis or spinal joint disease.

In some embodiments, the present application providesbispecific antibodies specifically binding to *K. pneumoniae* O2 antigen and *K. pneumoniae* O1 antigen and pharmaceutical compositions containing antibodies specifically binding to *K. pneumoniae* O2 antigen and *K. pneumoniae* O1 antigen. Also provided are methods of use thereof for preventing and treating*Klebsiella* infections.

In one aspect, the present application provides a bispecificantibody comprising a first antigen-binding domainspecifically binding to *K. pneumoniae* O2 antigen, and a second antigen-binding domain specifically binding to *K. pneumoniae* O1 antigen, wherein the first antigen-binding domain comprises:(a)a heavy chain variable domain (V_{H}) comprising an HC-CDR1 comprising the amino acid sequence of SEQ ID NO: 1; an HC-CDR2 comprising the amino acid sequence of SEQ ID NO: 4; and an HC-CDR3 comprising the amino acid sequence of SEQ ID NO: 6; and a light chain variable domain (V_{L}) comprising an LC-CDR1 comprising the amino acid sequence of SEQ ID NO: 11; an LC-CDR2 comprising the amino acid sequence of SEQ ID NO: 14; and an LC-CDR3 comprising the amino acid sequence of SEQ ID NO: 16; or(b)a heavy chain variable domain (V_{H}) comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 7; and a light chain variable domain (V_{L}) comprising an LC-CDR1 comprising the amino acid sequence of SEQ ID NO: 10; an LC-CDR2 comprising the amino acid sequence of SEQ ID NO: 12; and an LC-CDR3 comprising the amino acid sequence of SEQ ID NO: 15.In some embodiments, the secondantigen-binding domain comprises:(a)a heavy chain variable domain (V_{H}) comprising an HC-CDR1 comprising the amino acid sequence of SEQ ID NO: 35; an HC-CDR2 comprising the amino acid sequence of SEQ ID NO: 37; and an HC-CDR3 comprising the amino acid sequence of SEQ ID NO: 39; and a light chain variable domain (V_{L}) comprising an LC-CDR1 comprising the amino acid sequence of SEQ ID NO: 41; an LC-CDR2 comprising the amino acid sequence of SEQ ID NO: 43; and an LC-CDR3 comprising the amino acid sequence of SEQ ID NO: 45; or(b) a heavy chain variable domain (V_{H}) comprising an HC-CDR1 comprising the amino acid sequence of SEQ ID NO: 36; an HC-CDR2 comprising the amino acid sequence of SEQ ID NO: 38; and an HC-CDR3 comprising the amino acid sequence of SEQ ID NO: 40; and a light chain variable domain (V_{L}) comprising an LC-CDR1 comprising the amino acid sequence of SEQ ID NO: 42; an LC-CDR2 comprising the amino acid sequence of SEQ ID NO: 44; and an LC-CDR3 comprising the amino acid sequence of SEQ ID NO: 46.

In some embodiments, according to any one of the bispecific antibodies described herein, the first antigen-binding domain comprising: a V_{H} comprising an HC-CDR1 comprising the amino acid sequence of SEQ ID NO: 1; an HC-CDR2 comprising the amino acid sequence of SEQ ID NO: 4; and an HC-CDR3 comprising the amino acid sequence of SEQ ID NO: 6; anda V_{L} comprising an LC-CDR1 comprising the amino acid sequence of SEQ ID NO: 11; an LC-CDR2 comprising the amino acid sequence of SEQ ID NO: 14; and an LC-CDR3 comprising the amino acid sequence of SEQ ID NO: 16; and the secondantigen-binding domain comprising: a V_{H} comprising an HC-CDR1 comprising the amino acid sequence of SEQ ID NO: 35; an HC-CDR2 comprising the amino acid sequence of SEQ ID NO: 37; and an HC-CDR3 comprising the amino acid sequence of SEQ ID NO: 39; and a V_{L}comprising an LC-CDR1 comprising the amino acid sequence of SEQ ID NO: 41; an LC-CDR2 comprising the amino acid sequence of SEQ ID NO: 43; and an LC-CDR3 comprising the amino acid sequence of SEQ ID NO: 45. In some embodiments, the first antigen-binding domain comprising: a V_{H} comprising an HC-CDR1 comprising the amino acid sequence of SEQ ID NO: 1; an HC-CDR2 comprising the amino acid sequence of SEQ ID NO: 4; and an HC-CDR3 comprising the amino acid sequence of SEQ ID NO: 6; anda V_{L} comprising an LC-CDR1 comprising the amino acid sequence of SEQ ID NO: 11; an LC-CDR2 comprising the amino acid sequence of SEQ ID NO: 14; and an LC-CDR3 comprising the amino acid sequence of SEQ ID NO: 16; and the secondantigen-binding domain comprising: a V_{H} comprising an HC-CDR1 comprising the amino acid sequence of SEQ ID NO: 36; an HC-CDR2 comprising the amino acid sequence of SEQ ID NO: 38; and an HC-CDR3 comprising the amino acid sequence of SEQ ID NO: 40; and a V_{L} comprising an LC-CDR1 comprising the amino acid sequence of SEQ ID NO: 42; an LC-CDR2 comprising the amino acid sequence of SEQ ID NO: 44; and an LC-CDR3 comprising the amino acid sequence of SEQ ID NO: 46. In some embodiments, the first antigen-binding domain comprising: a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 7; anda V_{L} comprising an LC-CDR1 comprising the amino acid sequence of SEQ ID NO: 10; an LC-CDR2 comprising the amino acid sequence of SEQ ID NO: 12; and an LC-CDR3 comprising the amino acid sequence of SEQ ID NO: 15; and the secondantigen-binding domain comprising: a V_{H} comprising an HC-CDR1 comprising the amino acid sequence of SEQ ID NO: 35; an HC-CDR2 comprising the amino acid sequence of SEQ ID NO: 37; and an HC-CDR3 comprising the amino acid sequence of SEQ ID NO: 39; and a V_{L}comprising an LC-CDR1 comprising the amino acid sequence of SEQ ID NO: 41; an LC-CDR2 comprising the amino acid sequence of SEQ ID NO: 43; and an LC-CDR3 comprising the amino acid sequence of SEQ ID NO: 45. In some embodiments, the first antigen-binding domain comprising: a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 7; anda V_{L} comprising an LC-CDR1 comprising the amino acid sequence of SEQ ID NO: 10; an LC-CDR2 comprising the amino acid sequence of SEQ ID NO: 12; and an LC-CDR3 comprising the amino acid sequence of SEQ ID NO: 15; and the secondantigen-binding domain comprising: a V_{H} comprising an HC-CDR1 comprising the amino acid sequence of SEQ ID NO: 36; an HC-CDR2 comprising the amino acid sequence of SEQ ID NO: 38; and an HC-CDR3 comprising the amino acid sequence of SEQ ID NO: 40; and a V_{L} comprising an LC-CDR1 comprising the amino acid sequence of SEQ ID NO: 42; an LC-CDR2 comprising the amino acid sequence of SEQ ID NO: 44; and an LC-CDR3 comprising the amino acid sequence of SEQ ID NO: 46.

In one aspect, the present application provides a bispecific antibody comprising a first antigen-binding domain specifically binding to *K. pneumoniae* O2 antigen, and a second antigen-binding domain specifically binding to *K. pneumoniae* O1 antigen, wherein the first antigen-binding domain comprises:(a)a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; or a V_{H} comprisingan HC-CDR1, an HC-CDR2 and an HC-CDR3 of the V_{H} comprising the amino acid sequence of SEQ ID NO: 19 or 28; and a V_{L} comprising an LC-CDR1, an LC-CDR2, and an LC-CDR3 of the V_{L} comprising the amino acid sequence of SEQ ID NO: 24 or 33; or(b)a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; or a V_{H} comprisingan HC-CDR1, an HC-CDR2 and an HC-CDR3 of the V_{H} comprising the amino acid sequence of SEQ ID NO: 20 or 29; and a V_{L} comprising an LC-CDR1, an LC-CDR2, and an LC-CDR3 of the V_{L} comprising the amino acid sequence of SEQ ID NO: 25 or 34. In some embodiments, the secondantigen-binding domain comprises:(a)a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53; or a V_{H} comprisingan HC-CDR1, an HC-CDR2 and an HC-CDR3 of the V_{H} comprising the amino acid sequence of SEQ ID NO: 47 or 51; and a V_{L} comprising an LC-CDR1, an LC-CDR2, and an LC-CDR3 of the V_{L} comprising the amino acid sequence of SEQ ID NO: 49 or 53; or(b)a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54; or a V_{H} comprisingan HC-CDR1, an HC-CDR2 and an HC-CDR3 of the V_{H} comprising the amino acid sequence of SEQ ID NO: 48 or 52; and a V_{L} comprising an LC-CDR1, an LC-CDR2, and an LC-CDR3 of the V_{L} comprising the amino acid sequence of SEQ ID NO: 50 or 54.

In some embodiments,the first antigen-binding domain comprises: a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; and wherein the second antigen-binding domain comprises:a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53. In some embodiments,the first antigen-binding domain comprises: a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; and wherein the second antigen-binding domain comprises:a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54. In some embodiments,the first antigen-binding domain comprises: a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; and wherein the second antigen-binding fragment comprises:a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53. In some embodiments,the first antigen-binding domain comprises: a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; and wherein the second antigen-binding domain comprises:a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54.

In some embodiments, the bispecific antibody has the formats selected fromthe group consisting of DVD-Ig, Bs4Ab, Hetero H, CrossMab, IgG-(scFv)₂and scFv-FabIgG.

In some embodiments, the bispecific antibody adopts DVD-Ig format. In some embodiments, the bispecific antibody comprises four polypeptide chains: wherein two of the polypeptide chains comprise V_{H}1-L-V_{H}2-C_{H}1 structurefrom N- terminus to C-terminus, wherein V_{H}1 is a heavy chain variable domainspecifically binding to *K. pneumoniae* O2 antigen; V_{H}2 is a heavy chain variable domainspecifically binding to *K. pneumoniae* O1 antigen; L is a peptide linker; C_{H}1 is a heavy chain constant domain 1; wherein the polypeptide chain further comprises an Fccomprising C_{H}2 and C_{H}3 domains; the other two of the polypeptide chains comprise V_{L}1-L-V_{L}2-C_{L} structurefrom N- terminus to C-terminus, wherein V_{L}1 is a light chain variable domainspecifically binding to *K. pneumoniae* O2 antigen; V_{L}2 is a lightchain variable domainspecifically binding to *K. pneumoniae* O1 antigen; L is a peptide linker; C_{L} is a light chain constant domain; wherein the V_{H}1 and V_{L}1 form the antigen-binding domain(Fv) specifically binding to *K. pneumoniae* O2 antigen; the V_{H}2-C_{H}1 and V_{L}2-C_{L}form the antigen-binding domain(Fab) specifically binding to *K. pneumoniae* O1 antigen. In other embodiments, the bispecific antibodycomprises four polypeptide chains: wherein two of the polypeptide chains comprise V_{H}1-L-V_{H}2-C_{H}1 structurefrom N- terminus to C-terminus, wherein V_{H}1 is a heavy chain variable domainspecifically binding to *K. pneumoniae* O1 antigen; V_{H}2 is a heavy chain variable domainspecifically binding to *K. pneumoniae* O2 antigen; L is apeptide linker; C_{H}1 is a heavy chain constant domain 1; wherein the polypeptide chain further comprises an Fccomprising C_{H}2 and C_{H}3 domains; the other two of the polypeptide chains comprise V_{L}1-L-V_{L}2-C_{L} structurefrom N- terminus to C-terminus, wherein V_{L}1 is a light chain variable domainspecifically binding to *K. pneumoniae* O1 antigen; V_{L}2 is a lightchain variable domainspecifically binding to *K. pneumoniae* O2 antigen; L is apeptide linker; C_{L} is a light chain constant domain; wherein the V_{H}1 and V_{L}1 form the antigen-binding domain(Fv) specifically binding to *K. pneumoniae* O1 antigen; the V_{H}2-C_{H}1 and V_{L}2-C_{L}form the antigen-binding domain(Fab) specifically binding to *K. pneumoniae* O2 antigen.In some embodiments, the bispecific antibodycomprises: (a)the amino acid sequence of SEQ ID NO: 61, or a variant thereof having at least about 80% sequence identity to the amino acid sequenceof SEQ ID NO: 61; and/or theamino acid sequence of SEQ ID NO: 62, or a variant thereof having at least about 80% sequence identity to the amino acid sequence of SEQ ID NO: 62; or (b)theamino acid sequence of SEQ ID NO: 63, or a variant thereof having at least about 80% sequence identity to the amino acid sequenceof SEQ ID NO: 63; and/or theamino acid sequence of SEQ ID NO: 64, or a variant thereof having at least about 80% sequence identity to the amino acid sequence of SEQ ID NO: 64; or (c)theamino acid sequence of SEQ ID NO: 65, or a variant thereof having at least about 80% sequence identity to the amino acid sequenceof SEQ ID NO: 65; and/or theamino acid sequence of SEQ ID NO: 66, or a variant thereof having at least about 80% sequence identity to the amino acid sequence of SEQ ID NO: 66; or (d)theamino acid sequence of SEQ ID NO: 67, or a variant thereof having at least about 80% sequence identity to the amino acid sequenceof SEQ ID NO: 67; and/or theamino acid sequence of SEQ ID NO: 68, or a variant thereof having at least about 80% sequence identity to the amino acid sequence of SEQ ID NO: 68.In some embodiments, the bispecific antibodycomprises: (a)theamino acid sequence of SEQ ID NO: 86, or a variant thereof having at least about 80% sequence identity to the amino acid sequenceof SEQ ID NO: 86; and/or theamino acid sequence of SEQ ID NO: 62, or a variant thereof having at least about 80% sequence identity to the amino acid sequence of SEQ ID NO: 62; or (b)theamino acid sequence of SEQ ID NO: 87, or a variant thereof having at least about 80% sequence identity to the amino acid sequenceof SEQ ID NO: 87; and/or theamino acid sequence of SEQ ID NO: 64, or a variant thereof having at least about 80% sequence identity to the amino acid sequence of SEQ ID NO: 64; or (c)theamino acid sequence of SEQ ID NO: 88, or a variant thereof having at least about 80% sequence identity to the amino acid sequenceof SEQ ID NO: 88; and/or theamino acid sequence of SEQ ID NO: 66, or a variant thereof having at least about 80% sequence identity to the amino acid sequence of SEQ ID NO: 66; or (d)theamino acid sequence SEQ ID NO: 89, or a variant thereof having at least about 80% sequence identity to the amino acid sequenceof SEQ ID NO: 89; and/ortheamino acid sequence of SEQ ID NO: 68, or a variant thereof having at least about 80% sequence identity to the amino acid sequence of SEQ ID NO: 68; or (e) theamino acid sequence of SEQ ID NO: 115, or a variant thereof having at least about 80% sequence identity to the amino acid sequenceof SEQ ID NO: 115; and/or theamino acid sequence of SEQ ID NO: 66, or a variant thereof having at least about 80% sequence identity to the amino acid sequence of SEQ ID NO: 66.

In some embodiments, the bispecific antibody adopts Bs4Abformat. In some embodiments, the bispecific antibody comprises four polypeptide chains: wherein two of the polypeptide chains comprise V_{H}1-C_{H}1-L1-V_{H}2-L3-V_{L}2 structurefrom N- terminus to C-terminus, wherein V_{H}1 is a heavy chain variable domainspecifically binding to *K. pneumoniae* O2 antigen; V_{H}2 is a heavy chain variable domainspecifically binding to *K. pneumoniae* O1 antigen; L1 and L3 arepeptide linkers; C_{H}1 is a heavy chain constant domain 1; wherein the polypeptide chain further comprises an Fccomprising C_{H}2 and C_{H}3 domains; and the other two of the polypeptide chains comprise V_{L}1-C_{L} structurefrom N- terminus to C-terminus, wherein V_{L}1 is a light chain variable domainspecifically binding to *K. pneumoniae* O2 antigen, C_{L} is a light chain constant domain; wherein the V_{H}1-C_{H}1 and V_{L}1-C_{L}1 form the antigen-binding domain(Fab) specifically binding to *K. pneumoniae* O2 antigen; the V_{H}2-L3-V_{L}2form the antigen-binding domain(scFv) specifically binding to *K. pneumoniae* O1 antigen. In some embodiments, the bispecific antibody comprises four polypeptide chains: wherein two of the polypeptide chains comprise V_{H}1-C_{H}1-L1-V_{H}2-L3-V_{L}2 structurefrom N- terminus to C-terminus, wherein V_{H}1 is a heavy chain variable domainspecifically binding to *K. pneumoniae* O1 antigen; V_{H}2 is a heavy chain variable domainspecifically binding to *K. pneumoniae* O2 antigen; L1 and L3 arepeptide linkers; C_{H}1 is a heavy chain constant domain 1; wherein the polypeptide chain further comprises an Fccomprising C_{H}2 and C_{H}3 domains; and the other two of the polypeptide chains comprise V_{L}1-C_{L} structurefrom N- terminus to C-terminus, wherein V_{L}1 is a light chain variable domainspecifically binding to *K. pneumoniae* O1 antigen, C_{L} is a light chain constant domain; wherein the V_{H}1-C_{H}1 and V_{L}1-C_{L}1 form the antigen-binding domain(Fab) specifically binding to *K. pneumoniae* O1 antigen; the V_{H}2-L3-V_{L}2form the antigen-binding domain(scFv) specifically binding to *K. pneumoniae* O2 antigen. In some embodiments, the bispecific antibodycomprises: (a)theamino acid sequence of SEQ ID NO: 69, or a variant thereof having at least about 80% sequence identity to the amino acid sequenceof SEQ ID NO: 69; and/or amino acid sequence of SEQ ID NO: 70, or a variant thereof having at least about 80% sequence identity to the amino acid sequence of SEQ ID NO: 70; or (b)theamino acid sequence of SEQ ID NO: 71, or a variant thereof having at least about 80% sequence identity to the amino acid sequenceof SEQ ID NO: 71; and/or theamino acid sequence of SEQ ID NO: 72, or a variant thereof having at least about 80% sequence identity to the amino acid sequence of SEQ ID NO: 72; or (c)theamino acid sequence of SEQ ID NO: 73, or a variant thereof having at least about 80% sequence identity to the amino acid sequenceof SEQ ID NO: 73; and/or theamino acid sequence of SEQ ID NO: 74, or a variant thereof having at least about 80% sequence identity to the amino acid sequence of SEQ ID NO: 74; or (d)theamino acid sequence of SEQ ID NO: 75, or a variant thereof having at least about 80% sequence identity to the amino acid sequenceof SEQ ID NO: 75; and/or theamino acid sequence of SEQ ID NO: 74, or a variant thereof having at least about 80% sequence identity to the amino acid sequence of SEQ ID NO: 74.In some embodiments, the bispecific antibodycomprises: (a)theamino acid sequence of SEQ ID NO: 90, or a variant thereof having at least about 80% sequence identity to the amino acid sequenceof SEQ ID NO: 90; and/or theamino acid sequence of SEQ ID NO: 70, or a variant thereof having at least about 80% sequence identity to the amino acid sequence of SEQ ID NO: 70; or (b)theamino acid sequence of SEQ ID NO: 91, or a variant thereof having at least about 80% sequence identity to the amino acid sequenceof SEQ ID NO: 91; and/or theamino acid sequence of SEQ ID NO: 72, or a variant thereof having at least about 80% sequence identity to the amino acid sequence of SEQ ID NO: 72; or (c) theamino acid sequence of SEQ ID NO: 92, or a variant thereof having at least about 80% sequence identity to the amino acid sequenceof SEQ ID NO: 92; and/or theamino acid sequence of SEQ ID NO: 74, or a variant thereof having at least about 80% sequence identity to the amino acid sequence of SEQ ID NO: 74; or (d) theamino acid sequence of SEQ ID NO: 93, or a variant thereof having at least about 80% sequence identity to the amino acid sequence ofSEQ ID NO: 93; and/or theamino acid sequence of SEQ ID NO: 74, or a variant thereof having at least about 80% sequence identity to the amino acid sequence of SEQ ID NO: 74.

In some embodiments, the bispecific antibody adopts Hetero H, CrossMabformat. In some embodiments, the bispecific antibodycomprises four polypeptide chains: wherein one of the polypeptide chains comprises V_{H}1-C_{H}1 structurefrom N- terminus to C-terminus, wherein V_{H}1 is a heavy chain variable domainspecifically binding to *K. pneumoniae* O2 antigen; C_{H}1 is a heavy chain constant domain 1; wherein the polypeptide chain further comprises an Fccomprising C_{H}2 and C_{H}3 domains; and one of the polypeptide chains comprises V_{L}1-C_{L} structurefrom N- terminus to C-terminus, wherein V_{L}1 is a light chain variable domainspecifically binding to *K. pneumoniae* O2 antigen, C_{L} is a light chain constant domain; and one of the polypeptide chains comprises V_{H}2-C_{L} structurefrom N- terminus to C-terminus, wherein V_{H}2 is a heavy chain variable domainspecifically binding to *K. pneumoniae* O1 antigen; C_{L} is a light chain constant domain; wherein the polypeptide chain further comprises an Fccomprising C_{H}2 and C_{H}3 domains; and one of the polypeptide chains comprises V_{L}2-C_{H}1 structurefrom N- terminus to C-terminus, wherein V_{L}2 is a light chain variable domainspecifically binding to *K. pneumoniae* O1 antigen, C_{H}1 is a heavy chain constant domain 1; wherein the V_{H}1-C_{H}1 and V_{L}1-C_{L}form the antigen-binding domain(Fab) specifically binding to *K. pneumoniae* O2 antigen; V_{H}2-C_{L} and V_{L}2-C_{H}1form the antigen-binding domain(Fab) specifically binding to *K. pneumoniae* O1 antigen. In some embodiments, the bispecific antibody comprises four polypeptide chains: wherein one of the polypeptide chains comprises V_{H}1-C_{H}1 structurefrom N- terminus to C-terminus, wherein V_{H}1 is a heavy chain variable domainspecifically binding to *K. pneumoniae* O1 antigen; C_{H}1 is a heavy chain constant domain 1; wherein the polypeptide chain further comprises an Fccomprising C_{H}2 and C_{H}3 domains; and one of the polypeptide chains comprises V_{L}1-C_{L} structurefrom N- terminus to C-terminus, wherein V_{L}1 is a light chain variable domainspecifically binding to *K. pneumoniae* O1 antigen, C_{L} is a light chain constant domain; and one of the polypeptide chains comprises V_{H}2-C_{L} structurefrom N- terminus to C-terminus, wherein V_{H}2 is a heavy chain variable domainspecifically binding to *K. pneumoniae* O2 antigen; C_{L} is a light chain constant domain; wherein the polypeptide chain further comprises an Fccomprising C_{H}2 and C_{H}3 domains; and one of the polypeptide chains comprises V_{L}2-C_{H}1 structurefrom N- terminus to C-terminus, wherein V_{L}2 is a light chain variable domainspecifically binding to *K. pneumoniae* O2 antigen, C_{H}1 is a heavy chain constant domain 1; wherein the V_{H}1-C_{H}1 and V_{L}1-C_{L}form the antigen-binding domain(Fab) specifically binding to *K. pneumoniae* O1 antigen; V_{H}2-C_{L} and V_{L}2-C_{H}1form the antigen-binding domain(Fab) specifically binding to *K. pneumoniae* O2 antigen. In some embodiments, the bispecific antibodycomprises: (a)the amino acid sequence of SEQ ID NO: 76, or a variant thereof having at least about 80% sequence identity to the amino acid sequenceof SEQ ID NO: 76; and/or theamino acid sequence of SEQ ID NO: 77, or a variant thereof having at least about 80% sequence identity to the amino acid sequenceof SEQ ID NO: 77; and/ortheamino acid sequence of SEQ ID NO: 78, or a variant thereof having at least about 80% sequence identity to the amino acid sequenceof SEQ ID NO: 78; and/ortheamino acid sequence of SEQ ID NO: 74, or a variant thereof having at least about 80% sequence identity to the amino acid sequenceof SEQ ID NO: 74; or (b) theamino acid sequence of SEQ ID NO: 79, or a variant thereof having at least about 80% sequence identity to the amino acid sequenceof SEQ ID NO: 79; and/or theamino acid sequence of SEQ ID NO: 80, or a variant thereof having at least about 80% sequence identity to the amino acid sequenceof SEQ ID NO: 80; and/ortheamino acid sequence of SEQ ID NO: 78, or a variant thereof having at least about 80% sequence identity to the amino acid sequenceof SEQ ID NO: 78; and/ortheamino acid sequence of SEQ ID NO: 74, or a variant thereof having at least about 80% sequence identity to the amino acid sequenceof SEQ ID NO: 74. In some embodiments, the bispecific antibodycomprises: (a)theamino acid sequence of SEQ ID NO: 94, or a variant thereof having at least about 80% sequence identity to the amino acid sequenceof SEQ ID NO: 94; and/ortheamino acid sequence of SEQ ID NO: 77, or a variant thereof having at least about 80% sequence identity to the amino acid sequenceof SEQ ID NO: 77; and/ortheamino acid sequence of SEQ ID NO: 95, or a variant thereof having at least about 80% sequence identity to the amino acid sequenceof SEQ ID NO: 95; and/ortheamino acid sequence of SEQ ID NO: 74, or a variant thereof having at least about 80% sequence identity to the amino acid sequenceof SEQ ID NO: 74; or (b)theamino acid sequence of SEQ ID NO: 96, or a variant thereof having at least about 80% sequence identity to the amino acid sequenceof SEQ ID NO: 96; and/or theamino acid sequence of SEQ ID NO: 80, or a variant thereof having at least about 80% sequence identity to the amino acid sequenceof SEQ ID NO: 80; and/ortheamino acid sequence of SEQ ID NO: 95, or a variant thereof having at least about 80% sequence identity to the amino acid sequenceof SEQ ID NO: 95; and/ortheamino acid sequence of SEQ ID NO: 74, or a variant thereof having at least about 80% sequence identity to the amino acid sequenceof SEQ ID NO: 74.

In some embodiments, the bispecific antibody adopts IgG-(scFv)₂format. In some embodiments, the bispecific antibodycomprises four polypeptide chains: wherein two of the polypeptide chains comprise V_{H}1-C_{H}1-C_{H}2-C_{H}3-L-V_{H}2-L3-V_{L}2 structurefrom N- terminus to C-terminus, wherein V_{H}1 is a heavy chain variable domainspecifically binding to *K. pneumoniae* O2 antigen; V_{H}2 is a heavy chain variable domainspecifically binding to *K. pneumoniae* O1 antigen; V_{L}2 is a light chain variable domainspecifically binding to *K. pneumoniae* O1 antigen; L and L3 arepeptide linkers; C_{H}1 is a heavy chain constant domain 1; C_{H}2 is a heavy chain constant domain 2; C_{H}3 is a heavy chain constant domain 3; and the other two of the polypeptide chains comprise V_{L}1-C_{L} structurefrom N- terminus to C-terminus, wherein V_{L}1 is a light chain variable domainspecifically binding to *K. pneumoniae* O2 antigen, C_{L} is a light chain constant domain; wherein the V_{H}1-C_{H}1 and V_{L}1-C_{L}form the antigen-binding domain(Fab) specifically binding to *K. pneumoniae* O2 antigen; V_{H}2-L3-V_{L}2form the antigen-binding domain(scFv) specifically binding to *K. pneumoniae* O1 antigen. In some embodiments, the bispecific antibody comprises four polypeptide chains: wherein two of the polypeptide chains comprise V_{H}1-C_{H}1-C_{H}2-C_{H}3-L-V_{H}2-L3-V_{L}2 structurefrom N- terminus to C-terminus, wherein V_{H}1 is a heavy chain variable domainspecifically binding to *K. pneumoniae* O1 antigen; V_{H}2 is a heavy chain variable domainspecifically binding to *K. pneumoniae* O2 antigen; V_{L}2 is a light chain variable domainspecifically binding to *K. pneumoniae* O2 antigen; L and L3 arepeptide linkers; C_{H}1 is a heavy chain constant domain 1; C_{H}2 is a heavy chain constant domain 2; C_{H}3 is a heavy chain constant domain 3; and the other two of the polypeptide chains comprise V_{L}1-C_{L} structurefrom N- terminus to C-terminus, wherein V_{L}1 is a light chain variable domainspecifically binding to *K. pneumoniae* O1 antigen, C_{L} is a light chain constant domain; wherein the V_{H}1-C_{H}1 and V_{L}1-C_{L}form the antigen-binding domain(Fab) specifically binding to *K. pneumoniae* O1 antigen; V_{H}2-L3-V_{L}2form the antigen-binding domain(scFv) specifically binding to *K. pneumoniae* O2 antigen. In some embodiments, the bispecific antibodycomprises: (a)theamino acid sequence of SEQ ID NO: 97, or a variant thereof having at least about 80% sequence identity to the amino acid sequenceof SEQ ID NO: 97; and/or theamino acid sequence of SEQ ID NO: 83, or a variant thereof having at least about 80% sequence identity to the amino acid sequence of SEQ ID NO: 83; or (b)theamino acid sequence of SEQ ID NO: 98, or a variant thereof having at least about 80% sequence identity to the amino acid sequence ofSEQ ID NO: 98; and/or theamino acid sequence of SEQ ID NO: 85, or a variant thereof having at least about 80% sequence identity to the amino acid sequence of SEQ ID NO: 85.

In some embodiments, the bispecific antibody adopts scFv-Fab IgGformat. In some embodiments, the bispecific antibodycomprisesthree polypeptide chains: wherein one of the polypeptide chains comprises V_{H}1-C_{H}1 structurefrom N- terminus to C-terminus, wherein V_{H}1 is a heavy chain variable domainspecifically binding to *K. pneumoniae* O2 antigen; is a heavy chain constant domain 1; wherein the polypeptide chain further comprises an Fccomprising C_{H}2 and C_{H}3 domains; and one of the polypeptide chains comprises V_{L}1-C_{L} structurefrom N- terminus to C-terminus, wherein V_{L}1 is a light chain variable domainspecifically binding to *K. pneumoniae* O2 antigen, C_{L} is a light chain constant domain; and one of the polypeptide chains comprises V_{H}2-L3-V_{L}2 structurefrom N- terminus to C-terminus, wherein V_{H}2 is a heavy chain variable domainspecifically binding to *K. pneumoniae* O1 antigen; V_{L}2 is a light chain variable domainspecifically binding to *K. pneumoniae* O1 antigen; L3 isa peptide linker; wherein the polypeptide chain further comprises an Fccomprising C_{H}2 and C_{H}3 domains; wherein the V_{H}1-C_{H}1 and V_{L}1-C_{L}form the antigen-binding domain(Fab) specifically binding to *K. pneumoniae* O2 antigen; V_{H}2-L3-V_{L}2form the antigen-binding domain(scFv) specifically binding to *K. pneumoniae* O1 antigen. In some embodiments, the bispecific antibodycomprisesthree polypeptide chains: wherein one of the polypeptide chains comprises V_{H}1-C_{H}1 structurefrom N- terminus to C-terminus, wherein V_{H}1 is a heavy chain variable domainspecifically binding to *K. pneumoniae* O1 antigen; is a heavy chain constant domain 1; wherein the polypeptide chain further comprises an Fccomprising C_{H}2 and C_{H}3 domains; and one of the polypeptide chains comprises V_{L}1-C_{L} structurefrom N- terminus to C-terminus, wherein V_{L}1 is a light chain variable domainspecifically binding to *K. pneumoniae* O1 antigen, C_{L} is a light chain constant domain; andone of the polypeptide chains comprises V_{H}2-L3-V_{L}2 structurefrom N-terminus to C-terminus, wherein V_{H}2 is a heavy chain variable domainspecifically binding to *K. pneumoniae* O2 antigen; V_{L}2 is a light chain variable domainspecifically binding to *K. pneumoniae* O2 antigen; L3 isa peptide linker; wherein the polypeptide chain further comprises an Fccomprising C_{H}2 and C_{H}3 domains; wherein the V_{H}1-C_{H}1 and V_{L}1-C_{L}form the antigen-binding domain(Fab) specifically binding to *K. pneumoniae* O1 antigen; V_{H}2-L3-V_{L}2form the antigen-binding domain(scFv) specifically binding to *K. pneumoniae* O2 antigen. In some embodiments, the bispecific antibodycomprises: (a)theamino acid sequence of SEQ ID NO: 81, or a variant thereof having at least about 80% sequence identity to the amino acid sequenceof SEQ ID NO: 81; and/ortheamino acid sequence of SEQ ID NO: 82, or a variant thereof having at least about 80% sequence identity to the amino acid sequenceof SEQ ID NO: 82; and/or theamino acid sequence of SEQ ID NO: 83, or a variant thereof having at least about 80% sequence identity to the amino acid sequenceof SEQ ID NO: 83; or (b)theamino acid sequence of SEQ ID NO: 84, or a variant thereof having at least about 80% sequence identity to the amino acid sequenceof SEQ ID NO: 84; and/or theamino acid sequence of SEQ ID NO: 82, or a variant thereof having at least about 80% sequence identity to the amino acid sequenceof SEQ ID NO: 82; and/ortheamino acid sequence of SEQ ID NO: 85, or a variant thereof having at least about 80% sequence identity to the amino acid sequenceof SEQ ID NO: 85. In some embodiments, the bispecific antibodycomprises: (a)theamino acid sequence of SEQ ID NO: 99, or a variant thereof having at least about 80% sequence identity to the amino acid sequenceof SEQ ID NO: 99; and/or theamino acid sequence of SEQ ID NO: 100, or a variant thereof having at least about 80% sequence identity to the amino acid sequenceof SEQ ID NO: 100; and/or theamino acid sequence of SEQ ID NO: 83, or a variant thereof having at least about 80% sequence identity to the amino acid sequenceof SEQ ID NO: 83; or (b)theamino acid sequence of SEQ ID NO: 101, or a variant thereof having at least about 80% sequence identity to the amino acid sequenceof SEQ ID NO: 101; and/or theamino acid sequence of SEQ ID NO: 100, or a variant thereof having at least about 80% sequence identity to the amino acid sequenceof SEQ ID NO: 100; and/ortheamino acid sequence of SEQ ID NO: 85, or a variant thereof having at least about 80% sequence identity to the amino acid sequenceof SEQ ID NO: 85.

In some embodiments, the bispecific antibody comprises a Fc region, wherein the Fc region is selected from the group consisting of an Fc region from an IgGl, IgG2, IgG3, IgG4, IgA, IgM, IgE, and IgD. In some embodiments, the Fc region comprises a variant Fc region. In some embodiments, the Fc region is aglycosylated. In some embodiments, the Fc region is deglycosylated. In some embodiments, the Fc region has reduced fucosylation or is afucosylated.

In one aspect, the present application provides a pharmaceutical composition comprises: (i) antibody orantigen-binding fragment specificallybinding to *K. pneumoniae* O2 antigen and (ii) antibody orantigen-binding fragment specifically binding to *K. pneumoniae* O1 antigen; wherein the antibody orantigen-binding fragment specificallybinding to *K. pneumoniae* O2 antigen comprises:(a) a heavy chain variable domain (V_{H}) comprising a heavy chain complementarity determining region (HC-CDR) 1 comprising the amino acid sequence of SEQ ID NO: 1; an HC-CDR2 comprising the amino acid sequence of SEQ ID NO: 4; and an HC-CDR3 comprising the amino acid sequence of SEQ ID NO: 6; and a light chain variable domain (V_{L}) comprising a light chain complementarity determining region (LC-CDR) 1 comprising the amino acid sequence of SEQ ID NO: 11; an LC-CDR2 comprising the amino acid sequence of SEQ ID NO: 14; and an LC-CDR3 comprising the amino acid sequence of SEQ ID NO: 16; or(b) a heavy chain variable domain (V_{H}) comprising a heavy chain complementarity determining region (HC-CDR) 1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 7; and a light chain variable domain (V_{L}) comprising a light chain complementarity determining region (LC-CDR) 1 comprising the amino acid sequence of SEQ ID NO: 10; an LC-CDR2 comprising the amino acid sequence of SEQ ID NO: 12; and an LC-CDR3 comprising the amino acid sequence of SEQ ID NO: 15.

In one aspect, the present application provides a method of treating and/or preventing a disease or condition in an individual in need thereof, comprising administering to the individual an effective amount of(i) antibody orantigen-binding fragment specificallybinding to *K. pneumoniae* O2 antigen and (ii) antibody orantigen-binding fragment specifically binding to *K. pneumoniae* O1 antigen; wherein the antibody orantigen-binding fragment specificallybinding to *K. pneumoniae* O2 antigen comprises:(a)a heavy chain variable domain (V_{H}) comprising a heavy chain complementarity determining region (HC-CDR) 1 comprising the amino acid sequence of SEQ ID NO: 1; an HC-CDR2 comprising the amino acid sequence of SEQ ID NO: 4; and an HC-CDR3 comprising the amino acid sequence of SEQ ID NO: 6; and a light chain variable domain (V_{L}) comprising a light chain complementarity determining region (LC-CDR) 1 comprising the amino acid sequence of SEQ ID NO: 11; an LC-CDR2 comprising the amino acid sequence of SEQ ID NO: 14; and an LC-CDR3 comprising the amino acid sequence of SEQ ID NO: 16; or(b)a heavy chain variable domain (V_{H}) comprising a heavy chain complementarity determining region (HC-CDR) 1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 7; and a light chain variable domain (V_{L}) comprising a light chain complementarity determining region (LC-CDR) 1 comprising the amino acid sequence of SEQ ID NO: 10; an LC-CDR2 comprising the amino acid sequence of SEQ ID NO: 12; and an LC-CDR3 comprising the amino acid sequence of SEQ ID NO: 15.

In some embodiments, the pharmaceutical composition or method provided herein, wherein the antibody orantigen-binding fragment specificallybinding to *K. pneumoniae* O1 antigen comprises:(a) a V_{H} comprising a HC-CDR1 comprising the amino acid sequence of SEQ ID NO: 35; an HC-CDR2 comprising the amino acid sequence of SEQ ID NO: 37; and an HC-CDR3 comprising the amino acid sequence of SEQ ID NO: 39; and V_{L} comprising a LC-CDR1 comprising the amino acid sequence of SEQ ID NO: 41; an LC-CDR2 comprising the amino acid sequence of SEQ ID NO: 43; and an LC-CDR3 comprising the amino acid sequence of SEQ ID NO: 45; or(b)a V_{H} comprising a HC-CDR1 comprising the amino acid sequence of SEQ ID NO: 36; an HC-CDR2 comprising the amino acid sequence of SEQ ID NO: 38; and an HC-CDR3 comprising the amino acid sequence of SEQ ID NO: 40; and a V_{L} comprising a LC-CDR1 comprising the amino acid sequence of SEQ ID NO: 42; an LC-CDR2 comprising the amino acid sequence of SEQ ID NO: 44; and an LC-CDR3 comprising the amino acid sequence of SEQ ID NO: 46.

In some embodiments, the pharmaceutical composition or method provided herein, wherein the antibody orantigen-binding fragment specificallybinding to *K. pneumoniae* O2 antigen comprises:a V_{H} comprising an HC-CDR1 comprising the amino acid sequence SEQ ID NO: 1, an HC-CDR2 comprising the amino acid sequence of SEQ ID NO: 2, and an HC-CDR3 comprising the amino acid sequence of SEQ ID NO: 7; and a V_{L} comprising: an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15;and the antibody orantigen-binding fragment specificallybinding to *K. pneumoniae* O1 antigen comprises:a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising: an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45. In some embodiments, the pharmaceutical composition or method provided herein,wherein the antibody orantigen-binding fragment specificallybinding to *K. pneumoniae* O2 antigen comprises:a V_{H} comprising an HC-CDR1 comprising the amino acid sequence SEQ ID NO: 1, an HC-CDR2 comprising the amino acid sequence of SEQ ID NO: 2, and an HC-CDR3 comprising the amino acid sequence of SEQ ID NO: 7; and a V_{L} comprising: an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15;and the antibody orantigen-binding fragment specificallybinding to *K. pneumoniae* O1 antigen comprises:a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising: an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46. In some embodiments, the pharmaceutical composition or method provided herein,wherein the antibody orantigen-binding fragment specificallybinding to *K. pneumoniae* O2 antigen comprises:a V_{H} comprising an HC-CDR1 comprising the amino acid sequence SEQ ID NO: 1, an HC-CDR2 comprising the amino acid sequence of SEQ ID NO: 4, and an HC-CDR3 comprising the amino acid sequence of SEQ ID NO: 6; and a V_{L} comprising: an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16;and the antibody orantigen-binding fragment specificallybinding to *K. pneumoniae* O1 antigen comprises:a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising: an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45. In some embodiments, the pharmaceutical composition or method provided herein, wherein the antibody orantigen-binding fragment specificallybinding to *K. pneumoniae* O2 antigen comprises:a V_{H} comprising an HC-CDR1 comprising the amino acid sequence SEQ ID NO: 1, an HC-CDR2 comprising the amino acid sequence of SEQ ID NO: 4, and an HC-CDR3 comprising the amino acid sequence of SEQ ID NO: 6; and a V_{L} comprising: an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16;and the antibody orantigen-binding fragment specificallybinding to *K. pneumoniae* O1 antigen comprises:a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising: an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

In one aspect, the present application provides a pharmaceutical composition comprises: (i) antibody orantigen-binding fragment specificallybinding to *K. pneumoniae* O2 antigen and (ii) antibody orantigen-binding fragment specifically binding to *K. pneumoniae* O1 antigen; wherein the antibody orantigen-binding fragment specificallybinding to *K. pneumoniae* O2 antigen comprises:(a)a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; or a V_{H} comprisingan HC-CDR1, an HC-CDR2 and an HC-CDR3 of the V_{H} comprising the amino acid sequence of SEQ ID NO: 19 or 28; and a V_{L} comprising an LC-CDR1, an LC-CDR2, and an LC-CDR3 of the V_{L} comprising the amino acid sequence of SEQ ID NO: 24 or 33; or(b)a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; or a V_{H} comprisingan HC-CDR1, an HC-CDR2 and an HC-CDR3 of the V_{H} comprising the amino acid sequence of SEQ ID NO: 20 or 29; and a V_{L} comprising an LC-CDR1, an LC-CDR2, and an LC-CDR3 of the V_{L} comprising the amino acid sequence of SEQ ID NO: 25 or 34.

In one aspect, the present application provides a method of treating and/or preventing a disease or condition in an individual in need thereof, comprising administering to the individual an effective amount of(i) antibody orantigen-binding fragment specificallybinding to *K. pneumoniae* O2 antigen and (ii) antibody orantigen-binding fragment specifically binding to *K. pneumoniae* O1 antigen; wherein the antibody orantigen-binding fragment specificallybinding to *K. pneumoniae* O2 antigen comprises:(a)a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; or a V_{H} comprisingan HC-CDR1, an HC-CDR2 and an HC-CDR3 of the V_{H} comprising the amino acid sequence of SEQ ID NO: 19 or 28; and a V_{L} comprising an LC-CDR1, an LC-CDR2, and an LC-CDR3 of the V_{L} comprising the amino acid sequence of SEQ ID NO: 24 or 33; or(b)a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; or a V_{H} comprisingan HC-CDR1, an HC-CDR2 and an HC-CDR3 of the V_{H} comprising the amino acid sequence of SEQ ID NO: 20 or 29; and a V_{L} comprising an LC-CDR1, an LC-CDR2, and an LC-CDR3 of the V_{L} comprising the amino acid sequence of SEQ ID NO: 25 or 34.

In some embodiments, the pharmaceutical composition or method provided herein, wherein the antibody orantigen-binding fragment specificallybinding to *K. pneumoniae* O1 antigen comprises:(a)a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53; or a V_{H} comprisingan HC-CDR1, an HC-CDR2 and an HC-CDR3 of the V_{H} comprising the amino acid sequence of SEQ ID NO: 47 or 51; and a V_{L} comprising an LC-CDR1, an LC-CDR2, and an LC-CDR3 of the V_{L} comprising the amino acid sequence of SEQ ID NO: 49 or 53; or(b)a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54; or a V_{H} comprisingan HC-CDR1, an HC-CDR2 and an HC-CDR3 of the V_{H} comprising the amino acid sequence of SEQ ID NO: 48 or 52; and a V_{L} comprising an LC-CDR1, an LC-CDR2, and an LC-CDR3 of the V_{L} comprising the amino acid sequence of SEQ ID NO: 50 or 54.

In some embodiments, the pharmaceutical composition or method provided herein, wherein the antibody orantigen-binding fragment specificallybinding to *K. pneumoniae* O2 antigen comprises:a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34;and the antibody orantigen-binding fragment specificallybinding to *K. pneumoniae* O1 antigen comprises:a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53. In some embodiments, the pharmaceutical composition or method provided herein, wherein the antibody orantigen-binding fragment specificallybinding *to K. pneumoniae* O2 antigen comprises:a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34;and the antibody orantigen-binding fragment specificallybinding to *K. pneumoniae* O1 antigen comprises:a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54. In some embodiments, the pharmaceutical composition or method provided herein, wherein the antibody orantigen-binding fragment specificallybinding to *K. pneumoniae* O2 antigen comprises:a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33;and the antibody orantigen-binding fragment specificallybinding to *K. pneumoniae* O1 antigen comprises:a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53. In some embodiments, the pharmaceutical composition or method provided herein, wherein the antibody orantigen-binding fragment specificallybinding *to K. pneumoniae* O2 antigen comprises:a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO:24 or 33;and the antibody orantigen-binding fragment specificallybinding to *K. pneumoniae* O1 antigen comprises:a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54.

In some embodiments, the antibody orantigen-binding fragment specificallyrecognizing *K. pneumoniae* O2 antigen and the antibody orantigen-binding fragment specifically recognizing *K. pneumoniae* O1 antigen are administered to the individual concurrently. In some embodiments, the antibody orantigen-binding fragment specificallyrecognizing K. *pneumoniae* O2 antigen and the antibody orantigen-binding fragment specifically recognizing *K. pneumoniae* O1 antigen are administered to the individual consecutively.

In some embodiments, there is provided a method of treating and/or preventing a disease or condition in an individual in need thereof, comprising administering to the individual an effective amount of any one of the antibodies orantigen-binding fragments described herein and/or any one of the pharmaceutical compositions and/or bispecific antibodies described herein.

In some embodiments, according to any one of the methods provided herein, the disease or conditioncomprises one or more symptoms caused by *Klebsiella* infection. In some embodiments, the *Klebsiella* is *Klebsiella pneumoniae.*In some embodiments, the disease or condition comprises pneumonia, urinary tract infection, septicaemia/bacteremia/sepsis, neonatal septicaemia/bacteremia/sepsis, diarrhea, soft tissue infection, infection after organ transplantation, surgical infection, wound infection, lung infection, purulent liver abscess, lung abscess, cellulitis, necrotizing myositis, myositis, endophthalmitis, peritonitis, meningitis, necrotizing meningitis, ankylosing spondylitis or spinal joint disease.

In some embodiments, there is provided an isolated nucleic acid moleculethat encodes any one of the bispecific antibodiesdescribed above. In some embodiments, there is provided a vector comprising any one of the nucleic acid molecules described above.In some embodiments, there is provided a host cell comprising any one of the bispecific antibodies, any one of the nucleic acid molecules or any one of the vectors described above. In some embodiments, there is provided a method of producing a bispecific antibody specifically binding to *K. pneumoniae* O2 antigen and O1 antigen, comprising: a) culturing any one of the host cells described above under conditions effective to express the bispecific antibody specifically binding to *K. pneumoniae* O2 antigen and O1 antigen; and b) obtaining the expressed bispecific antibody from the host cell.

Also provided are pharmaceutical compositions, kits and articles of manufacture comprising any one of the bispecific antibodies, nucleic acids, vectors or host cells described above.

### BRIEF DESCRIPTION OF THE DRAWINGS

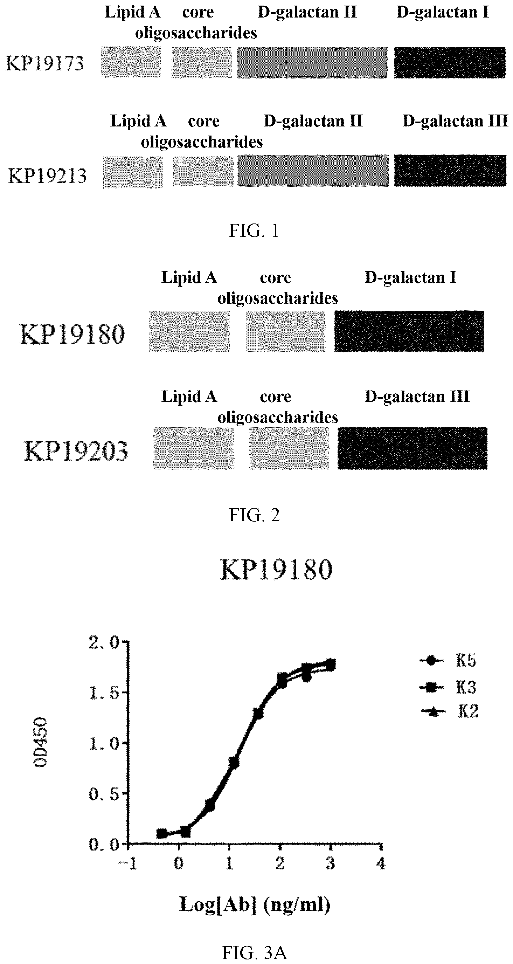

FIG. 1 shows the schematic diagram of the structure of *K. pneumoniae* O1 serotype KP19173 strain LPS and KP19213 strain LPS.
FIG. 2 shows the schematic diagram of the structure of *K. pneumoniae* O2 serotype KP19180 strain LPS and KP19203 strain LPS.

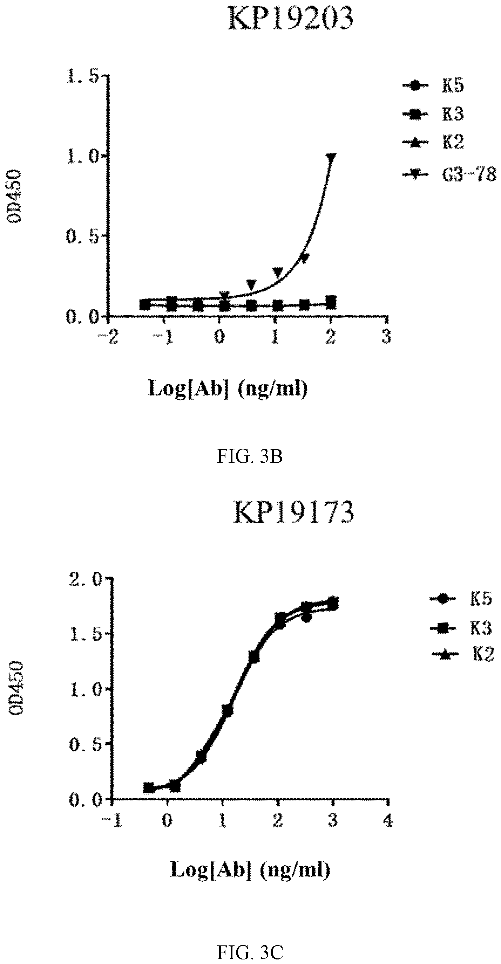

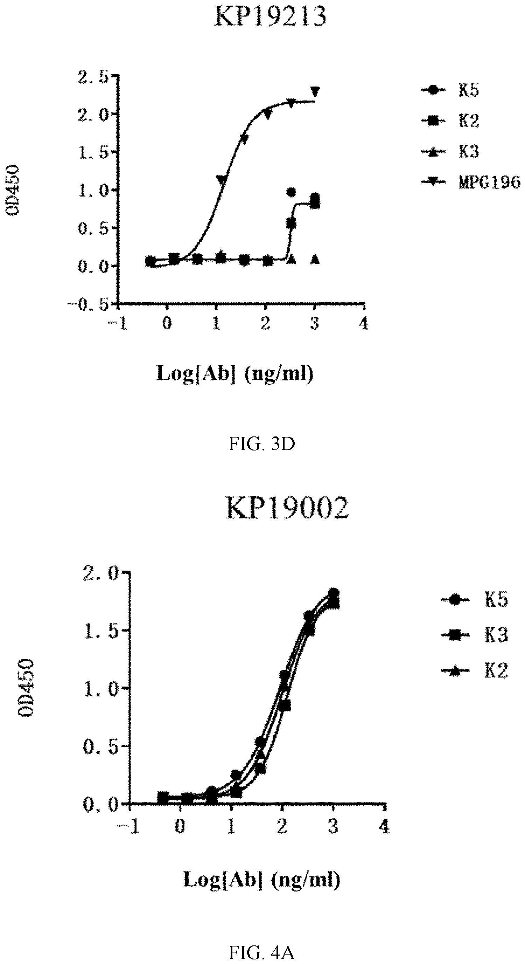

FIG. 3A shows ELISA binding results of K2, K3 or K5 antibodies specifically binding to *K. pneumoniae* O2 antigen to KP19180 strain O2-type LPS; FIG. 3B shows ELISA binding results of K2,K3 or K5 antibodies specifically binding to *K. pneumoniae* O2 antigen to KP19203 strain O2-type LPS; FIG. 3C showsELISA binding results of K2, K3 or K5 antibodies specifically binding to *K. pneumoniae* O2 antigen toKP19173 strain O1-type LPS; FIG. 3D shows ELISA binding results of K2, K3 or K5 antibodies specifically binding toK. *pneumoniae* O2 antigen to KP19213 strain O1-type LPS.

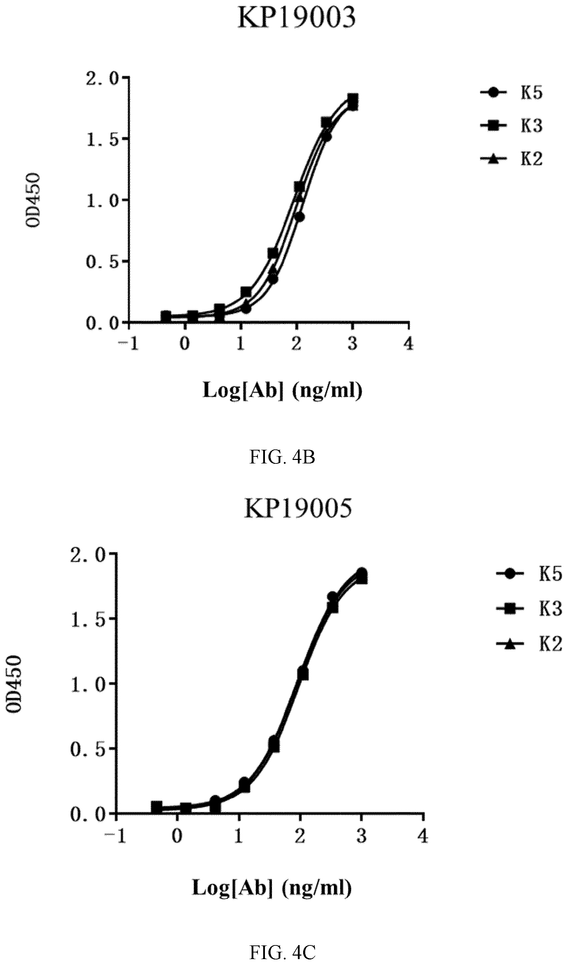

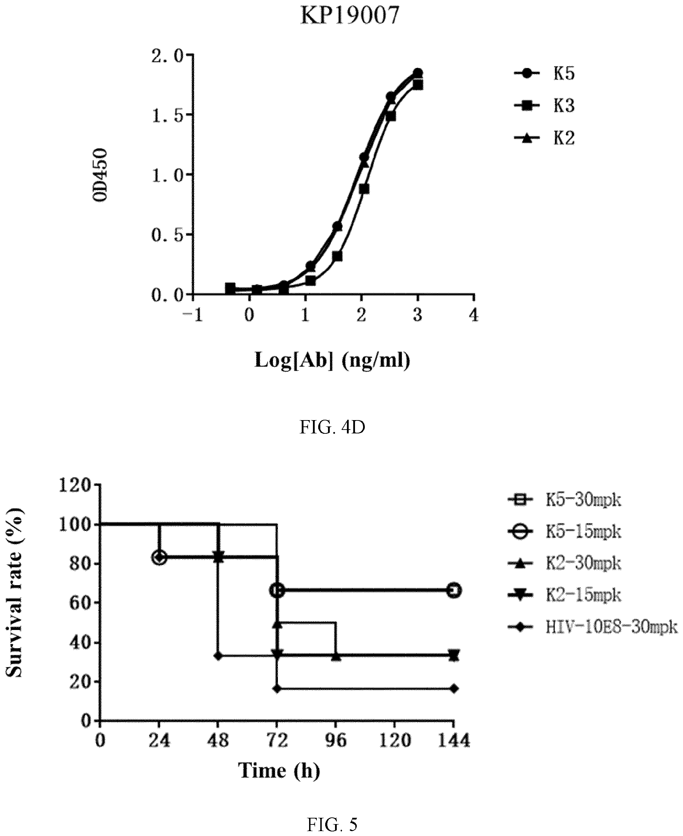

FIGS. 4A-4D show the results of exemplary K2, K3 and K5 antibodies specifically binding toK. *pneumoniae* O2 antigen bind to *K. pneumoniae* strains over a broad spectrum. FIG. 4A showsthe ELISA binding results of antibodies specifically binding to *K. pneumoniae* O2 antigen to *K. pneumoniae* strain KP19002O2-type LPS; FIG. 4B showsthe ELISA binding results of antibodies specifically bindingto *K. pneumoniae* O2 antigen to *K. pneumoniae* strain KP19003O2-type LPS; FIG. 4C shows theELISA binding results of antibodies specifically binding to *K. pneumoniae* O2 antigen to *K. pneumoniae* strain KP19005O2-type LPS; FIG. 4D shows the ELISA binding results of antibodies specificallybinding to *K. pneumoniae* O2 antigen to *K. pneumoniae* strain KP19007 O2-type LPS.
FIG. 5 shows the prophylactic protective effects of exemplary K2 or K5 antibodiesspecifically binding to *K. pneumoniae* O2 antigen in a mouse bacteremia model induced byK. *pneumoniae* KP19180.

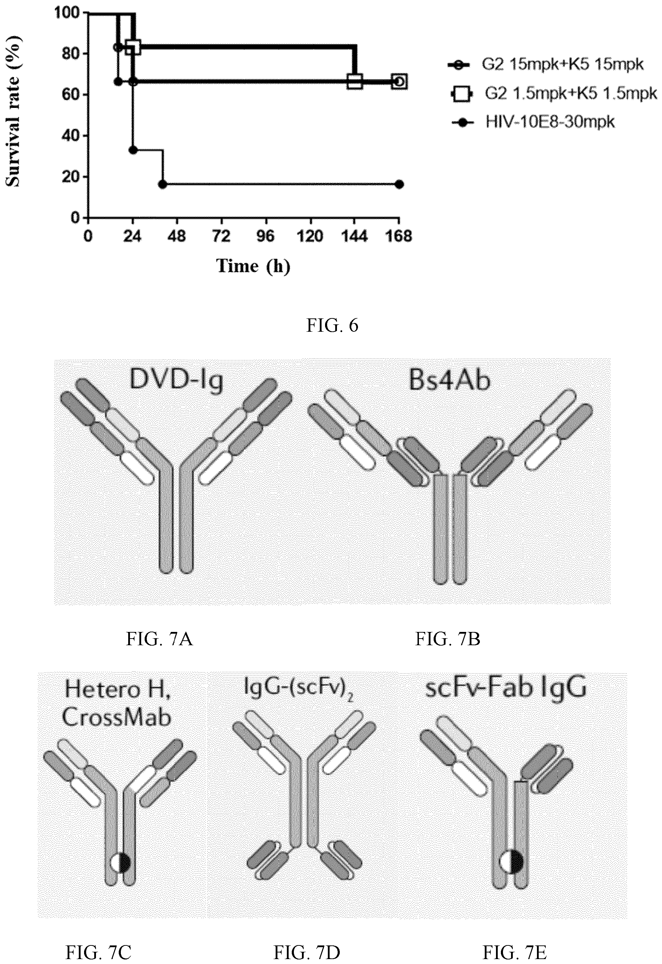

FIG. 6 shows the therapeutic effects of the combination application of K5antibody specifically binding to *K. pneumoniae* O2 antigen and G2 antibody specifically binding to *K. pneumoniae* O1 antigen in amouse pneumonia model induced byK. *pneumoniae* KP19173.
FIG. 7A shows the schematic diagramof DVD-Ig (Dual-variable domain-Ig) formatbispecificantibody; FIG. 7B shows the schematic diagramof Bs4Abformatbispecificantibody; FIG. 7C shows the schematic diagramof Hetero H, CrossMabformatbispecificantibody; FIG. 7D shows the schematic diagramof IgG-(scFv)₂formatbispecificantibody; FIG. 7E shows the schematic diagramof scFv-Fab IgGformatbispecificantibody.

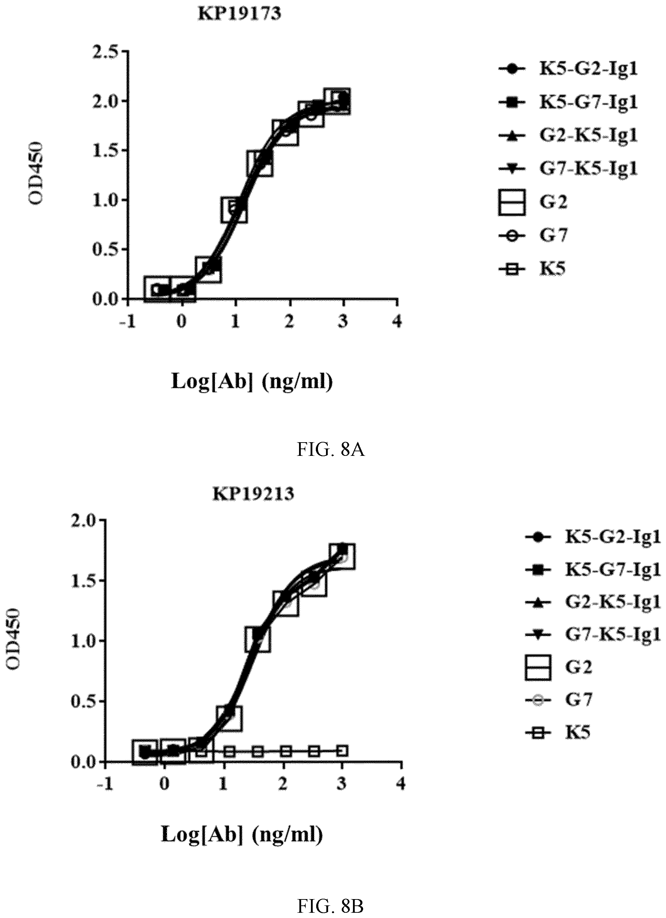

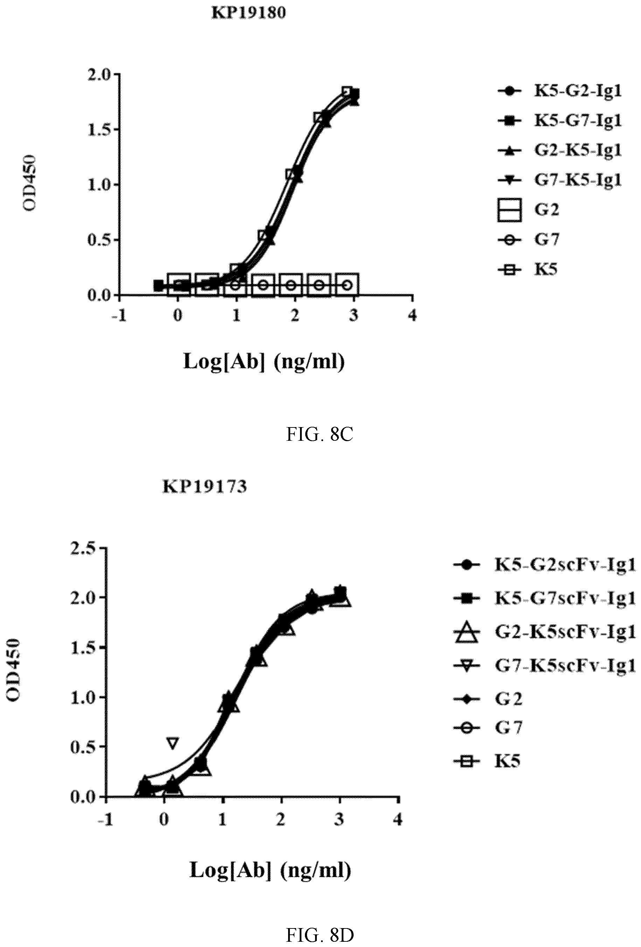

FIGS. 8A-8C show ELISA binding results of exemplary DVD-Ig formatbispecific antibodies toLPS of different serotypes of *K. pneumoniae* strains, respectively. FIG. 8A shows the binding results of KP19173 strain O1-type LPS; FIG. 8B shows the binding results of KP19213 strain O1-type LPS; FIG. 8C shows the bindingresults of KP19180 strain O2-type LPS.

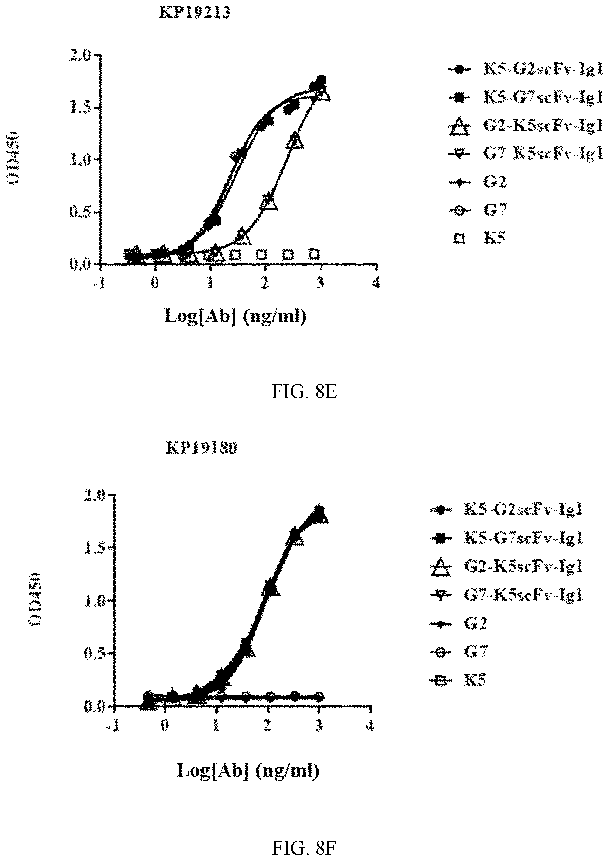

FIGS. 8D-8F show ELISA binding results of exemplary Bs4Ab formatbispecific antibodies to LPSof different serotypes of *K. pneumoniae* strains, respectively. FIG. 8D shows the binding results of KP19173 strain O1-type LPS; FIG. 8E shows the binding results of KP19213 strain O1-type LPS; FIG. 8F shows the bindingresults of KP19180 strain O2-type LPS.

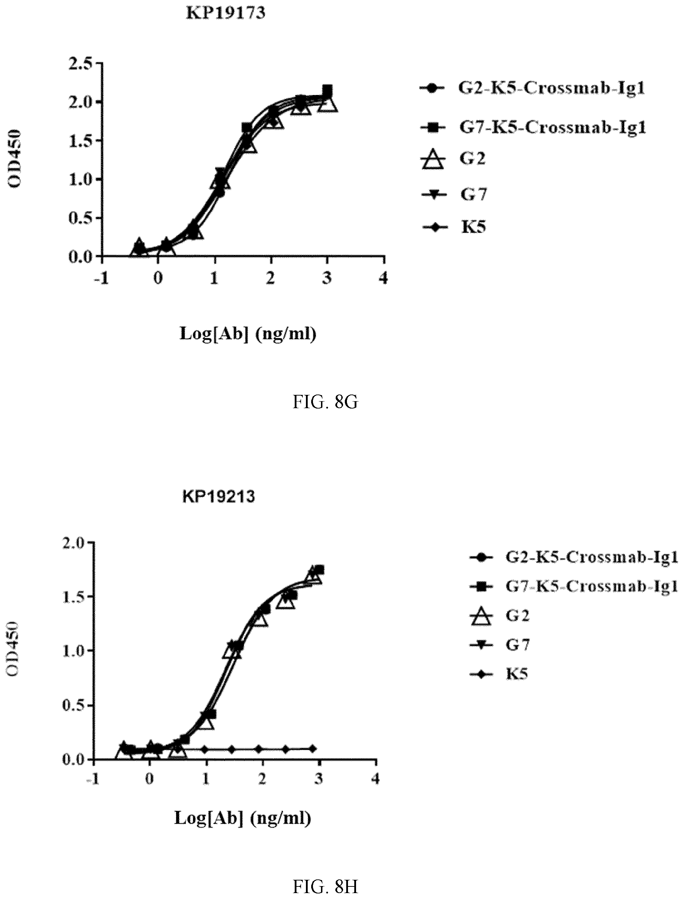

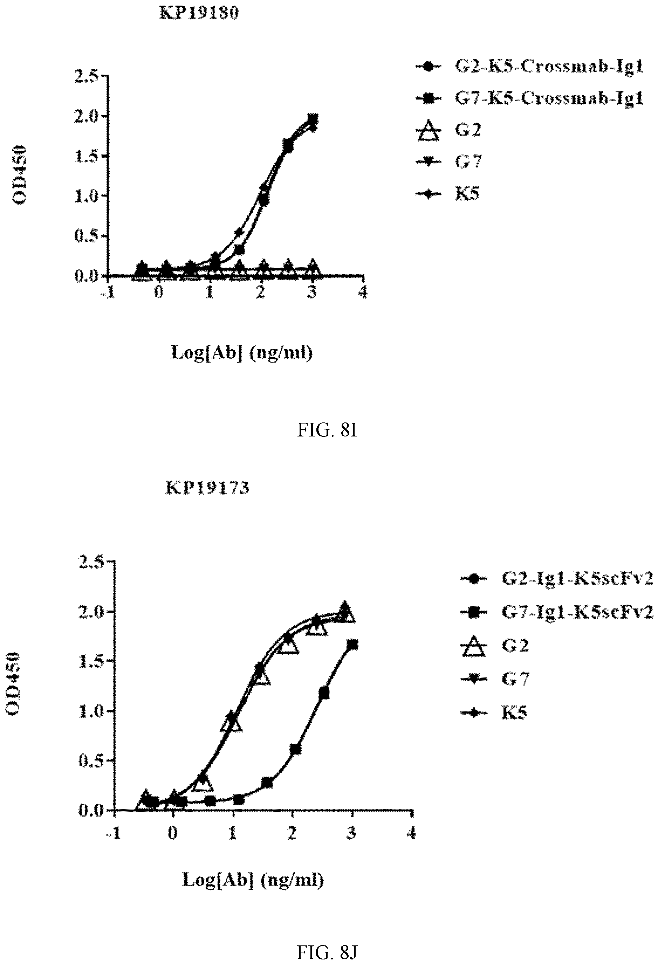

FIGS. 8G-8I show ELISA binding results of exemplary Hetero H, CrossMabformatbispecific antibodies to LPSof different serotypes *ofK. pneumoniae* strains, respectively. FIG. 8G shows the binding results of KP19173 strain O1-type LPS; FIG. 8H shows the binding results of KP19213 strain O1-type LPS; FIG. 8I shows the bindingresults of KP19180 strain O2-type LPS.

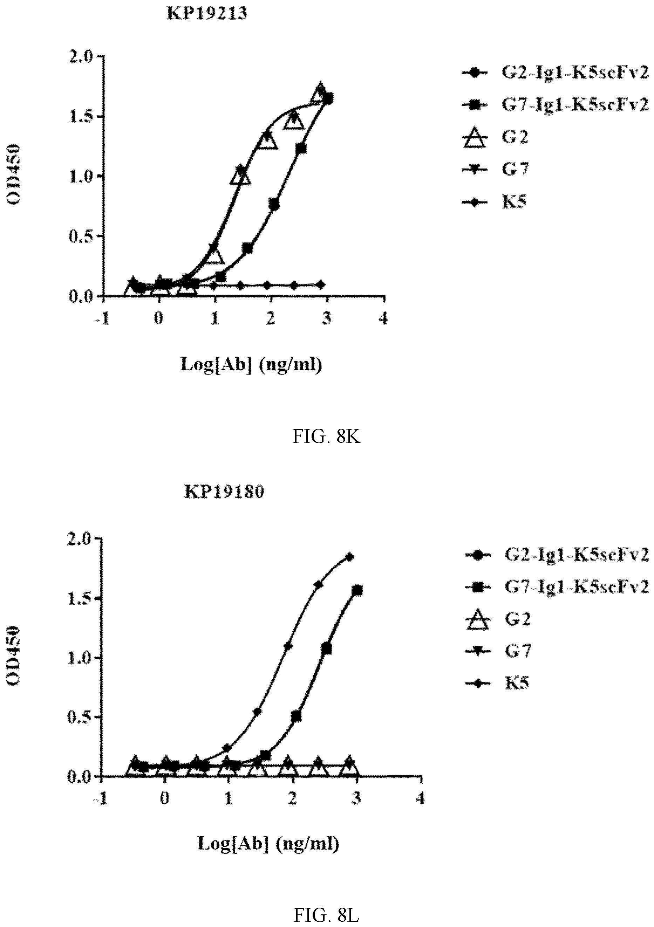

FIGS. 8J-8L showELISA binding results of exemplary IgG-(scFv)₂formatbispecific antibodies to LPSof different serotypes of *K. pneumoniae* strains, respectively. FIG. 8J shows the binding results of KP19173 strain O1-type LPS; FIG. 8K shows the binding results of KP19213 strain O1-type LPS; FIG. 8L shows the bindingresults of KP19180 strain O2-type LPS.

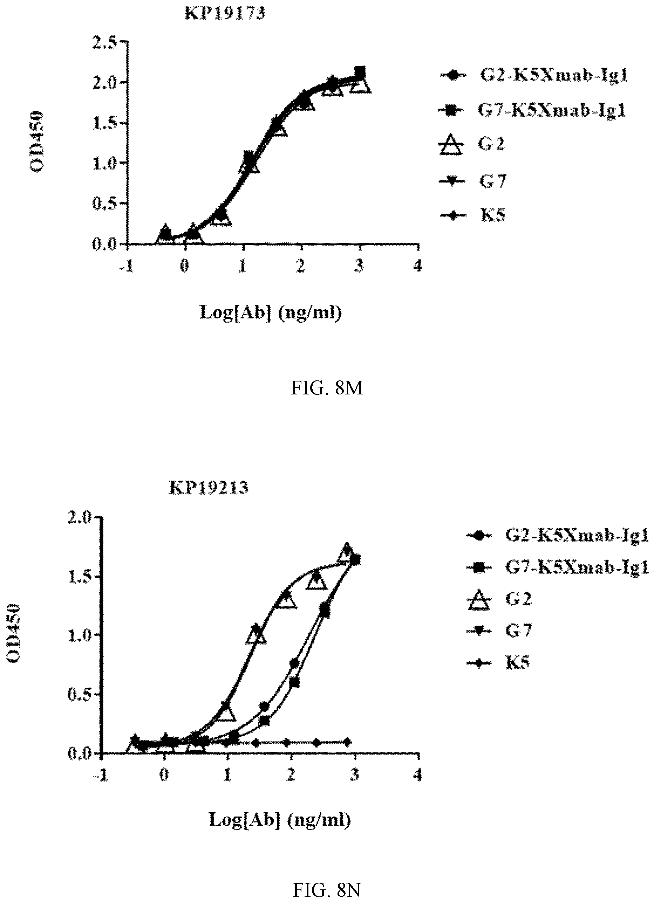

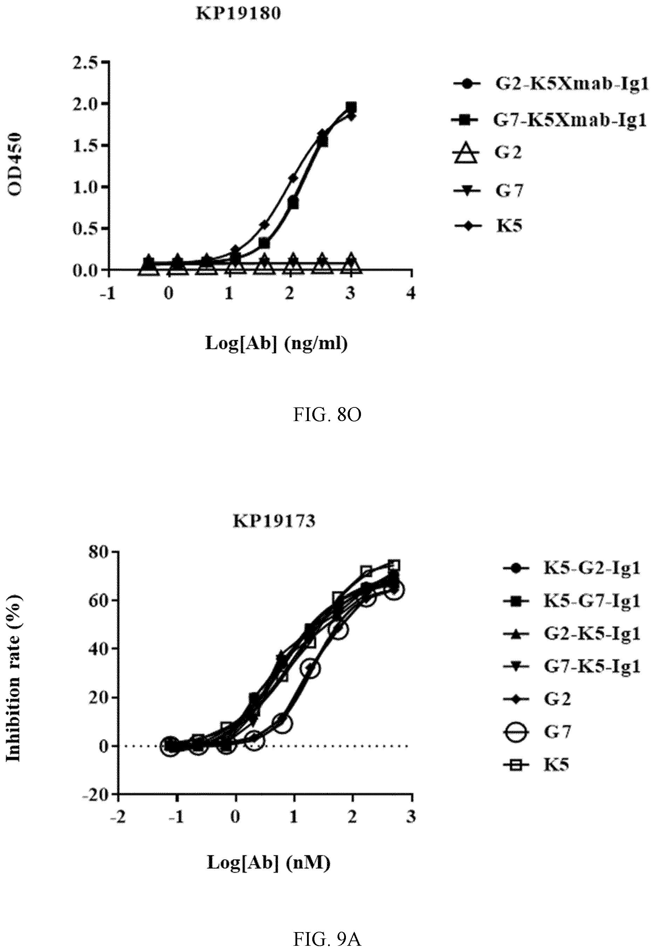

FIGS. 8M-8O ELISA binding results of exemplary scFv-Fab IgGformatbispecific antibodies to LPSof different serotypes of *K. pneumoniae* strains, respectively. FIG. 8M shows the binding results of KP19173 strain O1-type LPS; FIG. 8N shows the binding results of KP19213 strain O1-type LPS; FIG. 8O shows the bindingresults of KP19180 strain O2-type LPS.

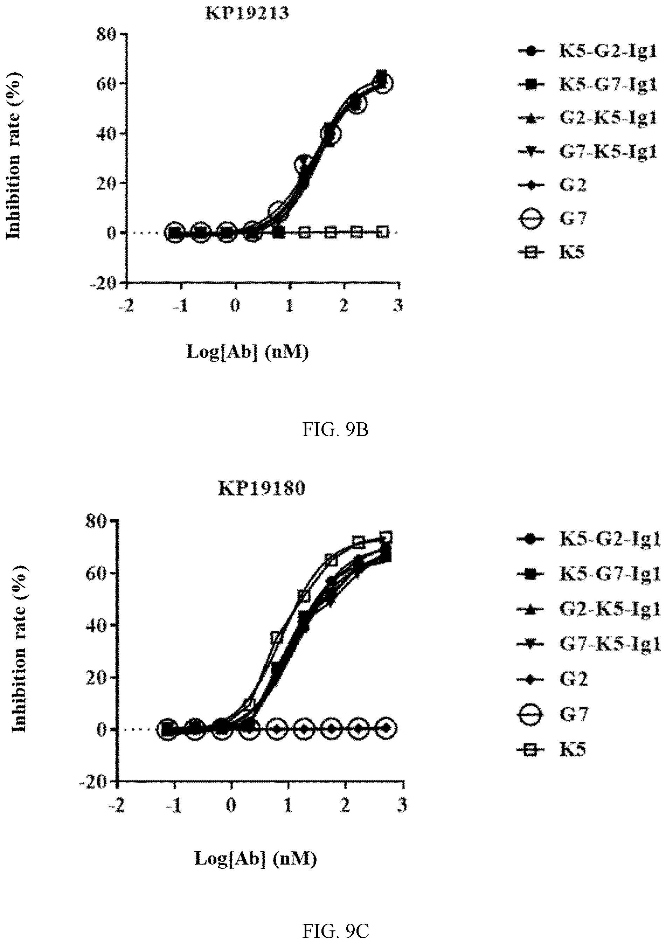

FIGS. 9A-9C show the results of neutralization activity of exemplaryDVD-Ig formatbispecific antibodiestoLPS of different serotypes of *K. pneumoniae* strains, respectively. FIG. 9A shows the neutralization activity to KP19173 strain O1-type LPS; FIG. 9B shows the result of neutralization activity to KP19213 strain O1-type LPS; FIG. 9C shows the result of neutralization activity toKP19180 strain O2-type LPS.

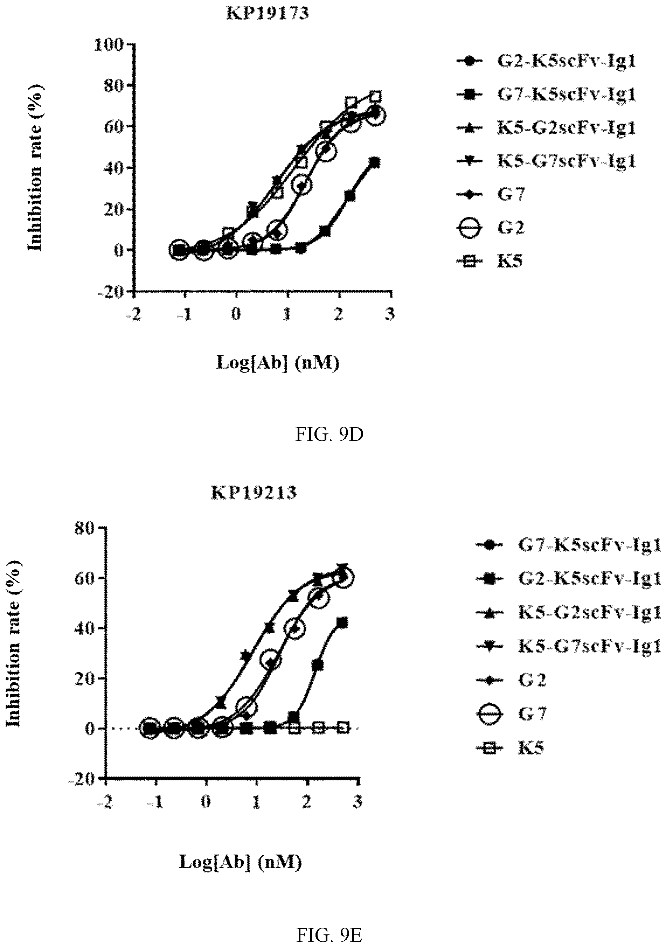

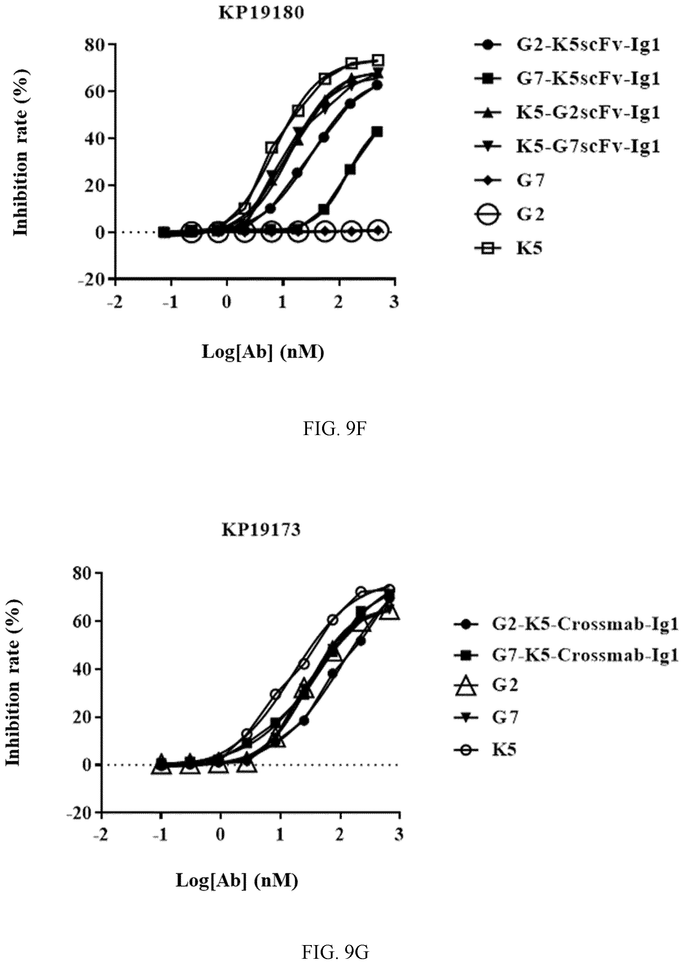

FIGS. 9D-9F show the results of neutralization activity of exemplaryBs4Abformatbispecific antibodiesto LPS of different serotypes of *K. pneumoniae* strains, respectively. FIG. 9D shows the neutralization activity to KP19173 strain O1-type LPS; FIG. 9E shows the result of neutralization activity to KP19213 strain O1-type LPS; FIG. 9F shows the result of neutralization activity to KP19180 strain O2-type LPS.

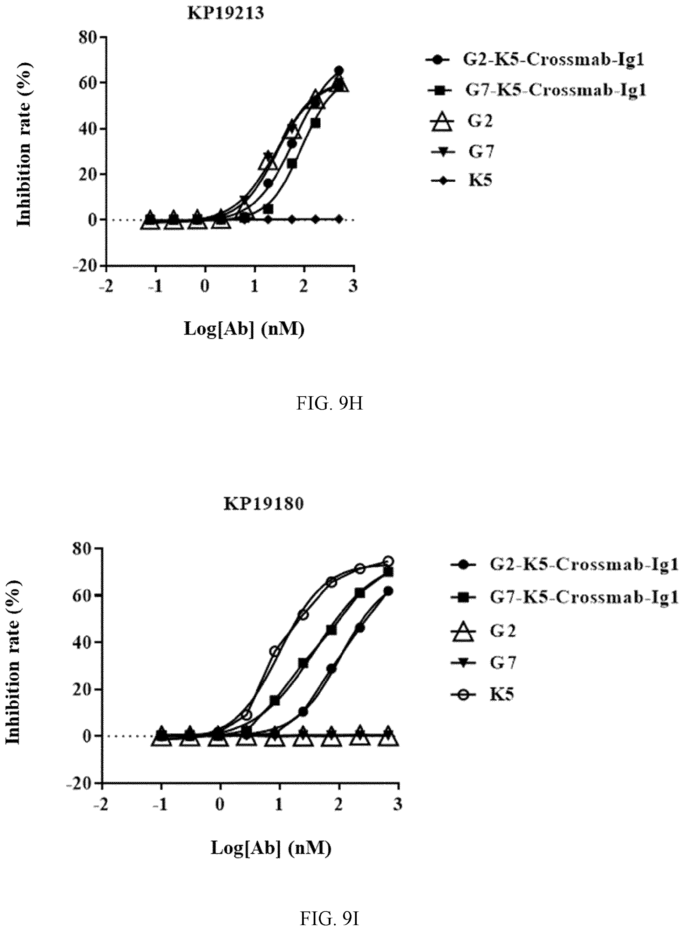

FIGS. 9G-9I show the results of neutralization activity of exemplary Hetero H, CrossMabformatbispecific antibodiesto LPS of different serotypes of *K. pneumoniae* strains, respectively. FIG. 9G shows the neutralization activity to KP19173 strain O1-type LPS; FIG. 9H shows the result of neutralization activity to KP19213 strain O1-type LPS; FIG. 9I shows the result of neutralization activity to KP19180 strain O2-type LPS.

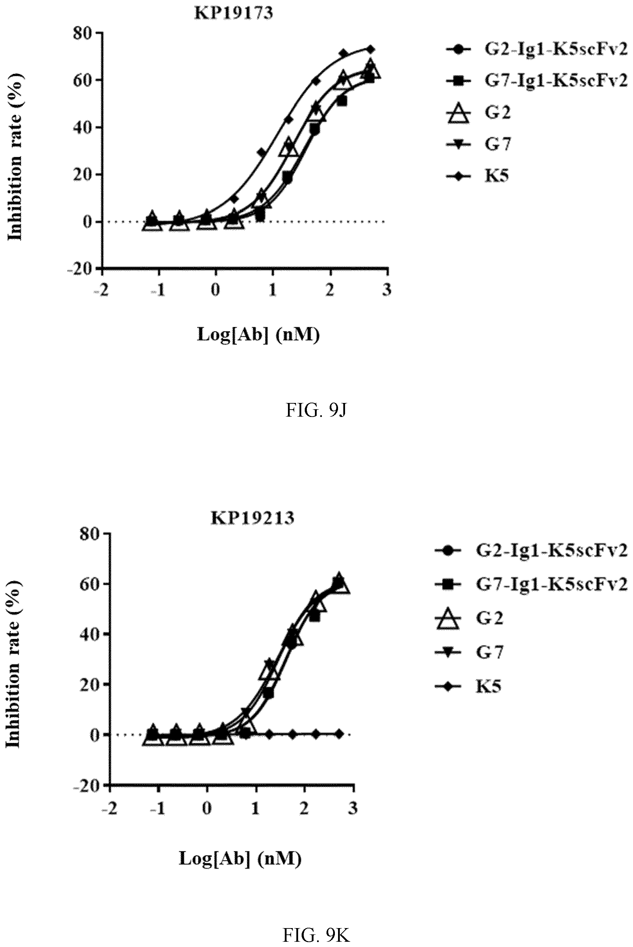

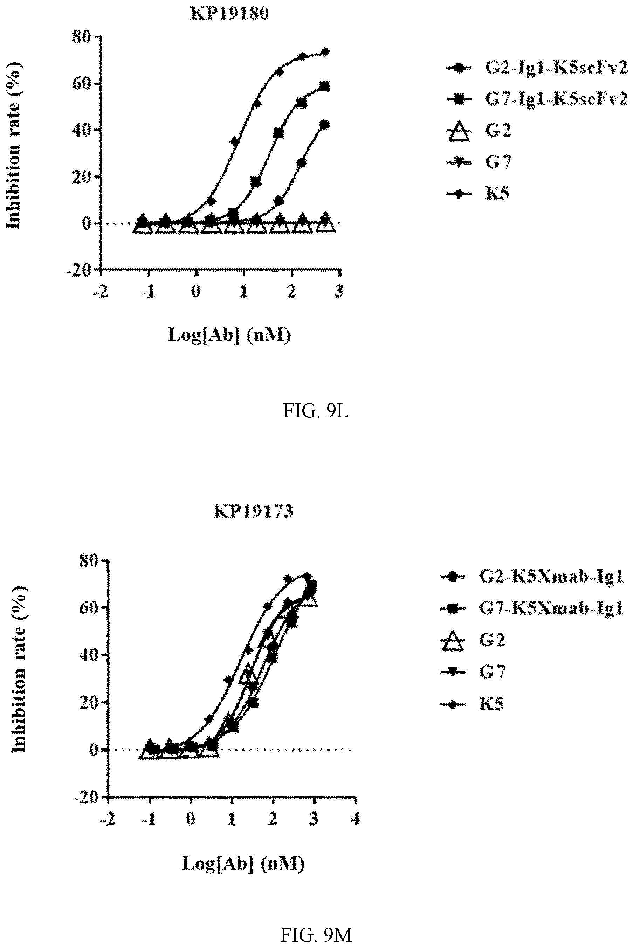

FIGS. 9J-9L show the results of neutralization activity of exemplaryIgG-(scFv)₂formatbispecific antibodiesto LPS of different serotypes of *K. pneumoniae* strains, respectively. FIG. 9J shows the neutralization activity toKP19173 strain O1-type LPS; FIG. 9K shows the result of neutralization activity to KP19213 strain O1-type LPS; FIG. 9L shows the result of neutralization activity to KP19180 strain O2-type LPS.

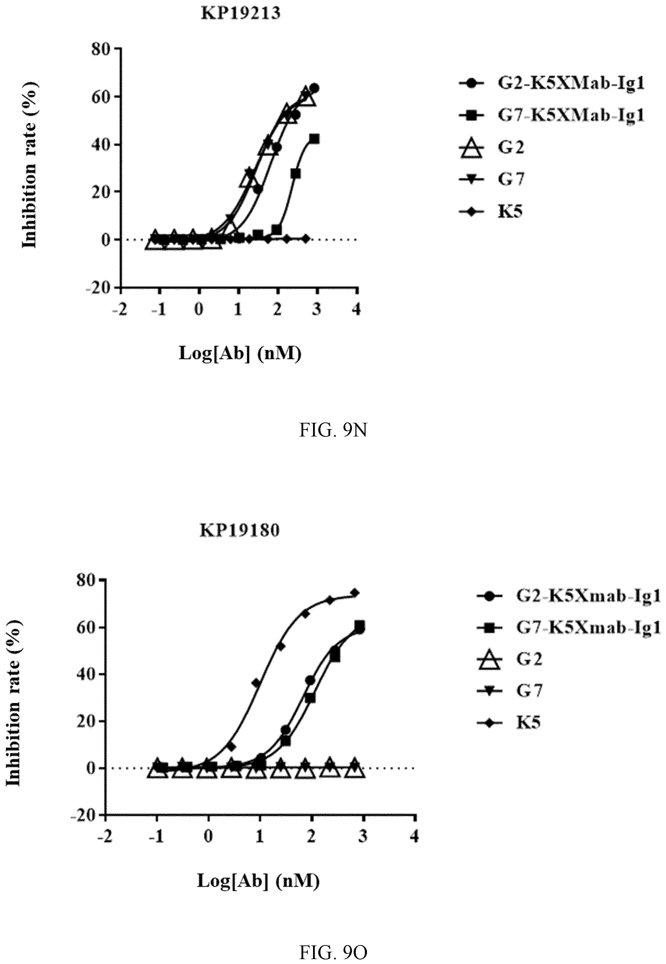

FIGS. 9M-9O show the results of neutralization activity of exemplaryscFv-Fab IgGformatbispecific antibodiesto LPS of different serotypes of *K. pneumoniae* strains, respectively. FIG. 9M shows the neutralization activity to KP19173 strain O1-type LPS; FIG. 9N shows the result of neutralization activity to KP19213 strain O1-type LPS; FIG. 9O shows the result of neutralization activity to KP19180 strain O2-type LPS.

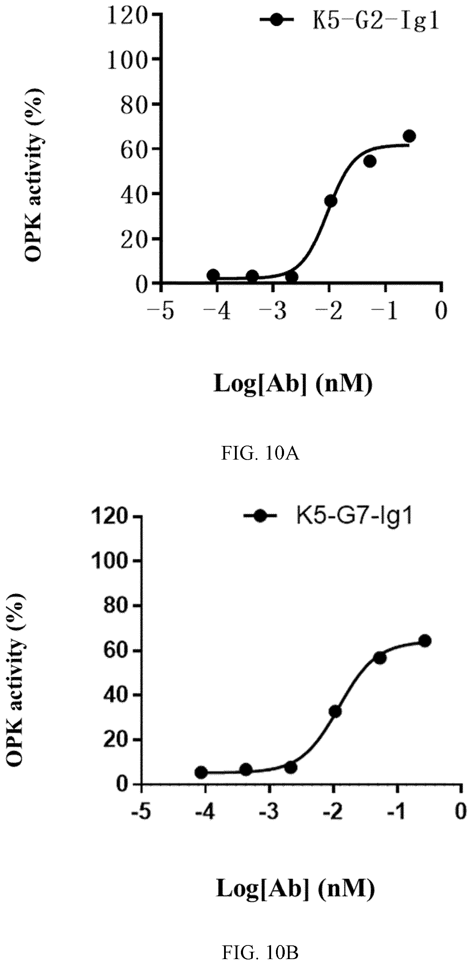

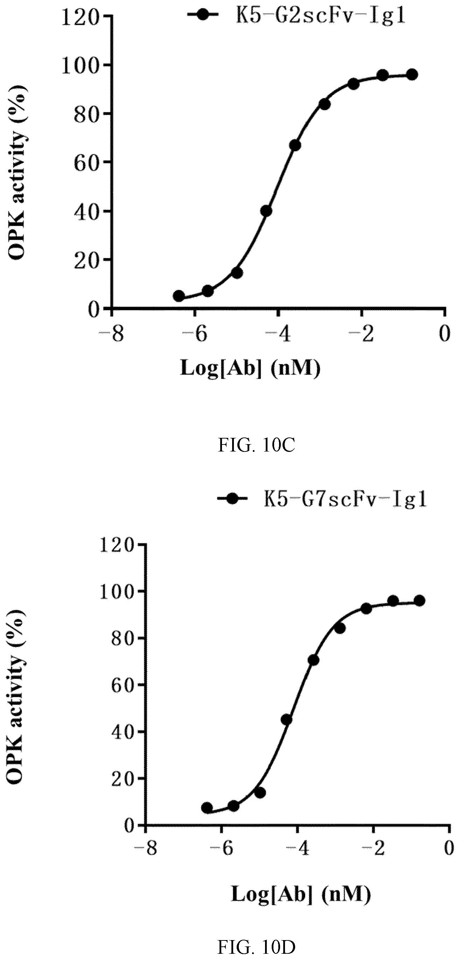

FIG. 10A shows the ability of DVD-Ig format bispecific antibody K5-G2-Ig1 in promoting opsonophagocytic killingof KP19173 strain; FIG. 10B shows the ability of DVD-Ig format bispecific antibody K5-G7-Ig1 in promotingopsonophagocytic killing of KP19173 strain; FIG. 10C shows the ability of Bs4Abformat bispecific antibody K5-G2scFv-Ig1 in promoting opsonophagocytic killing of KP19173 strain; FIG. 10D shows the ability of Bs4Abformat bispecific antibody K5-G7scFv-Ig1 in promoting opsonophagocytic killing of KP19173 strain.

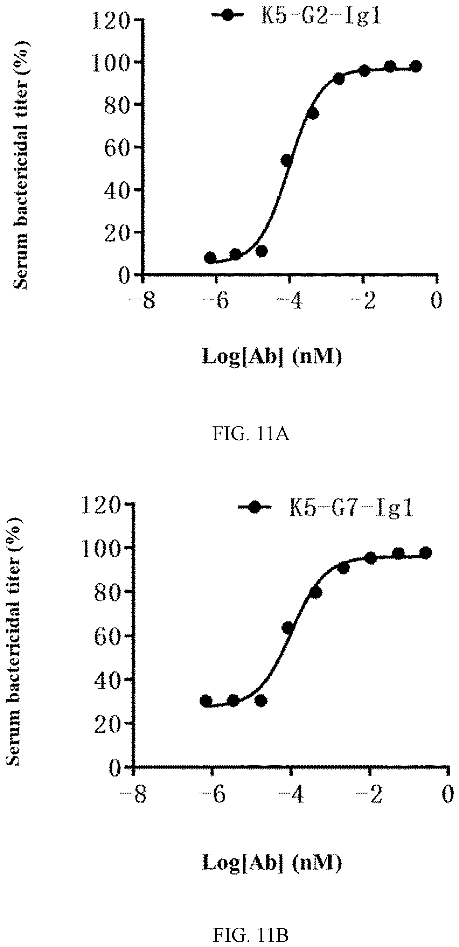

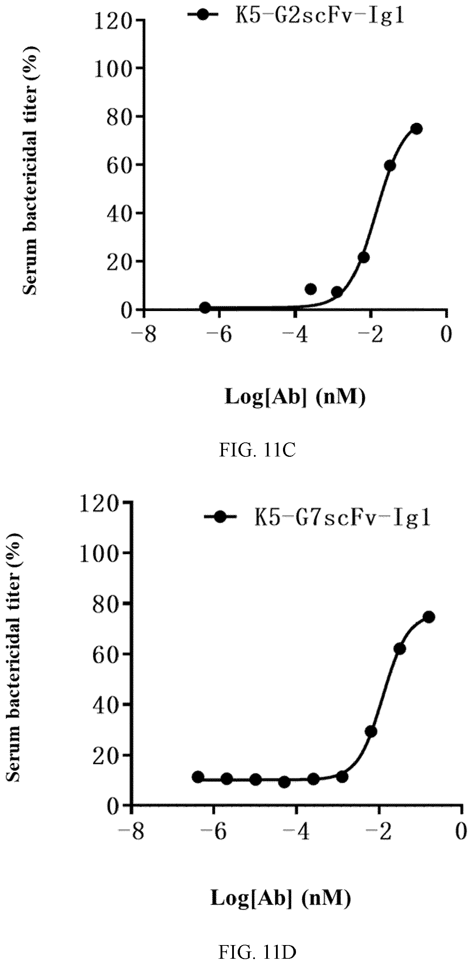

FIG. 11A shows the results of serum bactericidal activity of DVD-Ig format bispecific antibody K5-G2-Ig1 against KP19173 strain; FIG. 11B shows the results of serum bactericidal activity of DVD-Ig format bispecific antibody K5-G7-Ig1 against KP19173 strain; FIG. 11C shows the results of serum bactericidal activity of Bs4Ab format bispecific antibody K5-G2scFv-Ig1 against KP19173 strain; FIG. 11D shows the results of serum bactericidal activity of Bs4Ab format bispecific antibody K5-G7scFv-Ig1 against KP19173 strain.

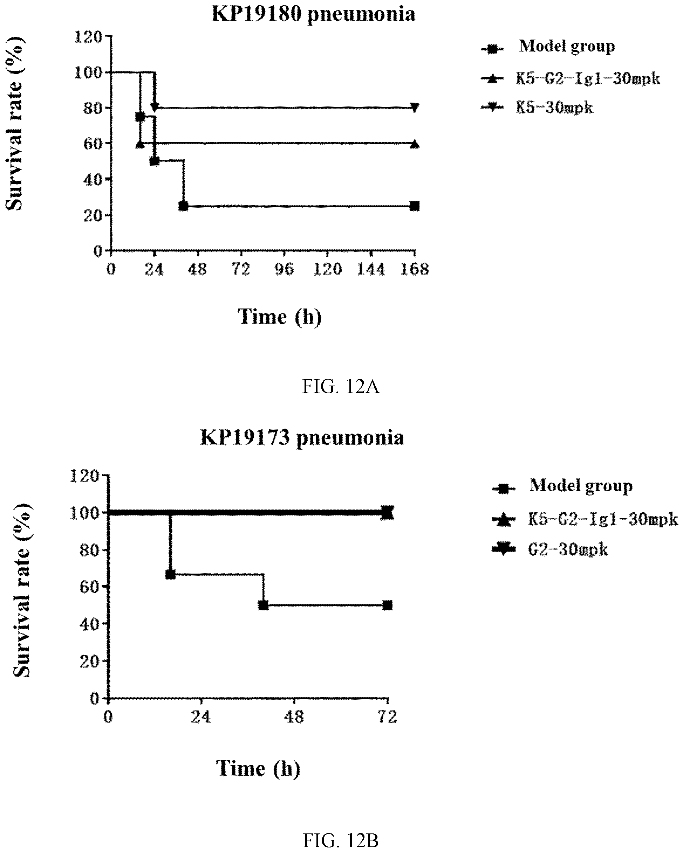

FIG. 12A shows the therapeutic effect of bispecific antibody K5-G2-Ig1 in mouse pneumonia model caused by *K. pneumoniae* KP19180 infection; FIG. 12B shows the therapeutic effect of bispecific antibody K5-G2-Ig1 in mouse pneumonia model caused by K. *pneumoniae* KP19173 infection.

### DETAILED DESCRIPTION OF THE APPLICATION

The present application in one aspect provides antibodies or antigen-binding fragments specifically binding to *K. pneumoniae* O2 antigen, in one aspect provides bispecific antibodies specifically binding to *K. pneumoniae* O2 antigen and O1 antigen. In one aspect provides combinations comprisingantibodies or antigen-binding fragments specifically binding to *K. pneumoniae* O2 antigen and antibodies or antigen-binding fragments specifically binding to *K. pneumoniae* O1 antigen. By using a combination of selections on scFv phage libraries, affinity maturation and appropriately designed biochemical and biological assays, we have identified the antibodies or antigen-binding fragments specifically binding to *K. pneumoniae* O2 antigen and the antibodies or antigen-binding fragments specifically binding to *K. pneumoniae* O1 antigen. The bispecific antibodiesspecifically binding to *K. pneumoniae* O2 antigen and O1 antigen were also produced. When the disease is treated in the form of (i) a pharmaceutical composition, or (ii) a bispecific antibody, or (iii) a combination, it can cover different serotypes of *K. pneumoniae* to achieve cumulative or synergistic effect and improve the broad spectrum of antibacterial.

Also provided are nucleic acids encoding the antibodies or antigen-binding fragments specifically binding to *K. pneumoniae* O2 antigen, or the bispecific antibodies specifically binding to *K. pneumoniae* O2 antigen and O1 antigen, compositions comprising the antibodies or antigen-binding fragments specifically binding to *K. pneumoniae* O2 and antibodies or antigen-binding fragments specifically binding to *K. pneumoniae* O1 antigen, and methods of making and using the antibodies specifically binding to *K. pneumoniae O2* antigen or the antibodiesspecifically binding to *K. pneumoniae* O1 antigen or bispecific antibodies specifically binding to *K. pneumoniae* O1 antigen and O2 antigen, and the compositions comprising thereof.

### Definitions

As used herein, "O antigen" refers to one of the components of *Klebsiella* LPS, forms LPS together with lipid A and core oligosaccharides. The "O1 antigen" refers to the O antigen portion of the O1 serotype *Klebsiella* LPS, and the "O2 antigen" refers to the O antigen portion of the O2 serotype *Klebsiella* LPS, and the O antigen contains the D-galactan-I disaccharide unit.

As used herein, "treatment" or "treating" is an approach for obtaining beneficial or desired results, including clinical results. For purposes of this application, beneficial or desired clinical results include, but are not limited to, one or more of the following: alleviating one or more symptoms resulting from the disease, diminishing the extent of the disease, stabilizing the disease (*e.g.,* preventing or delaying the worsening of the disease), preventing or delaying the spread (*e.g.,* systemic spread of a pathogen) of the disease, preventing or delaying the recurrence of the disease, delaying or slowing the progression of the disease, ameliorating the disease state, providing a remission (partial or total) of the disease, decreasing the dose of one or more other medications required to treat the disease, delaying the progression of the disease, increasing or improving the quality of life, increasing weight gain, and/or prolonging survival. Also encompassed by "treatment" is a reduction of pathological consequence of infection (such as, for example, host cell lysis or necrosis). The methods of the application contemplate any one or more of these aspects of treatment.

The term "prevent," and similar words such as "prevented," "preventing," "prevention" or "prophylactic" etc., indicate an approach for preventing, inhibiting, or reducing the likelihood of the occurrence or recurrence of, a disease or condition, *e.g.,* a pathogenic infection. It also refers to delaying the occurrence or recurrence of a disease or condition, or delaying the occurrence or recurrence of the symptoms of a disease or condition. As used herein, "prevention" and similar words also includes reducing the intensity, effect, symptoms and/or burden of a disease or condition prior to occurrence or recurrence of the disease or condition. As used herein, "prevention" and similar words also includes reducing the risk and susceptibility to occurrence or recurrence of the disease or condition, e.g., a pathogenic infection.

The antibody or antigen-binding fragment as used herein, the term "antibody" is used in the broadest sense and encompasses a variety of antibody structures, including, but not limited to, monoclonal antibodies, polyclonal antibodies, monospecific, multi-specific antibodies (e.g., bispecific antibodies), full-length antibodies and antigen-binding fragments thereof, so long as they exhibit the desired antigen binding activity. A full-length antibody comprises two heavy chains and two light chains. The variable domains of the light and heavy chains are responsible for antigen binding. The variable domains in both chains generally contain three highly variable loops called the complementarity determining regions (CDRs), light chain (LC) CDRs including LC-CDR1, LC-CDR2, and LC-CDR3, heavy chain (HC) CDRs including HC-CDR1, HC-CDR2, and HC-CDR3. CDR boundaries for the antibodies and antigen-binding fragments disclosed herein may be defined or identified by the conventions of Kabat, Chothia, or Al-Lazikani (Al-Lazikani 1997; Chothia 1985; Chothia 1987; Chothia 1989; Kabat 1987; Kabat 1991). The three CDRs of the heavy or light chains are interposed between flanking stretches known as framework regions (FRs), which are more highly conserved than the CDRs and form a scaffold to support the hypervariable loops. The constant regions of the heavy and light chains are not involved in antigen binding, but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, δ, ε, γ, and µ heavy chains, respectively. Several of the major antibody classes are divided into subclasses such as IgG1 (γ1 heavy chain), IgG2 (γ2 heavy chain), IgG3 (γ3 heavy chain), IgG4 (γ4 heavy chain), IgA1 (α1 heavy chain), or IgA2 (α2 heavy chain).

The term "antigen-binding fragment" as used herein includes an antibody fragment including, for example, a diabody, a Fab, a Fab', a F(ab')2, an Fv fragment, a disulfide stabilized Fv fragment (dsFv), a (dsFv)2, a bispecific dsFv (dsFv-dsFv'), a disulfide stabilized diabody (ds diabody), a single-chain Fv (scFv), an scFv dimer (bivalent diabody), a multispecific antibody formed from a portion of an antibody comprising one or more CDRs, a camelized single domain antibody, a nanobody, a domain antibody, a bivalent domain antibody, or any other antibody fragment that binds to an antigen but does not comprise a complete antibody structure. Wherein the Fab(fragment antigen-binding)as used herein is monovalent fragment containing the V_{L} domain, V_{H} domain, C_{L} domain, and domain of antibodies. An antigen-binding fragment also includes a fusion protein comprising the antibody fragment described above. An antigen-binding fragment is capable of binding to the same antigen to which the parent antibody or a parent antibody fragment (e.g., a parent scFv) binds. In some embodiments, an antigen-binding fragment may comprise one or more CDRs from a particular human antibody grafted to a framework region from one or more different human antibodies.

The term "bispecific antibody" as used herein refers to an antibody having binding specificity to two different antigen or epitopes in one molecule. Bispecific antibody is produced through a process that involves design of the intact molecule, synthesis and cloning of the nucleotide sequences for each domain, expression in mammalian cells and purification of the final product. Exemplary bispecific antibodies formats include those known in the art, e.g., DVD-Ig format, Bs4Ab format, Hetero H, CrossMab format, IgG- (scFv)₂format or scFv-Fab IgG format, etc. (see, e.g., Labrijn AF, et al. Nat Rev Drug Discov. 2019 Aug;18(8):585-608).

The DVD-Ig (Dual-variable domain-Ig) bispecific antibody, the formatof whichisconnectingthe V_{L} andV_{H} domain of another antibody respectivelyto the N-terminus of the light chainand heavy chains of a normal IgG antibody, and formingan antigen-binding domain (Fv)by the interaction between V_{H} and V_{L} of two antibodies, which can simultaneously bind to the corresponding antigen to achieve bispecific binding. Exemplary DVD-Ig formatbispecific antibody is described in Wu C,et al. Molecular construction and optimization of anti-human IL-1alpha/beta dual variable region immunoglobulin (DVD-Ig) molecules. MAbs. 2009 Jul-Aug;1(4):339-47. The Bs4Ab format bispecific antibodyisa tetravalent bispecific antibody comprising a full length IgG1 with another binding unit scFv inserted into the hinge domain to achieve bispecific.The Bs4Abformat bispecific antibodyis described in document Bezabeh B,et al. Insertion of scFv into the hinge domain of full-length IgG1 monoclonal antibody results in tetravalent bispecific molecule with robust properties. MAbs. 2017 Feb/Mar;9(2):240-256. The Hetero H, crossMab format bispecific antibody, i.e.,the knob-in-hole structure (KIH) isdesigned in the Fc region, introduces two Cys residue mutations that form stable disulfide bridges (S354C on the "knob" side and Y349C on the "hole" side). CrossMab technology was applied simultaneouslyto ensure correct pairing between the light and heavy chains of the antibody. CrossMAb technology isbased on the exchange of antibody domains in one Fab arm of bispecific IgG antibodies, either as an exchange ofintact Fab domains (CrossMAb Fab), or as an exchange of only variable domains(CrossMAb V_{H}-V_{L}) or only constant domains (CrossMAb C_{H}1-C_{L})in the Fab domain. The bispecific antibody of Hetero H, CrossMabformat is described in Klein C, et al. The use of CrossMAb technology for the generation of bi- and multispecific antibodies. MAbs. 2016 Aug-Sep;8(6): 1010-20. The bispecific antibody of IgG- (scFv)₂format, i.e.,bispecific is achieved by connecting the scFv fragment of another antibody to the Fc-terminus of two heavy chains of an IgG antibody. The bispecific antibody of IgG- (scFv)₂ formatis described inColoma MJ, Morrison SL. Design and production of novel tetravalent bispecific antibodies. Nat Biotechnol. 1997 Feb; 15(2): 159-63. The bispecific antibody of scFv-Fab IgGformat isa heterodimeric antibody, with IgG antibody structure, wherein one Fab arm is replaced with an scFv structure, wherein the first monomer comprises scFvand IgG Fc, the scFv is linked to IgG FcC_{H}2 domain by a peptide linker and the second monomer comprises Fab andIgG Fc.

The term "antigen-binding fragment" as used herein refers to the portion of an antigen binding molecule that specifically binds to an antigen. More specifically, the term "antigen-binding fragment" refers to a portion of an antibody that comprises a region that specifically binds to and is complementary to a portion or all of an antigen. In the case of large antigens, the antigen binding molecule may bind only a specific part of the antigen, which part is called an epitope. The antigen-binding fragment may be provided by, for example, one or more variable domains (also referred to as variable regions). Preferably, the antigen-binding fragment comprises an antibody light chain variable domain (V_{L}) and an antibody heavy chain variable domain (V_{H}). In one aspect, the antigen-binding fragment is capable of binding its antigen and blocking or partially blocking the function of said antigen. antigen-binding fragments that specifically bind *Klebsiella*O2 antigen or O1 antigen include antibodies and fragments thereof as further defined herein.

The term "epitope" as used herein refers to the specific group of atoms or amino acids on an antigen to which an antibody or antibody moiety binds. Two antibodies or antibody moieties may bind the same epitope within an antigen if they exhibit competitive binding for the antigen.

As used herein, a first antibody "competes" for binding to a target *Klebsiella*O2 antigen with a second antibody when the first antibody inhibits target *Klebsiella*O2 antigen binding of the second antibody by at least about 50% (such as at least about any of 55%, 60%, 65%, 70%, 75%, 80%, 85%, 90%, 95%, 98% or 99%) in the presence of an equimolar concentration of the first antibody, or *vice versa.* A high throughput process for "binning" antibodies based upon their cross-competition is described in PCT Publication No. WO 03/48731.

As used herein, the term "specifically binds," "specifically recognizing," or "is specific for" refers to measurable and reproducible interactions, such as binding between a target and an antibody, that is determinative of the presence of the target in the presence of a heterogeneous population of molecules, including biological molecules. For example, an antibody that specifically recognizes a target (which can be an epitope) is an antibody that binds to this target with greater affinity, avidity, more readily, and/or with greater duration than its bindings to other targets. In some embodiments, an antibody that specifically recognizes an antigen reacts with one or more antigenic determinants of the antigen with a binding affinity that is at least about 10 times its binding affinity for other targets.

An "isolated" antibody as used herein refers to an antibody that (1) is not associated with proteins found in nature, (2) is free of other proteins from the same source, (3) is expressed by a cell from a different species, or, (4) does not occur in nature.

The term "isolated nucleic acid" as used herein is intended to mean a nucleic acid of genomic, cDNA, or synthetic origin or some combination thereof, which by virtue of its origin the "isolated nucleic acid" (1) is not associated with all or a portion of a polynucleotide in which the "isolated nucleic acid" is found in nature, (2) is operably linked to a polynucleotide which it is not linked to in nature, or (3) does not occur in nature as part of a larger sequence.

As used herein, the term "CDR" or "complementarity determining region" is intended to mean the non-contiguous antigen combining sites found within the variable domain of both heavy and light chain polypeptides. These particular regions have been described by Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), where the definitions include overlapping or subsets of amino acid residues when compared against each other. Nevertheless, application of either definition to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**TABLE 1: CDR DEFINITIONS**

| | **Kabat¹** | | **Chothia²** | | **MacCallum³** | | **IMGT⁴** | **AHo⁵** |
|---|---|---|---|---|---|---|---|---|
| V_{H} CDR1 | | 31-35 | | 26-32 | | 30-35 | 27-38 | 25-40 |
| V_{H} CDR2 | | 50-65 | | 53-55 | | 47-58 | 56-65 | 58-77 |
| V_{H} CDR3 | | 95-102 | | 96-101 | | 93-101 | 105-117 | 109-137 |
| V_{L} CDR1 | | 24-34 | | 26-32 | | 30-36 | 27-38 | 25-40 |
| V_{L} CDR2 | | 50-56 | | 50-52 | | 46-55 | 56-65 | 58-77 |
| V_{L} CDR3 | | 89-97 | | 91-96 | | 89-96 | 105-117 | 109-137 |

| | | | | | | | | |
|---|---|---|---|---|---|---|---|---|
| 'Residue numbering follows the nomenclature of Kabat *et al.*, *supra* ²Residue numbering follows the nomenclature of Chothia *et al.*, *supra* ³Residue numbering follows the nomenclature of MacCallum *et al.*, *supra* ⁴Residue numbering follows the nomenclature of Lefranc *et al.*, *supra* ⁵Residue numbering follows the nomenclature of Honegger and Plückthun, *supra* | | | | | | | | |

The term "chimeric antibodies" refer to antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit a biological activity of this application (*see* U.S. Patent No. 4,816,567; and Morrison et al., Proc. Natl. Acad. Sci. USA, 81:6851-6855 (1984)).

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the heavy and light chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv," also abbreviated as "sFv" or "scFv," are antibody fragments that comprise the V_{H} and V_{L} antibody domains connected into a single polypeptide chain. In some embodiments, the scFv polypeptide further comprises a peptide linker between the V_{H} and V_{L} domains which enables the scFv to form the desired structure for antigen binding. For a review of scFv, *see* Pluckthun in The Pharmacology of Monoclonal Antibodies, vol. 113, Rosenburg and Moore eds., Springer-Verlag, New York, pp. 269-315 (1994).

The term "diabodies" refers to small antibody fragments prepared by constructing scFv fragments (see preceding paragraph) typically with short linkers (such as about 5 to about 10 residues) between the V_{H} and V_{L} domains such that inter-chain but not intra-chain pairing of the V domains is achieved, resulting in a bivalent fragment, *i.e.,* fragment having two antigen-binding sites. Bispecific diabodies are heterodimers of two "crossover" scFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

"Humanized" forms of non-human (e.g., rodent) antibodies are chimeric antibodies that contain minimal sequence derived from the non-human antibody. For the most part, humanized antibodies are human immunoglobulins (recipient antibody) in which residues from a hypervariable region (HVR) of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies can comprise residues that are not found in the recipient antibody or in the donor antibody. These modifications are made to further refine antibody performance. In general, the humanized antibody will comprise substantially at least one, and typically two, variable regions, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, *see* Jones et al., Nature 321:522-525 (1986); Riechmann et al., Nature 332:323-329 (1988); and Presta, Curr. Op. Struct. Biol. 2:593-596 (1992).

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skilled in the art, for instance, using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program MUSCLE (Edgar, R.C., Nucleic Acids Research 32(5): 1792-1797, 2004; Edgar, R.C., BMC Bioinformatics 5(1): 113, 2004).

The terms "Fc (fragment crystallizable)" or "Fc region" refers to polypeptides comprising the complete antibody constant region, excluding the CH₁ domain, and in some cases comprising a partial hinge region, whether in monomeric form or multimeric form. The primary immunoglobulin source of the natural Fc is preferably of human origin and can be any immunoglobulin, such as IgG1, IgG2, IgG3, or IgG4. Natural Fc is composed of monomeric peptides, which can be covalently (*i.e.,* disulfide bonds) and non-covalently linked into dimeric or multimeric forms. The Fc region of immunoglobulins generally comprises the CH₂ and CH₃ domains of the heavy chain constant region, and can optionally comprise the CH₄ domain.

In some embodiments, each of the two Fc monomers in the Fc dimer comprises an amino acidsubstitution that promotes heterodimerization of the two monomers. In some embodiments, heterodimerization ofthe Fc monomers may be facilitated by introducing different but compatible substitutions in the two Fc monomers, such as "knob-into-hole"residue pairs. The "knob-into-hole" technology is also disclosed in U.S. patent No. 8,216,805. In some embodiments, one Fc monomer comprises the knob mutation T366W and the other Fcmonomer comprises the hole mutations T366S, L358A and Y407V. In some embodiments, two Cys residueswere introduced to form a stabilized disulfide bridge (S354C in the "knob" side and Y349C in the "hole" side).

The terms "Fc receptor" or "FcR" are used to describe a receptor that binds to the Fc region of an antibody. In some embodiments, an FcR of this application is one that binds an IgG antibody (a γ receptor) and includes receptors of the FcyRI, FcyRII, and FcyRIII subclasses, including allelic variants and alternatively spliced forms of these receptors. FcyRII receptors include FcγRIIA (an "activating receptor") and FcyRIIB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibiting receptor FcyRIIB contains an immunoreceptor tyrosine-based inhibition motif (ITIM) in its cytoplasmic domain *(see* review M. in Daëron, Annu. Rev. Immunol. 15:203-234 (1997)). The term includes allotypes, such as FcγRIIIA allotypes: FcγRIIIA-Phe158, FcγRIIIA-Val158, FcyRIIA-R131 and/or FcyRIIA-H131. FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

The term "FcRn" refers to the neonatal Fc receptor (FcRn). FcRn is structurally similar to major histocompatibility complex (MHC) and consists of an α-chain noncovalently bound to β2-microglobulin. The multiple functions of the neonatal Fc receptor FcRn are reviewed in Ghetie and Ward (2000) Annu. Rev. Immunol. 18, 739-766. FcRn plays a role in the passive delivery of immunoglobulin IgGs from mother to young and the regulation of serum IgG levels. FcRn can act as a salvage receptor, binding and transporting pinocytosed IgGs in intact form both within and across cells, and rescuing them from a default degradative pathway.

The "CH1 domain" of a human IgG heavy chain constant region usually extends from about amino acid 118 to about amino acid 215 (EU numbering system).

"Hinge region" is generally defined as stretching from Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "CH2 domain" of a human IgG Fc region usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec Immunol. 22:161-206 (1985).

The "CH3 domain" comprises the stretch of residues C-terminal to a CH2 domain in an Fc region (*i.e.,* from about amino acid residue 341 to the C-terminal end of an antibody sequence, typically at amino acid residue 446 or 447 of an IgG).

A "functional Fc fragment" possesses an "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (*e.g.* B cell receptor; BCR), etc. Such effector functions generally require the Fc region to be combined with a binding domain (*e.g.* an antibody variable domain) and can be assessed using various assays known in the art.

An antibody with a variant IgG Fc with "altered" FcR binding affinity or ADCC activity is one which has either enhanced or diminished FcR binding activity (*e.g.,* FcyR or FcRn) and/or ADCC activity compared to a parent polypeptide or to a polypeptide comprising a native sequence Fc region. The variant Fc which "exhibits increased binding" to an FcR binds at least one FcR with higher affinity (*e.g.,* lower apparent Kd or IC₅₀ value) than the parent polypeptide or a native sequence IgG Fc. According to some embodiments, the improvement in binding compared to a parent polypeptide is about 3-fold, such as about any of 5, 10, 25, 50, 60, 100, 150, 200, or up to 500-fold, or about 25% to 1000% improvement in binding. The polypeptide variant which "exhibits decreased binding" to an FcR, binds at least one FcR with lower affinity (*e.g.,* higher apparent Kd or higher IC₅₀ value) than a parent polypeptide. The decrease in binding compared to a parent polypeptide may be about 40% or more decrease in binding.

"Antibody-dependent cell-mediated cytotoxicity" or "ADCC" refers to a form of cytotoxicity in which secreted Ig bound to Fc receptors (FcRs) present on certain cytotoxic cells *(e.g.,* Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are required for such killing. The primary cells for mediating ADCC, NK cells, express FcyRIII only, whereas monocytes express FcyRI, FcyRII and FcyRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. PNAS(USA) 95:652-656 (1998).

The polypeptide comprising a variant Fc region which "exhibits increased ADCC" or mediates ADCC in the presence of human effector cells more effectively than a polypeptide having wild type IgG Fc or a parent polypeptide is one which *in vitro* or *in vivo* is substantially more effective at mediating ADCC, when the amounts of polypeptide with variant Fc region and the polypeptide with wild type Fc region (or the parent polypeptide) in the assay are essentially the same. Generally, such variants will be identified using any *in vitro* ADCC assay known in the art, such as assays or methods for determining ADCC activity, *e.g.,* in an animal model *etc.* In some embodiments, the variant is from about 5-fold to about 100-fold, *e.g.* from about 25 to about 50-fold, more effective at mediating ADCC than the wild type Fc (or parent polypeptide).

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, *e.g.* as described in Gazzano-Santoro et al., J. Immunol. Methods 202:163 (1996), may be performed. Polypeptide variants with altered Fc region amino acid sequences and increased or decreased C1q binding capability are described in US patent No. 6,194,551B1 and WO99/51642. The contents of those patent publications are specifically incorporated herein by reference. *See* also, Idusogie et al. J. Immunol. 164: 4178-4184 (2000).

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

"Homologous" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared times 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

An "effective amount" of an antibody (including bispecific antibody) or composition as disclosed herein, is an amount sufficient to carry out a specifically stated purpose. An "effective amount" can be determined empirically and by known methods relating to the stated purpose.

The term "therapeutically effective amount" refers to an amount of an antibody (including bispecific antibody) or composition as disclosed herein, effective to "treat" a disease or disorder in an individual. In the case of *K. pneumoniae* infection, the therapeutically effective amount of the antibody or composition as disclosed herein can reduce the number of infected cells; inhibit (*i.e.,* slow to some extent and preferably stop) the spread of infection; and/or relieve to some extent one or more of the symptoms associated with the infection. To the extent the antibody or composition as disclosed herein can prevent *K. pneumoniae* growth and/or kill *K. pneumoniae* in an infection, the antibody can be cytostatic and/or cytotoxic. In some embodiments, the therapeutically effective amount is an amount that inhibits infection in a patient. In some embodiments, the therapeutically effective amount is an amount that completely eradicates infection in a patient.

As used herein, by "pharmaceutically acceptable" or "pharmacologically compatible" is meant a material that is not biologically or otherwise undesirable, *e.g.,* the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug Administration.

It is understood that embodiments of the application described herein include "consisting of" and/or "consisting essentially of' embodiments.

Reference to "about" a value or parameter herein includes (and describes) variations that are directed to that value or parameter per se. For example, description referring to "about X" includes description of "X".

As used herein, reference to "not" a value or parameter generally means and describes "other than" a value or parameter. For example, the method is not used to treat infection of type X means the method is used to treat infection of types other than X.

As used herein and in the appended claims, the singular forms "a," "or," and "the" i**nc**lude plural referents unless the context clearly dictates otherwise.

### Antibodies specifically binding toK. pneumoniae O2 antigen

In one aspect, the present application provides antibodies or antigen-binding fragments specifically binding toK. *pneumoniae* O2 antigenincluding, but not limited to, humanized antibodies, chimeric antibodies, mouse antibodies, human antibodies, and antibodies comprising the heavy chain and/or light chain CDRs discussed herein. In one aspect, the antibodies or antigen-binding fragments are isolated antibodies that bind to O2 antigen. Contemplated antibodies or antigen-binding fragments specifically binding to *K. pneumoniae* O2 antigen can include the entire or a fragment of full-length antibodies specifically binding to *K. pneumoniae* O2 antigen (e.g., full-length IgG1, IgG2 or IgG4), scFvsspecifically binding to *K. pneumoniae* O2 antigen, multi-specific (such as bispecific) antibodiesspecifically binding to *K. pneumoniae* O2 antigen, immunoconjugatesspecifically binding to *K. pneumoniae* O2 antigen, and the like. In some embodiments, the antibody or antigen-binding fragment specifically binding to *K. pneumoniae* O2 antigen is a Fab, a Fab', a F(ab)'2, a Fab'-SH, a single-chain Fv (scFv), an Fv fragment, a dAb, a Fd, a nanobody, or a diabody. In some embodiments, the antibody or antigen-binding fragment specifically binding to *K. pneumoniae* O2 antigen means that the antibody or antigen-binding fragment binds to *K. pneumoniae* O2 with an affinity that is at least about 10 times (including for example at least about any of 10, 10², 10³, 10⁴, 10⁵, 10⁶, or 10⁷times) of its binding affinity for non-target. In some embodiments, the non-target is an antigen that is not *K. pneumoniae* O2 antigen.

Binding affinity can be determined by methods known in the art, such as ELISA, fluorescence activated cell sorting (FACS) analysis, or radioimmunoprecipitation assay (RIA). Kd can be determined by methods known in the art, such assurface plasmon resonance (SPR) assay or biolayer interferometry (BLI).

In certain aspects, the antibody or antigen-binding fragment specifically binding to *K. pneumoniae* O2 antigen can (a) promote, mediate, or enhance the opsonophagocytic killing (OPK) of *K. pneumoniae,* and/or (b) promote, mediate, or enhance the serum bactericidal activity (SBA) of *K. pneumoniae.*

Although the antibodies or antigen-binding fragments specifically binding to *K. pneumoniae* O2 antigen and containing human sequences (e.g., human heavy and light chain variable region sequences comprising human CDR sequences) are extensively discussed herein, non-human antibodies are also contemplated. In some embodiments, non-human antibodies comprise human CDR sequences from an antibody or antigen-binding fragment specifically binding to *K. pneumoniae* O2 antigenas described herein and non-human framework sequences. Non-human framework sequences include, in some embodiments, any sequence that can be used for generating synthetic heavy and/or light chain variable regions using one or more human CDR sequences as described herein, including, *e*.*g*., mammals, *e.g*., mouse, rat, rabbit, pig, bovine (*e.g*., cow, bull, buffalo), deer, sheep, goat, chicken, cat, dog, ferret, primate (*e*.*g*.**,** marmoset, rhesus monkey), etc. In some embodiments, a non-human antibody or antigen-binding fragment specifically binding to *K. pneumoniae* O2 antigen includes an antibody or antigen-binding fragment specifically binding to *K. pneumoniae* O2 antigengenerated by grafting one or more human CDR sequences as described herein onto a non-human framework sequence (*e*.*g*., a mouse or chicken framework sequence).

In some embodiments, the antibody or antigen-binding fragment specifically binding to *K. pneumoniae*O2 antigenbinds to the D-galactan I domain of *K. pneumoniae* LPS. In some embodiments, the antibody or antigen-binding fragment specifically binding to *K. pneumoniae*O2 antigen is specific for *K. pneumoniae*O2 antigen and does not exhibit species cross-reactivity or other types of non-K. *pneumoniae* O2 antigencross-reactivity. In some embodiments, the antibody or antigen-binding fragment specifically binding to *K. pneumoniae*O2 antigen exhibits other types of non-K. *pneumoniae* O2 antigencross-reactivity.

In some embodiments, the antibody specifically binding to *K. pneumoniae*O2 antigen comprises: (i) a V_{H} comprising an HC-CDR1, an HC-CDR2, and an HC-CDR3 of a V_{H} comprising the amino acid sequence of SEQ ID NO: 17; and a V_{L} comprising an LC-CDR1, an LC-CDR2, and an LC-CDR3 of a V_{L} comprising the amino acid sequence of SEQ ID NO: 21; (ii) a V_{H} comprising an HC-CDR1, an HC-CDR2, and an HC-CDR3 of a V_{H} comprising the amino acid sequence of SEQ ID NO: 18; and a V_{L} comprising an LC-CDR1, an LC-CDR2, and an LC-CDR3 of a V_{L} comprising the amino acid sequence of SEQ ID NO: 22; (iii) a V_{H} comprising an HC-CDR1, an HC-CDR2, and an HC-CDR3 of a V_{H} comprising the amino acid sequence of SEQ ID NO: 19; and a V_{L} comprising an LC-CDR1, an LC-CDR2, and an LC-CDR3 of a V_{L} comprising the amino acid sequence of SEQ ID NO: 23; (iv) a V_{H} comprising an HC-CDR1, an HC-CDR2, and an HC-CDR3 of a V_{H} comprising the amino acid sequence of SEQ ID NO: 19; and a V_{L} comprising an LC-CDR1, an LC-CDR2, and an LC-CDR3 of a V_{L} comprising the amino acid sequence of SEQ ID NO: 24; or(v) a V_{H} comprising an HC-CDR1, an HC-CDR2, and an HC-CDR3 of a V_{H} comprising the amino acid sequence of SEQ ID NO: 20; and a V_{L} comprising an LC-CDR1, an LC-CDR2, and an LC-CDR3 of a V_{L} comprising the amino acid sequence of SEQ ID NO: 25.

In some embodiments, the antibody specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 12, and an LC-CDR3 comprising the amino acid sequence of SEQ ID NO: 15, or a variant thereof comprising up to about 5 amino acid substitutions in the LC-CDRs.

In some embodiments, the antibody specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 9, an LC-CDR2 comprising the amino acid sequence of SEQ ID NO: 12, and an LC-CDR3 comprising the amino acid sequence of SEQ ID NO: 16, or a variant thereof comprising up to about 5 amino acid substitutions in the LC-CDRs.

In some embodiments, the antibody specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 10, an LC-CDR2 comprising the amino acid sequence of SEQ ID NO: 13, and an LC-CDR3 comprising the amino acid sequence of SEQ ID NO: 16, or a variant thereof comprising up to about 5 amino acid substitutions in the LC-CDRs.

In some embodiments, the antibody specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16, or a variant thereof comprising up to about 5 amino acid substitutions in the LC-CDRs.

In some embodiments, the antibody specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7, or a variant thereof comprising up to about 5 amino acid substitutions in the HC-CDRs; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15, or a variant thereof comprising up to about 5 amino acid substitutions in the LC-CDRs.

In some embodiments, the antibody specifically binding to *K. pneumoniae*O2 antigen comprises: a V_{H} comprising the amino acid sequence of SEQ ID NO: 17, or a variant thereof having at least about 80% (e.g., at least 80%, 85%, 90%, 95%, 96%, 97%, 98%, or 99%) sequence identity to the amino acid sequence of SEQ ID NO: 17; and a V_{L} comprising the amino acid sequence of SEQ ID NO: 21, or a variant thereof having at least about 80% (e.g., at least 80%, 85%, 90%, 95%, 96%, 97%, 98%, or 99%) sequence identity to the amino acid sequence of SEQ ID NO: 21.

In some embodiments, the antibody specifically binding to *K. pneumoniae*O2 antigen comprises: a V_{H} comprising the amino acid sequence of SEQ ID NO: 18, or a variant thereof having at least about 80% (e.g., at least 80%, 85%, 90%, 95%, 96%, 97%, 98%, or 99%) sequence identity to the amino acid sequence of SEQ ID NO: 18; and a V_{L} comprising the amino acid sequence of SEQ ID NO: 22, or a variant thereof having at least about 80% (e.g., at least 80%, 85%, 90%, 95%, 96%, 97%, 98%, or 99%) sequence identity to the amino acid sequence of SEQ ID NO: 22.

In some embodiments, the antibody specifically binding to *K. pneumoniae*O2 antigen comprises: a V_{H} comprising the amino acid sequence of SEQ ID NO: 19, or a variant thereof having at least about 80% (e.g., at least 80%, 85%, 90%, 95%, 96%, 97%, 98%, or 99%) sequence identity to the amino acid sequence of SEQ ID NO: 19; and a V_{L} comprising the amino acid sequence of SEQ ID NO: 23, or a variant thereof having at least about 80% (e.g., at least 80%, 85%, 90%, 95%, 96%, 97%, 98%, or 99%) sequence identity to the amino acid sequence of SEQ ID NO: 23.

In some embodiments, the antibody specifically binding to *K. pneumoniae*O2 antigen comprises: a V_{H} comprising the amino acid sequence of SEQ ID NO: 19, or a variant thereof having at least about 80% (e.g., at least 80%, 85%, 90%, 95%, 96%, 97%, 98%, or 99%) sequence identity to the amino acid sequence of SEQ ID NO: 19; and a V_{L} comprising the amino acid sequence of SEQ ID NO: 24, or a variant thereof having at least about 80% (e.g., at least 80%, 85%, 90%, 95%, 96%, 97%, 98%, or 99%) sequence identity to the amino acid sequence of SEQ ID NO: 24.

In some embodiments, the antibody specifically binding to *K. pneumoniae*O2 antigen comprises: a V_{H} comprising the amino acid sequence of SEQ ID NO: 20, or a variant thereof having at least about 80% (e.g., at least 80%, 85%, 90%, 95%, 96%, 97%, 98%, or 99%) sequence identity to the amino acid sequence of SEQ ID NO: 20; and a V_{L} comprising the amino acid sequence of SEQ ID NO: 25, or a variant thereof having at least about 80% (e.g., at least 80%, 85%, 90%, 95%, 96%, 97%, 98%, or 99%) sequence identity to the amino acid sequence of SEQ ID NO: 25.

In some embodiments, the amino acid substitutions described herein are limited to "exemplary substitutions" shown in Table 10 of this application. In some embodiments, the amino acid substitutions are limited to "preferred substitutions" shown in Table 10 of this application.

In some embodiments, the application provides antibodies specifically binding to *K. pneumoniae,* which bind to *K. pneumoniae* with D-galactan I domain competitively with any one of the isolated antibodies described above. In some embodiments, the application provides antibodies, which binds to the same epitope as any one ofthe antibodies specifically binding to *K. pneumoniae* O2 antigen described above.

In some embodiments, competition assays may be used to identify a monoclonal antibody of *K. pneumoniae* that competes with the antibodies specifically binding to *K. pneumoniae* O2 antigen described herein for binding to D-galactan I domain. Competition assays can be used to determine whether two antibodies bind to the same epitope by recognizing identical or sterically overlapping epitopes or one antibody competitively inhibits binding of another antibody to the antigen. In certain embodiments, such a competing antibody binds to the same epitope that is bound by an antibody described herein. Exemplary competition assays include, but are not limited to, routine assays such as those provided in Harlow and Lane (1988) Antibodies: A Laboratory Manual ch. 14 (Cold Spring Harbor Laboratory, Cold Spring Harbor, N.Y.). Detailed exemplary methods for mapping an epitope to which an antibody binds are provided in Morris (1996) "Epitope Mapping Protocols", in Methods in Molecular Biology vol. 66 (Humana Press, Totowa, N.J.). In some embodiments, two antibodies are said to bind to the same epitope if each blocks binding of the other by 50% or more. In some embodiments, the antibodies that compete withthe antibodies specifically binding to *K. pneumoniae* O2 antigen described herein are chimeric, humanized or human antibodies.

Exemplary antibodies specifically binding to *K. pneumoniae* O2 antigen sequences are shown in Tables 2, wherein the CDR numbering is according to the Kabat index. Those skilled in the art will recognize that many algorithms (Kabat index) are known for prediction of CDR positions and for delimitation of antibody heavy chain and light chain variable domains. Antibodies specifically binding to *K. pneumoniae* O2 antigen comprising CDRs, V_{H} and/or V_{L} sequences from antibodies described herein, but based on prediction algorithms other than those exemplified in the tables below, are within the scope of this invention.

### Klebsiella

Most of the *Klebsiellainfections* are associated with hospitalization. As a conditional pathogen, *Klebsiella*mainlyattacks individuals with severe underlying diseases (such as diabetes or chronic pulmonary obstruction) and withlow immune function. With the lapse of time and the continuous development of taxonomies, the classification *ofKlebsiellais* continuously revised, and three main classifications are appeared: Cowan, Bascomb and Orskov. Wherein theØrskov classify *Klebsiella* into 5 categories, including *Klebsiella pneumoniae,Klebsiella oxytoca, Klebsiella terrestris, Klebsiella planticola and Klebsiella ornithine,* wherein the *Klebsiella pneumoniae* also includes *ozaenaeKlebsiella pneumoniae* and *rhinoscleromaKlebsiella pneumoniaesubspecies* (Podschun, R, and U Ullmann. Clinical microbiology reviews vol. 11,4 (1998): 589-603.). Furthermore, the *Klebsiella granuloma* and the like according to other classifications/nomenclature is also within the contemplation of the present application. In medicine, *Klebsiella pneumoniae* is the species that causes the most infections, and is also the most important species in *Klebsiella. Klebsiella pneumoniae*can cause, for example, sepsis, pneumonia, urinary tract infection, cartilage diseases, etc. (Podschun, R, and U Ullmann. Clinical microbiology reviews vol. 11,4 (1998): 589-603.).

### Lipopolysaccharide (LPS)

LPS (Lipopolysaccharide) is a component of the outermost layer present in the cell wall of gram-negative bacteriaconsisting of lipid A, core oligosaccharides and O antigen. O antigen, or O polysaccharide, is the outermost part oflipopolysaccharide, comprising varying numbers of oligosaccharide Repeat Units (RU). The unique O antigenstructure defines the LPS serotype of the *Klebsiella* strain. According to the variability of *Klebsiella* O antigen, there are currently9 major LPS serotypes: O1, O2, O2ac, O3, O4, O5, O7, O8, 012 (Hansen, D S et al. Journal of clinical microbiology vol. 37,1 (1999): 56-62.) and some subtypes of these serogroups (Kelly, R F, and C Whitfield. Journal of bacteriology vol. 178,17 (1996): 5205-14.). According to the published epidemiological data, O1 and O2 serotypepathogens account for 50-68% of all *Klebsiella* infections (Hansen, D S et al. Journal of clinical microbiology vol. 37,1 (1999): 56-62.; Follador, Rainer et al. Microbial genomics vol. 2,8 e000073. 25 Aug. 2016.). O1 and O2 serotype strains express LPS containing O polysaccharide built of homopolymers of galactose (galactans, gal). O1 serotypesexpressed D-galactan-I (D-gal-I)built of→3)-β-D-Galf -(1→3)-α-D-Galp-(1→ as repeat units and different antigenicity D-galactan-II (D-gal-II)built by →3)-α-D-Gal*p*-(1→3)-β-D-Gal*p*-(1→as repeat units (Whitfield, C et al. Journal of bacteriology vol. 173,4 (1991): 1420-31.; Kol, O et al. Carbohydrate research vol. 236 (1992): 339-44.). The D-gal-II is a unique structure of O1-type LPS (Pennini, Meghan E et al. Nature communications vol. 8,1 1991. 8 Dec. 2017). On the other hand, the O2-type LPS consists only consist of D-gal-I (Whitfield, C et al. Journal of bacteriology vol. 174,15 (1992): 4913-9.). For both serotypes, the synthesis of D-gal-I was encoded by the his-linked rfb (wb)operon (Clarke, B R, and C Whitfield. Journal of bacteriology vol. 174,14 (1992): 4614-21.; Kelly, RF, and C Whitfield. Journal of bacteriology vol. 178,17 (1996): 5205-14.). Furthermore, from the genetic perspective, the O1serotype strain carries an unlinked locus (wbbYZ) responsible for the synthesis of D-gal-II (Hsieh, Pei-Fang et al. Frontiers in microbiology vol. 5 608. 19 Nov. 2014.). Previous studies have shown that the D-gal-I of O2 serotype can bemodified by stoichiometric or non-stoichiometric with the addition of O-acetyl or terminal D-galactose (Kelly *et al*, 1995).Recent studies have revealed that the terminal α-D-Galp residue modified gal-I backbone repeat units, *i*.*e*.,→3)-β-D-Gal*f*-(1→3)-[α-D-Gal*p*-(1→4)]-α-D-Gal*p*-(1→), known as D-galactan-III (gal-III), frequently occur inO2 serogroups, and that the genetic background of such modifications has been established (Szijártó, Valéria et al. International journal of medical microbiology : IJMM vol. 306,2 (2016): 89-98.). The results indicate that theconversion of gal-I to gal-III is encoded by gmlABC, which is adjacent to the rfb (wb) operon encoding gal-I. Inaddition, studies have shown that about 40% of the clinical isolates of the O1 serotype carry the gmlABC gene(Szijártó, Valéria et al. International journal of medical microbiology : IJMM vol. 306,2 (2016): 89-98.), which suggests that D-gal-III is also expressed within the O1 serotype. Structural analysis of the extractedLPS or isolated O antigen revealed that D-gal-II and D-gal-III could be present simultaneously. Finally, D-gal-II can be combined with D-gal-I or D-gal-III homopolymers, resulting in two cases of O1 serotype described above. Then, the O2 serotype only consist of D-gal-I or D-gal-III homopolymers (Stojkovic, Katarina et al. Frontiers in microbiology vol. 8 684. 25 Apr. 2017.).

### Full-length antibodies specifically binding to K. pneumoniae O2 antigen

In some embodiments, the antibody specifically binding to *K. pneumoniae* O2 antigen is a full-length antibody. In some embodiments, the full-length antibody specifically binding to *K. pneumoniae* O2 antigen is an IgA, IgD, IgE, IgG, or IgM. In some embodiments, the antibody or antigen-binding fragment specifically binding to *K. pneumoniae* O2 antigen comprises antibody heavy chain constant region and antibody light chain constant region. In some embodiments, the full-length antibody specifically binding to *K. pneumoniae* O2 antigen comprises IgG constant regions, such as constant regions of any one of IgG1, IgG2, IgG3, and IgG4 including variants thereof. In some embodiments, the antibody or antigen-binding fragment specifically binding to *K. pneumoniae* O2 antigen comprises IgGlheavy chain constant region.In some embodiments, the antibody or antigen-binding fragment specifically binding to *K. pneumoniae* O2 antigen comprises IgG2 heavy chain constant region. In some embodiments, the antibody or antigen-binding fragment specifically binding to *K. pneumoniae* O2 antigen comprises IgG3 heavy chain constant region. In some embodiments, the antibody or antigen-binding fragment specifically binding to *K. pneumoniae* O2 antigen comprises IgG4 heavy chain constant region. In some embodiments, theIgG1refers to human IgG. In some embodiments, the heavy chain constant region comprises or consists of the amino acid sequence of SEQ ID NO: 55. In some embodiments, the heavy chain constant region comprises or consists of the amino acid sequence of SEQ ID NO: 56. In some embodiments, the antibody or antigen-binding fragment specifically binding to *K. pneumoniae* O2 antigen comprises a kappa light chain constant region. In some embodiments, the light chain constant region comprises or consists of the amino acid sequence of SEQ ID NO: 57.In some embodiments, the antibody or antigen-binding fragment specifically binding to *K. pneumoniae* O2 antigen comprises a lambda light chain constant region. In some embodiments, the light chain constant region comprises or consists of the amino acid sequence of SEQ ID NO: 58.In some embodiments, the antibody or antigen-binding fragment specifically binding to *K. pneumoniae* O2 antigen comprisesantibody heavy chain variable domain and antibody light chain variable domain.

In some embodiments, there is provided a full-length antibody specifically binding to *K. pneumoniae* O2 antigencomprising IgG1 constant regions, wherein the antibody specifically binding to *K. pneumoniae* O2 antigen comprises: a) a heavy chain variable domain, the heavy chain variable domaincomprises: an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 5; and b) a light chain variable domain, the light chain variable domain comprises an LC-CDR1 comprising the amino acid sequence of SEQ ID NO: 8, an LC-CDR2 comprising the amino acid sequence of SEQ ID NO: 12, and an LC-CDR3 comprising the amino acid sequence of SEQ ID NO: 15. In some embodiments, the IgG1 is human IgG1. In some embodiments, the heavy chain constant region comprises or consists of the amino acid sequence of SEQ ID NO: 55. In some embodiments, the light chain constant region comprises or consists of the amino acid sequence of SEQ ID NO: 57. In some embodiments, the light chain constant region comprises or consists of the amino acid sequence of SEQ ID NO: 58. In some embodiments, the heavy chain constant region comprises or consists of the amino acid sequence of SEQ ID NO: 55 and the light chain constant region comprises or consists of the amino acid sequence of SEQ ID NO: 57. In some embodiments, the heavy chain constant region comprises or consists of the amino acid sequence of SEQ ID NO: 55 and the light chain constant region comprises or consists of the amino acid sequence of SEQ ID NO: 58.

In some embodiments, there is provided a full-length antibody specifically binding to *K. pneumoniae* O2 antigencomprising IgG1 constant regions, wherein the antibody specifically binding to *K. pneumoniae* O2 antigen comprises: a) a heavy chain variable domain, the heavy chain variable domaincomprises: an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 6; and b) a light chain variable domain, the light chain variable domain comprises an LC-CDR1 comprising the amino acid sequence of SEQ ID NO: 9, an LC-CDR2 comprising the amino acid sequence of SEQ ID NO: 12, and an LC-CDR3 comprising the amino acid sequence of SEQ ID NO: 16. In some embodiments, the IgG1 is human IgG1. In some embodiments, the heavy chain constant region comprises or consists of the amino acid sequence of SEQ ID NO: 55. In some embodiments, the light chain constant region comprises or consists of the amino acid sequence of SEQ ID NO: 57. In some embodiments, the light chain constant region comprises or consists of the amino acid sequence of SEQ ID NO: 58. In some embodiments, the heavy chain constant region comprises or consists of the amino acid sequence of SEQ ID NO: 55 and the light chain constant region comprises or consists of the amino acid sequence of SEQ ID NO: 57. In some embodiments, the heavy chain constant region comprises or consists of the amino acid sequence of SEQ ID NO: 55 and the light chain constant region comprises or consists of the amino acid sequence of SEQ ID NO: 58.

In some embodiments, there is provided a full-length antibody specifically binding to *K. pneumoniae* O2 antigencomprising IgG1 constant regions, wherein the antibody specifically binding to *K. pneumoniae* O2 antigen comprises: a) a heavy chain variable domain, the heavy chain variable domaincomprises: an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and b) a light chain variable domain, the light chain variable domain comprises an LC-CDR1 comprising the amino acid sequence of SEQ ID NO: 10, an LC-CDR2 comprising the amino acid sequence of SEQ ID NO: 13, and an LC-CDR3 comprising the amino acid sequence of SEQ ID NO: 16. In some embodiments, the IgG1 is human IgG1. In some embodiments, the heavy chain constant region comprises or consists of the amino acid sequence of SEQ ID NO: 55. In some embodiments, the light chain constant region comprises or consists of the amino acid sequence of SEQ ID NO: 57. In some embodiments, the light chain constant region comprises or consists of the amino acid sequence of SEQ ID NO: 58. In some embodiments, the heavy chain constant region comprises or consists of the amino acid sequence of SEQ ID NO: 55 and the light chain constant region comprises or consists of the amino acid sequence of SEQ ID NO: 57. In some embodiments, the heavy chain constant region comprises or consists of the amino acid sequence of SEQ ID NO: 55 and the light chain constant region comprises or consists of the amino acid sequence of SEQ ID NO: 58.

In some embodiments, there is provided a full-length antibody specifically binding to *K. pneumoniae* O2 antigencomprising IgG1 constant regions, wherein the antibody specifically binding to *K. pneumoniae* O2 antigen comprises: a) a heavy chain variable domain, the heavy chain variable domaincomprises: an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and b) a light chain variable domain, the light chain variable domain comprises an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16. In some embodiments, the IgG1 is human IgG1. In some embodiments, the heavy chain constant region comprises or consists of the amino acid sequence of SEQ ID NO: 55. In some embodiments, the light chain constant region comprises or consists of the amino acid sequence of SEQ ID NO: 57. In some embodiments, the light chain constant region comprises or consists of the amino acid sequence of SEQ ID NO: 58. In some embodiments, the heavy chain constant region comprises or consists of the amino acid sequence of SEQ ID NO: 55 and the light chain constant region comprises or consists of the amino acid sequence of SEQ ID NO: 57. In some embodiments, the heavy chain constant region comprises or consists of the amino acid sequence of SEQ ID NO: 55 and the light chain constant region comprises or consists of the amino acid sequence of SEQ ID NO: 58.

In some embodiments, there is provided a full-length antibody specifically binding to *K. pneumoniae* O2 antigencomprising IgG1 constant regions, wherein the antibody specifically binding to *K. pneumoniae* O2 antigen comprises: a) a heavy chain variable domain, the heavy chain variable domaincomprises: an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7; and b) a light chain variable domain, the light chain variable domain comprises an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15. In some embodiments, the IgG1 is human IgG1. In some embodiments, the heavy chain constant region comprises or consists of the amino acid sequence of SEQ ID NO: 55. In some embodiments, the light chain constant region comprises or consists of the amino acid sequence of SEQ ID NO: 57. In some embodiments, the light chain constant region comprises or consists of the amino acid sequence of SEQ ID NO: 58. In some embodiments, the heavy chain constant region comprises or consists of the amino acid sequence of SEQ ID NO: 55 and the light chain constant region comprises or consists of the amino acid sequence of SEQ ID NO: 57. In some embodiments, the heavy chain constant region comprises or consists of the amino acid sequence of SEQ ID NO: 55 and the light chain constant region comprises or consists of the amino acid sequence of SEQ ID NO: 58.

In some embodiments, there is provided a full-length antibody specifically binding to *K. pneumoniae* O2 antigencomprising IgG4constant regions, wherein the antibody specifically binding to *K. pneumoniae* O2 antigen comprises: a) a heavy chain variable domain, the heavy chain variable domaincomprises: an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 5; and b) a light chain variable domain, the light chain variable domain comprises an LC-CDR1 comprising the amino acid sequence of SEQ ID NO: 8, an LC-CDR2 comprising the amino acid sequence of SEQ ID NO: 12, and an LC-CDR3 comprising the amino acid sequence of SEQ ID NO: 15. In some embodiments, the IgG4 is human IgG4. In some embodiments, the heavy chain constant region comprises or consists of the amino acid sequence of SEQ ID NO: 56. In some embodiments, the light chain constant region comprises or consists of the amino acid sequence of SEQ ID NO: 57. In some embodiments, the light chain constant region comprises or consists of the amino acid sequence of SEQ ID NO: 58. In some embodiments, the heavy chain constant region comprises or consists of the amino acid sequence of SEQ ID NO: 56 and the light chain constant region comprises or consists of the amino acid sequence of SEQ ID NO: 57. In some embodiments, the heavy chain constant region comprises or consists of the amino acid sequence of SEQ ID NO: 56 and the light chain constant region comprises or consists of the amino acid sequence of SEQ ID NO: 58.

In some embodiments, there is provided a full-length antibody specifically binding to *K. pneumoniae* O2 antigencomprising IgG4constant regions, wherein the antibody specifically binding to *K. pneumoniae* O2 antigen comprises: a) a heavy chain variable domain, the heavy chain variable domaincomprises: an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 6; and b) a light chain variable domain, the light chain variable domain comprises an LC-CDR1 comprising the amino acid sequence of SEQ ID NO: 9, an LC-CDR2 comprising the amino acid sequence of SEQ ID NO: 12, and an LC-CDR3 comprising the amino acid sequence of SEQ ID NO: 16. In some embodiments, the IgG4 is human IgG4. In some embodiments, the heavy chain constant region comprises or consists of the amino acid sequence of SEQ ID NO: 56. In some embodiments, the light chain constant region comprises or consists of the amino acid sequence of SEQ ID NO: 57. In some embodiments, the light chain constant region comprises or consists of the amino acid sequence of SEQ ID NO: 58. In some embodiments, the heavy chain constant region comprises or consists of the amino acid sequence of SEQ ID NO: 56 and the light chain constant region comprises or consists of the amino acid sequence of SEQ ID NO: 57. In some embodiments, the heavy chain constant region comprises or consists of the amino acid sequence of SEQ ID NO: 56 and the light chain constant region comprises or consists of the amino acid sequence of SEQ ID NO: 58.

In some embodiments, there is provided a full-length antibody specifically binding to *K. pneumoniae* O2 antigencomprising IgG4constant regions, wherein the antibody specifically binding to *K. pneumoniae* O2 antigen comprises: a) a heavy chain variable domain, the heavy chain variable domaincomprises: an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and b) a light chain variable domain, the light chain variable domain comprises an LC-CDR1 comprising the amino acid sequence of SEQ ID NO: 10, an LC-CDR2 comprising the amino acid sequence of SEQ ID NO: 13, and an LC-CDR3 comprising the amino acid sequence of SEQ ID NO: 16. In some embodiments, the IgG4 is human IgG4. In some embodiments, the heavy chain constant region comprises or consists of the amino acid sequence of SEQ ID NO: 56. In some embodiments, the light chain constant region comprises or consists of the amino acid sequence of SEQ ID NO: 57. In some embodiments, the light chain constant region comprises or consists of the amino acid sequence of SEQ ID NO: 58. In some embodiments, the heavy chain constant region comprises or consists of the amino acid sequence of SEQ ID NO: 56 and the light chain constant region comprises or consists of the amino acid sequence of SEQ ID NO: 57. In some embodiments, the heavy chain constant region comprises or consists of the amino acid sequence of SEQ ID NO: 56 and the light chain constant region comprises or consists of the amino acid sequence of SEQ ID NO: 58.

In some embodiments, there is provided a full-length antibody specifically binding to *K. pneumoniae* O2 antigencomprising IgG4constant regions, wherein the antibody specifically binding to *K. pneumoniae* O2 antigen comprises: a) a heavy chain variable domain, the heavy chain variable domaincomprises: an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and b) a light chain variable domain, the light chain variable domain comprises an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16. In some embodiments, the IgG4 is human IgG4. In some embodiments, the heavy chain constant region comprises or consists of the amino acid sequence of SEQ ID NO: 56. In some embodiments, the light chain constant region comprises or consists of the amino acid sequence of SEQ ID NO: 57. In some embodiments, the light chain constant region comprises or consists of the amino acid sequence of SEQ ID NO: 58. In some embodiments, the heavy chain constant region comprises or consists of the amino acid sequence of SEQ ID NO: 56 and the light chain constant region comprises or consists of the amino acid sequence of SEQ ID NO: 57. In some embodiments, the heavy chain constant region comprises or consists of the amino acid sequence of SEQ ID NO: 56 and the light chain constant region comprises or consists of the amino acid sequence of SEQ ID NO: 58.

In some embodiments, there is provided a full-length antibody specifically binding to *K. pneumoniae* O2 antigencomprising IgG4constant regions, wherein the antibody specifically binding to *K. pneumoniae* O2 antigen comprises: a) a heavy chain variable domain, the heavy chain variable domaincomprises: an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7; and b) a light chain variable domain, the light chain variable domain comprises an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15. In some embodiments, the IgG4 is human IgG4. In some embodiments, the heavy chain constant region comprises or consists of the amino acid sequence of SEQ ID NO: 56. In some embodiments, the light chain constant region comprises or consists of the amino acid sequence of SEQ ID NO: 57. In some embodiments, the light chain constant region comprises or consists of the amino acid sequence of SEQ ID NO: 58. In some embodiments, the heavy chain constant region comprises or consists of the amino acid sequence of SEQ ID NO: 56 and the light chain constant region comprises or consists of the amino acid sequence of SEQ ID NO: 57. In some embodiments, the heavy chain constant region comprises or consists of the amino acid sequence of SEQ ID NO: 56 and the light chain constant region comprises or consists of the amino acid sequence of SEQ ID NO: 58.

### Antibodies specifically binding toK. pneumoniae O1 antigen

In some embodiments, theantibody or antigen-binding fragment specifically binding to*K*. *pneumoniae* O1 antigenincludes, but is not limited to, humanized antibodies, chimeric antibodies, mouse antibodies, human antibodies, and antibodies comprising the heavy chain and/or light chain CDRs discussed herein. In one aspect, the antibodies or antigen-binding fragments are isolated antibodies that bind to O1 antigen. Contemplated antibodies or antigen-binding fragments specifically binding to *K. pneumoniae* O1 antigen can include the entire or a fragment of full-length antibodies specifically binding to *K. pneumoniae* O1 antigen (*e.g.*, full-length IgG1, IgG2 or IgG4), scFvsspecifically binding to *K. pneumoniae* O1 antigen, multi-specific (such as bispecific) antibodiesspecifically binding to *K. pneumoniae* O1 antigen, immunoconjugatesspecifically binding to *K. pneumoniae* O1 antigen, and the like. In some embodiments, the antibody or antigen-binding fragment specifically binding to *K. pneumoniae* O1 antigen is a Fab, a Fab', a F(ab)'2, a Fab'-SH, a single-chain Fv (scFv), an Fv fragment, a dAb, a Fd, a nanobody, or a diabody. In some embodiments, the antibody or antigen-binding fragment specifically binding to *K. pneumoniae* O1 antigen means that the antibody or antigen-binding fragment binds to *K. pneumoniae* O1 with an affinity that is at least about 10 times (including for example at least about any of 10, 10², 10³, 10⁴, 10⁵, 10⁶, or 10⁷times) of its binding affinity for non-target. In some embodiments, the non-target is an antigen that is not *K. pneumoniae* O1 antigen.

Binding affinity can be determined by methods known in the art, such as ELISA, fluorescence activated cell sorting (FACS) analysis, or radioimmunoprecipitation assay (RIA). Kd can be determined by methods known in the art, such assurface plasmon resonance (SPR) assay or biolayer interferometry (BLI).

In certain aspects, the antibody or antigen-binding fragment specifically binding to *K. pneumoniae* O1 antigen can (a) promote, mediate, or enhance the opsonophagocytic killing (OPK) of *K. pneumoniae,* and/or (b) promote, mediate, or enhance the serum bactericidal activity (SBA) of *K. pneumoniae.*

Although the antibodies or antigen-binding fragments specifically binding to *K. pneumoniae* O1 antigen and containing human sequences (*e.g.*, human heavy and light chain variable domain sequences comprising human CDR sequences) are extensively discussed herein, non-human antibodies are also contemplated. In some embodiments, non-human antibodies comprise human CDR sequences from an antibody or antigen-binding fragment described herein and non-human framework sequences. In some embodiments, non-human framework sequences include any sequence that can be used for generating synthetic heavy and/or light chain variable domains using one or more human CDR sequences as described herein, including, *e.g.*, mammals, *e.g.*, mouse, rat, rabbit, pig, bovine (*e.g.*, cow, bull, buffalo), deer, sheep, goat, chicken, cat, dog, ferret, primate (*e.g.*, marmoset, rhesus monkey), etc. In some embodiments, a non-human antibody or antigen-binding fragment includes an antibody or antigen-binding fragment generated by grafting one or more human CDR sequences as described herein onto a non-human framework sequence (*e.g.*, a mouse or chicken framework sequence).

In some embodiments, the antibody or antigen-binding fragment specifically binding to *K. pneumoniae*O1 antigendescribed herein binds specifically to one of the epitopes of *K. pneumoniae*O1 antigen. In some embodiments, the antibody or antigen-binding fragment specifically binding to *K. pneumoniae*O1 antigen is specific for *K. pneumoniae*O1 antigen and does not exhibit species cross-reactivity or other types of non-K. *pneumoniae* O1 antigencross-reactivity. In some embodiments, the antibody or antigen-binding fragment specifically binding to *K. pneumoniae*O1 antigen exhibits other types of non-*K*. *pneumoniae* O1 antigencross-reactivity.

In some embodiments, the antibody or antigen-binding fragment specifically binding to *K. pneumoniae* O1 antigen comprises antibody heavy chain constant region and antibody light chain constant region. In some embodiments, the antibody or antigen-binding fragment specifically binding to *K. pneumoniae* O1 antigen comprises IgG1heavy chain constant region. In some embodiments, the antibody or antigen-binding fragment specifically binding to *K. pneumoniae* O1 antigen comprises IgG2 heavy chain constant region. In some embodiments, the antibody or antigen-binding fragment specifically binding to *K. pneumoniae* O1 antigen comprises IgG3 heavy chain constant region. In some embodiments, the antibody or antigen-binding fragment specifically binding to *K. pneumoniae* O1 antigen comprises IgG4 heavy chain constant region. In some embodiments, theIgGrefers to human IgG. In some embodiments, the heavy chain constant region comprises or consists of the amino acid sequence of SEQ ID NO: 55. In some embodiments, the heavy chain constant region comprises or consists of the amino acid sequence of SEQ ID NO: 56. In some embodiments, the antibody or antigen-binding fragment specifically binding to *K. pneumoniae* O1 antigen comprises a kappa light chain constant region. In some embodiments, the light chain constant region comprises or consists of the amino acid sequence of SEQ ID NO: 57. In some embodiments, the light chain constant region comprises or consists of the amino acid sequence of SEQ ID NO: 58.In some embodiments, the antibody or antigen-binding fragment specifically binding to *K. pneumoniae* O1 antigen comprisesantibody heavy chain variable domain and antibody light chain variable domain.

In some embodiments, the antibody or antigen-binding fragment specifically binding to *K. pneumoniae* O1 antigencan be selected from the antibody specifically binding to *K. pneumoniae* O1 antigen described in Chinese patent application 202110980272.2.

### Bispecific antibodies specifically binding to K. pneumoniae O2 antigen and O1 antigen

In one aspect, the present application provides a bispecific antibody comprising a first antigen-binding domain specifically binding to *K. pneumoniae* O2 antigen, and a second antigen-binding domain specifically binding to *K. pneumoniae* O1 antigen.

In some embodiments, the first antigen-binding domain specifically binding to *K. pneumoniae* O2 antigencomprises: a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 5; and a V_{L} comprising an LC-CDR1 comprising the amino acid sequence of SEQ ID NO: 8; an LC-CDR2 comprising the amino acid sequence of SEQ ID NO: 12; and an LC-CDR3 comprising the amino acid sequence of SEQ ID NO: 15.

In some embodiments, the first antigen-binding domain specifically binding to *K. pneumoniae* O2 antigencomprises: a V_{H} comprising an HC-CDR1 comprising the amino acid sequence of SEQ ID NO: 1; an HC-CDR2 comprising the amino acid sequence of SEQ ID NO: 3; and an HC-CDR3 comprising the amino acid sequence of SEQ ID NO: 6; and a V_{L} comprising an LC-CDR1 comprising the amino acid sequence of SEQ ID NO: 9; an LC-CDR2 comprising the amino acid sequence of SEQ ID NO: 12; and an LC-CDR3 comprising the amino acid sequence of SEQ ID NO: 16.

In some embodiments, the first antigen-binding domain specifically binding to *K. pneumoniae* O2 antigencomprises: a V_{H} comprising an HC-CDR1 comprising the amino acid sequence of SEQ ID NO: 1; an HC-CDR2 comprising the amino acid sequence of SEQ ID NO: 4; and an HC-CDR3 comprising the amino acid sequence of SEQ ID NO: 6; and a V_{L} comprising an LC-CDR1 comprising the amino acid sequence of SEQ ID NO: 10; an LC-CDR2 comprising the amino acid sequence of SEQ ID NO: 13; and an LC-CDR3 comprising the amino acid sequence of SEQ ID NO: 16.

In some embodiments, the first antigen-binding domain specifically binding to *K. pneumoniae* O2 antigencomprises: a V_{H} comprising an HC-CDR1 comprising the amino acid sequence of SEQ ID NO: 1; an HC-CDR2 comprising the amino acid sequence of SEQ ID NO: 4; and an HC-CDR3 comprising the amino acid sequence of SEQ ID NO: 6; and a V_{L} comprising an LC-CDR1 comprising the amino acid sequence of SEQ ID NO: 11; an LC-CDR2 comprising the amino acid sequence of SEQ ID NO: 14; and an LC-CDR3 comprising the amino acid sequence of SEQ ID NO: 16.

In some embodiments, the first antigen-binding domain specifically binding to *K. pneumoniae* O2 antigencomprises: a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 7; and a V_{L} comprising an LC-CDR1 comprising the amino acid sequence of SEQ ID NO: 10; an LC-CDR2 comprising the amino acid sequence of SEQ ID NO: 12; and an LC-CDR3 comprising the amino acid sequence of SEQ ID NO: 15.

In some embodiments, the second antigen-binding domain specifically binding to *K. pneumoniae* O1 antigencomprises: a V_{H} comprising an HC-CDR1 comprising the amino acid sequence of SEQ ID NO: 35; an HC-CDR2 comprising the amino acid sequence of SEQ ID NO: 37; and an HC-CDR3 comprising the amino acid sequence of SEQ ID NO: 39; and a V_{L} comprising an LC-CDR1 comprising the amino acid sequence of SEQ ID NO: 41; an LC-CDR2 comprising the amino acid sequence of SEQ ID NO: 43; and an LC-CDR3 comprising the amino acid sequence of SEQ ID NO: 45.

In some embodiments, the second antigen-binding domain specifically binding to *K. pneumoniae* O1 antigencomprises: a V_{H} comprising an HC-CDR1 comprising the amino acid sequence of SEQ ID NO: 36; an HC-CDR2 comprising the amino acid sequence of SEQ ID NO: 38; and an HC-CDR3 comprising the amino acid sequence of SEQ ID NO: 40; and a V_{L} comprising an LC-CDR1 comprising the amino acid sequence of SEQ ID NO: 42; an LC-CDR2 comprising the amino acid sequence of SEQ ID NO: 44; and an LC-CDR3 comprising the amino acid sequence of SEQ ID NO: 46.

In some embodiments, the first antigen-binding domain specifically binding to *K. pneumoniae* O2 antigencomprises: a V_{H} comprising the amino acid sequence of SEQ ID NO: 17 or 26, or a variant thereof having at least about 80% (*e.g.*, at least 80%, 85%, 90%, 95%, 96%, 97%, 98%, or 99%) sequence identity to the amino acid sequence of SEQ ID NO: 17 or 26; and a V_{L} comprising the amino acid sequence of SEQ ID NO: 21 or 30, or a variant thereof having at least about 80% (*e.g.*, at least 80%, 85%, 90%, 95%, 96%, 97%, 98%, or 99%) sequence identity to the amino acid sequence of SEQ ID NO: 21 or 30; or a V_{H} comprisingan HC-CDR1, an HC-CDR2 and an HC-CDR3 of the V_{H} comprising the amino acid sequence of SEQ ID NO: 17 or 26; and a V_{L} comprising an LC-CDR1, an LC-CDR2, and an LC-CDR3 of the V_{L} comprising the amino acid sequence of SEQ ID NO: 21 or 30.

In some embodiments, the first antigen-binding domain specifically binding to *K. pneumoniae* O2 antigencomprises: a V_{H} comprising the amino acid sequence of SEQ ID NO: 18 or 27, or a variant thereof having at least about 80% (*e.g.*, at least 80%, 85%, 90%, 95%, 96%, 97%, 98%, or 99%) sequence identity to the amino acid sequence of SEQ ID NO: 18 or 27; and a V_{L} comprising the amino acid sequence of SEQ ID NO: 22 or 31, or a variant thereof having at least about 80% (*e.g.*, at least 80%, 85%, 90%, 95%, 96%, 97%, 98%, or 99%) sequence identity to the amino acid sequence of SEQ ID NO: 22 or 31; or a V_{H} comprisingan HC-CDR1, an HC-CDR2 and an HC-CDR3 of the V_{H} comprising the amino acid sequence of SEQ ID NO: 18 or 27; and a V_{L} comprising an LC-CDR1, an LC-CDR2, and an LC-CDR3 of the V_{L} comprising the amino acid sequence of SEQ ID NO: 22 or 31.

In some embodiments, the first antigen-binding domain specifically binding to *K. pneumoniae* O2 antigencomprises: a V_{H} comprising the amino acid sequence of SEQ ID NO: 19 or 28, or a variant thereof having at least about 80% (*e.g.*, at least 80%, 85%, 90%, 95%, 96%, 97%, 98%, or 99%) sequence identity to the amino acid sequence of SEQ ID NO: 19 or 28; and a V_{L} comprising the amino acid sequence of SEQ ID NO: 23 or 32, or a variant thereof having at least about 80% (*e.g.*, at least 80%, 85%, 90%, 95%, 96%, 97%, 98%, or 99%) sequence identity to the amino acid sequence of SEQ ID NO: 23 or 32; or a V_{H}comprisingan HC-CDR1, an HC-CDR2 and an HC-CDR3 of the V_{H} comprising the amino acid sequence of SEQ ID NO: 19 or 28; and a V_{L} comprising an LC-CDR1, an LC-CDR2, and an LC-CDR3 of the V_{L} comprising the amino acid sequence of SEQ ID NO: 23 or 32.

In some embodiments, the first antigen-binding domain specifically binding to *K. pneumoniae* O2 antigencomprises: a V_{H} comprising the amino acid sequence of SEQ ID NO: 19 or 28, or a variant thereof having at least about 80% (*e.g.*, at least 80%, 85%, 90%, 95%, 96%, 97%, 98%, or 99%) sequence identity to the amino acid sequence of SEQ ID NO: 19 or 28; and a V_{L} comprising the amino acid sequence of SEQ ID NO: 24 or 33, or a variant thereof having at least about 80% (*e.g.*, at least 80%, 85%, 90%, 95%, 96%, 97%, 98%, or 99%) sequence identity to the amino acid sequence of SEQ ID NO: 24 or 33; or a V_{H} comprisingan HC-CDR1, an HC-CDR2 and an HC-CDR3 of the V_{H} comprising the amino acid sequence of SEQ ID NO: 19 or 28; and a V_{L} comprising an LC-CDR1, an LC-CDR2, and an LC-CDR3 of the V_{L} comprising the amino acid sequence of SEQ ID NO: 24 or 33.

In some embodiments, the first antigen-binding domain specifically binding to *K. pneumoniae* O2 antigencomprises: a V_{H} comprising the amino acid sequence of SEQ ID NO: 20 or 29, or a variant thereof having at least about 80% (*e.g.*, at least 80%, 85%, 90%, 95%, 96%, 97%, 98%, or 99%) sequence identity to the amino acid sequence of SEQ ID NO: 20 or 29; and a V_{L} comprising the amino acid sequence of SEQ ID NO: 25 or 34, or a variant thereof having at least about 80% (*e.g.*, at least 80%, 85%, 90%, 95%, 96%, 97%, 98%, or 99%) sequence identity to the amino acid sequence of SEQ ID NO: 25 or 34; or a V_{H} comprisingan HC-CDR1, an HC-CDR2 and an HC-CDR3 of the V_{H} comprising the amino acid sequence of SEQ ID NO: 20 or 29; and a V_{L} comprising an LC-CDR1, an LC-CDR2, and an LC-CDR3 of the V_{L} comprising the amino acid sequence of SEQ ID NO: 25 or 34.

In some embodiments, the second antigen-binding domain specifically binding to *K. pneumoniae* O1 antigencomprises: a V_{H} comprising the amino acid sequence of SEQ ID NO: 47 or 51, or a variant thereof having at least about 80% (*e.g.*, at least 80%, 85%, 90%, 95%, 96%, 97%, 98%, or 99%) sequence identity to the amino acid sequence of SEQ ID NO: 47 or 51; and a V_{L} comprising the amino acid sequence of SEQ ID NO: 49 or 53, or a variant thereof having at least about 80% (*e.g.*, at least 80%, 85%, 90%, 95%, 96%, 97%, 98%, or 99%) sequence identity to the amino acid sequence of SEQ ID NO: 49 or 53; or a V_{H} comprisingan HC-CDR1, an HC-CDR2 and an HC-CDR3 of the V_{H} comprising the amino acid sequence of SEQ ID NO: 47 or 51; and a V_{L} comprising an LC-CDR1, an LC-CDR2, and an LC-CDR3 of the V_{L} comprising the amino acid sequence of SEQ ID NO: 49 or 53.

In some embodiments, the second antigen-binding domain specifically binding to *K. pneumoniae* O1 antigencomprises: a V_{H} comprising the amino acid sequence of SEQ ID NO: 48 or 52, or a variant thereof having at least about 80% (*e.g.*, at least 80%, 85%, 90%, 95%, 96%, 97%, 98%, or 99%) sequence identity to the amino acid sequence of SEQ ID NO: 48 or 52; and a V_{L} comprising the amino acid sequence of SEQ ID NO: 50 or 54, or a variant thereof having at least about 80% (*e.g.*, at least 80%, 85%, 90%, 95%, 96%, 97%, 98%, or 99%) sequence identity to the amino acid sequence of SEQ ID NO: 50 or 54; or a V_{H} comprisingan HC-CDR1, an HC-CDR2 and an HC-CDR3 of the V_{H} comprising the amino acid sequence of SEQ ID NO: 48 or 52; and a V_{L} comprising an LC-CDR1, an LC-CDR2, and an LC-CDR3 of the V_{L} comprising the amino acid sequence of SEQ ID NO: 50 or 54.

In some embodiments, the present application provides a bispecific antibody comprising a first antigen-binding domainspecifically binding to *K. pneumoniae* O2 antigen, and a second antigen-binding domain specifically binding to *K. pneumoniae* O1 antigen, wherein the first antigen-binding domain comprises:a V_{H} comprising an HC-CDR1 comprising the amino acid sequence SEQ ID NO: 1, an HC-CDR2 comprising the amino acid sequence of SEQ ID NO: 4, and an HC-CDR3 comprising the amino acid sequence of SEQ ID NO: 6; and a V_{L} comprising: an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16;and wherein the second antigen-binding domain comprises:a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising: an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45.

In some embodiments, the present application provides a bispecific antibody comprising a first antigen-binding domain specifically binding to *K. pneumoniae* O2 antigen, and a second antigen-binding domain specifically binding to *K. pneumoniae* O1 antigen, wherein the first antigen-binding domain comprises:a V_{H} comprising an HC-CDR1 comprising the amino acid sequence SEQ ID NO: 1, an HC-CDR2 comprising the amino acid sequence of SEQ ID NO: 4, and an HC-CDR3 comprising the amino acid sequence of SEQ ID NO: 6; and a V_{L} comprising: an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16;and wherein the second antigen-binding domain comprises:a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising: an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

In some embodiments, the present application provides a bispecific antibody comprising a first antigen-binding domain specifically binding to *K. pneumoniae* O2 antigen, and a second antigen-binding domain specifically binding to *K. pneumoniae* O1 antigen, wherein the first antigen-binding domain comprises:a V_{H} comprising an HC-CDR1 comprising the amino acid sequence SEQ ID NO: 1, an HC-CDR2 comprising the amino acid sequence of SEQ ID NO: 2, and an HC-CDR3 comprising the amino acid sequence of SEQ ID NO: 7; and a V_{L} comprising: an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15;and wherein the second antigen-binding domain comprises:a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising: an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45.

In some embodiments, the present application provides a bispecific antibody comprising a first antigen-binding domain specifically binding to *K. pneumoniae* O2 antigen, and a second antigen-binding domain specifically binding to *K. pneumoniae* O1 antigen, wherein the first antigen-binding domain comprises:a V_{H} comprising an HC-CDR1 comprising the amino acid sequence SEQ ID NO: 1, an HC-CDR2 comprising the amino acid sequence of SEQ ID NO: 2, and an HC-CDR3 comprising the amino acid sequence of SEQ ID NO: 7; and a V_{L} comprising: an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15;and wherein the second antigen-binding domain comprises:a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising: an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

In some embodiments, the present application provides a bispecific antibody comprising a first antigen-binding domain specifically binding to *K. pneumoniae* O2 antigen, and a second antigen-binding domain specifically binding to *K. pneumoniae* O1 antigen, wherein the first antigen-binding domain comprises:a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; and wherein the second antigen-binding domain comprises:a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53.

In some embodiments, the present application provides a bispecific antibody comprising a first antigen-binding domain specifically binding to *K. pneumoniae* O2 antigen, and a second antigen-binding domain specifically binding to *K. pneumoniae* O1 antigen, wherein the first antigen-binding domain comprises:a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; and wherein the second antigen-binding domain comprises:a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54.

In some embodiments, the present application provides a bispecific antibody comprising a first antigen-binding domain specifically binding to *K. pneumoniae* O2 antigen, and a second antigen-binding domain specifically binding to *K. pneumoniae* O1 antigen, wherein the first antigen-binding domain comprises:a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; and wherein the second antigen-binding domain comprises:a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53.

In some embodiments, the present application provides a bispecific antibody comprising a first antigen-binding domain specifically binding to *K. pneumoniae* O2 antigen, and a second antigen-binding domain specifically binding to *K. pneumoniae* O1 antigen, wherein the first antigen-binding domain comprises:a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; and wherein the second antigen-binding domain comprises:a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54.

### DVD-Ig format Bispecific antibodies

In some embodiments, any one of the bispecific antibodies described herein has a dual variable region immunoglobulin molecule (DVD-Ig) format, connecting the V_{L} and V_{H} domains of another antibody respectivelyto the N-terminus of the V_{L} and V_{H} of a normal IgG antibody, formingan antigen-binding domain by the interaction between V_{H} and V_{L} of two antibodies, which can simultaneously bind to the corresponding antigen to achieve bispecific binding.In some embodiments, the DVD-IgG formatis a homodimeric structure, consisting of twoidentical monomers, each monomer comprising two antigen binding domains, one of which is Fv and the other ofwhich is Fab.The two domains described above are connected in tandemthrough a peptide linker (L). In some embodiments, theDVD-Ig formatfurther comprises an Fc. An exemplary schematic diagram of DVD-Ig formatis shown in FIG. 7A.

In one embodiment of the application, one of the antigen-binding domains specifically binds to *K. pneumoniae* O1 antigen and the other antigen-binding domain specifically binds to *K. pneumoniae* O2antigen. In some embodiments, the bispecific antibody can bind to *K. pneumoniae* O2 antigen and*K*. *pneumoniae* O1 antigen simultaneously.

In some embodiments, the bispecific antibody consists of two identical monomers, each of which comprises two polypeptide chains, a heavy chain and a light chain, for a total of four polypeptide chains. Wherein, the heavy chain comprises V_{H}1-L-V_{H}2-C_{H}1from N- terminus to C-terminus, the light chain comprises V_{L}1-L-V_{L}2-C_{L}from N- terminus to C-terminus. In some embodiments, the heavy chain further comprises an Fccomprising C_{H}2 and C_{H}3 domains. In other embodiments,the heavy chain comprises V_{H}1-L-V_{H}2-C_{H}1-C_{H}2-C_{H}3from N- terminus to C-terminus, the light chain comprises V_{L}1-L-V_{L}2-C_{L}from N- terminus to C-terminus. Wherein the V_{H}1 and V_{L}1are the heavy chain variable domain and light chain variable domain that specifically bind to one of the antigens, respectively; the VH₂ and VL₂ are the heavy chain variable domain and light chain variable domain that specifically bind to another antigen, respectively; L is apeptide linker; C_{H}1 is a heavy chain constantdomain 1; C_{L}is a light chain constant domain. Wherein, the V_{H}1 and V_{L}1 form one of the antigen-binding domains (Fv) of the bispecific antibody; the V_{H}2-C_{H}1 and V_{L}2-C_{L}form the other antigen-binding domain (Fab) of the bispecific antibody.

In some embodiments, the V_{H}1 and V_{L}1are the heavy chain variable domain and light chain variable domain that specifically bind to *K. pneumoniae* O1 antigen, respectively, *i.e.,* the V_{H}1 and V_{L}1 form the antigen-binding domain (Fv) specifically binding to *K. pneumoniae* O1 antigen; the VH₂ and V_{L}2 are the heavy chain variable domain and light chain variable domain that specifically bind to *K. pneumoniae* O2 antigen, respectively, *i.e.,* the V_{H}2-C_{H}1 and V_{L}2-C_{L} form the antigen-binding domain (Fab) specifically binding to *K. pneumoniae* O2 antigen. In some embodiments, the V_{H}1 and V_{L}1are the heavy chain variable domain and light chain variable domain that specifically bind to *K. pneumoniae* O2 antigen, respectively, *i.e.,* the V_{H}1 and V_{L}1 form the antigen-binding domain (Fv) specifically binding to *K. pneumoniae* O2 antigen; the V_{H2} and V_{L}2 are the heavy chain variable domain and light chain variable domain that specifically bind to *K. pneumoniae* O1 antigen, respectively, *i.e.,* the V_{H}2-C_{H}1 and V_{L}2-C_{L} form the antigen-binding domain (Fab) specifically binding to *K. pneumoniae* O1 antigen.

Peptide linker (or linker) sequence can be single amino acid or polypeptide sequence. In some embodiments, thepeptide linker (or linker) comprises or consists of a Gly-Ser linker.As described herein, the term "Gly-Ser linker" refers to a peptide that consists of glycine and serine residues. An exemplary Gly-Ser linker comprises an amino acid sequence of the formula (Gly₄Ser)ₙ, wherein n is a positive integer (e.g., 1, 2, 3, 4, 5, 6, 7, 8, 9 or 10). A preferred Gly-Ser linker is (Gly₄Ser)₂, *i*.*e*.,GGGGSGGGGS(SEQ ID NO: 102), and (Gly₄Ser)₄,*i*.*e*.,GGGGSGGGGSGGGGSGGGGS(SEQ ID NO: 103).Another preferred Gly-Ser linker is (Gly₄Ser)₃,*i*.*e*.,, GGGGSGGGGSGGGGS (SEQ ID NO: 104). In other aspects, two or more Gly-Ser linker are incorporated in series in a peptide linker. In some aspects, the peptide linker comprises at least a portion of a hinge region (e.g., derived from an IgGl, IgG2, IgG3, or IgG4 molecule) and a series of Gly- Ser amino acid residues (e.g., a Gly-Ser linker such as (Gly₄Ser)ₙ).In some embodiments, the peptide linker can also be selected to comprise amino acid sequence ASTKGP (SEQ ID NO: 105) or amino acid sequence TVAAP(SEQ ID NO: 106).

In some embodiments, any one of the bispecific antibodies described herein comprises theamino acid sequence of SEQ ID NO: 61, or a variant thereof having at least about 80% *(e.g.,* at least 80%, 85%, 90%, 95%, 96%, 97%, 98%, or 99%) sequence identity to the amino acid sequenceof SEQ ID NO: 61.

In some embodiments, any one of the bispecific antibodies described herein comprises theamino acid sequence of SEQ ID NO: 62, or a variant thereof having at least about 80% *(e.g.,* at least 80%, 85%, 90%, 95%, 96%, 97%, 98%, or 99%) sequence identity to the amino acid sequenceof SEQ ID NO: 62.

In some embodiments, any one of the bispecific antibodies described herein comprises theamino acid sequence of SEQ ID NO: 63, or a variant thereof having at least about 80% *(e.g.,* at least 80%, 85%, 90%, 95%, 96%, 97%, 98%, or 99%) sequence identity to the amino acid sequenceof SEQ ID NO: 63.

In some embodiments, any one of the bispecific antibodies described herein comprises theamino acid sequence of SEQ ID NO: 64, or a variant thereof having at least about 80% *(e.g.,* at least 80%, 85%, 90%, 95%, 96%, 97%, 98%, or 99%) sequence identity to the amino acid sequenceof SEQ ID NO: 64.

In some embodiments, any one of the bispecific antibodies described herein comprises theamino acid sequence of SEQ ID NO: 65, or a variant thereof having at least about 80% *(e.g.,* at least 80%, 85%, 90%, 95%, 96%, 97%, 98%, or 99%) sequence identity to the amino acid sequenceof SEQ ID NO: 65.

In some embodiments, any one of the bispecific antibodies described herein comprises theamino acid sequence of SEQ ID NO: 66, or a variant thereof having at least about 80% *(e.g.,* at least 80%, 85%, 90%, 95%, 96%, 97%, 98%, or 99%) sequence identity to the amino acid sequenceof SEQ ID NO: 66.

In some embodiments, any one of the bispecific antibodies described herein comprises theamino acid sequence of SEQ ID NO: 67, or a variant thereof having at least about 80% *(e.g.,* at least 80%, 85%, 90%, 95%, 96%, 97%, 98%, or 99%) sequence identity to the amino acid sequenceof SEQ ID NO: 67.

In some embodiments, any one of the bispecific antibodies described herein comprises theamino acid sequence of SEQ ID NO: 68, or a variant thereof having at least about 80% *(e.g.,* at least 80%, 85%, 90%, 95%, 96%, 97%, 98%, or 99%) sequence identity to the amino acid sequenceof SEQ ID NO: 68.

In some embodiments, any one of the bispecific antibodies described herein comprises theamino acid sequence of SEQ ID NO: 61, or a variant thereof having at least about 80% *(e.g.,* at least 80%, 85%, 90%, 95%, 96%, 97%, 98%, or 99%) sequence identity to the amino acid sequenceof SEQ ID NO: 61; and theamino acid sequence of SEQ ID NO: 62, or a variant thereof having at least about 80% *(e.g.,* at least 80%, 85%, 90%, 95%, 96%, 97%, 98%, or 99%) sequence identity to the amino acid sequenceof SEQ ID NO: 62.

In some embodiments, any one of the bispecific antibodies described herein comprises theamino acid sequence of SEQ ID NO: 63, or a variant thereof having at least about 80% *(e.g.,* at least 80%, 85%, 90%, 95%, 96%, 97%, 98%, or 99%) sequence identity to the amino acid sequenceof SEQ ID NO: 63; and theamino acid sequence of SEQ ID NO: 64, or a variant thereof having at least about 80% *(e.g.,* at least 80%, 85%, 90%, 95%, 96%, 97%, 98%, or 99%) sequence identity to the amino acid sequenceof SEQ ID NO: 64.

In some embodiments, any one of the bispecific antibodies described herein comprises the amino acid sequence of SEQ ID NO: 65, or a variant thereof having at least about 80% (*e.g.*, at least 80%, 85%, 90%, 95%, 96%, 97%, 98%, or 99%) sequence identity to the amino acid sequenceof SEQ ID NO: 65; and theamino acid sequence of SEQ ID NO: 66, or a variant thereof having at least about 80% *(e.g.,* at least 80%, 85%, 90%, 95%, 96%, 97%, 98%, or 99%) sequence identity to the amino acid sequenceof SEQ ID NO: 66.

In some embodiments, any one of the bispecific antibodies described herein comprises theamino acid sequence of SEQ ID NO: 67, or a variant thereof having at least about 80% (*e.g.*, at least 80%, 85%, 90%, 95%, 96%, 97%, 98%, or 99%) sequence identity to the amino acid sequenceof SEQ ID NO: 67; and the amino acid sequence of SEQ ID NO: 68, or a variant thereof having at least about 80% (*e.g.*, at least 80%, 85%, 90%, 95%, 96%, 97%, 98%, or 99%) sequence identity to the amino acid sequenceof SEQ ID NO: 68.

In some embodiments, any one of the bispecific antibodies described herein comprises theamino acid sequence of SEQ ID NO: 86, or a variant thereof having at least about 80% (*e.g.*, at least 80%, 85%, 90%, 95%, 96%, 97%, 98%, or 99%) sequence identity to the amino acid sequenceof SEQ ID NO: 86; and theamino acid sequence of SEQ ID NO: 62, or a variant thereof having at least about 80% (*e.g.*, at least 80%, 85%, 90%, 95%, 96%, 97%, 98%, or 99%) sequence identity to the amino acid sequenceof SEQ ID NO: 62.

In some embodiments, any one of the bispecific antibodies described herein comprises theamino acid sequence of SEQ ID NO: 87, or a variant thereof having at least about 80% (*e.g.*, at least 80%, 85%, 90%, 95%, 96%, 97%, 98%, or 99%) sequence identity to the amino acid sequenceof SEQ ID NO: 87; and theamino acid sequence of SEQ ID NO: 64, or a variant thereof having at least about 80% (*e.g.*, at least 80%, 85%, 90%, 95%, 96%, 97%, 98%, or 99%) sequence identity to the amino acid sequenceof SEQ ID NO: 64.

In some embodiments, any one of the bispecific antibodies described herein comprises theamino acid sequence of SEQ ID NO: 88, or a variant thereof having at least about 80% (*e.g.*, at least 80%, 85%, 90%, 95%, 96%, 97%, 98%, or 99%) sequence identity to the amino acid sequenceof SEQ ID NO: 88; and the amino acid sequence of SEQ ID NO: 66, or a variant thereof having at least about 80% (*e.g.*, at least 80%, 85%, 90%, 95%, 96%, 97%, 98%, or 99%) sequence identity to the amino acid sequenceof SEQ ID NO: 66.

In some embodiments, any one of the bispecific antibodies described herein comprises theamino acid sequence of SEQ ID NO: 89, or a variant thereof having at least about 80% *(e.g.,* at least 80%, 85%, 90%, 95%, 96%, 97%, 98%, or 99%) sequence identity to the amino acid sequence ofSEQ ID NO: 89; and theamino acid sequence of SEQ ID NO: 68, or a variant thereof having at least about 80% (*e.g.*, at least 80%, 85%, 90%, 95%, 96%, 97%, 98%, or 99%) sequence identity to the amino acid sequenceof SEQ ID NO: 68.

In some embodiments, any one of the bispecific antibodies described herein comprises theamino acid sequence of SEQ ID NO: 115, or a variant thereof having at least about 80% *(e.g.,* at least 80%, 85%, 90%, 95%, 96%, 97%, 98%, or 99%) sequence identity to the amino acid sequenceof SEQ ID NO: 115; and theamino acid sequence of SEQ ID NO: 66, or a variant thereof having at least about 80% *(e.g.,* at least 80%, 85%, 90%, 95%, 96%, 97%, 98%, or 99%) sequence identity to the amino acid sequenceof SEQ ID NO: 66.

### Bs4Abformat Bispecific antibodies

In some embodiments, the bispecific antibody described herein has Bs4Abformat, which consists of twoidentical monomers, each monomer comprising two antigen-binding domains, one of which is Fab and the other ofwhich is scFv. In some embodiments, theBs4Abformat bispecific antibodyfurther comprises an Fccomprising C_{H}2 and C_{H}3 domains. The scFv connects to the Fab through the first peptide linker (L1) and the Fc through the second peptide linker (L2). An exemplary schematic diagram ofBs4Abformatis shown in FIG. 7B.

In some embodiments of the application, one of the antigen-binding domains (Fab or scFv) specifically binds to *K. pneumoniae* O1 antigen and the other antigen-binding domain (Fab or scFv) specifically binds to *K. pneumoniae* O2antigen. In some embodiments, the bispecific antibody can bind to *K. pneumoniae* O2 antigen and*K*. *pneumoniae* O1 antigen simultaneously.

In some embodiments, the bispecific antibody described herein consists of two identical monomers, each of which comprises two polypeptide chains, a heavy chain and a light chain, for a total of four polypeptide chains. In some embodiments, the heavy chain of the bispecific antibodycomprises V_{H}1-C_{H}1-L1-V_{H}2-L3-V_{L}2 structure from N- terminus to C-terminus. In other embodiments, the heavy chain of the bispecific antibodycomprises V_{H}1-C_{H}1-L1-V_{L}2-L3-V_{H}2 structure from N- terminus to C-terminus. In some embodiments, the heavy chain further comprises an Fccomprising C_{H}2 and C_{H}3 domains. In some embodiments, the heavy chain of the bispecific antibodycomprises V_{H}1-C_{H}1-L1-V_{H}2-L3-V_{L}2-L2-C_{H}2-C_{H}3 structure from N- terminus to C-terminus. In other embodiments, the heavy chain of the bispecific antibodycomprises V_{H}1-C_{H}1-L1-V_{L}2-L3-V_{H}2-L2-C_{H}2-C_{H}3 structure from N- terminus to C-terminus. In some embodiments, the light chain of the bispecific antibodycomprises V_{L}1-C_{L} structure from N-terminus to C-terminus. Wherein, the V_{H}1 and V_{L}1are the heavy chain variable domain and light chain variable domain that specifically bind to oneof the antigens, respectively; the VH₂ and VL₂ are the heavy chain variable domain and light chain variable domain that specifically bind to the other antigen, respectively; C_{H}1 is a heavy chain constant domain 1; C_{L}is a light chain constant domain; L1, L2 and L3arepeptide linkers. The V_{H}1-C_{H}1 and V_{L}l-C_{L}form one of the antigen-binding domains (Fab) of the bispecific antibody; the V_{H}2-L3-V_{L}2 or V_{L}2-L3-V_{H}2form the other antigen-binding domain (scFv) of the bispecific antibody.

In some embodiments, the V_{H}1 and V_{L}1are the heavy chain variable domain and light chain variable domain that specifically bind to *K. pneumoniae* O1 antigen, respectively, *i.e.,* the V_{H}1-C_{H}1 and V_{L}1-C_{L} form the antigen-binding domain (Fab) specifically binding to *K. pneumoniae* O1 antigen; the V_{H}2 and V_{L}2 are the heavy chain variable domain and light chain variable domain that specifically bind to *K. pneumoniae* O2 antigen, respectively, *i.e.,* the V_{H}2-L3-V_{L}2 or V_{L}2-L3-V_{H}2 form the antigen-binding domain (scFv) specifically binding to *K. pneumoniae* O2 antigen. In other embodiments, the V_{H}1 and V_{L}1are the heavy chain variable domain and light chain variable domain that specifically bind to *K. pneumoniae* O2 antigen, respectively, *i.e.,* the V_{H}1-C_{H}1 and V_{L}1-C_{L} form the antigen-binding domain (Fab) specifically binding to *K. pneumoniae* O2 antigen; the V_{H}2 and V_{L}2 are the heavy chain variable domain and light chain variable domain that specifically bind to *K. pneumoniae* O1 antigen, respectively, *i.e.,* the V_{H}2-L3-V_{L}2 or V_{L}2-L3-V_{H}2 form the antigen-binding domain (scFv) specifically binding to *K. pneumoniae* O1 antigen. In some embodiments, the bispecific antibodycomprisesC_{H}1comprising the amino acid sequence of SEQ ID No: 60. In some embodiments, the bispecific antibodycomprisesC_{H}2-C_{H}3comprising theamino acid sequence of SEQ ID No: 59.

In some embodiments, according to any one of the bispecific antibodiesdescribed herein, the antigen-binding domain scFv specifically binding to *K. pneumoniae* O2 antigen or *K. pneumoniae* O1 antigen comprises genetic engineering cysteine mutations. Bispecific antibodies with disulfide bond stability are obtained through introducing two cysteine mutations at the V_{H} and V_{L} interfaces.

Peptide linker (or linker) may be used to join domains and/or regions of the chimeric heavy chain of the bispecific antibody into a contiguous molecule. In some embodiments, the bispecific antibody includes at least two peptide linkers, L1 and L2. In some embodiments, the bispecific antibody may include additional linkers, such as a flexible linker interconnecting the variable heavy and light chains of an scFv. In some embodiments, the bispecific antibody may include additional linkers, such as a flexible linker interconnecting the variable heavy and light chains of an scFv and other linkers that connect other binding units to the core structure of the bispecific antibody.

An exemplary, non-limiting example of a linker is a polypeptide chain comprising at least 4 residues. Portions of such linkers may be flexible, hydrophilic and have little or no secondary structure of their own (linker portions or flexible linker portions). Linkers of at least 4 amino acids may be used to join domains and/or regions that are positioned near to one another after the molecule has assembled. Longer linkers may also be used. In some embodiments, linkers may be about any one of: 1, 2, 3, 4, 5, 6, 7, 8, 9, 10, 11, 12, 13, 14, 15, 16, 17, 18, 19, 20, 25, 30, 35, 40, 45, 50, 100, 125, 150, 175 or 200 residues. When multiple linkers are used to interconnect portions of the molecule, the linkers may be the same or different (e.g., the same or different length and/or amino acid sequence).

In some aspects, thepeptide linker comprises or consists of a Gly-Ser linker.As described herein, the term "Gly-Ser linker" refers to a peptide that consists of glycine and serine residues. An exemplary Gly-Ser linker comprises an amino acid sequence of the formula (Gly₄Ser)ₙ, wherein n is a positive integer (e.g., 1, 2, 3, 4, 5, 6, 7, 8, 9 or 10). A preferred Gly-Ser linker is (Gly₄Ser)₂, i.e.,GGGGSGGGGS(SEQ ID NO: 102), and (Gly₄Ser)₄,*i*.*e*.,GGGGSGGGGSGGGGSGGGGS(SEQ ID NO: 103).Another preferred Gly-Ser linker is (Gly₄Ser)₃,*i*.*e*.,, GGGGSGGGGSGGGGS (SEQ ID NO: 104). In other aspects, two or more Gly-Ser linker are incorporated in series in a peptide linker. In some aspects, the peptide linker comprises at least a portion of a hinge region (e.g., derived from an IgGl, IgG2, IgG3, or IgG4 molecule) and a series of Gly- Ser amino acid residues (e.g., a Gly-Ser linker such as (Gly₄Ser)ₙ).

In some embodiments, L1 and/or L2 include both a hinge portion and a linker portion, such as a linker portion comprising a Gly-Ser linker. In other aspects, L1 and/or L2 include only a hinge portion or only a linker portion, such as a Gly-Ser linker. In some embodiments, L1 and L2 include a Gly-Ser linker portion. In certain aspects, the Gly-Ser linker portion of L1 and L2 is the same length, whereas in other aspects, the Gly-Ser linker portion of LI and L2 are different lengths. When a bispecific molecule comprises an scFv, the heavy and light chains of the scFv may be connected by a flexible linker. In some embodiments, this flexible linker generally does not include a hinge portion, but rather, is a Gly-Ser linker or other flexible linker. The length and amino acid sequence of a flexible linker interconnecting domains of an scFv may be selected and optimized.

In some embodiments, the peptide linker (for example L1 and/or L2) comprises a Gly-Ser or all Gly linker and a portion or modified portion of a hinge domain. In some aspects, the peptide linker (L1) connecting one of the antigen-binding domains (e.g. the Fab or scFv) to the other antigen-binding domain (e.g. scFv or Fab) of the bispecific antibody comprises the amino acid sequence EPKSDKTGGGGSGGGGS (SEQ ID NO: 107) or EPKSCGKTGGGGSGGGGS (SEQ ID NO: 108) or EPKSCGGGGSGGGGS (SEQ ID NO: 109). In some aspects the peptide linker (L2) connecting the antigen-binding domainscFvto the Fc domain of the bispecific antibody comprises the amino acid sequence GGGGSGGGGSEPKSDKTHTCPPCP (SEQ ID NO: 110) or GGGGSGGGGSCPPCP (SEQ ID NO: 111) or GGGGSGGGGSDKTHTCPPCP (SEQ ID NO: 112).

In some embodiments, regardless of the peptide linker used to interconnect one antigen-binding domain to the other antigen-binding domainorone of the antigen-binding domains to Fc (e.g., L1 and L2), the bispecific antibody may optionally comprise additional peptide linkers. The lengths and sequence of such additional peptide linkers are independently selected. For example, the bispecific antibody may further comprise a flexible peptide linker (L3) interconnecting the variable heavy and light chains of a scFv (V_{HSCFV} and V_{LSCFV}). This flexible peptide linker may comprise a Gly-Ser linker. Generally, this linker does not include a hinge portion. In some embodiments, this flexible peptide linker (L3) interconnecting the variable heavy and light chains of the scFv comprises the sequence of GGGGSGGGGSGGGGSGGGGS (SEQ ID NO: 103).

In some embodiments, any one of the bispecific antibodies described herein comprises the amino acid sequence of SEQ ID NO: 69, or a variant thereof having at least about 80% *(e.g.,* at least 80%, 85%, 90%, 95%, 96%, 97%, 98%, or 99%) sequence identity to the amino acid sequenceof SEQ ID NO: 69.

In some embodiments, any one of the bispecific antibodies described herein comprises theamino acid sequence of SEQ ID NO: 70, or a variant thereof having at least about 80% *(e.g.,* at least 80%, 85%, 90%, 95%, 96%, 97%, 98%, or 99%) sequence identity to the amino acid sequenceof SEQ ID NO: 70.

In some embodiments, any one of the bispecific antibodies described herein comprises theamino acid sequence of SEQ ID NO: 71, or a variant thereof having at least about 80% *(e.g.,* at least 80%, 85%, 90%, 95%, 96%, 97%, 98%, or 99%) sequence identity to the amino acid sequence ofSEQ ID NO: 71.

In some embodiments, any one of the bispecific antibodies described herein comprises theamino acid sequence of SEQ ID NO: 72, or a variant thereof having at least about 80% *(e.g.,* at least 80%, 85%, 90%, 95%, 96%, 97%, 98%, or 99%) sequence identity to the amino acid sequence ofSEQ ID NO: 72.

In some embodiments, any one of the bispecific antibodies described herein comprises theamino acid sequence of SEQ ID NO: 73, or a variant thereof having at least about 80% *(e.g.,* at least 80%, 85%, 90%, 95%, 96%, 97%, 98%, or 99%) sequence identity to the amino acid sequence ofSEQ ID NO: 73.

In some embodiments, any one of the bispecific antibodies described herein comprises theamino acid sequence of SEQ ID NO: 74, or a variant thereof having at least about 80% *(e.g.,* at least 80%, 85%, 90%, 95%, 96%, 97%, 98%, or 99%) sequence identity to the amino acid sequenceof SEQ ID NO: 74.

In some embodiments, any one of the bispecific antibodies described herein comprises theamino acid sequence of SEQ ID NO: 75, or a variant thereof having at least about 80% *(e.g.,* at least 80%, 85%, 90%, 95%, 96%, 97%, 98%, or 99%) sequence identity to the amino acid sequenceof SEQ ID NO: 75.

In some embodiments, any one of the bispecific antibodies described herein comprises theamino acid sequence of SEQ ID NO: 69, or a variant thereof having at least about 80% *(e.g.,* at least 80%, 85%, 90%, 95%, 96%, 97%, 98%, or 99%) sequence identity to the amino acid sequenceof SEQ ID NO: 69; and theamino acid sequence of SEQ ID NO: 70, or a variant thereof having at least about 80% *(e.g.,* at least 80%, 85%, 90%, 95%, 96%, 97%, 98%, or 99%) sequence identity to the amino acid sequenceof SEQ ID NO: 70.

In some embodiments, any one of the bispecific antibodies described herein comprises theamino acid sequence of SEQ ID NO: 71, or a variant thereof having at least about 80% *(e.g.,* at least 80%, 85%, 90%, 95%, 96%, 97%, 98%, or 99%) sequence identity to the amino acid sequenceof SEQ ID NO: 71; and theamino acid sequence of SEQ ID NO: 72, or a variant thereof having at least about 80% *(e.g.,* at least 80%, 85%, 90%, 95%, 96%, 97%, 98%, or 99%) sequence identity to the amino acid sequenceof SEQ ID NO: 72.

In some embodiments, any one of the bispecific antibodies described herein comprises theamino acid sequence of SEQ ID NO: 73, or a variant thereof having at least about 80% *(e.g.,* at least 80%, 85%, 90%, 95%, 96%, 97%, 98%, or 99%) sequence identity to the amino acid sequenceof SEQ ID NO: 73; and theamino acid sequence of SEQ ID NO: 74, or a variant thereof having at least about 80% *(e.g.,* at least 80%, 85%, 90%, 95%, 96%, 97%, 98%, or 99%) sequence identity to the amino acid sequenceof SEQ ID NO: 74.

In some embodiments, any one of the bispecific antibodies described herein comprises theamino acid sequence of SEQ ID NO: 75, or a variant thereof having at least about 80% *(e.g.,* at least 80%, 85%, 90%, 95%, 96%, 97%, 98%, or 99%) sequence identity to the amino acid sequence ofSEQ ID NO: 75; and theamino acid sequence of SEQ ID NO: 74, or a variant thereof having at least about 80% *(e.g.,* at least 80%, 85%, 90%, 95%, 96%, 97%, 98%, or 99%) sequence identity to the amino acid sequenceof SEQ ID NO: 74.

In some embodiments, the chimeric heavy chain of the bispecific antibodycomprises V_{H}1-C_{H}1-L1-V_{H}2-L3-V_{L}2-L2-C_{H}2-C_{H}3from N- terminus to C-terminus. Insomeembodiments, the light chain of the bispecific antibodycomprises V_{L}1-C_{L}from N- terminus to C-terminus. In some embodiments, the bispecific antibodies comprises theamino acid sequence of SEQ ID NO: 90, or a variant thereof having at least about 80% *(e.g.,* at least 80%, 85%, 90%, 95%, 96%, 97%, 98%, or 99%) sequence identity to the amino acid sequence ofSEQ ID NO: 90; and theamino acid sequence of SEQ ID NO: 70, or a variant thereof having at least about 80% *(e.g.,* at least 80%, 85%, 90%, 95%, 96%, 97%, 98%, or 99%) sequence identity to the amino acid sequenceof SEQ ID NO: 70.

In some embodiments, the chimeric heavy chain of the bispecific antibodycomprises V_{H}1-C_{H}1-L1-V_{H}2-L3-V_{L}2-L2-C_{H}2-C_{H}3from N- terminus to C-terminus. Insomeembodiments, the light chain of the bispecific antibodycomprises V_{L}1-C_{L}from N- terminus to C-terminus. In some embodiments, the bispecific antibodies comprises theamino acid sequence of SEQ ID NO: 91, or a variant thereof having at least about 80% *(e.g.,* at least 80%, 85%, 90%, 95%, 96%, 97%, 98%, or 99%) sequence identity to the amino acid sequenceof SEQ ID NO: 91; and theamino acid sequence of SEQ ID NO: 72, or a variant thereof having at least about 80% *(e.g.,* at least 80%, 85%, 90%, 95%, 96%, 97%, 98%, or 99%) sequence identity to the amino acid sequenceof SEQ ID NO: 72.

In some embodiments, the chimeric heavy chain of the bispecific antibodycomprises V_{H}1-C_{H}1-L1-V_{H}2-L3-V_{L}2-L2-C_{H}2-C_{H}3from N- terminus to C-terminus. In other embodiments, the light chain of the bispecific antibodycomprises V_{L}1-C_{L}from N- terminus to C-terminus. In some embodiments, the bispecific antibodies comprises theamino acid sequence of SEQ ID NO: 92, or a variant thereof having at least about 80% (*e.g.*, at least 80%, 85%, 90%, 95%, 96%, 97%, 98%, or 99%) sequence identity to the amino acid sequenceof SEQ ID NO: 92; and theamino acid sequence of SEQ ID NO: 74, or a variant thereof having at least about 80% (*e.g.*, at least 80%, 85%, 90%, 95%, 96%, 97%, 98%, or 99%) sequence identity to the amino acid sequenceof SEQ ID NO: 74.

In some embodiments, the chimeric heavy chain of the bispecific antibodycomprises V_{H}1-C_{H}1-L1-V_{H}2-L3-V_{L}2-L2-C_{H}2-C_{H}3from N- terminus to C-terminus. In other embodiments, the light chain of the bispecific antibodycomprises V_{L}1-C_{L}from N- terminus to C-terminus. In some embodiments, the bispecific antibodies comprises theamino acid sequence of SEQ ID NO: 93, or a variant thereof having at least about 80% (*e.g.,* at least 80%, 85%, 90%, 95%, 96%, 97%, 98%, or 99%) sequence identity to the amino acid sequenceof SEQ ID NO: 93; and theamino acid sequence of SEQ ID NO: 74, or a variant thereof having at least about 80% (*e.g.*, at least 80%, 85%, 90%, 95%, 96%, 97%, 98%, or 99%) sequence identity to the amino acid sequenceof SEQ ID NO: 74.

### Hetero H, CrossMabformat Bispecific antibodies

In some embodiments, any one of the bispecific antibodies described herein has Hetero H, CrossMab structure, which is a bivalent bispecific antibody consisting of heterodimer, comprising two antigen-bindingdomains Fab. In other embodiments, the bispecific antibodyfurther comprises two Fc domainscomprising C_{H}2 and C_{H}3 domains.In some embodiments, the amino acid residue in C_{H}3 domain of one of the Fc is substituted with a larger side chain volume amino acid residue to form a "knob", the amino acid residue in C_{H}3 domain of the other Fc is substituted with a smaller side chain volume amino acid residue to form a "hole", which can promote the binding of heterodimers. Wherein, in the Fab arm, the location of the light chain constant domain(C_{L}) and the heavy chain constant domain 1can be exchanged with each other; or the location of the heavy chain variable domain (V_{H} ) and the light chain variable domain (V_{L}) can be exchanged with each other; or the light chain constant domain(C_{L}) and the heavy chain constant domain 1(C_{H}1) as well as the heavy chain variable domain (V_{H}) and the light chain variable domain (V_{L}) can be exchanged with each other simultaneously. An exemplary schematic diagram of the Hetero H, CrossMabformatis shown in FIG. 7C.

In some embodiments, theFc is derived from wild-type human IgG1 Fc. In other embodiments, the C_{H}3 domain of the Fc comprises, but is not limited to the amino acid substitution described below: S354C, T366W, Y349C, T366S, L368A and/or Y407V, wherein the numbering is according to EU index of Kabat.

In other embodiments of the application, one of the antigen-binding domains specifically binds to *K. pneumoniae* O2 antigen and the other antigen-binding domainspecifically binds to *K. pneumoniae* O1 antigen. In some embodiments, the bispecific antibody can bind to *K. pneumoniae* O2 antigen and*K*. *pneumoniae* O1 antigen simultaneously.

In some embodiments, the bispecific antibodydescribed herein has Hetero H, CrossMabformat, which is a heterodimer consisting of two different monomers, each of which comprises two polypeptide chains. Wherein the first monomer comprises the first heavy chain and the first light chain specifically binding to one of the antigens, and the second monomer comprises the second heavy chain and the second light chain specifically binding to the other antigen. In some embodiments, the first heavy chain of the bispecific antibodycomprises V_{H}1-C_{H}1 structure from N- terminus to C-terminus; the first light chain of the bispecific antibodycomprises V_{L}1-C_{L} structure from N- terminus to C-terminus. In some embodiments, the first heavy chain further comprises an Fccomprising C_{H}2 and C_{H}3 domains. In some embodiments, the first heavy chain of the bispecific antibodycomprises V_{H}1-C_{H}1-C_{H}2-C_{H}3 structure from N- terminus to C-terminus; the first light chain of the bispecific antibodycomprises V_{L}1-C_{L} structure from N- terminus to C-terminus. The V_{H}1 and V_{L}1are the heavy chain variable domain and light chain variable domain that specifically bind to oneof the antigens, respectively, C_{H}1 is a heavy chain constant domain 1, C_{L}is a light chain constant domain. Wherein, the V_{H}1-C_{H}1 and V_{L}1-C_{L}form the antigen-binding domain (Fab) binding to one of the antigens. In some embodiments, the second heavy chain of the bispecific antibodycomprises V_{H}2-C_{L} structure from N- terminus to C-terminus; the second light chain of the bispecific antibodycomprises V_{L}2-C_{H}1 structure from N- terminus to C-terminus. In some embodiments, the second heavy chain further comprises an Fccomprising C_{H}2 and C_{H}3 domains. In some embodiments, the second heavy chain of the bispecific antibodycomprises V_{H}2-C_{L}-C_{H}2-C_{H}3 structure from N- terminus to C-terminus; the second light chain of the bispecific antibodycomprises V_{L}2-C_{H}1 structure from N- terminus to C-terminus. The VH₂ and V_{L}2 are the heavy chain variable domain and light chain variable domain that specifically bind to the other antigen, respectively, C_{H}1 is a heavy chain constant domain 1, C_{L}is a light chain constant domain. Wherein, the V_{H}2-C_{L} and V_{L}2-C_{H}1form the antigen-binding domain (Fab) binding to the other antigen. In some embodiments, the location of C_{L} and C_{H}1 of the first monomer or the second monomer of the bispecific antibody can be exchanged with each other. In some embodiments, the location of V_{H}1 and V_{L}1 can be exchanged with each other. In other embodiments, the location of V_{H}2 and V_{L}2 can be exchanged with each other.In some embodiments, the amino acid residue in C_{H}3 domain of the first heavy chain is substituted with a larger side chain volume amino acid residue to form a "knob", and the amino acid residue in C_{H}3 domain of the second heavy chain is substituted with a smaller side chain volume amino acid residue to form a "hole". In someembodiments, the amino acid residue in C_{H}3 domain of the second heavy chain is substituted with a larger side chain volume amino acid residue to form a "knob", and the amino acid residue in C_{H}3 domain of the first heavy chain is substituted with a smaller side chain volume amino acid residue to form a "hole". In some embodiments, the C_{H}3 domain comprises, but is not limited to the amino acid substitution described below: S354C, T366W, Y349C, T366S, L368A and/or Y407V, wherein the numbering is according to EU index of Kabat.

In some embodiments, the V_{H}1 and V_{L}1are the heavy chain variable domain and light chain variable domain that specifically bind to *K. pneumoniae* O1 antigen, respectively, the VH₂ and V_{L}2 are the heavy chain variable domain and light chain variable domain that specifically bind to *K. pneumoniae* O2 antigen, respectively, *i.e.,* the V_{H}1-C_{H}1 and V_{L}1-C_{L} form the antigen-binding domain (Fab) specifically binding to *K. pneumoniae* O1 antigen, V_{H}2-C_{L} andV_{L}2-C_{H}1 form the antigen-binding domain (Fab) specifically binding to *K. pneumoniae* O2 antigen. In another embodiment, the V_{H}1 and V_{L}1are the heavy chain variable domain and light chain variable domain that specifically bind to *K. pneumoniae* O2 antigen, respectively, the V_{H}2 and V_{L}2 are the heavy chain variable domain and light chain variable domain that specifically bind to *K. pneumoniae* O1 antigen, respectively, *i.e.,* the V_{H}1-C_{H}1 and V_{L}1-C_{L} form the antigen-binding domain (Fab) specifically binding to *K. pneumoniae* O2 antigen, V_{H}2-C_{L} andV_{L}2-C_{H}1 form the antigen-binding domain (Fab) specifically binding to *K. pneumoniae* O1 antigen.

In some embodiments, any one of the bispecific antibodies described herein comprises the amino acid sequence of SEQ ID NO: 76, or a variant thereof having at least about 80% *(e.g.,* at least 80%, 85%, 90%, 95%, 96%, 97%, 98%, or 99%) sequence identity to the amino acid sequenceof SEQ ID NO: 76.

In some embodiments, any one of the bispecific antibodies described herein comprises theamino acid sequence of SEQ ID NO: 77, or a variant thereof having at least about 80% *(e.g.,* at least 80%, 85%, 90%, 95%, 96%, 97%, 98%, or 99%) sequence identity to the amino acid sequenceof SEQ ID NO: 77.

In some embodiments, any one of the bispecific antibodies described herein comprises theamino acid sequence of SEQ ID NO: 78, or a variant thereof having at least about 80% *(e.g.,* at least 80%, 85%, 90%, 95%, 96%, 97%, 98%, or 99%) sequence identity to the amino acid sequenceof SEQ ID NO: 78.

In some embodiments, any one of the bispecific antibodies described herein comprises theamino acid sequence of SEQ ID NO: 74, or a variant thereof having at least about 80% *(e.g.,* at least 80%, 85%, 90%, 95%, 96%, 97%, 98%, or 99%) sequence identity to the amino acid sequenceof SEQ ID NO: 74.

In some embodiments, any one of the bispecific antibodies described herein comprises theamino acid sequence of SEQ ID NO: 79, or a variant thereof having at least about 80% *(e.g.,* at least 80%, 85%, 90%, 95%, 96%, 97%, 98%, or 99%) sequence identity to the amino acid sequenceof SEQ ID NO: 79.

In some embodiments, any one of the bispecific antibodies described herein comprises theamino acid sequence of SEQ ID NO: 80, or a variant thereof having at least about 80% *(e.g.,* at least 80%, 85%, 90%, 95%, 96%, 97%, 98%, or 99%) sequence identity to the amino acid sequenceof SEQ ID NO: 80.

In some embodiments, any one of the bispecific antibodies described herein comprises theamino acid sequence of SEQ ID NO: 76, or a variant thereof having at least about 80% *(e.g.,* at least 80%, 85%, 90%, 95%, 96%, 97%, 98%, or 99%) sequence identity to the amino acid sequenceof SEQ ID NO: 76; and theamino acid sequence of SEQ ID NO: 77, or a variant thereof having at least about 80% *(e.g.,* at least 80%, 85%, 90%, 95%, 96%, 97%, 98%, or 99%) sequence identity to the amino acid sequenceof SEQ ID NO: 77; and theamino acid sequence of SEQ ID NO: 78, or a variant thereof having at least about 80% *(e.g.,* at least 80%, 85%, 90%, 95%, 96%, 97%, 98%, or 99%) sequence identity to the amino acid sequenceof SEQ ID NO: 78; and theamino acid sequence of SEQ ID NO: 74, or a variant thereof having at least about 80% *(e.g.,* at least 80%, 85%, 90%, 95%, 96%, 97%, 98%, or 99%) sequence identity to the amino acid sequence ofSEQ ID NO: 74.

In some embodiments, any one of the bispecific antibodies described herein comprises theamino acid sequence of SEQ ID NO: 79, or a variant thereof having at least about 80% *(e.g.,* at least 80%, 85%, 90%, 95%, 96%, 97%, 98%, or 99%) sequence identity to the amino acid sequenceof SEQ ID NO: 79; and theamino acid sequence of SEQ ID NO: 80, or a variant thereof having at least about 80% *(e.g.,* at least 80%, 85%, 90%, 95%, 96%, 97%, 98%, or 99%) sequence identity to the amino acid sequenceof SEQ ID NO: 80; and amino acid sequence of SEQ ID NO: 78, or a variant thereof having at least about 80% *(e.g.,* at least 80%, 85%, 90%, 95%, 96%, 97%, 98%, or 99%) sequence identity to the amino acid sequenceof SEQ ID NO: 78; and theamino acid sequence of SEQ ID NO: 74, or a variant thereof having at least about 80% *(e.g.,* at least 80%, 85%, 90%, 95%, 96%, 97%, 98%, or 99%) sequence identity to the amino acid sequenceof SEQ ID NO: 74.

In some embodiments, any one of the bispecific antibodies described herein comprises theamino acid sequence of SEQ ID NO: 94, or a variant thereof having at least about 80% *(e.g.,* at least 80%, 85%, 90%, 95%, 96%, 97%, 98%, or 99%) sequence identity to the amino acid sequenceof SEQ ID NO: 94; and theamino acid sequence of SEQ ID NO: 77, or a variant thereof having at least about 80% *(e.g.,* at least 80%, 85%, 90%, 95%, 96%, 97%, 98%, or 99%) sequence identity to the amino acid sequenceof SEQ ID NO: 77; and theamino acid sequence of SEQ ID NO: 95, or a variant thereof having at least about 80% *(e.g.,* at least 80%, 85%, 90%, 95%, 96%, 97%, 98%, or 99%) sequence identity to the amino acid sequenceof SEQ ID NO: 95; and theamino acid sequence of SEQ ID NO: 74, or a variant thereof having at least about 80% *(e.g.,* at least 80%, 85%, 90%, 95%, 96%, 97%, 98%, or 99%) sequence identity to the amino acid sequenceof SEQ ID NO: 74.

In some embodiments, any one of the bispecific antibodies described herein comprises theamino acid sequence of SEQ ID NO: 96, or a variant thereof having at least about 80% *(e.g.,* at least 80%, 85%, 90%, 95%, 96%, 97%, 98%, or 99%) sequence identity to the amino acid sequenceof SEQ ID NO: 96; and theamino acid sequence of SEQ ID NO: 80, or a variant thereof having at least about 80% *(e.g.,* at least 80%, 85%, 90%, 95%, 96%, 97%, 98%, or 99%) sequence identity to the amino acid sequenceof SEQ ID NO: 80; and theamino acid sequence of SEQ ID NO: 95, or a variant thereof having at least about 80% *(e.g.,* at least 80%, 85%, 90%, 95%, 96%, 97%, 98%, or 99%) sequence identity to the amino acid sequenceof SEQ ID NO: 95; and theamino acid sequence of SEQ ID NO: 74, or a variant thereof having at least about 80% *(e.g.,* at least 80%, 85%, 90%, 95%, 96%, 97%, 98%, or 99%) sequence identity to the amino acid sequenceof SEQ ID NO: 74.

### IgG-(scFv)₂format Bispecific antibodies

In some embodiments, the bispecific antibody described herein has IgG-(scFv)₂format, which consists of twoidentical monomers, each monomer comprising two antigen-binding domains, one of which is Fab and the other ofwhich is scFv. In some embodiments, thebispecific antibody further comprises an Fccomprising C_{H}2 and C_{H}3 domains. The scFv connects to the carboxyl terminal of the Fc through the peptide linker (L). An exemplary schematic diagram of theIgG-(scFv)₂formatis shown in FIG. 7D.

In some embodiments of the application, one of the antigen-binding domains (Fab or scFv) specifically binds to *K. pneumoniae* O1 antigen and the other antigen-binding domain (Fab or scFv) specifically binds to *K. pneumoniae* O2antigen. In some embodiments, the bispecific antibody can bind to *K. pneumoniae* O2 antigen and*K*. *pneumoniae* O1 antigen simultaneously.

In some embodiments, the bispecific antibodydisclosed herein consists of two identical monomers, each of which comprises a heavy chain and a light chain. In some embodiments, the heavy chain of the bispecific antibodycomprises V_{H}1-C_{H}1-C_{H}2-C_{H}3 structure from N-terminus to C-terminus. In some embodiments, the light chain of the bispecific antibodycomprises V_{L}1-C_{L} structure from N- terminus to C-terminus. In some embodiments, the heavy chain further comprises an Fccomprising C_{H}2 and C_{H}3 domains. In other embodiments, the heavy chain of the bispecific antibodycomprises V_{H}1-C_{H}1-C_{H}2-C_{H}3-L-V_{H}2-L3-V_{L}2 structure from N- terminus to C-terminus.In other embodiments, the heavy chain of the bispecific antibodycomprises V_{H}1-C_{H}1-C_{H}2-C_{H}3-L-V_{L}2-L3-V_{H}2 structure from N- terminus to C-terminus. In some embodiments, the light chain of the bispecific antibodycomprises V_{L}1-C_{L}structure from N-terminus to C-terminus. Wherein, the V_{H}1 and V_{L}1are the heavy chain variable domain and light chain variable domain that specifically bind to oneof the antigens, respectively; the VH₂ and VL₂ are the heavy chain variable domain and light chain variable domain that specifically bind to the other antigen, respectively; C_{H}1 is a heavy chain constant domain 1; C_{L}is a light chain constant domain; L and L3arepeptide linkers. The V_{H}1-C_{H}1 and V_{L}l-C_{L}form one of the antigen-binding domains (Fab) of the bispecific antibody; the V_{H}2-L3-V_{L}2 or V_{L}2-L3-V_{H}2form the other antigen-binding domain (scFv) of the bispecific antibody.

In some embodiments, the V_{H}1 and V_{L}1are the heavy chain variable domain and light chain variable domain that specifically bind to *K. pneumoniae* O1 antigen, respectively, *i.e.,* the V_{H}1-C_{H}1 and V_{L}1-C_{L} form the antigen-binding domain (Fab) specifically binding to *K. pneumoniae* O1 antigen; the V_{H}2 and V_{L}2 are the heavy chain variable domain and light chain variable domain that specifically bind to *K. pneumoniae* O2 antigen, respectively, *i.e.,* the V_{H}2-L3-V_{L}2 or V_{L}2-L3-V_{H}2 form the antigen-binding domain (scFv) specifically binding to *K. pneumoniae* O2 antigen. In someembodiments, the V_{H}1 and V_{L}1are the heavy chain variable domain and light chain variable domain that specifically bind to *K. pneumoniae* O2 antigen, respectively, *i.e.,* the V_{H}1-C_{H}1 and V_{L}1-C_{L} form the antigen-binding domain (Fab) specifically binding to *K. pneumoniae* O2 antigen; the V_{H}2 and V_{L}2 are the heavy chain variable domain and light chain variable domain that specifically bind to *K. pneumoniae* O1 antigen, respectively, *i.e.,* the V_{H}2-L3-V_{L}2 or V_{L}2-L3-V_{H}2 form the antigen-binding domain (scFv) specifically binding to *K. pneumoniae* O1 antigen. In some embodiments, the bispecific antibodycomprisesC_{H}1comprising theamino acid sequence of SEQ ID No: 60. In some embodiments, the bispecific antibodycomprisesC_{H}2-C_{H}3comprising theamino acid sequence of SEQ ID No: 59.

Peptide linker (or linker) may be used to join domains and/or regions of the chimeric heavy chain of the bispecific antibody into a contiguous molecule. In some embodiments, the bispecific antibody may include additional linkers, such as a flexible linker interconnecting the variable heavy and light chains of an scFv. In some embodiments, the bispecific antibody may include additional linkers, such as a flexible linker interconnecting the variable heavy and light chains of an scFv and other linkers that connect other binding units to the core structure of the bispecific antibody.

An exemplary, non-limiting example of a linker is a polypeptide chain comprising at least 4 residues. Portions of such linkers may be flexible, hydrophilic and have little or no secondary structure of their own (linker portions or flexible linker portions). Linkers of at least 4 amino acids may be used to join domains and/or regions that are positioned near to one another after the molecule has assembled. Longer linkers may also be used. In some embodiments, linkers may be about any one of: 1, 2, 3, 4, 5, 6, 7, 8, 9, 10, 11, 12, 13, 14, 15, 16, 17, 18, 19, 20, 25, 30, 35, 40, 45, 50, 100, 125, 150, 175 or 200 residues. When multiple linkers are used to interconnect portions of the molecule, the linkers may be the same or different (e.g., the same or different length and/or amino acid sequence).

In some aspects, thepeptide linker (linker) comprises or consists of a Gly-Ser linker.As described herein, the term "Gly-Ser linker" refers to a peptide that consists of glycine and serine residues. An exemplary Gly-Ser linker comprises an amino acid sequence of the formula (Gly₄Ser)ₙ, wherein n is a positive integer (e.g., 1, 2, 3, 4, 5, 6, 7, 8, 9 or 10). A preferred Gly-Ser linker is (Gly₄Ser)₂, i.e.,GGGGSGGGGS(SEQ ID NO: 102), and (Gly₄Ser)₄,*i*.*e*.,GGGGSGGGGSGGGGSGGGGS(SEQ ID NO: 103).Another preferred Gly-Ser linker is (Gly₄Ser)₃,*i*.*e*.,, GGGGSGGGGSGGGGS (SEQ ID NO: 104). In yet other aspects, two or more Gly-Ser linker are incorporated in series in a peptide linker. In some aspects, the peptide linker comprises at least a portion of a hinge region (e.g., derived from an IgGl, IgG2, IgG3, or IgG4 molecule) and a series of Gly- Ser amino acid residues (e.g., a Gly-Ser linker such as (Gly₄Ser)ₙ).

In some embodiments, the peptide linker comprises a Gly-Ser or all Gly linker and a portion or modified portion of a hinge domain. In some embodiments, the peptide linker (e.g., L) of the bispecific antibody connecting the antigen-binding domain to the C_{H}3 of the Fc carboxyl terminal comprises the amino acid sequence GGGGSGGGGTGGGGS(SEQ ID NO: 114).

In some embodiments, regardless of the peptide linker used to interconnect the antigen-binding domain to Fc (e.g., L), the bispecific antibody may optionally comprise additional peptide linkers. The lengths and sequence of such additional peptide linkers are independently selected. For example, the bispecific antibody may further comprise a flexible peptide linker (L3) interconnecting the variable heavy and light chains of a scFv (V_{HSCFV} and V_{LSCFV}). This flexible peptide linker may comprise a Gly-Ser linker. Generally, this linker does not include a hinge portion. In some embodiments, this flexible peptide linker (L3) interconnecting the variable heavy and light chains of the scFv comprises the sequence of GGGGSGGGGSGGGGSGGGGS (SEQ ID NO: 103).

In some embodiments, the chimeric heavy chain of the bispecific antibodycomprises V_{H}1-C_{H}1-C_{H}2-C_{H}3-L-V_{H}2-L3-V_{L}2from N- terminus to C-terminus. Insomeembodiments, the light chain of the bispecific antibodycomprises V_{L}1-C_{L}from N- terminus to C-terminus. In some embodiments, the bispecific antibodies comprises the amino acid sequence of SEQ ID NO: 97, or a variant thereof having at least about 80% *(e.g.,* at least 80%, 85%, 90%, 95%, 96%, 97%, 98%, or 99%) sequence identity to the amino acid sequenceof SEQ ID NO: 97; and/ortheamino acid sequence of SEQ ID NO: 83, or a variant thereof having at least about 80% (*e.g.*, at least 80%, 85%, 90%, 95%, 96%, 97%, 98%, or 99%) sequence identity to the amino acid sequenceof SEQ ID NO: 83.

In some embodiments, the chimeric heavy chain of the bispecific antibodycomprises V_{H}1-C_{H}1-C_{H}2-C_{H}3-L-V_{H}2-L3-V_{L}2from N- terminus to C-terminus. Insomeembodiments, the light chain of the bispecific antibodycomprises V_{L}1-C_{L}from N- terminus to C-terminus. In some embodiments, the bispecific antibodies comprises theamino acid sequence of SEQ ID NO: 98, or a variant thereof having at least about 80% *(e.g.,* at least 80%, 85%, 90%, 95%, 96%, 97%, 98%, or 99%) sequence identity to the amino acid sequenceof SEQ ID NO: 98; and/ortheamino acid sequence of SEQ ID NO: 85, or a variant thereof having at least about 80% (*e.g.*, at least 80%, 85%, 90%, 95%, 96%, 97%, 98%, or 99%) sequence identity to the amino acid sequence ofSEQ ID NO: 85.

### scFv-Fab IgGformat Bispecific antibodies

In some embodiments, the bispecific antibody described herein has scFv-Fab IgGformat, which is in the form of heterodimer. The heterodimer comprises a first monomer and a second monomer, while the first monomer comprises the antigen-binding domain (Fab) binding to one of the antigens, the second monomer comprises the antigen-binding domain (scFv) binding to the other antigen. In some embodiments, thebispecific antibody further comprises two Fccomprising C_{H}2 and C_{H}3 domains. The two Fc further comprise amino acid substitution, which can promote the binding of heterodimers. An exemplary schematic diagram of scFv-Fab IgGformatis shown in FIG. 7E.

In some embodiments, one of the antigen-binding domains (Fab or scFv) specifically binds to *K. pneumoniae* O2 antigen and the other antigen-binding domain (scFv or Fab) specifically binds to *K. pneumoniae* O1 antigen. In some embodiments, the bispecific antibody can bind to *K. pneumoniae* O2 antigen and*K*. *pneumoniae* O1 antigen simultaneously.

In some embodiments, the bispecific antibody described herein has scFv-Fab IgGformat, which is in the form of heterodimer. The heterodimer comprises a first monomer and a second monomer, while the first monomer comprises two polypeptide chains: the first heavy chain and light chain, wherein the first heavy chain comprises V_{H}1-C_{H}1 structure from N- terminus to C-terminus, the light chain comprises V_{L}1-C_{L} structure. In some embodiments, the first heavy chain further comprises an Fccomprising C_{H}2 and C_{H}3 domains. In some embodiments, the first heavy chain comprises V_{H}1-C_{H}1-C_{H}2-C_{H}3 structure from N- terminus to C-terminus, the light chain comprises V_{L}1-C_{L} structure. The V_{H}1 and V_{L}1are the heavy chain variable domain and light chain variable domain that specifically bind to oneof the antigens, respectively; C_{H}1 is a heavy chain constant domain 1; C_{L}is a light chain constant domain. The V_{H}1-C_{H}1 and V_{L}l-C_{L}form one of the antigen-binding domains (Fab). The second monomer comprises one polypeptide chain: the second heavy chain. The secondheavy chain comprises V_{H}2-L3-V_{L}2 structure or V_{L}2-L3-V_{H}2 structure from N- terminus to C-terminus. In some embodiments, the secondheavy chain further comprises an Fccomprising C_{H}2 and C_{H}3 domains. In some embodiments, the secondheavy chain comprises V_{H}2-L3-V_{L}2-C_{H}2-C_{H}3 structure or V_{L}2-L3-V_{H}2-C_{H}2-C_{H}3 structure from N- terminus to C-terminus. The V_{H}2 and V_{L}2 are the heavy chain variable domain and light chain variable domain that specifically bind to the other antigen, respectively; L3 is a peptide linker. The V_{H}2-L3-V_{L}2 or V_{L}2-L3-V_{H}2form the other antigen-binding domains (scFv).

In some embodiments, the V_{H}1 and V_{L}1are the heavy chain variable domain and light chain variable domain that specifically bind to *K. pneumoniae* O1 antigen, respectively, *i.e.,* the V_{H}1-C_{H}1 and V_{L}1-C_{L} form the antigen-binding domain (Fab) specifically binding to *K. pneumoniae* O1 antigen; the V_{H}2 and V_{L}2 are the heavy chain variable domain and light chain variable domain that specifically bind to *K. pneumoniae* O2 antigen, respectively, *i.e.,* the V_{H}2-L3-V_{L}2 or V_{L}2-L3-V_{H}2 form the antigen-binding domain (scFv) specifically binding to *K. pneumoniae* O2 antigen. In someembodiments, the V_{H}1 and V_{L}1are the heavy chain variable domain and light chain variable domain that specifically bind to *K. pneumoniae* O2 antigen, respectively, *i.e.,* the V_{H}1-C_{H}1 and V_{L}1-C_{L} form the antigen-binding domain (Fab) specifically binding to *K. pneumoniae* O2 antigen. The V_{H}2 and V_{L}2 are the heavy chain variable domain and light chain variable domain that specifically bind to *K. pneumoniae* O1 antigen, respectively, *i.e.,* the V_{H}2-L3-V_{L}2 or V_{L}2-L3-V_{H}2 form the antigen-binding domain (scFv) specifically binding to *K. pneumoniae* O1 antigen.

In some embodiments, theFc is derived from human wild-type IgG1. In another embodiment, relative to human wild-type IgG1, theFcin one monomer comprises, but is not limited to the amino acid substitution described below: E357Q and S364K; relative to human wild-type IgG1, theFcin the other monomer comprises, but is not limited to the amino acid substitution described below: Q295E, L368D, K370S, N384D, Q418E and N421D, wherein the numbering is according to EU index of Kabat. In some embodiments, exemplary peptide linker (e.g., L3) connecting V_{H}1 with V_{L}1 of scFv comprises GKPGSGKPGSGKPGSGKPGS (SEQ ID NO: 113).

In some embodiments, any one of the bispecific antibodies described herein comprises the amino acid sequence of SEQ ID NO: 81, or a variant thereof having at least about 80% *(e.g.,* at least 80%, 85%, 90%, 95%, 96%, 97%, 98%, or 99%) sequence identity to the amino acid sequenceof SEQ ID NO: 81.

In some embodiments, any one of the bispecific antibodies described herein comprises theamino acid sequence of SEQ ID NO: 82, or a variant thereof having at least about 80% *(e.g.,* at least 80%, 85%, 90%, 95%, 96%, 97%, 98%, or 99%) sequence identity to the amino acid sequenceof SEQ ID NO: 82.

In some embodiments, any one of the bispecific antibodies described herein comprises theamino acid sequence of SEQ ID NO: 83, or a variant thereof having at least about 80% *(e.g.,* at least 80%, 85%, 90%, 95%, 96%, 97%, 98%, or 99%) sequence identity to the amino acid sequenceof SEQ ID NO: 83.

In some embodiments, any one of the bispecific antibodies described herein comprises theamino acid sequence of SEQ ID NO: 84, or a variant thereof having at least about 80% *(e.g.,* at least 80%, 85%, 90%, 95%, 96%, 97%, 98%, or 99%) sequence identity to the amino acid sequenceof SEQ ID NO: 84.

In some embodiments, any one of the bispecific antibodies described herein comprises theamino acid sequence of SEQ ID NO: 85, or a variant thereof having at least about 80% *(e.g.,* at least 80%, 85%, 90%, 95%, 96%, 97%, 98%, or 99%) sequence identity to the amino acid sequenceof SEQ ID NO: 85.

In some embodiments, any one of the bispecific antibodies described herein comprises theamino acid sequence of SEQ ID NO: 81, or a variant thereof having at least about 80% *(e.g.,* at least 80%, 85%, 90%, 95%, 96%, 97%, 98%, or 99%) sequence identity to the amino acid sequenceof SEQ ID NO: 81; and theamino acid sequence of SEQ ID NO: 82, or a variant thereof having at least about 80% *(e.g.,* at least 80%, 85%, 90%, 95%, 96%, 97%, 98%, or 99%) sequence identity to the amino acid sequenceof SEQ ID NO: 82; and theamino acid sequence of SEQ ID NO: 83, or a variant thereof having at least about 80% *(e.g.,* at least 80%, 85%, 90%, 95%, 96%, 97%, 98%, or 99%) sequence identity to the amino acid sequenceof SEQ ID NO: 83.

In some embodiments, any one of the bispecific antibodies described herein comprises theamino acid sequence of SEQ ID NO: 84, or a variant thereof having at least about 80% *(e.g.,* at least 80%, 85%, 90%, 95%, 96%, 97%, 98%, or 99%) sequence identity to the amino acid sequenceof SEQ ID NO: 84; and theamino acid sequence of SEQ ID NO: 82, or a variant thereof having at least about 80% *(e.g.,* at least 80%, 85%, 90%, 95%, 96%, 97%, 98%, or 99%) sequence identity to the amino acid sequenceof SEQ ID NO: 82; and theamino acid sequence of SEQ ID NO: 85, or a variant thereof having at least about 80% *(e.g.,* at least 80%, 85%, 90%, 95%, 96%, 97%, 98%, or 99%) sequence identity to the amino acid sequenceof SEQ ID NO: 85.

In some embodiments, any one of the bispecific antibodies described herein comprises theamino acid sequence of SEQ ID NO: 99, or a variant thereof having at least about 80% *(e.g.,* at least 80%, 85%, 90%, 95%, 96%, 97%, 98%, or 99%) sequence identity to the amino acid sequenceof SEQ ID NO: 99; and theamino acid sequence of SEQ ID NO: 100, or a variant thereof having at least about 80% *(e.g.,* at least 80%, 85%, 90%, 95%, 96%, 97%, 98%, or 99%) sequence identity to the amino acid sequenceof SEQ ID NO: 100; and theamino acid sequence of SEQ ID NO: 83, or a variant thereof having at least about 80% *(e.g.,* at least 80%, 85%, 90%, 95%, 96%, 97%, 98%, or 99%) sequence identity to the amino acid sequenceof SEQ ID NO: 83.

In some embodiments, any one of the bispecific antibodies described herein comprises theamino acid sequence of SEQ ID NO: 101, or a variant thereof having at least about 80% *(e.g.,* at least 80%, 85%, 90%, 95%, 96%, 97%, 98%, or 99%) sequence identity to the amino acid sequence ofSEQ ID NO: 101; and theamino acid sequence of SEQ ID NO: 100, or a variant thereof having at least about 80% *(e.g.,* at least 80%, 85%, 90%, 95%, 96%, 97%, 98%, or 99%) sequence identity to the amino acid sequence ofSEQ ID NO: 100; and theamino acid sequence of SEQ ID NO: 85, or a variant thereof having at least about 80% *(e.g.,* at least 80%, 85%, 90%, 95%, 96%, 97%, 98%, or 99%) sequence identity to the amino acid sequenceof SEQ ID NO: 85.

Exemplary antibody sequences are shown in Tables 2-10, wherein the CDR numbering is according to the EU index of Kabat. Those skilled in the art will recognize that many algorithms are known for prediction of CDR positions and for delimitation of antibody heavy chain and light chain variable domains. CDRs, V_{H} and/or V_{L} sequences from the antibodies specifically binding to *K. pneumoniae* O2 antigen, the antibodies specifically binding to *K. pneumoniae* O1 antigen or the bispecific antibody described herein, but based on prediction algorithms other than those exemplified in the tables below, are within the scope of this invention. The antibody or antigen-binding fragment specifically binding to *K. pneumoniae* O1 antigen can selected from the anti-O1 antigen antibody described in the Chinese application No.202110980272.2, which was incorporated here in this invention.

**Table 2: Exemplary antibody CDR sequences specifically binding to K. pneumoniae 02 antigen**

| **Antibody name** | **HC-CDR1** | **HC-CDR2** | **HC-CDR3** |
|---|---|---|---|
| K1 | TYYWN (SEQ ID NO: 1) | NIHQSGTVYYNPSLKS (SEQ ID NO: 2) | ESDHGYKWNYFDY (SEQ ID NO: 5) |
| K2 | TYYWN (SEQ ID NO: 1) | NIHQSSFLYYNPSLKS (SEQ ID NO: 3) | ESDDGYKWNYFDY (SEQ ID NO: 6) |
| K3 | TYYWN (SEQ ID NO: 1) | NIHQSGTTYYNPSLKS (SEQ ID NO: 4) | ESDDGYKWNYFDY (SEQ ID NO: 6) |
| K4 | TYYWN (SEQ ID NO: 1) | NIHQSGTTYYNPSLKS (SEQ ID NO: 4) | ESDDGYKWNYFDY (SEQ ID NO: 6) |
| K5 | TYYWN (SEQ ID NO: 1) | NIHQSGTVYYNPSLKS (SEQ ID NO: 2) | ESDVGYKWNYFDY (SEQ ID NO: 7) |
| | | | |

| **Antibody name** | **LC-CDR1** | **LC-CDR2** | **LC-CDR3** |
|---|---|---|---|
| K1 | RASQTVTNYLA (SEQ ID NO: 8) | DMSIRAA (SEQ ID NO: 12) | QHRSNWPIFT (SEQ ID NO: 15) |
| K2 | RASQIVTNYLA (SEQ ID NO: 9) | DMSIRAA (SEQ ID NO: 12) | QHRSNWPLFT (SEQ ID NO: 16) |
| K3 | RASQVVTNYLA (SEQ ID NO: 10) | DKSIRAA (SEQ ID NO: 13) | QHRSNWPLFT (SEQ ID NO: 16) |
| K4 | RASQVVTDYLA (SEQ ID NO: 11) | DMIIRAA (SEQ ID NO: 14) | QHRSNWPLFT (SEQ ID NO: 16) |
| K5 | RASQVVTNYLA (SEQ ID NO: 10) | DMSIRAA (SEQ ID NO: 12) | QHRSNWPIFT (SEQ ID NO: 15) |

**Table 3-1: Exemplary antibody V_{H} and V_{L} sequences specifically binding to K. pneumoniae O2 antigen**

| **SEQ ID NO:** | **Description** | **Sequence** |
|---|---|---|
| 17 | K1 V_{H} | QVQLQESGPGLVKPSETLSLTCTVSGGSLSTYYWNWIRQPPGKGLEWIANIHQ SGTVYYNPSLKSRVTMSVDTSKNQFSLKLSSVTAADTAVYYCARESDHGYKW NYFDYWGQGTLVTVSS |
| 18 | K2 V_{H} | QVQLQESGPGLVKPSETLSLTCTVSGGAISTYYWNWIRQPPGKGLEWIANIHQ SSTLYYNPSLKSRVTMSVDTSKNQFSLKLSSVTAADTAVYYCARESDDGYKW NYFDYWGQGTLVTVSS |
| 19 | K3 V_{H} | QVQLQESGPGLVKPSETLSLTCTVSGGSISTYYWNWIRQPPGKGLEWIANIHQS GTTYYNPSLKSRVTMSVDTSKNQFSLKLSSVTAADTAVYYCARESDDGYKW NYFDYWGQGTLVTVSS |
| | K4 V_{H} | |
| 20 | K5 V_{H} | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYKW NYFDYWGQGTLVTVSS |
| | | |
| 21 | K1 V_{L} | EIVLTQSPATLSVSPGERATLSCRASQTVTNYLAWYQHKPGQAPRLLIFDMSIR AAGIPARFSGSGSGTEFTLTISSLQSEDFAVYYCQHRSNWPIFTFGQGTKVEIK |
| 22 | K2 V_{L} | EIVLTQSPASLSLSPGERATLSCRASQIVTNYLAWYQHKPGQAPRLLIFDMSIRA |
| | | AGIPARFSGSGSGTDFTLTISSLEPEDFAVYYCQHRSNWPLFTFGPGTKVDIK |
| 23 | K3 V_{L} | EIVLTQSPATLSVSPGERATLSCRASQVVTNYLAWYQHKPGQAPRLLIFDKSIR AAGIPARFSGSGSGTEFTLTISSLQSEDFAVYYCQHRSNWPLFTFGQGTKVEIK |
| 24 | K4 V_{L} | EIVLTQSPATLSVSPGERATLSCRASQVVTDYLAWYQHKPGQAPRLLIFDMIIR AAGIPARFSGSGSGTEFTLTISSLQSEDFAVYYCQHRSNWPLFTFGQGTKVEIK |
| 25 | K5 V_{L} | EIVLTQSPATLSVSPGERATLSCRASQVVTNYLAWYQHKPGQAPRLLIFDMSIR AAGIPARFSGSGSGTEFTLTISSLQSEDFAVYYCQHRSNWPIFTFGQGTKVEIK |

**Table 3-2: Exemplary antibody V_{H} and V_{L} cysteine variant sequences specifically binding to K. pneumoniae O2 antigen**

| **SEQ ID NO:** | **Description** | **Sequence** |
|---|---|---|
| 26 | K1 V_{H}cysteine variant | QVQLQESGPGLVKPSETLSLTCTVSGGSLSTYYWNWIRQPPGKCLEWIANI HQSGTVYYNPSLKSRVTMSVDTSKNQFSLKLSSVTAADTAVYYCARESDH GYKWNYFDYWGQGTLVTVSS |
| 27 | K2 V_{H} cysteine variant | QVQLQESGPGLVKPSETLSLTCTVSGGAISTYYWNWIRQPPGKCLEWIANI HQSSTLYYNPSLKSRVTMSVDTSKNQFSLKLSSVTAADTAVYYCARESDD GYKWNYFDYWGQGTLVTVSS |
| 28 | K3 V_{H} K4 V_{H} cysteine variant | QVQLQESGPGLVKPSETLSLTCTVSGGSISTYYWNWIRQPPGKCLEWIANIH QSGTTYYNPSLKSRVTMSVDTSKNQFSLKLSSVTAADTAVYYCARESDDG YKWNYFDYWGQGTLVTVSS |
| 29 | K5 V_{H} cysteine variant | QVQLQESGPGLVKPSETLSLTCTVSGGSIDTYYWNWIRQPPGKCLEWIANI HQSGTVYYNPSLKSRVTMSVDTSKNQFSLKLSSVTAADTAVYYCARESDV GYKWNYFDYWGQGTLVTVSS |
| | | |
| 30 | K1 V_{L} cysteine variant | EIVLTQSPATLSVSPGERATLSCRASQTVTNYLAWYQHKPGQAPRLLIFDM SIRAAGIPARFSGSGSGTEFTLTISSLQSEDFAVYYCQHRSNWPIFTFGCGTK VEIK |
| 31 | K2 V_{L} cysteine variant | EIVLTQSPASLSLSPGERATLSCRASQIVTNYLAWYQHKPGQAPRLLIFDMSI RAAGIPARFSGSGSGTDFTLTISSLEPEDFAVYYCQHRSNWPLFTFGCGTKV DIK |
| 32 | K3 V_{L} cysteine variant | EIVLTQSPATLSVSPGERATLSCRASQVVTNYLAWYQHKPGQAPRLLIFDKS IRAAGIPARFSGSGSGTEFTLTISSLQSEDFAVYYCQHRSNWPLFTFGCGTK VEIK |
| 33 | K4 V_{L} cysteine variant | EIVLTQSPATLSVSPGERATLSCRASQVVTDYLAWYQHKPGQAPRLLIFDMI IRAAGIPARFSGSGSGTEFTLTISSLQSEDFAVYYCQHRSNWPLFTFGCGTK VEIK |
| 34 | K5 V_{L} cysteine variant | EIVLTQSPATLSVSPGERATLSCRASQVVTNYLAWYQHKPGQAPRLLIFDM SIRAAGIPARFSGSGSGTEFTLTISSLQSEDFAVYYCQHRSNWPIFTFGCGTK VEIK |

**Table 4: Exemplary antibody CDR sequences specifically binding to K. pneumoniae O1 antigen**

| **Antibody name** | **HC-CDR1** | **HC-CDR2** | **HC-CDR3** |
|---|---|---|---|
| G2 | NAWMY (SEQ ID NO: 35) | RIRSYSDGGTTDYAALVEG (SEQ ID NO: 37) | PSGDFYPAS (SEQ ID NO: 39) |
| G7 | GYWMS (SEQ ID NO: 36) | NIKQDGSEQYYVDSVKG (SEQ ID NO: 38) | DRGIKMGSVWYPSFDL (SEQ ID NO: 40) |
| | | | |

| **Antibody name** | **LC-CDR1** | **LC-CDR2** | **LC-CDR3** |
|---|---|---|---|
| G2 | RSSQNLLHSNGYNYLD (SEQ ID NO: 41) | LSSNRAS (SEQ ID NO: 43) | MQALQTPYT (SEQ ID NO: 45) |
| G7 | RASRSISNYLN (SEQ ID NO: 42) | AASTLQS (SEQ ID NO: 44) | QQSYSAPRT (SEQ ID NO: 46) |

**Table 5-1: Exemplary antibody V_{H} and V_{L} sequences specifically binding to K. pneumoniae O1 antigen**

| **SEQ ID NO:** | **Description** | **Sequence** |
|---|---|---|
| 47 | G2 V_{H} | EVQLVESGGGLVRPGGSLRLSCVASGLTFSNAWMYWVRQAPGKGLEWL GRIRSYSDGGTTDYAALVEGRFTISRDDSINTVFLQMNNLKSEDTAVYYC GFPSGDFYPASWGQGTLVTVSS |
| 48 | G7 V_{H} | EVQLLESGGGLVQPGGSLRLSCAASGFIFSGYWMSWVRQAPGKGLEWV ANIKQDGSEQYYVDSVKGRFTISRDNAKSSLYLQMNSLRTEDTAIFYCAR DRGIKMGSVWYPSFDLWGQGTMVTVSS |
| | | |
| 49 | G2 V_{L} | DIVMTQTPLSLPVSPGEPASISCRSSQNLLHSNGYNYLDWYVQKPGQSPQ LLIYLSSNRASGVPDRFSGSGSGTDFTLKISRVEAEDVGVYYCMQALQTP YTFGQGTKLEIK |
| 50 | G7 V_{L} | EIVLTQSPSSLSASVGDRVTITCRASRSISNYLNWYQQKPGQPPKLLIYAAS TLQSGVPSRFRGSGSGAHFTLIISSLQPEDFASYYCQQSYSAPRTFGGGTRL EIK |

**Table 5-2: Exemplary antibody V_{H} and V_{L} cysteine variant sequences specifically binding to K. pneumoniae O1 antigen**

| **SEQ ID NO:** | **Description** | **Sequence** |
|---|---|---|
| 51 | G2 V_{H} cysteine variant | EVQLVESGGGLVRPGGSLRLSCVASGLTFSNAWMYWVRQAPGKCLEWL GRIRSYSDGGTTDYAALVEGRFTISRDDSINTVFLQMNNLKSEDTAVYYC GFPSGDFYPASWGQGTLVTVSS |
| 52 | G7 V_{H} cysteine variant | EVQLLESGGGLVQPGGSLRLSCAASGFIFSGYWMSWVRQAPGKCLEWV ANIKQDGSEQYYVDSVKGRFTISRDNAKSSLYLQMNSLRTEDTAIFYCAR DRGIKMGSVWYPSFDLWGQGTMVTVSS |
| | | |
| 53 | G2 V_{L} cysteine variant | DIVMTQTPLSLPVSPGEPASISCRSSQNLLHSNGYNYLDWYVQKPGQSPQ LLIYLSSNRASGVPDRFSGSGSGTDFTLKISRVEAEDVGVYYCMQALQTP YTFGCGTKLEIK |
| 54 | G7 V_{L} cysteine variant | EIVLTQSPSSLSASVGDRVTITCRASRSISNYLNWYQQKPGQPPKLLIYAAS TLQSGVPSRFRGSGSGAHFTLIISSLQPEDFASYYCQQSYSAPRTFGCGTRL EIK |

**Table 6: Exemplary antibody constant region sequences**

| **SEQ ID NO:** | **Description** | **Sequence** |
|---|---|---|
| 55 | IgG1 heavy chain constant region | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLN GKEYKCKVSNKALPAPIEKTISKAKGQPREPQVYTLPPSREEMTKNQVSL TCLVKGFYPSDIAVEWESNGQPENNYKTTPPVLDSDGSFFLYSKLTVDKS RWQQGNVFSCSVMHEALHNHYTQKSLSLSPGK |
| 56 | IgG4 heavy chain constant region | ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPSCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV |
| | | KGFYPSDIAVEWESNGQPENNYKTTPPVLDSDGSFFLYSRLTVDKSRWQE GNVFSCSVMHEALHNHYTQKSLSLSLGK |
| 57 | Light chain constant region(kappa) | |
| 58 | Light chain constant region(lambda ) | GQPKAAPSVTLFPPSSEELQANKATLVCLISDFYPGAVTVAWKADSSPVK AGVETTTPSKQSNNKYAASSYLSLTPEQWKSHRSYSCQVTHEGSTVEKT VAPTECS |
| 59 | Exemplary C_{H}2-C_{H}3 | APELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKAL PAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVM HEALHNHYTQKSLSLSPGK |
| 60 | Exemplary C_{H}1 | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKV |

**Table 7-1: Partial heavy chain and light chain sequences of exemplary DVD-Ig format bispecific antibody specifically binding to O2 antigen and O1 antigen**

| **SEQ ID NO:** | **Description** | **Sequence** |
|---|---|---|
| 61 | G7-K5-Ig1 partial heavy chain (G7V_{H}-K5V_{H}-IgG1C_{H}1) | EVQLLESGGGLVQPGGSLRLSCAASGFIFSGYWMSWVRQAPGKGLEWVANI KQDGSEQYYVDSVKGRFTISRDNAKSSLYLQMNSLRTEDTAIFYCARDRGIK MGSVWYPSFDLWGQGTMVTVSSGGGGSGGGGSQVQLQESGPGLVKPSETLS LTCTVSGGSIDTYYWNWIRQPPGKGLEWIANIHQSGTVYYNPSLKSRVTMSV DTSKNQFSLKLSSVTAADTAVYYCARESDVGYKWNYFDYWGQGTLVTVSS ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKV |
| 62 | G7-K5-Ig1 light chain (G7V_{L}-K5V_{L}-C_{L}) | EIVLTQSPSSLSASVGDRVTITCRASRSISNYLNWYQQKPGQPPKLLIYAASTL QSGVPSRFRGSGSGAHFTLIISSLQPEDFASYYCQQSYSAPRTFGGGTRLEIKG GGGSGGGGSEIVLTQSPATLSVSPGERATLSCRASQVVTNYLAWYQHKPGQA PRLLIFDMSIRAAGIPARFSGSGSGTEFTLTISSLQSEDFAVYYCQHRSNWPIFT FGQGTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQGLS SPVTKSFNRGEC |
| 63 | G2-K5-Ig1 partial heavy chain (G2V_{H}-K5V_{H}-IgG1C_{H}1) | EVQLVESGGGLVRPGGSLRLSCVASGLTFSNAWMYWVRQAPGKGLEWLGRI RSYSDGGTTDYAALVEGRFTISRDDSINTVFLQMNNLKSEDTAVYYCGFPSG DFYPASWGQGTLVTVSSGGGGSGGGGSQVQLQESGPGLVKPSETLSLTCTVS GGSIDTYYWNWIRQPPGKGLEWIANIHQSGTVYYNPSLKSRVTMSVDTSKNQ FSLKLSSVTAADTAVYYCARESDVGYKWNYFDYWGQGTLVTVSSASTKGPS VFPLAPSSKSTSGGTAALGCLVKDYFPEPVTVSWNSGALTSGVHTFPAVLQSS GLYSLSSVVTVPSSSLGTQTYICNVNHKPSNTKVDKKV |
| 64 | G2-K5-Ig1 light chain(G2V_{L}-K5V_{L}-C_{L}) | DIVMTQTPLSLPVSPGEPASISCRSSQNLLHSNGYNYLDWYVQKPGQSPQLLI YLSSNRASGVPDRFSGSGSGTDFTLKISRVEAEDVGVYYCMQALQTPYTFGQ GTKLEIKGGGGSGGGGSEIVLTQSPATLSVSPGERATLSCRASQVVTNYLAWY QHKPGQAPRLLIFDMSIRAAGIPARFSGSGSGTEFTLTISSLQSEDFAVYYCQH RSNWPIFTFGQGTKVEIKRTVAAPSVFIFPPSDEQLKSGTASVVCLLNNFYPRE AKVQWKVDNALQSGNSQESVTEQDSKDSTYSLSSTLTLSKADYEKHKVYAC EVTHQGLSSPVTKSFNRGEC |
| 65 | K5-G2-Ig1 partial heavy chain (K5V_{H}-G2V_{H}-IgG1C_{H}1) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSASTKGPEVQLVESGGGLVRPGGSLRLSCVASGLTF SNAWMYWVRQAPGKGLEWLGRIRSYSDGGTTDYAALVEGRFTISRDDSINT VFLQMNNLKSEDTAVYYCGFPSGDFYPASWGQGTLVTVSSASTKGPSVFPLA |
| | | PSSKSTSGGTAALGCLVKDYFPEPVTVSWNSGALTSGVHTFPAVLQSSGLYSL SSVVTVPSSSLGTQTYICNVNHKPSNTKVDKKV |
| 66 | K5-G2-Ig1 light chain (K5V_{L}-G2V_{L}-C_{L}) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KTVAAPDIVMTQTPLSLPVSPGEPASISCRSSQNLLHSNGYNYLDWYVQKPGQ SPQLLIYLSSNRASGVPDRFSGSGSGTDFTLKISRVEAEDVGVYYCMQALQTP YTFGQGTKLEIKRTVAAPSVFIFPPSDEQLKSGTASVVCLLNNFYPREAKVQW KVDNALQSGNSQESVTEQDSKDSTYSLSSTLTLSKADYEKHKVYACEVTHQG LSSPVTKSFNRGEC |
| 67 | K5-G7-Ig1partial heavy chain (K5V_{H}-G7V_{H}-IgG1C_{H}1) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSASTKGPEVQLLESGGGLVQPGGSLRLSCAASGFIFS GYWMSWVRQAPGKGLEWVANIKQDGSEQYYVDSVKGRFTISRDNAKSSLY LQMNSLRTEDTAIFYCARDRGIKMGSVWYPSFDLWGQGTMVTVSSASTKGP SVFPLAPSSKSTSGGTAALGCLVKDYFPEPVTVSWNSGALTSGVHTFPAVLQS SGLYSLSSVVTVPSSSLGTQTYICNVNHKPSNTKVDKKV |
| 68 | K5-G7-Ig1 light chain (K5V_{L}-G7V_{L}-C L) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KTVAAPEIVLTQSPSSLSASVGDRVTITCRASRSISNYLNWYQQKPGQPPKLLIY AASTLQSGVPSRFRGSGSGAHFTLIISSLQPEDFASYYCQQSYSAPRTFGGGTR LEIKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSF NRGEC |

**Table 7-2: Partial heavy chain and light chain sequences of exemplary Bs4Abformat bispecific antibody specifically binding to O2 antigen and O1 antigen**

| **SEQ ID NO:** | **Description** | **Sequence** |
|---|---|---|
| 69 | G7-K5scFv-Ig1 partial heavy chain (G7V_{H}-C_{H}1-K5scFv) | EVQLLESGGGLVQPGGSLRLSCAASGFIFSGYWMSWVRQAPGKGLEWVANI KQDGSEQYYVDSVKGRFTISRDNAKSSLYLQMNSLRTEDTAIFYCARDRGIK MGSVWYPSFDLWGQGTMVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSNTKVDKKVEPKSCGGGGSGGGGSQVQLQESGPGLVKPSETLSLTCT VSGGSIDTYYWNWIRQPPGKCLEWIANIHQSGTVYYNPSLKSRVTMSVDTSK NQFSLKLSSVTAADTAVYYCARESDVGYKWNYFDYWGQGTLVTVSSGGGG SGGGGSGGGGSGGGGSEIVLTQSPATLSVSPGERATLSCRASQVVTNYLAWY QHKPGQAPRLLIFDMSIRAAGIPARFSGSGSGTEFTLTISSLQSEDFAVYYCQH RSNWPIFTFGCGTKVEIK |
| 70 | G7-K5scFv-Ig1 light chain (G7V_{L}-C_{L}) | EIVLTQSPSSLSASVGDRVTITCRASRSISNYLNWYQQKPGQPPKLLIYAASTL QSGVPSRFRGSGSGAHFTLIISSLQPEDFASYYCQQSYSAPRTFGGGTRLEIKRT VAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC |
| 71 | G2-K5scFv-I g1 partial heavy chain(G2V_{H}-C_{H}1-K5scFv) | EVQLVESGGGLVRPGGSLRLSCVASGLTFSNAWMYWVRQAPGKGLEWLGRI RSYSDGGTTDYAALVEGRFTISRDDSINTVFLQMNNLKSEDTAVYYCGFPSG DFYPASWGQGTLVTVSSASTKGPSVFPLAPSSKSTSGGTAALGCLVKDYFPEP VTVSWNSGALTSGVHTFPAVLQSSGLYSLSSVVTVPSSSLGTQTYICNVNHKP SNTKVDKKVEPKSCGGGGSGGGGSQVQLQESGPGLVKPSETLSLTCTVSGGSI DTYYWNWIRQPPGKCLEWIANIHQSGTVYYNPSLKSRVTMSVDTSKNQFSLK LSSVTAADTAVYYCARESDVGYKWNYFDYWGQGTLVTVSSGGGGSGGGGS GGGGSGGGGSEIVLTQSPATLSVSPGERATLSCRASQVVTNYLAWYQHKPGQ APRLLIFDMSIRAAGIPARFSGSGSGTEFTLTISSLQSEDFAVYYCQHRSNWPIF TFGCGTKVEIK |
| 72 | G2-K5scFv-Ig1 light chain (G2V_{L}-C_{L}) | DIVMTQTPLSLPVSPGEPASISCRSSQNLLHSNGYNYLDWYVQKPGQSPQLLI YLSSNRASGVPDRFSGSGSGTDFTLKISRVEAEDVGVYYCMQALQTPYTFGQ GTKLEIKRTVAAPSVFIFPPSDEQLKSGTASVVCLLNNFYPREAKVQWKVDNA |
| | | LQSGNSQESVTEQDSKDSTYSLSSTLTLSKADYEKHKVYACEVTHQGLSSPVT KSFNRGEC |
| 73 | K5-G2scFv-I g1 partial heavy chain(K5V_{H}-C_{H}1-G2scFv) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSASTKGPSVFPLAPSSKSTSGGTAALGCLVKDYFPE PVTVSWNSGALTSGVHTFPAVLQSSGLYSLSSVVTVPSSSLGTQTYICNVNHK PSNTKVDKKVEPKSCGGGGSGGGGSEVQLVESGGGLVRPGGSLRLSCVASGL TFSNAWMYWVRQAPGKGLEWLGRIRSYSDGGTTDYAALVEGRFTISRDDSIN TVFLQMNNLKSEDTAVYYCGFPSGDFYPASWGQGTLVTVSSGGGGSGGGGS GGGGSGGGGSDIVMTQTPLSLPVSPGEPASISCRSSQNLLHSNGYNYLDWYVQ KPGQSPQLLIYLSSNRASGVPDRFSGSGSGTDFTLKISRVEAEDVGVYYCMQA LQTPYTFGQGTKLEIK |
| 74 | K5-G2scFv-Ig1 light chain (K5V_{L}-C_{L}) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GEC |
| 75 | K5-G7scFv-I g1 partial heavy chain(K5V_{H}-C_{H}1-G7scFv) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSASTKGPSVFPLAPSSKSTSGGTAALGCLVKDYFPE PVTVSWNSGALTSGVHTFPAVLQSSGLYSLSSVVTVPSSSLGTQTYICNVNHK PSNTKVDKKVEPKSCGGGGSGGGGSEVQLLESGGGLVQPGGSLRLSCAASGFI FSGYWMSWVRQAPGKGLEWVANIKQDGSEQYYVDSVKGRFTISRDNAKSSL YLQMNSLRTEDTAIFYCARDRGIKMGSVWYPSFDLWGQGTMVTVSSGGGGS GGGGSGGGGSGGGGSEIVLTQSPSSLSASVGDRVTITCRASRSISNYLNWYQQ KPGQPPKLLIYAASTLQSGVPSRFRGSGSGAHFTLIISSLQPEDFASYYCQQSYS APRTFGGGTRLEIK |
| 74 | K5-G7scFv-Ig1 light chain (K5V_{L}-C_{L}) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GEC |

**Table 7-3: Partial heavy chain and light chain sequences of exemplary Hetero H, CrossMabformat bispecific antibody specifically binding to O2 antigen and O1 antigen**

| **SEQ ID NO:** | **Description** | **Sequence** |
|---|---|---|
| 76 | G7-K5-CrossMab-Ig1 partial heavy chain (G7V_{H}-C_{L}) | EVQLLESGGGLVQPGGSLRLSCAASGFIFSGYWMSWVRQAPGKGLEWVANI KQDGSEQYYVDSVKGRFTISRDNAKSSLYLQMNSLRTEDTAIFYCARDRGIK MGSVWYPSFDLWGQGTMVTVSSASVAAPSVFIFPPSDEQLKSGTASVVCLLN NFYPREAKVQWKVDNALQSGNSQESVTEQDSKDSTYSLSSTLTLSKADYEKH KVYACEVTHQGLSSPVTKSFNRGEC |
| 77 | G7-K5-CrossMab-Ig1 light chain (G7V_{L}-C_{H}1) | EIVLTQSPSSLSASVGDRVTITCRASRSISNYLNWYQQKPGQPPKLLIYAASTL QSGVPSRFRGSGSGAHFTLIISSLQPEDFASYYCQQSYSAPRTFGGGTRLEIKSS ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSC |
| 78 | G7-K5-CrossMab-Ig1 partial heavy chain (K5V_{H}-IgG1C_{H}1) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSASTKGPSVFPLAPSSKSTSGGTAALGCLVKDYFPE PVTVSWNSGALTSGVHTFPAVLQSSGLYSLSSVVTVPSSSLGTQTYICNVNHK PSNTKVDKKV |
| 74 | G7-K5-CrossMab-Ig1 light chain (K5V_{L}-C_{L}) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR |
| | | GEC |
| 79 | G2-K5-Crossmab-Ig1 partial heavy chain (G2V_{H}-C_{L}) | EVQLVESGGGLVRPGGSLRLSCVASGLTFSNAWMYWVRQAPGKGLEWLGRI RSYSDGGTTDYAALVEGRFTISRDDSINTVFLQMNNLKSEDTAVYYCGFPSG DFYPASWGQGTLVTVSSASVAAPSVFIFPPSDEQLKSGTASVVCLLNNFYPRE AKVQWKVDNALQSGNSQESVTEQDSKDSTYSLSSTLTLSKADYEKHKVYAC EVTHQGLSSPVTKSFNRGEC |
| 80 | G2-K5-Crossmab-Ig1 light chain (G2V_{L}-C_{H}1) | DIVMTQTPLSLPVSPGEPASISCRSSQNLLHSNGYNYLDWYVQKPGQSPQLLI YLSSNRASGVPDRFSGSGSGTDFTLKISRVEAEDVGVYYCMQALQTPYTFGQ GTKLEIKSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKK VEPKSC |
| 78 | G2-K5-Crossmab-Ig1 partial heavy chain (K5V_{H}-IgG1C_{H}1) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSASTKGPSVFPLAPSSKSTSGGTAALGCLVKDYFPE PVTVSWNSGALTSGVHTFPAVLQSSGLYSLSSVVTVPSSSLGTQTYICNVNHK PSNTKVDKKV |
| 74 | G2-K5-Crossmab-Ig1 light chain (K5V_{L}-C_{L}) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GEC |

**Table 7-4: Partial heavy chain and light chain sequences of exemplary scFv-Fab IgGformat bispecific antibody specifically binding to O2 antigen and O1 antigen**

| **SEQ ID NO:** | **Description** | **Sequence** |
|---|---|---|
| 81 | G7-K5Xmab-Iglpartia 1 heavy chain (G7V_{H}-IgG1C_{H}1) | EVQLLESGGGLVQPGGSLRLSCAASGFIFSGYWMSWVRQAPGKGLEWVANI KQDGSEQYYVDSVKGRFTISRDNAKSSLYLQMNSLRTEDTAIFYCARDRGIK MGSVWYPSFDLWGQGTMVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSDTKVDKKV |
| 83 | G7-K5Xmab-Ig1 light chain (G7V_{L}-C_{L}) | EIVLTQSPSSLSASVGDRVTITCRASRSISNYLNWYQQKPGQPPKLLIYAASTL QSGVPSRFRGSGSGAHFTLIISSLQPEDFASYYCQQSYSAPRTFGGGTRLEIKRT VAAPSVFIFPPSDEQLKSGTASVVCLLNNFYPREAKVQWKVDNALQSGNSQE SVTEQDSKDSTYSLSNTLTLSKADYEKHKVYACEVTHQGLSSPVTKSFNRGE C |
| 82 | G7-K5Xmab-Iglpartia 1 heavy chain (K5scFv) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSGKPGSGKPGSGKPGSGKPGSEIVLTQSPATLSVSPG ERATLSCRASQVVTNYLAWYQHKPGQAPRLLIFDMSIRAAGIPARFSGSGSGT EFTLTISSLQSEDFAVYYCQHRSNWPIFTFGQGTKVEIK |
| 84 | G2-K5Xmab-Iglpartia 1 heavy chain (G2V_{H}-IgG1C_{H}1) | EVQLVESGGGLVRPGGSLRLSCVASGLTFSNAWMYWVRQAPGKGLEWLGRI RSYSDGGTTDYAALVEGRFTISRDDSINTVFLQMNNLKSEDTAVYYCGFPSG DFYPASWGQGTLVTVSSASTKGPSVFPLAPSSKSTSGGTAALGCLVKDYFPEP VTVSWNSGALTSGVHTFPAVLQSSGLYSLSSVVTVPSSSLGTQTYICNVNHKP SDTKVDKKV |
| 85 | G2-K5Xmab-Ig1 light chain (G2V_{L}-C_{L}) | DIVMTQTPLSLPVSPGEPASISCRSSQNLLHSNGYNYLDWYVQKPGQSPQLLI YLSSNRASGVPDRFSGSGSGTDFTLKISRVEAEDVGVYYCMQALQTPYTFGQ GTKLEIKRTVAAPSVFIFPPSDEQLKSGTASVVCLLNNFYPREAKVQWKVDNA LQSGNSQESVTEQDSKDSTYSLSNTLTLSKADYEKHKVYACEVTHQGLSSPV TKSFNRGEC |
| 82 | G2-K5Xmab-Iglpartia 1 heavy chain (K5scFv) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSGKPGSGKPGSGKPGSGKPGSEIVLTQSPATLSVSPG |
| | | ERATLSCRASQVVTNYLAWYQHKPGQAPRLLIFDMSIRAAGIPARFSGSGSGT EFTLTISSLQSEDFAVYYCQHRSNWPIFTFGQGTKVEIK |

**Table 8-1: Full-length heavy chain and light chain sequences of exemplary DVD-Ig format bispecific antibody specifically binding to O2 antigen and O1 antigen**

| **SEQ ID NO:** | **Description** | **Sequence** |
|---|---|---|
| 86 | G7-K5-Ig1 heavy chain (G7V_{H}-K5V_{H}-IgG1C_{H} ) | EVQLLESGGGLVQPGGSLRLSCAASGFIFSGYWMSWVRQAPGKGLEWVANI KQDGSEQYYVDSVKGRFTISRDNAKSSLYLQMNSLRTEDTAIFYCARDRGIK MGSVWYPSFDLWGQGTMVTVSSGGGGSGGGGSQVQLQESGPGLVKPSETLS LTCTVSGGSIDTYYWNWIRQPPGKGLEWIANIHQSGTVYYNPSLKSRVTMSV DTSKNQFSLKLSSVTAADTAVYYCARESDVGYKWNYFDYWGQGTLVTVSS ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNK ALPAPIEKTISKAKGQPREPQVYTLPPSREEMTKNQVSLTCLVKGFYPSDIAVE WESNGQPENNYKTTPPVLDSDGSFFLYSKLTVDKSRWQQGNVFSCSVMHEA LHNHYTQKSLSLSPGK |
| 62 | G7-K5-Ig1 light chain (G7V_{L}-K5V_{L}-C_{L}) | EIVLTQSPSSLSASVGDRVTITCRASRSISNYLNWYQQKPGQPPKLLIYAASTL QSGVPSRFRGSGSGAHFTLIISSLQPEDFASYYCQQSYSAPRTFGGGTRLEIKG GGGSGGGGSEIVLTQSPATLSVSPGERATLSCRASQVVTNYLAWYQHKPGQA PRLLIFDMSIRAAGIPARFSGSGSGTEFTLTISSLQSEDFAVYYCQHRSNWPIFT FGQGTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQGLS SPVTKSFNRGEC |
| 87 | G2-K5-Ig1 heavy chain (G2V_{H}-K5V_{H}-IgG1C_{H}) | EVQLVESGGGLVRPGGSLRLSCVASGLTFSNAWMYWVRQAPGKGLEWLGRI RSYSDGGTTDYAALVEGRFTISRDDSINTVFLQMNNLKSEDTAVYYCGFPSG DFYPASWGQGTLVTVSSGGGGSGGGGSQVQLQESGPGLVKPSETLSLTCTVS GGSIDTYYWNWIRQPPGKGLEWIANIHQSGTVYYNPSLKSRVTMSVDTSKNQ FSLKLSSVTAADTAVYYCARESDVGYKWNYFDYWGQGTLVTVSSASTKGPS VFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCP APELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIE KTISKAKGQPREPQVYTLPPSREEMTKNQVSLTCLVKGFYPSDIAVEWESNGQ PENNYKTTPPVLDSDGSFFLYSKLTVDKSRWQQGNVFSCSVMHEALHNHYT QKSLSLSPGK |
| 64 | G2-K5-Ig1 light chain (G2V_{L}-K5V_{L}-C_{L}) | DIVMTQTPLSLPVSPGEPASISCRSSQNLLHSNGYNYLDWYVQKPGQSPQLLI YLSSNRASGVPDRFSGSGSGTDFTLKISRVEAEDVGVYYCMQALQTPYTFGQ GTKLEIKGGGGSGGGGSEIVLTQSPATLSVSPGERATLSCRASQVVTNYLAWY QHKPGQAPRLLIFDMSIRAAGIPARFSGSGSGTEFTLTISSLQSEDFAVYYCQH RSNWPIFTFGQGTKVEIKRTVAAPSVFIFPPSDEQLKSGTASVVCLLNNFYPRE AKVQWKVDNALQSGNSQESVTEQDSKDSTYSLSSTLTLSKADYEKHKVYAC EVTHQGLSSPVTKSFNRGEC |
| 88 | K5-G2-Ig1 heavy chain (K5V_{H}-G2V_{H}-IgG1C_{H}) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSASTKGPEVQLVESGGGLVRPGGSLRLSCVASGLTF SNAWMYWVRQAPGKGLEWLGRIRSYSDGGTTDYAALVEGRFTISRDDSINT VFLQMNNLKSEDTAVYYCGFPSGDFYPASWGQGTLVTVSSASTKGPSVFPLA PSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKA KGQPREPQVYTLPPSREEMTKNQVSLTCLVKGFYPSDIAVEWESNGQPENNY |
| | | KTTPPVLDSDGSFFLYSKLTVDKSRWQQGNVFSCSVMHEALHNHYTQKSLSL SPGK |
| 66 | K5-G2-Ig1 light chain (K5V_{L}-G2V_{L}-C_{L}) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KTVAAPDIVMTQTPLSLPVSPGEPASISCRSSQNLLHSNGYNYLDWYVQKPGQ SPQLLIYLSSNRASGVPDRFSGSGSGTDFTLKISRVEAEDVGVYYCMQALQTP YTFGQGTKLEIKRTVAAPSVFIFPPSDEQLKSGTASVVCLLNNFYPREAKVQW KVDNALQSGNSQESVTEQDSKDSTYSLSSTLTLSKADYEKHKVYACEVTHQG LSSPVTKSFNRGEC |
| 89 | K5-G7-Ig1 heavy chain (K5V_{H}-G7V_{H}-IgG1C_{H}) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSASTKGPEVQLLESGGGLVQPGGSLRLSCAASGFIFS GYWMSWVRQAPGKGLEWVANIKQDGSEQYYVDSVKGRFTISRDNAKSSLY LQMNSLRTEDTAIFYCARDRGIKMGSVWYPSFDLWGQGTMVTVSSASTKGP SVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPC PAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIE KTISKAKGQPREPQVYTLPPSREEMTKNQVSLTCLVKGFYPSDIAVEWESNGQ PENNYKTTPPVLDSDGSFFLYSKLTVDKSRWQQGNVFSCSVMHEALHNHYT QKSLSLSPGK |
| 68 | K5-G7-Ig1 light chain (K5V_{L}-G7V_{L}-C_{L}) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KTVAAPEIVLTQSPSSLSASVGDRVTITCRASRSISNYLNWYQQKPGQPPKLLIY AASTLQSGVPSRFRGSGSGAHFTLIISSLQPEDFASYYCQQSYSAPRTFGGGTR LEIKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSF NRGEC |
| 115 | K5-G2-Ig1-LS heavy chain (K5V_{H}-G2V_{H}-IgG1C_{H}-LS) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSASTKGPEVQLVESGGGLVRPGGSLRLSCVASGLT FSNAWMYWVRQAPGKGLEWLGRIRSYSDGGTTDYAALVEGRFTISRDDSINT VFLQMNNLKSEDTAVYYCGFPSGDFYPASWGQGTLVTVSSASTKGPSVFPLA PSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKA KGQPREPQVYTLPPSREEMTKNQVSLTCLVKGFYPSDIAVEWESNGQPENNY KTTPPVLDSDGSFFLYSKLTVDKSRWQQGNVFSCSVLHEALHSHYTQKSLSLS PGK |
| 66 | K5-G2-Ig1-LS light chain (K5V_{L}-G2V_{L}-C_{L}) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KTVAAPDIVMTQTPLSLPVSPGEPASISCRSSQNLLHSNGYNYLDWYVQKPGQ SPQLLIYLSSNRASGVPDRFSGSGSGTDFTLKISRVEAEDVGVYYCMQALQTP YTFGQGTKLEIKRTVAAPSVFIFPPSDEQLKSGTASVVCLLNNFYPREAKVQW KVDNALQSGNSQESVTEQDSKDSTYSLSSTLTLSKADYEKHKVYACEVTHQG LSSPVTKSFNRGEC |

**Table 8-2: Full-length heavy chain and light chain sequences of exemplary Bs4Abformat bispecific antibody specifically binding to O2 antigen and O1 antigen**

| **SEQ ID NO:** | **Description** | **Sequence** |
|---|---|---|
| 90 | G7-K5scFv-Ig1 heavy chain (G7V_{H}-C_{H}1-K5scFv-Ig | EVQLLESGGGLVQPGGSLRLSCAASGFIFSGYWMSWVRQAPGKGLEWVANI KQDGSEQYYVDSVKGRFTISRDNAKSSLYLQMNSLRTEDTAIFYCARDRGIK MGSVWYPSFDLWGQGTMVTVSSASTKGPSVFPLAPSSKSTSGGTAALGCLV |
| | G1Fc) | KDYFPEPVTVSWNSGALTSGVHTFPAVLQSSGLYSLSSVVTVPSSSLGTQTYI CNVNHKPSNTKVDKKVEPKSCGGGGSGGGGSQVQLQESGPGLVKPSETLSL TCTVSGGSIDTYYWNWIRQPPGKCLEWIANIHQSGTVYYNPSLKSRVTMSVD TSKNQFSLKLSSVTAADTAVYYCARESDVGYKWNYFDYWGQGTLVTVSSG GGGSGGGGSGGGGSGGGGSEIVLTQSPATLSVSPGERATLSCRASQVVTNYL AWYQHKPGQAPRLLIFDMSIRAAGIPARFSGSGSGTEFTLTISSLQSEDFAVYY CQHRSNWPIFTFGCGTKVEIKGGGGSGGGGSDKTHTCPPCPAPELLGGPSVFL FPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPR EPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK |
| 70 | G7-K5scFv-Ig1 light chain (G7V_{L}-C_{L}) | EIVLTQSPSSLSASVGDRVTITCRASRSISNYLNWYQQKPGQPPKLLIYAASTL QSGVPSRFRGSGSGAHFTLIISSLQPEDFASYYCQQSYSAPRTFGGGTRLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC |
| 91 | G2-K5scFv-Ig1 heavy chain (G2V_{H}-C_{H}1-K5scFv-Ig G1Fc) | EVQLVESGGGLVRPGGSLRLSCVASGLTFSNAWMYWVRQAPGKGLEWLGR IRSYSDGGTTDYAALVEGRFTISRDDSINTVFLQMNNLKSEDTAVYYCGFPSG DFYPASWGQGTLVTVSSASTKGPSVFPLAPSSKSTSGGTAALGCLVKDYFPEP VTVSWNSGALTSGVHTFPAVLQSSGLYSLSSVVTVPSSSLGTQTYICNVNHKP SNTKVDKKVEPKSCGGGGSGGGGSQVQLQESGPGLVKPSETLSLTCTVSGGS IDTYYWNWIRQPPGKCLEWIANIHQSGTVYYNPSLKSRVTMSVDTSKNQFSL KLSSVTAADTAVYYCARESDVGYKWNYFDYWGQGTLVTVSSGGGGSGGG GSGGGGSGGGGSEIVLTQSPATLSVSPGERATLSCRASQVVTNYLAWYQHKP GQAPRLLIFDMSIRAAGIPARFSGSGSGTEFTLTISSLQSEDFAVYYCQHRSNW PIFTFGCGTKVEIKGGGGSGGGGSDKTHTCPPCPAPELLGGPSVFLFPPKPKDT LMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPP SREEMTKNQVSLTCLVKGFYPSDIAVEWESNGQPENNYKTTPPVLDSDGSFF LYSKLTVDKSRWQQGNVFSCSVMHEALHNHYTQKSLSLSPGK |
| 72 | G2-K5scFv-Ig1 light chain (G2V_{L}-C_{L}) | DIVMTQTPLSLPVSPGEPASISCRSSQNLLHSNGYNYLDWYVQKPGQSPQLLI YLSSNRASGVPDRFSGSGSGTDFTLKISRVEAEDVGVYYCMQALQTPYTFGQ GTKLEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGLSSP VTKSFNRGEC |
| 92 | K5-G2scFv-Ig1 heavy chain (K5V_{H}-C_{H}1-G2scFv-Ig G1Fc) | QVQLQESGPGLVKPSETLSLTCTVSGGSIDTYYWNWIRQPPGKGLEWIANIH QSGTVYYNPSLKSRVTMSVDTSKNQFSLKLSSVTAADTAVYYCARESDVGY KWNYFDYWGQGTLVTVSSASTKGPSVFPLAPSSKSTSGGTAALGCLVKDYF PEPVTVSWNSGALTSGVHTFPAVLQSSGLYSLSSVVTVPSSSLGTQTYICNVN HKPSNTKVDKKVEPKSCGGGGSGGGGSEVQLVESGGGLVRPGGSLRLSCVA SGLTFSNAWMYWVRQAPGKGLEWLGRIRSYSDGGTTDYAALVEGRFTISRD DSINTVFLQMNNLKSEDTAVYYCGFPSGDFYPASWGQGTLVTVSSGGGGSG GGGSGGGGSGGGGSDIVMTQTPLSLPVSPGEPASISCRSSQNLLHSNGYNYLD WYVQKPGQSPQLLIYLSSNRASGVPDRFSGSGSGTDFTLKISRVEAEDVGVY YCMQALQTPYTFGQGTKLEIKGGGGSGGGGSDKTHTCPPCPAPELLGGPSVF LFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQP REPQVYTLPPSREEMTKNQVSLTCLVKGFYPSDIAVEWESNGQPENNYKTTP PVLDSDGSFFLYSKLTVDKSRWQQGNVFSCSVMHEALHNHYTQKSLSLSPG K |
| 74 | K5-G2scFv-Ig1 light chain (K5V_{L}-C_{L}) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFN RGEC |
| 93 | K5-G7scFv-Ig1 heavy chain (K5V_{H}-C_{H}1-G7scFv-Ig G1Fc) | QVQLQESGPGLVKPSETLSLTCTVSGGSIDTYYWNWIRQPPGKGLEWIANIH QSGTVYYNPSLKSRVTMSVDTSKNQFSLKLSSVTAADTAVYYCARESDVGY KWNYFDYWGQGTLVTVSSASTKGPSVFPLAPSSKSTSGGTAALGCLVKDYF PEPVTVSWNSGALTSGVHTFPAVLQSSGLYSLSSVVTVPSSSLGTQTYICNVN HKPSNTKVDKKVEPKSCGGGGSGGGGSEVQLLESGGGLVQPGGSLRLSCAA SGFIFSGYWMSWVRQAPGKGLEWVANIKQDGSEQYYVDSVKGRFTISRDNA KSSLYLQMNSLRTEDTAIFYCARDRGIKMGSVWYPSFDLWGQGTMVTVSSG GGGSGGGGSGGGGSGGGGSEIVLTQSPSSLSASVGDRVTITCRASRSISNYLN WYQQKPGQPPKLLIYAASTLQSGVPSRFRGSGSGAHFTLIISSLQPEDFASYYC QQSYSAPRTFGGGTRLEIKGGGGSGGGGSDKTHTCPPCPAPELLGGPSVFLFP PKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREP QVYTLPPSREEMTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 74 | K5-G7scFv-Ig1 light chain (K5V_{L}-C_{L}) | EIVLTQSPATLSVSPGERATLSCRASQVVTNYLAWYQHKPGQAPRLLIFDMSI RAAGIPARFSGSGSGTEFTLTISSLQSEDFAVYYCQHRSNWPIFTFGQGTKVEI KRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFN RGEC |

**Table 8-3: Full-length heavy chain and light chain sequences of exemplary Hetero H, CrossMabformat bispecific antibody specifically binding to O2 antigen and O1 antigen**

| **SEQ ID NO:** | **Description** | **Sequence** |
|---|---|---|
| 94 | G7-K5-CrossMab-Ig1 heavy chain (G7V_{H}-C_{L}-IgG1Fc-hole) | EVQLLESGGGLVQPGGSLRLSCAASGFIFSGYWMSWVRQAPGKGLEWVANIK QDGSEQYYVDSVKGRFTISRDNAKSSLYLQMNSLRTEDTAIFYCARDRGIKMG SVWYPSFDLWGQGTMVTVSSASVAAPSVFIFPPSDEQLKSGTASVVCLLNNFY PREAKVQWKVDNALQSGNSQESVTEQDSKDSTYSLSSTLTLSKADYEKHKVY ACEVTHQGLSSPVTKSFNRGECDKTHTCPPCPAPELLGGPSVFLFPPKPKDTLMI SRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVCTLPPSRDEL TKNQVSLSCAVKGFYPSDIAVEWESNGQPENNYKTTPPVLDSDGSFFLVSKLT VDKSRWQQGNVFSCSVMHEALHNHYTQKSLSLSPGK |
| 77 | G7-K5-CrossMab-Ig1 light chain (G7V_{L}-C_{H}1) | EIVLTQSPSSLSASVGDRVTITCRASRSISNYLNWYQQKPGQPPKLLIYAASTLQ SGVPSRFRGSGSGAHFTLIISSLQPEDFASYYCQQSYSAPRTFGGGTRLEIKSSAS TKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSC |
| 95 | G7-K5-CrossMab-Ig1 heavy chain (K5V_{H}-IgG1C_{H}-knob) | QVQLQESGPGLVKPSETLSLTCTVSGGSIDTYYWNWIRQPPGKGLEWIANIHQS GTVYYNPSLKSRVTMSVDTSKNQFSLKLSSVTAADTAVYYCARESDVGYKWN YFDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVV VDVSHEDPEVKFNWYVDGVEVHNAKTKPREEQYNSTYRVVSVLTVLHQDWL NGKEYKCKVSNKALPAPIEKTISKAKGQPREPQVYTLPPCRDELTKNQVSLWC LVKGFYPSDIAVEWESNGQPENNYKTTPPVLDSDGSFFLYSKLTVDKSRWQQG NVFSCSVMHEALHNHYTQKSLSLSPGK |
| 74 | G7-K5-CrossMab-Ig1 light chain (K5V_{L}-C_{L}) | EIVLTQSPATLSVSPGERATLSCRASQVVTNYLAWYQHKPGQAPRLLIFDMSIR AAGIPARFSGSGSGTEFTLTISSLQSEDFAVYYCQHRSNWPIFTFGQGTKVEIKR TVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC |
| 96 | G2-K5-Crossmab-Ig1 heavy chain (G2V_{H}-C_{L}-IgG1Fc-hole) | EVQLVESGGGLVRPGGSLRLSCVASGLTFSNAWMYWVRQAPGKGLEWLGRIR SYSDGGTTDYAALVEGRFTISRDDSINTVFLQMNNLKSEDTAVYYCGFPSGDF YPASWGQGTLVTVSSASVAAPSVFIFPPSDEQLKSGTASVVCLLNNFYPREAKV QWKVDNALQSGNSQESVTEQDSKDSTYSLSSTLTLSKADYEKHKVYACEVTH QGLSSPVTKSFNRGECDKTHTCPPCPAPELLGGPSVFLFPPKPKDTLMISRTPEV |
| | | TCVVVDVSHEDPEVKFNWYVDGVEVHNAKTKPREEQYNSTYRVVSVLTVLH QDWLNGKEYKCKVSNKALPAPIEKTISKAKGQPREPQVCTLPPSRDELTKNQV SLSCAVKGFYPSDIAVEWESNGQPENNYKTTPPVLDSDGSFFLVSKLTVDKSR WQQGNVFSCSVMHEALHNHYTQKSLSLSPGK |
| 80 | G2-K5-Crossmab-Ig1 light chain (G2V_{L}-C_{H}1) | DIVMTQTPLSLPVSPGEPASISCRSSQNLLHSNGYNYLDWYVQKPGQSPQLLIY LSSNRASGVPDRFSGSGSGTDFTLKISRVEAEDVGVYYCMQALQTPYTFGQGT KLEIKSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPK SC |
| 95 | G2-K5-Crossmab-Ig1 heavy chain (K5V_{H}-IgG1C_{H}-knob) | QVQLQESGPGLVKPSETLSLTCTVSGGSIDTYYWNWIRQPPGKGLEWIANIHQS GTVYYNPSLKSRVTMSVDTSKNQFSLKLSSVTAADTAVYYCARESDVGYKWN YFDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVV VDVSHEDPEVKFNWYVDGVEVHNAKTKPREEQYNSTYRVVSVLTVLHQDWL NGKEYKCKVSNKALPAPIEKTISKAKGQPREPQVYTLPPCRDELTKNQVSLWC LVKGFYPSDIAVEWESNGQPENNYKTTPPVLDSDGSFFLYSKLTVDKSRWQQG NVFSCSVMHEALHNHYTQKSLSLSPGK |
| 74 | G2-K5-Crossmab-Ig1 light chain (K5V_{L}-C_{L}) | EIVLTQSPATLSVSPGERATLSCRASQVVTNYLAWYQHKPGQAPRLLIFDMSIR AAGIPARFSGSGSGTEFTLTISSLQSEDFAVYYCQHRSNWPIFTFGQGTKVEIKR TVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC |

**Table 8-4: Full-length heavy chain and light chain sequences of exemplary IgG-(scFv)₂format bispecific antibody specifically binding to O2 antigen and O1 antigen**

| **SEQ ID NO:** | **Description** | **Sequence** |
|---|---|---|
| 97 | G7-Ig1-K5scFv2 heavy chain (G7V_{H}-IgG1C_{H}-K5scFv) | EVQLLESGGGLVQPGGSLRLSCAASGFIFSGYWMSWVRQAPGKGLEWVANI KQDGSEQYYVDSVKGRFTISRDNAKSSLYLQMNSLRTEDTAIFYCARDRGIK MGSVWYPSFDLWGQGTMVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSNTKVDKKVEPKSCDKTHTCPPCPAPELLGGPSVFLFPPKPKDTLMIS RTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSRDEL TKNQVSLTCLVKGFYPSDIAVEWESNGQPENNYKTTPPVLDSDGSFFLYSKLT VDKSRWQQGNVFSCSVMHEALHNHYTQKSLSLSPGGGGGSGGGGTGGGGSQ VQLQESGPGLVKPSETLSLTCTVSGGSIDTYYWNWIRQPPGKGLEWIANIHQS GTVYYNPSLKSRVTMSVDTSKNQFSLKLSSVTAADTAVYYCARESDVGYKW NYFDYWGQGTLVTVSSGGGGSGGGGSGGGGSGGGGSEIVLTQSPATLSVSPG ERATLSCRASQVVTNYLAWYQHKPGQAPRLLIFDMSIRAAGIPARFSGSGSGT EFTLTISSLQSEDFAVYYCQHRSNWPIFTFGQGTKVEIK |
| 83 | G7-Ig1-K5scFv2 light chain (G7V_{L}-C_{L}) | EIVLTQSPSSLSASVGDRVTITCRASRSISNYLNWYQQKPGQPPKLLIYAASTL QSGVPSRFRGSGSGAHFTLIISSLQPEDFASYYCQQSYSAPRTFGGGTRLEIKRT VAAPSVFIFPPSDEQLKSGTASVVCLLNNFYPREAKVQWKVDNALQSGNSQE SVTEQDSKDSTYSLSNTLTLSKADYEKHKVYACEVTHQGLSSPVTKSFNRGE C |
| 98 | G2-Ig1-K5scFv2 heavy chain (G2V_{H}-IgG1C_{H}-K5scFv) | EVQLVESGGGLVRPGGSLRLSCVASGLTFSNAWMYWVRQAPGKGLEWLGRI RSYSDGGTTDYAALVEGRFTISRDDSINTVFLQMNNLKSEDTAVYYCGFPSG DFYPASWGQGTLVTVSSASTKGPSVFPLAPSSKSTSGGTAALGCLVKDYFPEP VTVSWNSGALTSGVHTFPAVLQSSGLYSLSSVVTVPSSSLGTQTYICNVNHKP SNTKVDKKVEPKSCDKTHTCPPCPAPELLGGPSVFLFPPKPKDTLMISRTPEVT CVVVDVSHEDPEVKFNWYVDGVEVHNAKTKPREEQYNSTYRVVSVLTVLH QDWLNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTVDKS RWQQGNVFSCSVMHEALHNHYTQKSLSLSPGGGGGSGGGGTGGGGSQVQLQ |
| | | ESGPGLVKPSETLSLTCTVSGGSIDTYYWNWIRQPPGKGLEWIANIHQSGTVY YNPSLKSRVTMSVDTSKNQFSLKLSSVTAADTAVYYCARESDVGYKWNYFD YWGQGTLVTVSSGGGGSGGGGSGGGGSGGGGSEIVLTQSPATLSVSPGERAT LSCRASQVVTNYLAWYQHKPGQAPRLLIFDMSIRAAGIPARFSGSGSGTEFTL TISSLQSEDFAVYYCQHRSNWPIFTFGQGTKVEIK |
| 85 | G2-Ig1-K5scFv2 light chain (G2V_{L}-C_{L}) | DIVMTQTPLSLPVSPGEPASISCRSSQNLLHSNGYNYLDWYVQKPGQSPQLLI YLSSNRASGVPDRFSGSGSGTDFTLKISRVEAEDVGVYYCMQALQTPYTFGQ GTKLEIKRTVAAPSVFIFPPSDEQLKSGTASVVCLLNNFYPREAKVQWKVDNA LQSGNSQESVTEQDSKDSTYSLSNTLTLSKADYEKHKVYACEVTHQGLSSPV TKSFNRGEC |

**Table 8-5: Full-length heavy chain and light chain sequences of exemplary scFv-Fab IgGformat bispecific antibody specifically binding to O2 antigen and O1 antigen**

| **SEQ ID NO:** | **Description** | **Sequence** |
|---|---|---|
| 99 | G7-K5Xmab-Ig1 heavy chain (G7V_{H}-IgG1C_{H}) | EVQLLESGGGLVQPGGSLRLSCAASGFIFSGYWMSWVRQAPGKGLEWVANI KQDGSEQYYVDSVKGRFTISRDNAKSSLYLQMNSLRTEDTAIFYCARDRGIK MGSVWYPSFDLWGQGTMVTVSSASTKGPSVFPLAPSSKSTSGGTAALGCLV KDYFPEPVTVSWNSGALTSGVHTFPAVLQSSGLYSLSSVVTVPSSSLGTQTYI CNVNHKPSDTKVDKKVEPKSCDKTHTCPPCPAPELLGGPSVFLFPPKPKDTL MISRTPEVTCVVVDVSHEDPEVKFNWYVDGVEVHNAKTKPREEEYNSTYRV VSVLTVLHQDWLNGKEYKCKVSNKALPAPIEKTISKAKGQPREPQVYTLPPS REEMTKNQVSLTCDVSGFYPSDIAVEWESDGQPENNYKTTPPVLDSDGSFFL YSKLTVDKSRWEQGDVFSCSVMHEALHNHYTQKSLSLSPGK |
| 100 | G7-K5Xmab-Ig1 heavy chain (K5scFv-IgG1Fc) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSGKPGSGKPGSGKPGSGKPGSEIVLTQSPATLSVSP GERATLSCRASQVVTNYLAWYQHKPGQAPRLLIFDMSIRAAGIPARFSGSGS GTEFTLTISSLQSEDFAVYYCQHRSNWPIFTFGQGTKVEIKEPKSSDKTHTCPP CPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPA PIEKTISKAKGQPREPQVYTLPPSREQMTKNQVKLTCLVKGFYPSDIAVEWES NGQPENNYKTTPPVLDSDGSFFLYSKLTVDKSRWQQGNVFSCSVMHEALHN HYTQKSLSLSPGK |
| 83 | G7-K5Xmab-Ig1 light chain (G7V_{L}-C_{L}) | EIVLTQSPSSLSASVGDRVTITCRASRSISNYLNWYQQKPGQPPKLLIYAASTL QSGVPSRFRGSGSGAHFTLIISSLQPEDFASYYCQQSYSAPRTFGGGTRLEIKR TVAAPSVFIFPPSDEQLKSGTASVVCLLNNFYPREAKVQWKVDNALQSGNSQ ESVTEQDSKDSTYSLSNTLTLSKADYEKHKVYACEVTHQGLSSPVTKSFNRG EC |
| 101 | G2-K5Xmab-Ig1 heavy chain (G2V_{H}-IgG1C_{H}) | EVQLVESGGGLVRPGGSLRLSCVASGLTFSNAWMYWVRQAPGKGLEWLGRI RSYSDGGTTDYAALVEGRFTISRDDSINTVFLQMNNLKSEDTAVYYCGFPSG DFYPASWGQGTLVTVSSASTKGPSVFPLAPSSKSTSGGTAALGCLVKDYFPEP VTVSWNSGALTSGVHTFPAVLQSSGLYSLSSVVTVPSSSLGTQTYICNVNHKP SDTKVDKKVEPKSCDKTHTCPPCPAPELLGGPSVFLFPPKPKDTLMISRTPEVT CVVVDVSHEDPEVKFNWYVDGVEVHNAKTKPREEEYNSTYRVVSVLTVLH QDWLNGKEYKCKVSNKALPAPIEKTISKAKGQPREPQVYTLPPSREEMTKNQ VSLTCDVSGFYPSDIAVEWESDGQPENNYKTTPPVLDSDGSFFLYSKLTVDKS RWEQGDVFSCSVMHEALHNHYTQKSLSLSPGK |
| 100 | G2-K5Xmab-Ig1 heavy chain (K5scFv-IgG1Fc) | QVQLQESGPGLVKPSETLSLTCTVSGGSIDTYYWNWIRQPPGKGLEWIANIHQ SGTVYYNPSLKSRVTMSVDTSKNQFSLKLSSVTAADTAVYYCARESDVGYK WNYFDYWGQGTLVTVSSGKPGSGKPGSGKPGSGKPGSEIVLTQSPATLSVSP GERATLSCRASQVVTNYLAWYQHKPGQAPRLLIFDMSIRAAGIPARFSGSGS GTEFTLTISSLQSEDFAVYYCQHRSNWPIFTFGQGTKVEIKEPKSSDKTHTCPP CPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPA |
| | | PIEKTISKAKGQPREPQVYTLPPSREQMTKNQVKLTCLVKGFYPSDIAVEWES NGQPENNYKTTPPVLDSDGSFFLYSKLTVDKSRWQQGNVFSCSVMHEALHN HYTQKSLSLSPGK |
| 85 | G2-K5Xmab-Ig1 light chain (G2V_{L}-C_{L}) | DIVMTQTPLSLPVSPGEPASISCRSSQNLLHSNGYNYLDWYVQKPGQSPQLLI YLSSNRASGVPDRFSGSGSGTDFTLKISRVEAEDVGVYYCMQALQTPYTFGQ GTKLEIKRTVAAPSVFIFPPSDEQLKSGTASVVCLLNNFYPREAKVQWKVDN ALQSGNSQESVTEQDSKDSTYSLSNTLTLSKADYEKHKVYACEVTHQGLSSP VTKSFNRGEC |

**Table 9: Exemplary popypeptide linker (or linker) sequences**

| **SEQ ID NO:** | **Sequence** |
|---|---|
| 102 | GGGGSGGGGS |
| 103 | GGGGSGGGGSGGGGSGGGGS |
| 104 | GGGGSGGGGSGGGGS |
| 105 | ASTKGP |
| 106 | TVAAP |
| 107 | EPKSDKTGGGGSGGGGS |
| 108 | EPKSCGKTGGGGSGGGGS |
| 109 | EPKSCGGGGSGGGGS |
| 110 | GGGGSGGGGSEPKSDKTHTCPPCP |
| 111 | GGGGSGGGGSCPPCP |
| 112 | GGGGSGGGGSDKTHTCPPCP |
| 113 | GKPGSGKPGSGKPGSGKPGS |
| 114 | GGGGSGGGGTGGGGS |

### Combination of antibodies specifically binding toK. pneumoniaeO2 antigen and K. pneumoniaeO1 antigen

In one aspect, the present application provides a pharmaceutical composition comprising (i) an antibody or antigen-binding fragment specifically binding to*K*. ***pneumoniae*O2** antigenand (ii) an antibody or antigen-binding fragment specifically binding toK. *pneumoniae*O1 antigen.

In some embodiments, there is provided a pharmaceutical composition comprising (i) an antibody or antigen-binding fragment specifically binding toK. ***pneumoniae*O2** antigenand (ii) an antibody or antigen-binding fragment specifically binding toK. *pneumoniae*O1*,* wherein: the antibody or antigen-binding fragment specifically binding toK. *pneumoniae*O2 antigen comprises: (a) a heavy chain variable domain (V_{H}) comprising anHC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 12, and an LC-CDR3 comprising the amino acid sequence of SEQ ID NO: 15, or a variant thereof comprising up to about 5 amino acid substitutions in the LC-CDRs; or (b) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 9, an LC-CDR2 comprising the amino acid sequence of SEQ ID NO: 12, and an LC-CDR3 comprising the amino acid sequence of SEQ ID NO: 16, or a variant thereof comprising up to about 5 amino acid substitutions in the LC-CDRs; or (c) a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 10, an LC-CDR2 comprising the amino acid sequence of SEQ ID NO: 13, and an LC-CDR3 comprising the amino acid sequence of SEQ ID NO: 16, or a variant thereof comprising up to about 5 amino acid substitutions in the LC-CDRs; or (d) a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16, or a variant thereof comprising up to about 5 amino acid substitutions in the LC-CDRs; or (e) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7, or a variant thereof comprising up to about 5 amino acid substitutions in the HC-CDRs; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15, or a variant thereof comprising up to about 5 amino acid substitutions in the LC-CDRs.

In some embodiments, there is provided a pharmaceutical composition comprising (i) an antibody or antigen-binding fragment specifically binding to*K*. *pneumoniae*O2 antigenand (ii) an antibody or antigen-binding fragment specifically binding to*K*. *pneumoniae*O1, wherein: the antibody or antigen-binding fragment specifically binding to*K*. *pneumoniae*O1 antigen comprises: (a) a V_{H} comprising a HC-CDR1comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39, or a variant thereof comprising up to about 5 amino acid substitutions in the HC-CDRs; and a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45, or a variant thereof comprising up to about 5 amino acid substitutions in the LC-CDRs; or (b) a V_{H} comprising a HC-CDR1comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40, or a variant thereof comprising up to about 5 amino acid substitutions in the HC-CDRs; and a V_{L} comprising an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46, or a variant thereof comprising up to about 5 amino acid substitutions in the LC-CDRs.

In some embodiments, the antibody or antigen-binding fragment specifically binding toK. *pneumoniae*O2 antigenin the pharmaceutical compositioncomprises:a V_{H} comprising the amino acid sequence of SEQ ID NO: 17; and a V_{L} comprising the amino acid sequence of SEQ ID NO: 21. In some embodiments, the antibody or antigen-binding fragmentspecifically binding to*K*. *pneumoniae*O2 antigen comprises:a V_{H} comprising an HC-CDR1, an HC-CDR2, and an HC-CDR3 of a V_{H} comprising the amino acid sequence of SEQ ID NO: 17; and a V_{L} comprising an LC-CDR1, an LC-CDR2, and an LC-CDR3 of a V_{L} comprising the amino acid sequence of SEQ ID NO: 21.

In some embodiments, the antibody or antigen-binding fragment specifically binding toK. *pneumoniae*O2 antigenin the pharmaceutical compositioncomprises:a V_{H} comprising the amino acid sequence of SEQ ID NO: 18; and a V_{L} comprising the amino acid sequence of SEQ ID NO: 22. In some embodiments, the antibody or antigen-binding fragment specifically binding to*K*. *pneumoniae*O2 antigen comprises:a V_{H} comprising an HC-CDR1, an HC-CDR2, and an HC-CDR3 of a V_{H} comprising the amino acid sequence of SEQ ID NO: 18; and a V_{L} comprising an LC-CDR1, an LC-CDR2, and an LC-CDR3 of a V_{L} comprising the amino acid sequence of SEQ ID NO: 22.

In some embodiments, the antibody or antigen-binding fragment specifically binding toK. *pneumoniae*O2 antigenin the pharmaceutical compositioncomprises:a V_{H} comprising the amino acid sequence of SEQ ID NO: 19; and a V_{L} comprising the amino acid sequence of SEQ ID NO: 23. In some embodiments, the antibody or antigen-binding fragment specifically binding to*K*. *pneumoniae*O2 antigen comprises:a V_{H} comprising an HC-CDR1, an HC-CDR2, and an HC-CDR3 of a V_{H} comprising the amino acid sequence of SEQ ID NO: 19; and a V_{L} comprising an LC-CDR1, an LC-CDR2, and an LC-CDR3 of a V_{L} comprising the amino acid sequence of SEQ ID NO: 23.

In some embodiments, the antibody or antigen-binding fragment specifically binding toK. *pneumoniae*O2 antigenin the pharmaceutical compositioncomprises:a V_{H} comprising the amino acid sequence of SEQ ID NO: 19; and a V_{L} comprising the amino acid sequence of SEQ ID NO: 24. In some embodiments, the antibody or antigen-binding fragment specifically binding to*K*. *pneumoniae*O2 antigen comprises:a V_{H} comprising an HC-CDR1, an HC-CDR2, and an HC-CDR3 of a V_{H} comprising the amino acid sequence of SEQ ID NO: 19; and a V_{L} comprising an LC-CDR1, an LC-CDR2, and an LC-CDR3 of a V_{L} comprising the amino acid sequence of SEQ ID NO: 24.

In some embodiments, the antibody or antigen-binding fragment specifically binding to*K*. *pneumoniae*O2 antigenin the pharmaceutical compositioncomprises:a V_{H} comprising the amino acid sequence of SEQ ID NO: 20; and a V_{L} comprising the amino acid sequence of SEQ ID NO: 25. In some embodiments, the antibody or antigen-binding fragment specifically binding to*K*. *pneumoniae*O2 antigen comprises:a V_{H} comprising an HC-CDR1, an HC-CDR2, and an HC-CDR3 of a V_{H} comprising the amino acid sequence of SEQ ID NO: 20; and a V_{L} comprising an LC-CDR1, an LC-CDR2, and an LC-CDR3 of a V_{L} comprising the amino acid sequence of SEQ ID NO: 25.

In some embodiments, the antibody or antigen-binding fragment specifically binding toK. *pneumoniae*O1 antigenin the pharmaceutical compositioncomprises:a V_{H} comprising the amino acid sequence of SEQ ID NO: 47; and a V_{L} comprising the amino acid sequence of SEQ ID NO: 49. In some embodiments, the antibody or antigen-binding fragment specifically binding to*K*. *pneumoniae*O1 antigen comprises:a V_{H} comprising an HC-CDR1, an HC-CDR2, and an HC-CDR3 of a V_{H} comprising the amino acid sequence of SEQ ID NO: 47; and a V_{L} comprising an LC-CDR1, an LC-CDR2, and an LC-CDR3 of a V_{L} comprising the amino acid sequence of SEQ ID NO: 49.

In some embodiments, the antibody or antigen-binding fragment specifically binding toK. *pneumoniae*O1 antigenin the pharmaceutical compositioncomprises:a V_{H} comprising the amino acid sequence of SEQ ID NO: 48; and a V_{L} comprising the amino acid sequence of SEQ ID NO: 50. In some embodiments, the antibody or antigen-binding fragment specifically binding to*K*. *pneumoniae*O1 antigen comprises:a V_{H} comprising an HC-CDR1, an HC-CDR2, and an HC-CDR3 of a V_{H} comprising the amino acid sequence of SEQ ID NO: 48; and a V_{L} comprising an LC-CDR1, an LC-CDR2, and an LC-CDR3 of a V_{L} comprising the amino acid sequence of SEQ ID NO: 50.

In some embodiments, there is provided a pharmaceutical composition comprising: an antibody or antigen-binding fragment specifically binding to*K*. *pneumoniae*O2 antigenand an antibody or antigen-binding fragment specifically binding to*K*. *pneumoniae*O1, wherein the antibody or antigen-binding fragment specifically binding to*K*. *pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16; and wherein the antibody or antigen-binding fragment specifically binding to*K*. *pneumoniae*O1 antigen comprises: a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45.

In some embodiments, there is provided a pharmaceutical composition comprising: an antibody or antigen-binding fragment specifically binding to*K*. *pneumoniae*O2 antigenand an antibody or antigen-binding fragment specifically binding to*K*. *pneumoniae*O1, wherein the antibody or antigen-binding fragment specifically binding to*K*. *pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16; and wherein the antibody or antigen-binding fragment specifically binding to*K*. *pneumoniae*O1 antigen comprises: a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

In some embodiments, there is provided a pharmaceutical composition comprising: an antibody or antigen-binding fragment specifically binding to*K*. *pneumoniae*O2 antigenand an antibody or antigen-binding fragment specifically binding to*K*. *pneumoniae*O1, wherein the antibody or antigen-binding fragment specifically binding to*K*. *pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15; and wherein the antibody or antigen-binding fragment specifically binding toK. *pneumoniae*O1 antigen comprises: a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45.

In some embodiments, there is provided a pharmaceutical composition comprising: an antibody or antigen-binding fragment specifically binding to*K*. *pneumoniae*O2 antigenand an antibody or antigen-binding fragment specifically binding to*K*. *pneumoniae*O1, wherein the antibody or antigen-binding fragment specifically binding to*K*. *pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15; and wherein the antibody or antigen-binding fragment specifically binding toK. *pneumoniae*O1 antigen comprises: a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

### Binding affinity

Binding affinity can be indicated by Kd, Koff, Kon, or Ka. The term "Koff', as used herein, is intended to refer to the off-rate constant for dissociation of an antigen-binding fragment from theantigen-binding fragment/antigen complex, as determined from a kinetic selection set up. The term "Kon", as used herein, is intended to refer to the on-rate constant for association of an antibody to the antigen to form the antigen-binding fragment/antigen complex. The term dissociation constant "Kd", as used herein, refers to thedissociation constant of a particular antibody-antigen interaction, and describes the concentration of antigen required to occupy one half of all of the antigen-binding fragments present in a solution of antibody molecules at equilibrium, and is equal to Koff/Kon. The measurement of Kd presupposes that all binding agents are in solution. In the case where the antigen-binding fragment is tethered to a cell wall, e.g., in a yeast expression system, the corresponding equilibrium rate constant is expressed as EC50, which gives a good approximation of Kd. The affinity constant, Ka, is the inverse of the dissociation constant, Kd.

The dissociation constant (Kd) is used as an indicator showing affinity of antigen-binding fragment moieties to antigens. For example, easy analysis is possible by the Scatchard method using antibodies marked with a variety of marker agents, as well as by using Biacore (made by Amersham Biosciences), analysis of biomolecular interactions by surface plasmon resonance, according to the user's manual and attached kit. The Kd value that can be derived using these methods is expressed in units of M.An antibody that specifically binds to a target may have a Kd of, for example, ≤ 10⁻⁷M, ≤ 10⁻⁸M, ≤ 10⁻⁹M, ≤ 10⁻¹⁰M, ≤ 10⁻¹¹M, ≤ 10⁻¹²M, or ≤ 10⁻¹³M.

Binding specificity of the antibody can be determined experimentally by methods known in the art. Such methods comprise, but are not limited to, Western blots, ELISA-, RIA-, ECL-, IRMA-, EIA-, BIAcore-tests and peptide scans.

In some embodiments, the antibody orantigen-binding fragment specifically binding to*K*. *pneumoniae* O2 antigenspecifically binds to a target *K. pneumoniae* O2 antigen with a Kdof about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M).Thus in some embodiments, the Kd of the binding between the antibody orantigen-binding fragment specifically binding to*K*. *pneumoniae* O2 antigen and *K. pneumoniae* O2 antigen, is about 10⁻⁷ M to about 10⁻¹³ M, about 1×10⁻⁷M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷ M to about 10⁻⁹M, about 10⁻⁸ M to about 10⁻¹³ M, about 1×10⁻⁸ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1×10⁻¹³ M, about 5×10⁻⁹ M to about 1×10⁻¹² M, about 5×10⁻⁹ M to about 1× 0⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1×10⁻¹³ M, about 5×10⁻¹⁰ M to about 1×10⁻¹² M, about 5×10⁻¹⁰ M to about 1×10⁻¹¹ M, about 10⁻¹⁰ M to about10⁻¹³ M, about 1×10⁻¹⁰ M to about 5×10⁻¹³ M, about 1×10⁻¹⁰ M to about 1×10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the antibody orantigen-binding fragment specifically binding to*K*. *pneumoniae* O2 antigen and *K. pneumoniae* O2 antigen is about 10⁻⁷ M to about 10⁻¹³ M.

In some embodiments, the Kd of the binding between the antibody orantigen-binding fragment specifically binding toK. *pneumoniae* O2 antigenand a non-target is higher than the Kd of the binding between the antibody orantigen-binding fragment specifically binding toK. *pneumoniae* O2 antigen and the target, and is herein referred to in some embodiments as the binding affinity of the antibody orantigen-binding fragment specifically binding toK. *pneumoniae* O2 antigento the target (*e.g.*,*K. pneumoniae* O2 antigen) is higher than that to a non-target. In some embodiments, the non-target is an antigen that is not *K. pneumoniae* O2 antigen. In some embodiments, the Kd of the binding between the antibody orantigen-binding fragment specifically binding to*K*. *pneumoniae* O2 antigen and non-*K*. *pneumoniae* O2 antigentarget can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the antibody orantigen-binding fragment specifically binding toK. *pneumoniae* O2 antigen and a target *K. pneumoniae* O2 antigen.

In some embodiments, the antibody orantigen-binding fragment specifically binding to*K*. *pneumoniae* O1 antigenspecifically binds to a target *K. pneumoniae* O1 antigen with a Kdof about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M).Thus in some embodiments, the Kd of the binding between the antibody orantigen-binding fragment specifically binding to*K*. *pneumoniae* O1 antigen and *K. pneumoniae* O1 antigen, is about 10⁻⁷ M to about 10⁻¹³ M, about 1×10⁻⁷ M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷ M to about 10⁻⁹M, about 10⁻⁸ M to about 10⁻¹³ M, about 1×10⁻⁸ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1×10⁻¹³ M, about 5×10⁻⁹ M to about 1×10⁻¹² M, about 5×10⁻⁹ M to about 1×10⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1×10⁻¹³ M, about 5×10⁻¹⁰ M to about 1×10⁻¹² M, about 5×10⁻¹⁰ M to about 1×10⁻¹¹ M, about 10⁻¹⁰ M to about10⁻¹³ M, about 1×10⁻¹⁰ M to about 5×10⁻¹³ M, about 1×10⁻¹⁰ M to about 1×10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the antibody orantigen-binding fragment specifically binding to*K*. *pneumoniae* O1 antigen and*K*. *pneumoniae* O1 antigenis about 10⁻⁷ M to about 10⁻¹³ M.

In some embodiments, the Kd of the binding between the antibody orantigen-binding fragment specifically binding to*K*. *pneumoniae* O1 antigen and a non-target is higher than the Kd of the binding between the antibody orantigen-binding fragment specifically binding toK. *pneumoniae* O1 antigen and the target, and is herein referred to in some embodiments as the binding affinity of the antibody orantigen-binding fragment specifically binding toK. *pneumoniae* O1 antigento the target (*e.g.*,*K. pneumoniae* O1 antigen) is higher than that to a non-target. In some embodiments, the non-target is an antigen that is not *K. pneumoniae* O1 antigen. In some embodiments, the Kd of the binding between the antibody orantigen-binding fragment specifically binding to*K*. *pneumoniae* O1 antigen and a non-*K*. *pneumoniae* O1 antigentarget can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the antibody orantigen-binding fragment specifically binding to*K*. *pneumoniae* O1 antigen and a target *K. pneumoniae* O1 antigen.

### Nucleic Acids

Nucleic acid molecules encoding the antibody orantigen-binding fragment specifically binding to*K*. *pneumoniae* O2 antigen, the antibody orantigen-binding fragment specifically binding to*K*. *pneumoniae* O1 antigen and the bispecific antibody are also contemplated. In some embodiments, there is provided a nucleic acid (or a set of nucleic acids) encoding a full-length antibody specifically binding to*K*. *pneumoniae* O2 antigen or the antibody specifically binding toK. *pneumoniae* O1 antigen or the bispecific antibody specifically binding to*K*. *pneumoniae* O2 antigen and O1 antigen, including any of the full-length antibody specifically binding to*K*. *pneumoniae* O2 antigen or the full-length antibody specifically binding to*K*. *pneumoniae* O1 antigen or the full-length bispecific antibody specifically binding to*K*. *pneumoniae* O2 antigen and O1 antigen described herein. In some embodiments, thenucleic acid (or a set of nucleic acids) encoding the antibody or antigen-binding fragment or bispecific antibody described herein may further comprises a nucleic acid sequence encoding a peptide tag (such as protein purification tag, *e*.*g*., His-tag, HA tag).

Also contemplated here are isolated host cells comprising anantibody specifically binding to*K*. *pneumoniae* O2 antigen, antibody specifically binding to*K*. *pneumoniae* O1 antigen, or bispecific antibody specifically binding to*K*. *pneumoniae* O2 antigen and O1 antigen; or a nucleic acid molecular coding the antibodies described herein; or a vector comprising a nucleic acid molecular described herein.

The present application also includes variants to these nucleic acid sequences. For example, the variants include nucleotide sequences that hybridize to the nucleic acid sequences encoding the antibodies or antigen-binding fragments or bispecific antibodies of the present application under at least moderately stringent hybridization conditions.

The present application also provides vectors in which a nucleic acid of the present application is inserted.

In brief summary, the expression of an antibody or antigen-binding fragment or bispecific antibody by a natural or synthetic nucleic acid encoding the antibody or antigen-binding fragment or bispecific antibody can be achieved by inserting the nucleic acid into an appropriate expression vector, such that the nucleic acid is operably linked to 5' and 3' regulatory elements, including for example a promoter (*e*.*g*., a lymphocyte-specific promoter) and a 3' untranslated region (UTR). The vectors can be suitable for replication and integration in eukaryotic host cells. Typical cloning and expression vectors contain transcription and translation terminators, initiation sequences, and promoters useful for regulation of the expression of the desired nucleic acid sequence.

The nucleic acids of the present application may also be used for nucleic acid immunization and gene therapy, using standard gene delivery protocols. Methods for gene delivery are known in the art. See, *e.g.*, U.S. Pat. Nos. 5,399,346; 5,580,859; 5,589,466, incorporated by reference herein in their entireties. In some embodiments, the application provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Green and Sambrook (2013, Molecular Cloning: A Laboratory Manual, Cold Spring Harbor Laboratory, New York), and in other virology and molecular biology manuals. Viruses which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers (*see*, *e.g.*, WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In some embodiments, lentivirus vectors are used. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

Additional promoter elements, *e*.*g*., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the application should not be limited to the use of constitutive promoters. Inducible promoters are also contemplated as part of the application. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

In some embodiments, the expression of the antibody or antigen-binding fragment or bispecific antibody is inducible. In some embodiments, a nucleic acid sequence encoding the antibody or antigen-binding fragment or bispecific antibody is operably linked to an inducible promoter, including any inducible promoter described herein.

### Inducible promoters

The use of an inducible promoter provides a molecular switch capable of turning on expression of the polynucleotide sequence which it is operatively linked when such expression is desired, or turning off the expression when expression is not desired. Exemplary inducible promoter systems for use in eukaryotic cells include, but are not limited to, hormone-regulated elements (e.g., *see* Mader, S. and White, J. H. (1993) Proc. Natl. Acad. Sci. USA 90:5603-5607), synthetic ligand-regulated elements (see, *e.g.*, Spencer, D. M. et al 1993) Science 262: 1019-1024) and ionizing radiation-regulated elements (*e.g.*, *see* Manome, Y. et al. (1993) Biochemistry 32: 10607-10613; Datta, R. et al. (1992) Proc. Natl. Acad. Sci. USA 89: 1014- 10153). Further exemplary inducible promoter systems for use in in vitro or in vivo mammalian systems are reviewed in Gingrich et al. (1998) Annual Rev. Neurosci 21:377-405. In some embodiments, the inducible promoter system for use to express the antibody or antigen-binding fragment or bispecific antibody is the Tet system. In some embodiments, the inducible promoter system for use to express the antibody or antigen-binding fragment or bispecific antibody is the lac repressor system from *E. coli.*

An exemplary inducible promoter system for use in the present application is the Tet system. Such systems are based on the Tet system described by Gossen et al. (1993). In an exemplary embodiment, a polynucleotide of interest is under the control of a promoter that comprises one or more Tet operator (TetO) sites. In the inactive state, Tet repressor (TetR) will bind to the TetO sites and repress transcription from the promoter. In the active state, *e*.*g*., in the presence of an inducing agent such as tetracycline (Tc), anhydrotetracycline, doxycycline (Dox), or an active analog thereof, the inducing agent causes release of TetR from TetO, thereby allowing transcription to take place. Doxycycline is a member of the tetracycline family of antibiotics having the chemical name of 1-dimethylamino-2,4a,5,7,12-pentahydroxy-11-methyl-4,6-dioxo-1,4a,11,11a,12,12a-hexahyd rotetracene-3 -carboxamide.

In one embodiment, a TetR is codon-optimized for expression in mammalian cells, *e*.*g*., murine or human cells. Most amino acids are encoded by more than one codon due to the degeneracy of the genetic code, allowing for substantial variations in the nucleotide sequence of a given nucleic acid without any alteration in the amino acid sequence encoded by the nucleic acid. However, many organisms display differences in codon usage, also known as "codon bias" (i.e., bias for use of a particular codon(s) for a given amino acid). Codon bias often correlates with the presence of a predominant species of tRNA for a particular codon, which in turn increases efficiency of mRNA translation. Accordingly, a coding sequence derived from a particular organism (*e*.*g*., a prokaryote) may be tailored for improved expression in a different organism (*e*.*g*., a eukaryote) through codon optimization.

Other specific variations of the Tet system include the following "Tet-Off" and "Tet-On" systems. In the Tet-Off system, transcription is inactive in the presence of Tc or Dox. In that system, a tetracycline-controlled transactivator protein (tTA), which is composed of TetR fused to the strong transactivating domain of VP16 from Herpes simplex virus, regulates expression of a target nucleic acid that is under transcriptional control of a tetracycline-responsive promoter element (TRE). The TRE is made up of TetO sequence concatamers fused to a promoter (commonly the minimal promoter sequence derived from the human cytomegalovirus (hCMV) immediate-early promoter). In the absence of Tc or Dox, tTA binds to the TRE and activates transcription of the target gene. In the presence of Tc or Dox, tTA cannot bind to the TRE, and expression from the target gene remains inactive.

Conversely, in the Tet-On system, transcription is active in the presence of Tc or Dox. The Tet-On system is based on a reverse tetracycline-controlled transactivator, rtTA. Like tTA, rtTA is a fusion protein comprised of the TetR repressor and the VP16 transactivation domain. However, a four amino acid change in the TetR DNA binding moiety alters rtTA's binding characteristics such that it can only recognize the tetO sequences in the TRE of the target transgene in the presence of Dox. Thus, in the Tet-On system, transcription of the TRE-regulated target gene is stimulated by rtTA only in the presence of Dox.

Another inducible promoter system is the lac repressor system from *E. coli* (*See* Brown et al., Cell 49:603-612 (1987)). The lac repressor system functions by regulating transcription of a polynucleotide of interest operably linked to a promoter comprising the lac operator (lacO). The lac repressor (lacR) binds to LacO, thus preventing transcription of the polynucleotide of interest. Expression of the polynucleotide of interest is induced by a suitable inducing agent, *e*.*g*., isopropyl-β-D-thiogalactopyranoside (IPTG).

In order to assess the expression of a polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, β-galactosidase, chloramphenicol acetyl transferase, secreted alkaline phosphatase, or the green fluorescent protein gene (*e.g.*, Ui-Tel et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

In some embodiments, there is provided nucleic acid encoding an antibody or antigen-binding fragment or bispecific antibody according to any of the antigen-binding fragment, antigen-binding protein or bispecific molecule described herein. In some embodiments, the nucleic acid comprises one or more nucleic acid sequences encoding the heavy and light chains of the antibody or antigen-binding fragment or bispecific antibody. In some embodiments, each of the one or more nucleic acid sequences is contained in separate vectors. In some embodiments, at least some of the nucleic acid sequences are contained in the same vector. In some embodiments, all of the nucleic acid sequences are contained in the same vector. Vectors may be selected, for example, from the group consisting of mammalian expression vectors and viral vectors (such as those derived from retroviruses, adenoviruses, adeno-associated viruses, herpes viruses, and lentiviruses).

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Green and Sambrook (2013, Molecular Cloning: A Laboratory Manual, Cold Spring Harbor Laboratory, New York). In some embodiments, the introduction of a polynucleotide into a host cell is carried out by calcium phosphate transfection.

Biological methods for introducing a polynucleotide of interest into a host cell include the use of DNA and RNA vectors. Viral vectors, and especially retroviral vectors, have become the most widely used method of inserting genes into mammalian, e.g., human cells. Other viral vectors can be derived from lentivirus, poxviruses, herpes simplex virus 1, adenoviruses and adeno-associated viruses, and the like. *See*, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (*in vitro*, *ex vivo* or *in vivo*)*.* In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Regardless of the method used to introduce exogenous nucleic acids into a host cell or otherwise expose a cell to the inhibitor of the present application, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, *e.g.*, by immunological means (ELISAs and Western blots) or by assays described herein to identify agents falling within the scope of the application.

### Preparation of antibodies or antigen-binding fragments or bispecific antibodies

In some embodiments, the antibody or antigen-binding fragment (e.g. an antibody or antigen-binding fragment specifically binding to *K. pneumoniae* O2 antigen or O1 antigen) is a monoclonal antibody. In some embodiments, the antibody or antigen-binding fragment or bispecific antibodyis derived from a monoclonal antibody. In some embodiments, the antibody or antigen-binding fragment or bispecific antibody comprises V_{H} and V_{L} domains, or variants thereof, from the monoclonal antibody. In some embodiments, the antibody or antigen-binding fragment or bispecific antibody further comprises C_{H}1 and C_{L} domains, or variants thereof, from the monoclonal antibody.Monoclonal antibodies can be prepared, *e.g.*, using known methods in the art, including hybridoma methods, yeast display,phage display methods, or using recombinant DNA methods. Additionally, exemplary yeast display and phage display methods are described herein and in the Examples below. The bispecific antibodycan be prepared by methods known in the art, including chemical coupling method, hybridoma method, and genetic engineering method, etc..

In a hybridoma method, a hamster, mouse, or other appropriate host animal is typically immunized with an immunizing agent to elicit lymphocytes that produce or are capable of producing antibodies that will specifically bind to the immunizing agent. Alternatively, the lymphocytes can be immunized *in vitro.* The immunizing agent can include a polypeptide or a fusion protein of the protein of interest. Generally, peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Immortalized cell lines are usually transformed mammalian cells, particularly myeloma cells of rodent, bovine, and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which prevents the growth of HGPRT-deficient cells.

In some embodiments, the immortalized cell lines fuse efficiently, support stable high-level expression of antibody by the selected antibody-producing cells, and are sensitive to a medium such as HAT medium. In some embodiments, the immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies.

The culture medium in which the hybridoma cells are cultured can then be assayed for the presence of monoclonal antibodies directed against the polypeptide. The binding specificity of monoclonal antibodies produced by the hybridoma cells can be determined by immunoprecipitation or by an *in vitro* binding assay, such as radioimmunoassay (RIA) or enzyme-linked immunosorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones can be sub cloned by limiting dilution procedures and grown by standard methods. Goding, *supra.* Suitable culture media for this purpose include, for example, Dulbecco's Modified Eagle's Medium and RPMI-1640 medium. Alternatively, the hybridoma cells can be grown *in vivo* as ascites in a mammal.

The monoclonal antibodies secreted by the sub clones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

In some embodiments, according to any of the antibodies or antigen-binding fragments or bispecific antibodies described herein, the antibody or antigen-binding fragment or bispecific antibody comprises sequences from a clone selected from an antibody library (such as a phage library presenting scFv or Fab fragments). The clone may be identified by screening combinatorial libraries for antibody fragments with the desired activity or activities. For example, a variety of methods are known in the art for generating phage display libraries and screening such libraries for antibodies possessing the desired binding characteristics. Such methods are reviewed, *e.g.*, in Hoogenboom et al., Methods in Molecular Biology 178:1-37 (O'Brien et al., ed., Human Press, Totowa, N.J., 2001) and further described, *e.g.*, in McCafferty et al., Nature 348:552-554; Clackson et al., Nature 352: 624-628 (1991); Marks et al., J. Mol. Biol. 222: 581-597 (1992); Marks and Bradbury, Methods in Molecular Biology248: 161-175 (Lo, ed., Human Press, Totowa, N.J., 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of V_{H} and V_{L} genes are separately cloned by polymerase chain reaction (PCR) and recombined randomly in phage libraries, which can then be screened for antigen-binding phage as described in Winter et al., Ann. Rev. Immunol., 12: 433-455 (1994). Phages typically display antibody fragments, either as scFv fragments or as Fab fragments. Libraries from immunized sources provide high-affinity antibodies to the immunogen without the requirement of constructing hybridomas. Alternatively, the naive repertoire can be cloned (*e.g.*, from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro*, as described by Hoogenboom and Winter, J. Mol. Biol., 227: 381-388 (1992). Patent publications describing human antibody phage libraries include, for example: U.S. Pat. No. 5,750,373, and US Patent Publication Nos. 2005/0079574, 2005/0119455, 2005/0266000, 2007/0117126, 2007/0160598, 2007/0237764, 2007/0292936, and 2009/0002360.

The antibodies or antigen-binding fragments or bispecific antibodies can be prepared using phage display to screen libraries for antigen-binding moieties specific to the target antigen (such as *K. pneumoniae*O2 antigen or O1 antigen). The library can be a human scFv phage display library having a diversity of at least 1 × 10⁹ (such as at least about any of 1 × 10⁹, 2.5 ×10⁹, 5 × 10⁹, 7.5 × 10⁹, 1 × 10¹⁰, 2.5 × 10¹⁰, 5 × 10¹⁰, 7.5 × 10¹⁰, or 1 × 10¹¹) unique human antibody fragments. In some embodiments, the library is a naïve human library constructed from DNA extracted from human PMBCs and spleens from healthy donors, encompassing all human heavy and light chain subfamilies. In some embodiments, the library is a naive human library constructed from DNA extracted from PBMCs isolated from patients with various diseases, such as patients with autoimmune diseases, cancer patients, and patients with infectious diseases. In some embodiments, the library is a semi-synthetic human library, wherein heavy chain CDR3 is completely randomized, with all amino acids (with the exception of cysteine) equally likely to be present at any given position (*see*, *e.g.*, Hoet, R.M. et al., Nat. Biotechnol. 23(3):344-348, 2005). In some embodiments, the heavy chain CDR3 of the semi-synthetic human library has a length from about 5 to about 24 (such as about any of 5, 6, 7, 8, 9, 10, 11, 12, 13, 14, 15, 16, 17, 18, 19, 20, 21, 22, 23, or 24) amino acids. In some embodiments, the library is a fully-synthetic phage display library. In some embodiments, the library is a non-human phage display library.

Phage clones that bind to the target antigen (such as *K. pneumoniae*O2 antigen or O1 antigen) with high affinity can be selected by iterative binding of phage to the target antigen, which is bound to a solid support (such as, for example, beads for solution panning or mammalian cells for cell panning), followed by removal of non-bound phage and by elution of specifically bound phage. The bound phage clones are then eluted and used to infect an appropriate host cell, such as *E. coli* XL1-Blue, for expression and purification. The panning can be performed for multiple (such as about any of 2, 3, 4, 5, 6 or more) rounds with solution panning, cell panning, or a combination of both, to enrich for phage clones binding specifically to the target antigen. Enriched phage clones can be tested for specific binding to the target antigen by any methods known in the art, including for example ELISA and FACS.

Monoclonal antibodies and bispecific molecules can also be made by recombinant DNA methods, such as those described in U.S. Patent No. 4,816,567. DNA encoding the monoclonal antibodies of the application can be readily isolated and sequenced using conventional procedures (*e*.*g*., by using oligonucleotide probes that are capable of binding specifically to genes encoding the heavy and light chains of murine antibodies). Hybridoma cells as described above or antigen-specific phage clones of the application can serve as a source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy- and light-chain constant regions and/or framework regions in place of the homologous non-human sequences (U.S. Patent No. 4,816,567; Morrison *et al.*, *supra*)or by covalently joining to the immunoglobulin coding sequence all or part of the coding sequence for a non-immunoglobulin polypeptide. Such a non-immunoglobulin polypeptide can be substituted for the constant regions of an antibody of the application, or can be substituted for the variable domains of one antigen-combining site of an antibody of the application to create a chimeric bivalent antibody. In some embodiments, additional variable domains targeting a different epitope or antigen can be included to generate a chimeric bispecific antibody.

The antigen-binding proteins can be monovalent antibodies. Methods for preparing monovalent antibodies are known in the art. For example, one method involves recombinant expression of immunoglobulin light chain and modified heavy chain. The heavy chain is truncated generally at any point in the Fc region so as to prevent heavy-chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly Fab fragments, can be accomplished using any method known in the art.

Chemical coupling method is the technology that was first applied to prepare bispecific antibodies. In 1985, Brennan used the chemical binding of two monoclonal antibody G1 fragments for the first time to prepare bispecific antibodies (Brennan M, et al. Preparation of bispecific antibodies by chemical recombination of monoclonal immunoglobulinG1 fragments [J] . Science, 1985, 229(4708):81-83). The chemical coupling method mainly comprises two modes, one is to directly couple two monoclonal antibodies or their derivatives to form bispecific antibodies; the other is to dissociate two monoclonal antibodies into free light chainsand heavy chains through various physical and chemical methodsfirstly, and then recombine these light chainsand heavy chains. The advantages of chemical coupling method are rapidness, simple operation and highrecovery rate, but it is also easy to disrupt the antigen-binding domain of antibodies, affect antibody activity, and easy to form polymers.

Preparation of bispecific antibodyby hybridoma cell lines refers to the fusion of two different hybridoma celllines by cell fusion techniques, followed by identification and isolation of cells that can produce antibodies withspecific therapeutics (Kohler, G, et al. Continuous cultures of fused cells secreting antibody of predefined specificity [J] . J Immunol., 2005, 174(5):2453-2455). Because two kinds of hybridoma cells can produce twodifferent light-heavy chains, and the light-heavy chains can be randomly combined, the bispecific antibodyprepared by the method has larger randomness and low preparation efficiency.

Genetic engineering technologies are also currently used to prepare a variety of bispecific antibodies(Roland E K. Antibody-cytokine fusion proteins [J] . Arch Biochem Biophys., 2012, 526(2): 194-205). Editingrecombinant antibodies by genetic engineering can solve the problem of random combination by limiting the binding selectivity of two pairs of light-heavy chain in variety ways. KiH (Knob into hole) and CrossMab aretwo common technologies currently applied to improve the problem of light-heavy chain pairing. KiH technology introduces asymmetric mutation structures into C_{H}3 domain ("knob" mutation refers to substituting a smaller residue with a larger amino acid residue inC_{H}3 domain, whereas "hole" mutation refers to substituting a larger residue with a smaller amino acid residue). The Fc region of engineered bispecific antibodies is more prone toheterodimerization than homodimerization due to steric effects (Ridgway J B, et al. "Knobs-into-holes" engineering of antibody CH3 domains for heavy chain heterodimerization [J] . Protein Eng. 1996, 9(7):617-621). While introducing a Y349C mutation in the glycosylated C_{H}3 domain can lead to the formation of disulfide bonds between glycosylated heavy chains, enhancing the stability of KiH (Kuglstatter A, et al. Structural differences between glycosylated, disulfide-linked heterodimeric knob-into-hole Fc fragment and its homodimeric knob-knob and hole-hole side products [J] . Protein Eng Des Sel. ,2017, 30(9):649-656).

In addition to steric effects, the charge effect of amino acid residues is also used to enhanceheterodimerization between two heavy chains of a bispecific antibody. "Positive electricity" is generated in one chain and negative electricity is generated in the pairedchain through structural simulation and moleculardesign modification, and the formation of heavy chain heterodimer is promoted by the mode that like charges repel each other and unlike charges attractive each other. The formation of heterodimer can be increased to some extent by the mode of respective mutation K409D and D399K, K409D/K392D and D399K/E356K, orE356K/E357K/D399K and K370E/K409D/K439E in two chains (IGAWA T, et al. Methods for producing polypeptides by regulating polypeptide; association: US, 20100015133A1 [P] .2006). Combining the KiH steric effect with the charge effect is also one of thestrategies to further enhance heterodimerization.

The CrossMab technology is a new antibody pairing technology developed by Roche based on KiHtechnology by exchanging the domain of light chain with the heavy chain of one Fab of bispecific antibodies, with no exchange on the other one. The exchanged light chain will contain a portion of the homologous heavy chain fragment,making them unable to pair with the non-exchanged heavy chain, thereby ensuring the correct combination between the light chainsandheavy chains (Schaefer W, et al. Immunoglobulin domain crossover as a generic approach for the production of bispecific IgG antibodies [J]. Proc Natl Acad Sci USA, 2011, 108 (27):11187-11192). Includes the form of "CrossMab Fab", "CrossMab V_{H}-V_{L}" or "CrossMab C_{H}1-C_{L}"etc.

Antibody variable domains with the desired binding specificities (antibody-antigen combining sites) can be fused to immunoglobulin constant-domain sequences. The fusion preferably is with an immunoglobulin heavy-chain constant region, comprising at least part of the hinge, CH2, and CH3 domains. In some embodiments, the heavy-chain constant domain 1(CH1) containing the site necessary for light-chain binding is present in at least one of the fusions. DNAs encoding the immunoglobulin heavy-chain fusions and, if desired, the immunoglobulin light chain, are inserted into separate expression vectors, and are co-transfected into a suitable host organism. In some embodiments, antibody variable domains targeting different epitopes or different antigens can be fused to immunoglobulin constant-domain sequences to generate a chimeric bispecific antibody.

### Human and Humanized Antibodies

The antibodies or antigen-binding fragments or bispecific antibodies can comprise humanized antibody moieties or human antibody moieties. Humanized forms of non-human (e.g., murine) antibody moieties are chimeric immunoglobulins, immunoglobulin chains, or fragments thereof (such as Fv, Fab, Fab', F(ab')₂, scFv, or other antigen-binding subsequences of antibodies) that typically contain minimal sequence derived from non-human immunoglobulin. Humanized antibody moieties include human immunoglobulins, immunoglobulin chains, or fragments thereof (recipient antibody) in which residues from a CDR of the recipient are replaced by residues from a CDR of a non-human species (donor antibody) such as mouse, rat, or rabbit having the desired specificity, affinity, and capacity. In some instances, Fv framework residues of the human immunoglobulin are replaced by corresponding non-human residues. Humanized antibody moieties can also comprise residues that are found neither in the recipient antibody nor in the imported CDR or framework sequences. In general, the humanized antibody can comprise substantially at least one, and typically two, variable domains, in which all or substantially all of the CDR regions correspond to those of a non-human immunoglobulin, and all or substantially all of the FR regions are those of a human immunoglobulin consensus sequence.

Generally, a humanized antibody or humanized antibody moiety has one or more amino acid residues introduced into it from a source that is non-human. These non-human amino acid residues are often referred to as "import" residues, which are typically taken from an "import" variable domain. According to some embodiments, humanization can be essentially performed following the method of Winter and co-workers (Jones et al., Nature, 321: 522-525 (1986); Riechmann et al., Nature, 332: 323-327 (1988); Verhoeyen et al., Science, 239: 1534-1536 (1988)), by substituting rodent CDRs or CDR sequences for the corresponding sequences of a human antibody. Accordingly, such "humanized" antibody moieties are antibody moieties (U.S. Patent No. 4,816,567), wherein substantially less than an intact human variable domain has been substituted by the corresponding sequence from a non-human species. In practice, humanized antibody moieties are typically human antibody moieties in which some CDR residues and possibly some FR residues are substituted by residues from analogous sites in rodent antibodies.

As an alternative to humanization, human antibody moieties can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, e.g., Jakobovits et al., PNAS USA, 90:2551 (1993); Jakobovits et al., Nature, 362:255-258 (1993); Bruggemann et al., Year in Immunol., 7:33 (1993); U.S. Patent Nos. 5,545,806, 5,569,825, 5,591,669; 5,545,807; and WO 97/17852. Alternatively, human antibodies can be made by introducing human immunoglobulin loci into transgenic animals, *e*.*g*., mice in which the endogenous immunoglobulin genes have been partially or completely inactivated. Upon challenge, human antibody production is observed that closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; and 5,661,016, and Marks et al., BiolTechnology, 10: 779-783 (1992); Lonberg et al., Nature, 368: 856-859 (1994); Morrison, Nature, 368: 812-813 (1994); Fishwild et al., Nature Biotechnology, 14: 845-851 (1996); Neuberger, Nature Biotechnology, 14: 826 (1996); Lonberg and Huszar, Intern. Rev. Immunol., 13: 65-93 (1995).

Human antibodies or human antibody moieties may also be generated by *in vitro* activated B cells (see U.S. Patents 5,567,610 and 5,229,275) or by using various techniques known in the art, including phage display libraries. Hoogenboom and Winter, J. Mol. Biol., 227:381 (1991); Marks et al., J. Mol. Biol., 222:581 (1991). The techniques of Cole *et al.* and Boerner *et al.* are also available for the preparation of human monoclonal antibodies. Cole et al., Monoclonal Antibodies and Cancer Therapy, Alan R. Liss, p. 77 (1985) and Boerner et al., J. Immunol., 147(1): 86-95 (1991).

### Variants of antibodies or antigen-binding fragments

In some embodiments, amino acid sequences of the antibodies or antigen-binding fragments(*e*.*g*., antibody specifically binding to*K*. *pneumoniae* O2 antigen or O1 antigen, or bispecific antibody specifically binding to*K*. *pneumoniae*O2 antigen and O1 antigen) variantsprovided herein are contemplated. For example, it may be desirable to improve the binding affinity and/or other biological properties of the antibody or antigen-binding fragment. Amino acid sequences of an antigen-binding entity variants may be prepared by introducing appropriate modifications into the nucleotide sequence encoding the antigen-binding entity, or by peptide synthesis. Such modifications include, for example, deletions from, and/or insertions into and/or substitutions of residues within the amino acid sequences of the antigen-binding entity. Any combination of deletion, insertion, and substitution can be made to arrive at the final construct, provided that the final construct possesses the desired characteristics, *e*.*g*., antigen-binding.

In some embodiments, variants of the antibodies or antigen-binding fragments having one or more amino acid substitutions are provided. Sites of interest for substitutional mutagenesis include the HVRs and FRs. Amino acid substitutions may be introduced into an antibody of interest and the products screened for a desired activity, *e.g*., improved bioactivity, retained/improved antigen binding, decreased immunogenicity, reduced pathogen binding to epithelial cells or improved opsonophagocytic killing (OPK) of pathogens, such as *K. pneumoniae.* In some embodiments, the amino acid substitutions described herein are limited to "exemplary substitutions" shown in Table 10 of this application. In some embodiments, the amino acid substitutions are limited to "preferred substitutions" shown in Table 10 of this application.

**Conservative substitutions are shown in Table 10 below**

| **TABLE 10: CONSERVATIVE SUBSTITITIONS** | | |
|---|---|---|
| **Original Residue** | **Exemplary Substitutions** | **Preferred Substitutions** |
| Ala (A) | Val; Leu; Ile | Val |
| Arg (R) | Lys; Gln; Asn | Lys |
| Asn (N) | Gln; His; Asp, Lys; Arg | Gin |
| Asp (D) | Glu; Asn | Glu |
| Cys (C) | Ser; Ala | Ser |
| Gin (Q) | Asn; Glu | Asn |
| Glu (E) | Asp; Gln | Asp |
| Gly (G) | Ala | Ala |
| His (H) | Asn; Gin; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped into different classes according to common side-chain properties:
a. hydrophobic: Norleucine, Met, Ala, Val, Leu, Ile;
b. neutral hydrophilic: Cys, Ser, Thr, Asn, Gln;
c. acidic: Asp, Glu;
d. basic: His, Lys, Arg;
e. residues that influence chain orientation: Gly, Pro;
f. aromatic: Trp, Tyr, Phe.

Non-conservative substitutions will entail exchanging a member of one of these classes for another class.

An exemplary substitutional variant is an affinity matured antibody, which may be conveniently generated, e.g., using phage display-based affinity maturation techniques. Briefly, one or more CDR residues are mutated and the variant antibody moieties displayed on phage and screened for a particular biological activity (*e*.*g*., bioactivity based on RBC lysis inhibition assay or binding affinity). Alterations (*e*.*g*., substitutions) may be made in HVRs, *e.g.*, to improve bioactivity based on RBC lysis inhibition assay or antibody affinity. Such alterations may be made in HVR "hotspots," *i.e.*, residues encoded by codons that undergo mutation at high frequency during the somatic maturation process (*see*, *e.g.*, Chowdhury, Methods Mol. Biol. 207:179-196 (2008)), and/or specificity determining residues (SDRs), with the resulting variant V_{H} and V_{L} being tested for binding affinity. Affinity maturation by constructing and reselecting from secondary libraries has been described, *e*.*g*., in Hoogenboom et al. in Methods in Molecular Biology 178:1-37 (O'Brien et al., ed., Human Press, Totowa, NJ, (2001).)

In some embodiments of affinity maturation, diversity is introduced into the variable genes chosen for maturation by any of a variety of methods *(e*.*g*., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues *(e.g.*, 4-6 residues at a time) are randomized. HVR residues involved in antigen binding may be specifically identified, *e*.*g*., using alanine scanning mutagenesis or modeling. CDR-H3 and CDR-L3 in particular are often targeted.

In some embodiments, substitutions, insertions, or deletions may occur within one or more HVRs so long as such alterations do not substantially reduce the ability of the antibody to bind antigen. For example, conservative alterations (*e*.*g*., conservative substitutions as provided herein) that do not substantially reduce binding affinity may be made in HVRs. Such alterations may be outside of HVR "hotspots" orSDRs. In some embodiments of the variant V_{H} and V_{L} sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (*e*.*g*., charged residues such as Arg, Asp, His, Lys, and Glu) are identified and replaced by a neutral or negatively charged amino acid (*e*.*g*., Ala or Glu) to determine whether the interaction of the antibody with antigen is affected. Further substitutions may be introduced at the amino acid locations to demonstrate functional sensitivity to the initial substitutions. Alternatively, or additionally, a crystal structure of an antigen-antibody complex can be determined to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antigen-binding moiety with an N-terminal methionyl residue. Other insertional variants of the antigen-binding moiety include the fusion to the N- or C-terminus of the antigen-binding moiety to an enzyme (*e*.*g*. for ADEPT) or a polypeptide which increases the serum half-life of the antigen-binding moiety.

### Fc Variants

In some embodiments, one or more amino acid modifications may be introduced into the Fc region of an antibody or antigen-binding fragment (e.g., a full-length antibody specifically binding to*K*. *pneumoniae*O2 antigen, full-length antibody specifically binding to*K*. *pneumoniae*O1 antigen, full-length bispecific antibody specifically binding toK. *pneumoniae*O2 antigen and O1 antigen, or fusion protein comprising the antibody or antigen-binding fragmentor bispecific antibody) provided herein, thereby generating an Fc variant. In some embodiments, the Fc variant has enhanced ADCC effector function, often related to binding to Fc receptors (FcRs). In some embodiments, the Fc variant has decreased ADCC effector function. There are many examples of changes or mutations to Fc sequences that can alter effector function. For example, WO 00/42072 and Shields et al. J Biol. Chem. 9(2): 6591-6604 (2001)describe antibody variants with improved or diminished binding to FcRs. The contents of those publications are specifically incorporated herein by reference.

Antibody-Dependent Cell-Mediated Cytotoxicity (ADCC) is a mechanism of action of therapeutic antibodies against tumor cells. ADCC is a cell-mediated immune defense whereby an effector cell of the immune system actively lyses a target cell (*e*.*g*., an infected cell), whose membrane-surface antigens have been bound by specific antigen-binding moieties (*e*.*g*., an antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen). The typical ADCC involves activation of NK cells by antibodies. An NK cell expresses CD16 which is an Fc receptor. This receptor recognizes, and binds to, the Fc portion of an antibody bound to the surface of a target cell. The most common Fc receptor on the surface of an NK cell is called CD 16 or FcγRIII. Binding of the Fc receptor to the Fc region of an antibody results in NK cell activation, release of cytolytic granules and consequent target cell apoptosis.

In some embodiments, the application contemplates the variant of antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a variant of full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) comprising an Fc region that possesses some but not all effector functions, which makes it a desirable candidate for applications in which the half-life of the antibody specifically binding *toK. pneumoniae*O2 antigen, antibody specifically binding to*K*. *pneumoniae*O1 antigen or bispecific antibody specifically binding to*K*. *pneumoniae*O2 antigen and O1 antigenin *vivo* is important yet certain effector functions (such as CDC and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Pat. No. 5,500,362 (see, *e.g.* Hellstrom, I. et al.Proc. Nat'l Acad. Sci. USA 83:7059-7063 (1986)) and Hellstrom, I et al., Proc. Nat'l Acad. Sci. USA 82:1499-1502 (1985); U.S. Pat. No. 5,821,337 (see Bruggemann, M. et al., J. Exp. Med. 166:1351-1361 (1987)). Alternatively, non-radioactive assay methods may be employed (see, for example, ACTI^{™} non-radioactive cytotoxicity assay for flow cytometry (CellTechnology, Inc. Mountain View, Calif.; and CytoTox 96^{™} non-radioactive cytotoxicity assay (Promega, Madison, Wis.). Useful effector cells for such assays include peripheral blood mononuclear cells (PBMC) and Natural Killer (NK) cells. Alternatively, or additionally, ADCC activity of the molecule of interest may be assessed *in vivo*, *e.g.*, in an animal model such as that disclosed in Clynes et al.Proc. Nat'l Acad. Sci. USA 95:652-656 (1998). C1q binding assays may also be carried out to confirm that the antibody is unable to bind C1q and hence lacks CDC activity. See, *e.g.,* C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M. S. et al., Blood 101:1045-1052 (2003); and Cragg, M. S. and M. J. Glennie, Blood 103:2738-2743 (2004)). FcRn binding and *in vivo* clearance/half-life determinations can also be performed using methods known in the art (see, *e.g.*, Petkova, S. B. et al., Int'l. Immunol. 18(12):1759-1769 (2006)).

Antibodies with reduced effector function include those with substitution of one or more of Fc region residues 238, 265, 269, 270, 297, 327 and 329 (U.S. Pat. No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (U.S. Pat. No. 7,332,581).

Certain antibody variants with improved or diminished binding to FcRs are described. (See, *e.g.*, U.S. Pat. No. 6,737,056; WO 2004/056312, and Shields et al., J. Biol. Chem. 9(2): 6591-6604 (2001).)

In some embodiments, alterations are made in the Fc region that result in altered (*i.e.*, either improved or diminished) opsonization, e.g. such as described in Moore et al., MAbs. 2(2): 181-189(2010).

In some embodiments, there is provided an antibody specifically binding toK. *pneumoniae*O2 antigen, antibody specifically binding toK. *pneumoniae*O1 antigen or bispecific antibody specifically binding toK. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) variant comprising a variant Fc region comprising one or more amino acid substitutions which increase half-life and/or improve binding to the neonatal Fc receptor (FcRn). Antibodies with increased half-lives and improved binding to FcRn are described in US2005/0014934A1 (Hinton et al*.*)*.* Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (U.S. Pat. No. 7,371,826).

*See* also Duncan & Winter, Nature 322:738-40 (1988); U.S. Pat. No. 5,648,260; U.S. Pat. No. 5,624,821; and WO 94/29351 concerning other examples of Fc variants.

Antibodies specifically binding toK. *pneumoniae*O2 antigen, antibodies specifically binding to*K*. *pneumoniae*O1 antigen or bispecific antibodies specifically binding toK. *pneumoniae*O2 antigen and O1 antigen (such as full-length antibodies specifically binding *toK. pneumoniae*O2 antigen, full-length antibodies specifically binding to*K*. *pneumoniae*O1 antigen or full-length bispecific antibodies specifically binding toK. *pneumoniae*O2 antigen and O1 antigen) comprising any of the Fc variants described herein, or combinations thereof, are contemplated.

### Glycosylation Variants

In some embodiments, an antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding toK. *pneumoniae*O1 antigen or full-length bispecific antibody specifically binding toK. *pneumoniae*O2 antigen and O1 antigen) provided herein is altered to increase or decrease the glycosylationextent of the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen. Addition or deletion of glycosylation sites to an antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen may be conveniently accomplished by altering the amino acid sequence of the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen or polypeptide portion thereof such that one or more glycosylation sites is created or removed.

Where the antibody specifically binding to*K*. *pneumoniae*O2 antigen, antibody specifically binding to*K*. *pneumoniae*O1 antigen or bispecific antibody specifically binding to*K. pneumoniae*O2 antigen and O1 antigen comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, *e.g.*, Wright et al., TIBTECH 15:26-32 (1997). The oligosaccharide may include various carbohydrates, *e*.*g*., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen of the application may be made in order to create antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen variants with certain improved properties.

The N-glycans attached to the CH2 domain of Fc is heterogeneous. Antibodies or Fc fusion proteins generated in CHO cells are fucosylated by fucosyltransferase activity. *See* Shoji-Hosaka et al., J. Biochem. 2006, 140:777- 83. Normally, a small percentage of naturally occurring afucosylated IgGs may be detected in human serum. N-glycosylation of the Fc is important for binding to FcγR; and afucosylation of the N-glycan increases Fc's binding capacity to FcγRIIIa. Increased FcγRIIIa binding can enhance ADCC, which can be advantageous in certain antibody therapeutic applications in which cytotoxicity is desirable.

In some embodiments, an enhanced effector function can be detrimental when Fc-mediated cytotoxicity is undesirable. In some embodiments, the Fc fragment or CH2 domain is not glycosylated. In some embodiments, the N-glycosylation site in the CH2 domain is mutated to prevent from glycosylation.

In some embodiments, the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) variants are provided comprising an Fc region wherein a carbohydrate structure attached to the Fc region has reduced fucose or lacks fucose, which may improve ADCC function. Specifically, the antibody specifically binding toK. *pneumoniae*O2 antigen, antibody specifically binding to*K*. *pneumoniae*O1 antigen or bispecific antibody specifically binding to*K*. *pneumoniae*O2 antigen and O1 antigenvariants are contemplated herein that have reduced fucose relative to the amount of fucose on the same antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen produced in a wild-type CHO cell. That is, they are characterized by having a lower amount of fucose than they would otherwise have if produced by native CHO cells (*e*.*g*., a CHO cell that produce a native glycosylation pattern, such as, a CHO cell containing a native FUT8 gene). In some embodiments, the antibody specifically binding to*K*. *pneumoniae*O2 antigen, antibody specifically binding to*K. pneumoniae*O1 antigen or bispecific antibody specifically binding to*K*. *pneumoniae*O2 antigen and O1 antigen is one wherein less than about 50%, 40%, 30%, 20%, 10%, or 5% of the N-linked glycans thereon comprise fucose.For example, the amount of fucose in such an antibody specifically binding to*K. pneumoniae*O2 antigen, antibody specifically binding to*K*. *pneumoniae*O1 antigen or bispecific antibody specifically binding to*K*. *pneumoniae*O2 antigen and O1 antigen may be from 1% to 80%, from 1% to 65%, from 5% to 65% or from 20% to 40%. In some embodiments, the antibody specifically binding to*K*. *pneumoniae*O2 antigen, antibody specifically binding to*K*. *pneumoniae*O1 antigen or bispecific antibody specifically binding *toK. pneumoniae*O2 antigen and O1 antigen is one wherein none of the N-linked glycans thereon comprise fucose, *i.e.*, wherein the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen is completely without fucose, or has no fucose or is afucosylated. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ±3 amino acids upstream or downstream of position 297, *i*.*e*., between positions 294 and 300, due to minor sequence variations in antibodies. Such fucosylation variants may have improved ADCC function. See, *e.g.*, US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al.J. Mol. Biol. 336:1239-1249 (2004); Yamane-Ohnuki et al.Biotech. Bioeng. 87: 614 (2004). Examples of cell lines capable of producing defucosylated antibodies include Lec13 CHO cells deficient in protein fucosylation (Ripka et al.Arch. Biochem. Biophys. 249:533-545 (1986); US Pat Appl No US 2003/0157108 A1, Presta, L; and WO 2004/056312 A1, Adams et al*.*, especially at Example 11), and knockout cell lines, such asα-1,6-fucosyltransferase gene, FUT8, knockout CHO cells (see, e.g., Yamane-Ohnuki et al.Biotech. Bioeng. 87: 614 (2004); Kanda, Y. et al., Biotechnol. Bioeng., 94(4):680-688 (2006); and WO2003/085107).

Antibody specifically binding toK. *pneumoniae*O2 antigen, antibody specifically binding *toK. pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) variants are further provided with bisected oligosaccharides, *e*.*g*., in which a biantennary oligosaccharide attached to the Fc region of the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen is bisected by GlcNAc. Such antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) variants may have reduced fucosylation and/or improved ADCC function. Examples of such antibody variants are described, *e*.*g*., in WO 2003/011878 (Jean-Mairet et al.); U.S. Pat. No. 6,602,684 (Umana et al.); US 2005/0123546 (Umana et al*.*), and Ferrara et al., Biotechnology and Bioengineering, 93(5): 851-861 (2006). Antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding toK. *pneumoniae*O2 antigen and O1 antigen) variants with at least one galactose residue in the oligosaccharide attached to the Fc region are also provided. Such antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen variants may have improved CDC function. Such antibody variants are described, *e.g.*, in WO 1997/30087 (Patel et al.); WO 1998/58964 (Raju, S.); and WO 1999/22764 (Raju, S.).

In some embodiments, the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) variants comprising an Fc region are capable of binding to an FcγRIII. In some embodiments, the antibody specifically binding toK. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) variants comprising an Fc region have ADCC activity in the presence of human effector cells (*e.g.*, T cell) or have increased ADCC activity in the presence of human effector cells compared to the otherwise same antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) comprising a human wild-type Fc region.

### Cysteine Engineered Variants

In some embodiments, it may be desirable to create cysteine engineered antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding toK. *pneumoniae*O2 antigen, full-length antibody specifically binding to*K*. *pneumoniae*O1 antigen or full-length bispecific antibody specifically binding to*K*. *pneumoniae*O2 antigen and O1 antigen) in which one or more amino acid residues are substituted with cysteine residues. In some embodiments, the substituted residues occur at accessible sites of the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen). By substituting those residues with cysteine, reactive thiol groups are thereby positioned at accessible sites of the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen and may be used to conjugate the antibody specifically binding to*K*. *pneumoniae*O2 antigen, antibody specifically binding to*K*. *pneumoniae*O1 antigen or bispecific antibody specifically binding toK. *pneumoniae*O2 antigen and O1 antigen to other moieties, such as drug moieties or linker-drug moieties, to create an antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen immunoconjugate, as described further herein. Cysteine engineered antibody specifically binding to*K*. *pneumoniae*O2 antigen, antibody specifically binding to*K*. *pneumoniae*O1 antigen or bispecific antibody specifically binding to*K. pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) may be generated as described, *e*.*g*., in U.S. Pat. No. 7,521,541.

### Derivatives

In some embodiments, a bispecific antibody specifically binding to*K*. *pneumoniae*O2 antigen and O1 antigen (such as a full-length bispecific antibody specifically binding to*K*. *pneumoniae*O2 antigen and O1 antigen) provided herein may be further modified to contain additional non-proteinaceous moieties that are known in the art and readily available. The moieties suitable for derivatization of the bispecific antibodies specifically binding to*K*. *pneumoniae*O2 antigen and O1 antigen include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone) polyethylene glycol, propropylene glycol homopolymers, prolypropylene oxide/ethylene oxide co-polymers, polyoxyethylated polyols (*e*.*g*., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the bispecific antibodies specifically binding to*K*. *pneumoniae*O2 antigen and O1 antigen may vary, and if more than one polymer are attached, they can be the same or different molecules. In general, the number and/or type of polymers used for derivatization can be determined based on considerations including, but not limited to, the particular properties or functions of the bispecific antibody specifically binding toK. *pneumoniae*O2 antigen and O1 antigen to be improved, whether the bispecific antibody specifically binding to*K*. *pneumoniae*O2 antigen and O1 antigen derivative will be used in a therapy under defined conditions, etc.

### Pharmaceutical Compositions

Also provided herein are compositions (such as pharmaceutical compositions, also referred to herein as formulations) comprising any of the antibodies specifically binding to*K*. *pneumoniae*O2 antigen, antibodies specifically binding to*K*. *pneumoniae*O1 antigen or bispecific antibodies specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen), nucleic acids encoding the antibodies, vectors comprising the nucleic acids encoding the antibodies, or host cells comprising the nucleic acids or vectors described herein. In some embodiments, there is provided a pharmaceutical composition comprising any one of the antibodies specifically binding toK. *pneumoniae*O2 antigen, antibodies specifically binding to*K*. *pneumoniae*O1 antigen or bispecific antibodies specifically binding to*K*. *pneumoniae*O2 antigen and O1 antigen described herein and a pharmaceutically acceptable carrier.

Suitable formulations of the antibodies specifically binding to*K*. *pneumoniae*O2 antigen, antibodies specifically binding to*K*. *pneumoniae*O1 antigen or bispecific antibodies specifically binding to*K*. *pneumoniae*O2 antigen and O1 antigen are obtained by mixing an antibody specifically binding to*K*. *pneumoniae*O2 antigen, antibody specifically binding to*K*. *pneumoniae*O1 antigen or bispecific antibody specifically binding toK. *pneumoniae*O2 antigen and O1 antigen having the desired degree of purity with optional pharmaceutically acceptable carriers, excipients or stabilizers *(*Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propylparaben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes *(e*.*g*. Zn-protein complexes); and/or non-ionic surfactants such as TWEEN^{™}, PLURONICS^{™} or polyethylene glycol (PEG). Exemplary formulations are described in WO98/56418, expressly incorporated herein by reference. Lyophilized formulations adapted for subcutaneous administration are described in WO97/04801. Such lyophilized formulations may be reconstituted with a suitable diluent to a high protein concentration and the reconstituted formulation may be administered subcutaneously to the individual to be treated herein. Lipofectins or liposomes can be used to deliver the antibodies specifically binding toK. *pneumoniae*O2 antigen, antibodies specifically binding to*K*. *pneumoniae*O1 antigen or bispecific antibodies specifically binding to*K*. *pneumoniae*O2 antigen and O1 antigen of this application into cells.

The formulation herein may also contain one or more active compounds in addition to the antibody specifically binding to*K*. *pneumoniae*O2 antigen, antibody specifically binding to*K. pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) as necessary for the particular indication being treated, preferably those with complementary activities that do not adversely affect each other. For example, it may be desirable to further provide other agents with therapeutic activity, such as antibiotics agent in addition to the antibody specifically binding to*K*. *pneumoniae*O2 antigen, antibody specifically binding to*K*. *pneumoniae*O1 antigen or bispecific antibody specifically binding to*K. pneumoniae*O2 antigen and O1 antigen. Such molecules are suitably present in combination in amounts that are effective for the purpose intended. The effective amount of such other active agents depends on the amount of antibody specifically binding toK. *pneumoniae*O2 antigen, antibody specifically binding to*K. pneumoniae*O1 antigen or bispecific antibody specifically binding to*K*. *pneumoniae*O2 antigen and O1 antigen present in the formulation, the type of disease or disorder or treatment, and other factors discussed above. These are generally used in the same dosages and with administration routes as described herein or about from 1 to 99% of the heretofore employed dosages.

The antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding to*K. pneumoniae*O2 antigen, full-length antibody specifically binding to*K*. *pneumoniae*O1 antigen or full-length bispecific antibody specifically binding to*K*. *pneumoniae*O2 antigen and O1 antigen) may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Sustained-release preparations may be prepared.

Sustained-release preparations of the antibodies specifically binding to*K*. *pneumoniae*O2 antigen, antibodies specifically binding to*K*. *pneumoniae*O1 antigen or bispecific antibodies specifically binding to*K*. *pneumoniae*O2 antigen and O1 antigen (such as full-length antibodies specifically binding to*K*. *pneumoniae*O2 antigen, full-length antibodies specifically binding to*K*. *pneumoniae*O1 antigen or full-length bispecific antibodies specifically binding to*K. pneumoniae*O2 antigen and O1 antigen) can be prepared. Suitable examples of sustained-release preparations include semipermeable matrices of solid hydrophobic polymers containing the antibody (or fragment thereof), which matrices are in the form of shaped articles, *e*.*g*., films, or microcapsules. Examples of sustained-release matrices include polyesters, hydrogels (for example, poly(2-hydroxyethyl-methacrylate ), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D (-)-3-hydroxybutyric acid. While polymers such as ethylene-vinyl acetate and lactic acid-glycolic acid enable release of molecules for over 100 days, certain hydro gels release proteins for shorter time periods. When encapsulated antibody remain in the body for a long time, they can denature or aggregate as a result of exposure to moisture at 37 °C, resulting in a loss of biological activity and possible changes in immunogenicity. Rational strategies can be devised for stabilization of antibodies specifically binding to*K*. *pneumoniae*O2 antigen, antibodies specifically binding to*K. pneumoniae*O1 antigen or bispecific antibodies specifically binding to*K*. *pneumoniae*O2 antigen and O1 antigen depending on the mechanism involved. For example, if the aggregation mechanism is discovered to be intermolecular S-S bond formation through thio-disulfide interchange, stabilization can be achieved by modifying sulfhydryl residues, lyophilizing from acidic solutions, controlling moisture content, using appropriate additives, and developing specific polymer matrix compositions.

In some embodiments, the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen or full-length bispecific antibody specifically binding to*K*. *pneumoniae*O2 antigen and O1 antigen) is formulated in a buffer comprising a citrate, NaCl, acetate, succinate, glycine, polysorbate 80 (Tween 80), or any combination of the foregoing. In some embodiments, the antibody specifically binding toK. *pneumoniae*O2 antigen, antibody specifically binding to*K*. *pneumoniae*O1 antigen or bispecific antibody specifically binding to*K*. *pneumoniae*O2 antigen and O1 antigen is formulated in a buffer comprising about 100 mM to about 150 mM glycine. In some embodiments, the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen is formulated in a buffer comprising about 50mM to about 100 mM NaCl. In some embodiments, the antibody specifically binding to*K*. *pneumoniae*O2 antigen, antibody specifically binding to*K*. *pneumoniae*O1 antigen or bispecific antibody specifically binding to*K. pneumoniae*O2 antigen and O1 antigen is formulated in a buffer comprising about 10mM to about 50 mM acetate. In some embodiments, the antibody specifically binding to*K*. *pneumoniae*O2 antigen, antibody specifically binding to*K. pneumoniae*O1 antigen or bispecific antibody specifically binding to*K*. *pneumoniae*O2 antigen and O1 antigen is formulated in a buffer comprising about 10mM to about 50 mM succinate. In some embodiments, the antibody specifically binding to*K*. *pneumoniae*O2 antigen, antibody specifically binding to*K*. *pneumoniae*O1 antigen or bispecific antibody specifically binding to*K. pneumoniae*O2 antigen and O1 antigen is formulated in a buffer comprising about 0.005% to about 0.02% polysorbate 80. In some embodiments, the antibody specifically binding to*K*. *pneumoniae*O2 antigen, antibody specifically binding to*K*. *pneumoniae*O1 antigen or bispecific antibody specifically binding to*K*. *pneumoniae*O2 antigen and O1 antigen is formulated in a buffer having a pH between about 5.1 and 5.6. In some embodiments, the antibody specifically binding to*K*. *pneumoniae*O2 antigen, antibody specifically binding to*K*. *pneumoniae*O1 antigen or bispecific antibody specifically binding to*K. pneumoniae*O2 antigen and O1 antigen is formulated in a buffer comprising 10 mM citrate, 100 mM NaCl, 100mM glycine, and 0.01% polysorbate 80, wherein the formulation is at pH 5.5.

The formulations to be used for *in vivo* administration must be sterile. This is readily accomplished by, e.g., filtration through sterile filtration membranes.

### Methods of preventingor treatingK. pneumoniae infection

In certain aspects, there is provided a method of preventing or treating a *K. pneumoniae* infection in an individual comprising administering to the individual an effective amount of a composition comprising any of the antibodies specifically binding to*K*. *pneumoniae*O2 antigen, and/or antibodies specifically binding toK. *pneumoniae*O1 antigen, and/or bispecific antibodies specifically binding to*K*. *pneumoniae*O2 antigen and O1 antigen described herein, and/or any of the pharmaceutical compositions comprising bispecific antibodies specifically binding to*K*. *pneumoniae*O2 antigen and O1 antigen, and/or pharmaceutical compositions comprisingantibodies specifically binding to*K*. *pneumoniae*O2 antigen and O1 antigen. In some embodiments, the method of treating *a K. pneumoniae* infection further provides therapeutic or prophylactic effect on diseases and/or conditions associated with*K*. *pneumoniae* infection. In some aspects, there is provided a method of preventing *a K. pneumoniae* infection in an individual comprising administering to the individual an effective amount of a composition comprising any of the bispecific antibodies or pharmaceutical compositions described herein. In some embodiments, there is provided theuse of any one of the bispecific antibodies or pharmaceutical compositions described above in the manufacture of a medicament for treating a disease or condition.

Diseases and/or conditions associated with*K*. *pneumoniae* infection include, but are not limited to pneumonia, urinary tract infection, septicaemia/bacteremia/sepsis, neonatal septicaemia/bacteremia/sepsis, diarrhea, soft tissue infection, infection after organ transplantation, surgical infection, wound infection, lung infection, purulent liver abscess, lung abscess, cellulitis, necrotizing myositis, myositis, endophthalmitis, peritonitis, meningitis, necrotizing meningitis, ankylosing spondylitis or spinal joint disease. In some embodiments, the method of treating or preventing *K. pneumoniae* infection reduces rate of mortality resulting from the *K. pneumoniae* infection.

In some embodiments, there is provided a method of treating or preventing *a K. pneumoniae* infection in an individual comprising administering to the individual an effective amount of a bispecific antibody specifically binding to*K*. *pneumoniae*O2 antigen and *K. pneumoniae*O1 antigen or a composition comprising the bispecific antibody, wherein the bispecific antibody comprises the first antigen-binding domain specifically binding to*K*. *pneumoniae*O2 antigen and the second antigen-binding domain specifically binding to*K*. *pneumoniae*O1 antigen, wherein the first antigen-binding domain specifically binding to*K*. *pneumoniae*O2 antigen comprises:a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising: an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16. In some embodiments, the first antigen-binding domain specifically binding toK. *pneumoniae*O2 domain comprises: a V_{H} comprising the amino acid sequence of SEQ ID NO: 19 or 28; and a V_{L} comprising the amino acid sequence of SEQ ID NO: 24 or 33. In some embodiments, the first antigen-binding domain specifically binding to*K*. *pneumoniae*O2 domain comprises a V_{H} comprising an HC-CDR1, an HC-CDR2, and an HC-CDR3 of a V_{H} comprising the amino acid sequence of SEQ ID NO: 19 or 28; and a V_{L} comprising an LC-CDR1, an LC-CDR2, and an LC-CDR3 of a V_{L} comprising the amino acid sequence of SEQ ID NO: 24 or 33.

In some embodiments, the first antigen-binding domain specifically binding to*K*. *pneumoniae*O2 antigenof the bispecific antibody in the method comprises:a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7; and a V_{L} comprising: an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15. In some embodiments, the first antigen-binding domain specifically binding to*K*. *pneumoniae*O2 domainof the bispecific antibody comprises: a V_{H} comprising the amino acid sequence of SEQ ID NO: 20 or 29; and a V_{L} comprising the amino acid sequence of SEQ ID NO: 25 or 34. In some embodiments, the first antigen-binding domain specifically binding toK. *pneumoniae*O2 domainof the bispecific antibody comprises a V_{H} comprising an HC-CDR1, an HC-CDR2, and an HC-CDR3 of a V_{H} comprising the amino acid sequence of SEQ ID NO: 20 or 29; and a V_{L} comprising an LC-CDR1, an LC-CDR2, and an LC-CDR3 of a V_{L} comprising the amino acid sequence of SEQ ID NO: 25 or 34.

In some embodiments, the secondantigen-binding domain specifically binding to*K*. *pneumoniae*O1 antigenof the bispecific antibody in the method comprises:a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising: an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45. In some embodiments, the secondantigen-binding domain specifically binding to*K*. *pneumoniae*O1 domainof the bispecific antibody comprises: a V_{H} comprising the amino acid sequence of SEQ ID NO: 47 or 51; and a V_{L} comprising the amino acid sequence of SEQ ID NO: 49 or 53. In some embodiments, the secondantigen-binding domain specifically binding to*K*. *pneumoniae*O1 domainof the bispecific antibody comprises a V_{H} comprising an HC-CDR1, an HC-CDR2, and an HC-CDR3 of a V_{H} comprising the amino acid sequence of SEQ ID NO: 47 or 51; and a V_{L} comprising an LC-CDR1, an LC-CDR2, and an LC-CDR3 of a V_{L} comprising the amino acid sequence of SEQ ID NO: 49 or 53.

In some embodiments, the secondantigen-binding domain specifically binding to*K*. *pneumoniae*O1 antigenof the bispecific antibody in the method comprises:a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising: an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46. In some embodiments, the secondantigen-binding domain specifically binding to*K*. *pneumoniae*O1 domainof the bispecific antibody comprises: a V_{H} comprising the amino acid sequence of SEQ ID NO: 48 or 52; and a V_{L} comprising the amino acid sequence of SEQ ID NO: 50 or 54. In some embodiments, the secondantigen-binding domain specifically binding to*K*. *pneumoniae*O1 domainof the bispecific antibody comprises a V_{H} comprising an HC-CDR1, an HC-CDR2, and an HC-CDR3 of a V_{H} comprising the amino acid sequence of SEQ ID NO: 48 or 52; and a V_{L} comprising an LC-CDR1, an LC-CDR2, and an LC-CDR3 of a V_{L} comprising the amino acid sequence of SEQ ID NO: 50 or 54.

In some embodiments, there is provided a method of treating or preventing a *K*. *pneumoniae* infection in an individual comprising administering to the individual an effective amount of a bispecific antibody specifically binding to*K*. *pneumoniae*O2 antigen and *K. pneumoniae*O1 antigen or a composition comprising the bispecific antibody, wherein the bispecific antibody comprises the first antigen-binding domain specifically binding to*K*. *pneumoniae*O2 antigen and the second antigen-binding domain specifically binding to*K*. *pneumoniae*O1 antigen, wherein the first antigen-binding domain comprises:a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising: an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16; the secondantigen-binding domain comprises:a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising: an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45.

In some embodiments, there is provided a method of treating or preventing a *K*. *pneumoniae* infection in an individual comprising administering to the individual an effective amount of a bispecific antibody specifically binding to*K*. *pneumoniae*O2 antigen and *K. pneumoniae*O1 antigen or a composition comprising the bispecific antibody, wherein the bispecific antibody comprises the first antigen-binding domain specifically binding to*K*. *pneumoniae*O2 antigen and the second antigen-binding domain specifically binding to*K*. *pneumoniae*O1 antigen, wherein the first antigen-binding domain comprises:a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7; and a V_{L} comprising: an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15; and the secondantigen-binding domain comprises:a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising: an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45.

In some embodiments, there is provided a method of treating or preventing a *K*. *pneumoniae* infection in an individual comprising administering to the individual an effective amount of a bispecific antibody specifically binding to*K*. *pneumoniae*O2 antigen and *K. pneumoniae*O1 antigen or a composition comprising the bispecific antibody, wherein the bispecific antibody comprises the first antigen-binding domain specifically binding to*K*. *pneumoniae*O2 antigen and the second antigen-binding domain specifically binding to*K*. *pneumoniae*O1 antigen, wherein the first antigen-binding domain comprises:a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising: an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16; and the secondantigen-binding domain comprises:a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising: an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

In some embodiments, there is provided a method of treating or preventing a *K*. *pneumoniae* infection in an individual comprising administering to the individual an effective amount of a bispecific antibody specifically binding to*K*. *pneumoniae*O2 antigen and *K. pneumoniae*O1 antigen or a composition comprising the bispecific antibody, wherein the bispecific antibody comprises the first antigen-binding domain specifically binding to*K*. *pneumoniae*O2 antigen and the second antigen-binding domain specifically binding to*K*. *pneumoniae*O1 antigen, wherein the first antigen-binding domain comprises:a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7; and a V_{L} comprising: an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15; and the secondantigen-binding domain comprises:a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising: an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

In some embodiments, there is provided a method of treating or preventing a *K*. *pneumoniae* infection in an individual comprising administering an effective amount of a composition comprising bispecific antibody specifically binding to*K*. *pneumoniae*O2 antigen and *K. pneumoniae*O1 antigenis more efficacious than administering a same valency amount of an antibody specifically binding to*K*. *pneumoniae*O2 antigen or a same valency amount of an antibody specifically bindingto *K. pneumoniae*O1 antigen. In some embodiments, the method comprising administering an effective amount of a bispecific antibody specifically binding to*K*. *pneumoniae*O2 antigen and *K. pneumoniae*O1 antigen enhances the opsonophagocytic killing (OPK) activity against*K*. *pneumoniae* by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody specifically binding to*K*. *pneumoniae*O2 antigen or a same valency amount of an antibody specifically binding to*K*. *pneumoniae*O1 antigen. In some embodiments, the method comprising administering an effective amount of a bispecific antibody specifically binding to*K*. *pneumoniae*O2 antigen and *K. pneumoniae*O1 antigenenhances the neutralization activity against *K. pneumoniae* by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody specifically binding to*K*. *pneumoniae*O2 antigen or a same valency amount of an antibody specifically binding to*K*. *pneumoniae*O1 antigen.In some embodiments, the methodcomprising administering an effective amount of a bispecific antibody specifically binding to*K*. *pneumoniae*O2 antigen and *K. pneumoniae*O1 antigenenhances the serum bactericidal activity (SBA) against *K. pneumoniaeby* about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody specifically binding to*K*. *pneumoniae*O2 antigen or a same valency amount of an antibody specifically binding to*K*. *pneumoniae*O1 antigen. In some embodiments, the method comprising administering an effective amount of a bispecific antibody specifically binding to*K. pneumoniae*O2 antigen and *K. pneumoniae*O1 antigen improves patient survival by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody specifically binding to*K*. *pneumoniae*O2 antigen or a same valency amount of an antibody specifically binding to*K*. *pneumoniae*O1 antigen.

### Methods ofpreventing or treating by using a pharmaceutical composition comprising antibody specifically binding toO2 antigen and antibody specifically binding toO1 antigen

In some embodiments, there is provided a method of preventing or treating a *K. pneumoniae* infection in an individual comprising administering to the individual an effective amount of a pharmaceutical composition comprising (i) an antibody or antigen-binding fragment specifically binding to*K*. *pneumoniae*O2 antigenand (ii) an antibody or antigen-binding fragment specifically binding to *K. pneumoniae*O1 antigen.

In some embodiments, there is provided a method of treating or preventing a *K. pneumoniae* infection in an individual comprising administering to the individual an effective amount of (i) an antibody or antigen-binding fragment specifically binding toK. *pneumoniae*O2 antigenand (ii) an antibody or antigen-binding fragment specifically binding to *K. pneumoniae*O1 antigen. In some embodiments, the antibody or antigen-binding fragment specifically binding to*K*. *pneumoniae*O2 antigen and the antibody or antigen-binding fragment specifically binding to*K*. *pneumoniae*O1 antigen are administered concurrently. In some embodiments, the antibody or antigen-binding fragment specifically binding to*K*. *pneumoniae*O2 antigen and the antibody or antigen-binding fragment specifically binding to*K*. *pneumoniae*O1 antigen are administered consecutively.

In some embodiments, the antibody or antigen-binding fragment specifically binding to *K. pneumoniae*O2 antigen in the method comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 5; and a V_{L} comprising an LC-CDR1 comprising the amino acid sequence of SEQ ID NO: 8, an LC-CDR2 comprising the amino acid sequence of SEQ ID NO: 12, and an LC-CDR3 comprising the amino acid sequence of SEQ ID NO: 15. In some embodiments, the antibody or antigen-binding fragment specifically binding to *K. pneumoniae*O2 antigen comprises: a V_{H} comprising the amino acid sequence of SEQ ID NO: 17; and a V_{L} comprising the amino acid sequence of SEQ ID NO: 21. In some embodiments, the antibody or antigen-binding fragment specifically binding to *K. pneumoniae*O2 antigen comprises: a V_{H} comprising an HC-CDR1, an HC-CDR2, and an HC-CDR3 of a V_{H} comprising the amino acid sequence of SEQ ID NO: 17; and a V_{L} comprising an LC-CDR1, an LC-CDR2, and an LC-CDR3 of a V_{L} comprising the amino acid sequence of SEQ ID NO: 21.

In some embodiments, the antibody or antigen-binding fragment specifically binding to *K. pneumoniae*O2 antigen in the method comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 6; and a V_{L} comprising an LC-CDR1 comprising the amino acid sequence of SEQ ID NO: 9, an LC-CDR2 comprising the amino acid sequence of SEQ ID NO: 12, and an LC-CDR3 comprising the amino acid sequence of SEQ ID NO: 16. In some embodiments, the antibody or antigen-binding fragment specifically binding to *K. pneumoniae*O2 antigen comprises: a V_{H} comprising the amino acid sequence of SEQ ID NO: 18; and a V_{L} comprising the amino acid sequence of SEQ ID NO: 22. In some embodiments, the antibody or antigen-binding fragment specifically binding to *K. pneumoniae*O2 antigen comprises: a V_{H} comprising an HC-CDR1, an HC-CDR2, and an HC-CDR3 of a V_{H} comprising the amino acid sequence of SEQ ID NO: 18; and a V_{L} comprising an LC-CDR1, an LC-CDR2, and an LC-CDR3 of a V_{L} comprising the amino acid sequence of SEQ ID NO: 22.

In some embodiments, the antibody or antigen-binding fragment specifically binding to *K. pneumoniae*O2 antigen in the method comprises: a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 10, an LC-CDR2 comprising the amino acid sequence of SEQ ID NO: 13, and an LC-CDR3 comprising the amino acid sequence of SEQ ID NO: 16. In some embodiments, the antibody or antigen-binding fragment specifically binding to *K. pneumoniae*O2 antigen comprises: a V_{H} comprising the amino acid sequence of SEQ ID NO: 19; and a V_{L} comprising the amino acid sequence of SEQ ID NO: 23. In some embodiments, the antibody or antigen-binding fragment specifically binding to *K. pneumoniae*O2 antigen comprises: a V_{H} comprising an HC-CDR1, an HC-CDR2, and an HC-CDR3 of a V_{H} comprising the amino acid sequence of SEQ ID NO: 19; and a V_{L} comprising an LC-CDR1, an LC-CDR2, and an LC-CDR3 of a V_{L} comprising the amino acid sequence of SEQ ID NO: 23.

In some embodiments, the antibody or antigen-binding fragment specifically binding to *K. pneumoniae*O2 antigen in the method comprises: a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16. In some embodiments, the antibody or antigen-binding fragment specifically binding to *K. pneumoniae*O2 antigen comprises: a V_{H} comprising the amino acid sequence of SEQ ID NO: 19; and a V_{L} comprising the amino acid sequence of SEQ ID NO: 24. In some embodiments, the antibody or antigen-binding fragment specifically binding to *K. pneumoniae*O2 antigen comprises: a V_{H} comprising an HC-CDR1, an HC-CDR2, and an HC-CDR3 of a V_{H} comprising the amino acid sequence of SEQ ID NO: 19; and a V_{L} comprising an LC-CDR1, an LC-CDR2, and an LC-CDR3 of a V_{L} comprising the amino acid sequence of SEQ ID NO: 24.

In some embodiments, the antibody or antigen-binding fragment specifically binding to *K. pneumoniae*O2 antigen in the method comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15. In some embodiments, the antibody or antigen-binding fragment specifically binding to *K. pneumoniae*O2 antigen comprises: a V_{H} comprising the amino acid sequence of SEQ ID NO: 20; and a V_{L} comprising the amino acid sequence of SEQ ID NO: 25. In some embodiments, the antibody or antigen-binding fragment specifically binding to *K. pneumoniae*O2 antigen comprises: a V_{H} comprising an HC-CDR1, an HC-CDR2, and an HC-CDR3 of a V_{H} comprising the amino acid sequence of SEQ ID NO: 20; and a V_{L} comprising an LC-CDR1, an LC-CDR2, and an LC-CDR3 of a V_{L} comprising the amino acid sequence of SEQ ID NO: 25.

In some embodiments, the antibody or antigen-binding fragment specifically binding to *K. pneumoniae*O1 antigen in the method comprises: a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45. In some embodiments, the antibody or antigen-binding fragment specifically binding to *K. pneumoniae*O1 antigen comprises: a V_{H} comprising the amino acid sequence of SEQ ID NO: 47; and a V_{L} comprising the amino acid sequence of SEQ ID NO: 49. In some embodiments, the antibody or antigen-binding fragment specifically binding to *K. pneumoniae*O1 antigen comprises: a V_{H} comprising an HC-CDR1, an HC-CDR2, and an HC-CDR3 of a V_{H} comprising the amino acid sequence of SEQ ID NO: 47; and a V_{L} comprising an LC-CDR1, an LC-CDR2, and an LC-CDR3 of a V_{L} comprising the amino acid sequence of SEQ ID NO: 49.

In some embodiments, the antibody or antigen-binding fragment specifically binding to *K. pneumoniae*O1 antigen in the method comprises: a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46. In some embodiments, the antibody or antigen-binding fragment specifically binding to *K. pneumoniae*O1 antigen comprises: a V_{H} comprising the amino acid sequence of SEQ ID NO: 48; and a V_{L} comprising the amino acid sequence of SEQ ID NO: 50. In some embodiments, the antibody or antigen-binding fragment specifically binding to *K. pneumoniae*O1 antigen comprises: a V_{H} comprising an HC-CDR1, an HC-CDR2, and an HC-CDR3 of a V_{H} comprising the amino acid sequence of SEQ ID NO: 48; and a V_{L} comprising an LC-CDR1, an LC-CDR2, and an LC-CDR3 of a V_{L} comprising the amino acid sequence of SEQ ID NO: 50.

In some embodiments, there is provided a pharmaceutical composition comprising anantibody or antigen-binding fragment specifically binding to *K. pneumoniae*O2 antigen and antibody or antigen-binding fragment specifically binding to *K. pneumoniae*O1 antigen, wherein the antibody or antigen-binding fragment specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 10, an LC-CDR2 comprising the amino acid sequence of SEQ ID NO: 13, and an LC-CDR3 comprising the amino acid sequence of SEQ ID NO: 16; and wherein the antibody or antigen-binding fragment specifically binding to *K. pneumoniae*O1 comprises: a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45.

In some embodiments, there is provided a pharmaceutical composition comprising anantibody or antigen-binding fragment specifically binding to *K. pneumoniae*O2 antigen and antibody or antigen-binding fragment specifically binding to *K. pneumoniae*O1 antigen, wherein the antibody or antigen-binding fragment specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 10, an LC-CDR2 comprising the amino acid sequence of SEQ ID NO: 13, and an LC-CDR3 comprising the amino acid sequence of SEQ ID NO: 16; and wherein the antibody or antigen-binding fragment specifically binding to *K. pneumoniae*O1 comprises: a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

In some embodiments, there is provided a pharmaceutical composition comprising anantibody or antigen-binding fragment specifically binding to *K. pneumoniae*O2 antigen and antibody or antigen-binding fragment specifically binding to *K. pneumoniae*O1 antigen, wherein the antibody or antigen-binding fragment specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16; and wherein the antibody or antigen-binding fragment specifically binding to *K. pneumoniae*O1 comprises: a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45.

In some embodiments, there is provided a pharmaceutical composition comprising anantibody or antigen-binding fragment specifically binding to *K. pneumoniae*O2 antigen and antibody or antigen-binding fragment specifically binding to *K. pneumoniae*O1 antigen, wherein the antibody or antigen-binding fragment specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16; and wherein the antibody or antigen-binding fragment specifically binding to *K. pneumoniae*O1 comprises: a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

In some embodiments, there is provided a pharmaceutical composition comprising anantibody or antigen-binding fragment specifically binding to *K. pneumoniae*O2 antigen and antibody or antigen-binding fragment specifically binding to *K. pneumoniae*O1 antigen, wherein the antibody or antigen-binding fragment specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15; and wherein the antibody or antigen-binding fragment specifically binding to *K. pneumoniae*O1 comprises: a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45.

In some embodiments, there is provided a pharmaceutical composition comprising anantibody or antigen-binding fragment specifically binding to *K. pneumoniae*O2 antigen and antibody or antigen-binding fragment specifically binding to *K. pneumoniae*O1 antigen, wherein the antibody or antigen-binding fragment specifically binding to *K. pneumoniae*O2 antigen comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15; and wherein the antibody or antigen-binding fragment specifically binding to *K. pneumoniae*O1 comprises: a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

In some embodiments, there is provided a method of preventing ortreating a *K. pneumoniae* infection comprising administering an effective amount of a composition comprising antibody or antigen-binding fragment specifically binding to*K*. *pneumoniae*O2 antigen and antibody or antigen-binding fragment specifically binding to*K*. *pneumoniae*O1 antigenis more efficacious than administering a same valency amount of an antibodyor antigen-binding fragment specifically binding to*K*. *pneumoniae*O2 antigen or a same valency amount of an antibodyor antigen-binding fragment specifically bindingto *K. pneumoniae*O1 antigen. In some embodiments, the method comprising administering an effective amount of a composition comprising antibody or antigen-binding fragment specifically binding to*K*. *pneumoniae*O2 antigen and antibody or antigen-binding fragment specifically binding to*K*. *pneumoniae*O1 antigen enhances the opsonophagocytic killing (OPK) activity against*K*. *pneumoniae* by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibodyor antigen-binding fragment specifically binding to*K*. *pneumoniae*O2 antigen or a same valency amount of an antibodyor antigen-binding fragment specifically binding to*K*. *pneumoniae*O1 antigen. In some embodiments, the methodcomprising administering an effective amount of a composition comprising antibody or antigen-binding fragment specifically binding to*K*. *pneumoniae*O2 antigen and antibody or antigen-binding fragment specifically binding to*K*. *pneumoniae*O1 antigenenhances the serum bactericidal activity (SBA) against *K. pneumoniae*by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to*K*. *pneumoniae*O2 antigen or a same valency amount of an antibodyor antigen-binding fragment specifically binding to*K*. *pneumoniae*O1 antigen.In some embodiments, the method comprising administering an effective amount of a composition comprising antibody or antigen-binding fragment specifically binding to*K*. *pneumoniae*O2 antigen and antibody or antigen-binding fragment specifically binding to*K*. *pneumoniae*O1 antigenenhances the neutralization activity against *K. pneumoniae* by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibodyor antigen-binding fragment specifically binding to*K*. *pneumoniae*O2 antigen or a same valency amount of an antibody or antigen-binding fragment specifically binding to*K*. *pneumoniae*O1 antigen. In some embodiments, the method comprising administering an effective amount of a bispecific antibody specifically binding to*K*. *pneumoniae*O2 antigen and *K. pneumoniae*O1 antigen improves patient survival by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibodyor antigen-binding fragment specifically binding toK. *pneumoniae*O2 antigen or a same valency amount of an antibodyor antigen-binding fragment specifically binding toK. *pneumoniae*O1 antigen.

### Articles of Manufacture and Kits

In some embodiments of the application, there is provided an article of manufacture containing materials useful for preventing or treating a *K. pneumoniae* infection in an individual, or for delivering a antibody or antigen-binding fragment (such as a antibody specifically binding toK. *pneumoniae*O2 antigen and antibody specifically binding toK. *pneumoniae*O1 antigen)or bispecific antibody (such as a bispecific antibody specifically binding toO2 antigen and O1 antigen) or a pharmaceutical composition comprising antibodiesor antigen-binding fragments specifically binding toK. *pneumoniae*O2 antigen or antibodiesor antigen-binding fragments specifically binding toK. *pneumoniae*O1 antigen, to a cell attached by a pathogen expressing *K. pneumoniae*O2 antigen and O1 antigen. The article of manufacture can comprise a container and a label or package insert on or associated with the container. Suitable containers include, for example, bottles, vials, syringes, etc. The containers may be formed from a variety of materials such as glass or plastic. Generally, the container holds a composition which is effective for treating a disease or disorder described herein, and may have a sterile access port (for example the container may be an intravenous solution bag or a vial having a stopper pierceable by a hypodermic injection needle). In some embodiments, at least one active agent in the composition is a antibody or antigen-binding fragment or bispecific antibody of the application. The label or package insert indicates that the composition is used for treating the particular condition. The label or package insert will further comprise instructions for administering the bispecific antibody or the pharmaceutical composition to the patient. Articles of manufacture and kits comprising combinatorial therapies described herein are also contemplated.

Package insert refers to instructions customarily included in commercial packages of therapeutic products that contain information about the indications, usage, dosage, administration, contraindications and/or warnings concerning the use of such therapeutic products. In some embodiments, the package insert indicates that the composition is used for treating bacterial infections. In some embodiments, the package insert indicates that the composition is used for treating *K. pneumoniae* infections.

Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Kits are also provided that are useful for various purposes, e.g., useful for preventing or treating a *K. pneumoniae* infection in an individual, or for delivering an antibody specifically binding to*K*. *pneumoniae*O2 antigen, antibody specifically binding to*K*. *pneumoniae*O1 antigen, or bispecific antibody specifically binding toK. *pneumoniae*O2 antigen and O1 antigen (such as a full-length antibody specifically binding toK. *pneumoniae*O2 antigen, full-length antibody specifically binding to*K*. *pneumoniae*O1 antigen, or full-length bispecific antibody specifically binding to*K*. *pneumoniae*O2 antigen and O1 antigen) to a cell attached by a pathogen expressing O2 antigen or O1 antigen, optionally in combination with the articles of manufacture. Kits of the application include one or more containers comprising antibody specifically binding to*K*. *pneumoniae*O2 antigen, antibody specifically binding to*K*. *pneumoniae*O1 antigen, or bispecific antibody specifically binding to*K. pneumoniae*O2 antigen and O1 antigencomposition (or unit dosage form and/or article of manufacture), and in some embodiments, further comprise another agent (such as the agents described herein) and/or instructions for use in accordance with any of the methods described herein. The kit may further comprise a description of selection of individuals suitable for treatment. Instructions supplied in the kits of the application are typically written instructions on a label or package insert (e.g., a paper sheet included in the kit), but machine-readable instructions (e.g., instructions carried on a magnetic or optical storage disk) are also acceptable.

For example, in some embodiments, the kit comprises a composition comprising an antibody specifically binding to*K*. *pneumoniae*O2 antigen, antibody specifically binding to*K*. *pneumoniae*O1 antigen, or bispecific antibody specifically binding *toK. pneumoniae*O2 antigen and O1 antigen (such as a full-length antibody specifically binding to*K*. *pneumoniae*O2 antigen, full-length antibody specifically binding to*K*. *pneumoniae*O1 antigen, or full-length bispecific antibody specifically binding to*K*. *pneumoniae*O2 antigen and O1 antigen). In some embodiments, the kit comprises a) a composition comprising any one of the antibodies specifically binding to*K*. *pneumoniae*O2 antigen, antibodies specifically binding *toK. pneumoniae*O1 antigen, or bispecific antibodies specifically binding toK. *pneumoniae*O2 antigen and O1 antigen described herein, and b) an effective amount of at least one other agent, wherein the other agent enhances the effect (e.g., treatment effect, detecting effect) of the antibody specifically binding to*K*. *pneumoniae*O2 antigen, antibody specifically binding to*K. pneumoniae*O1 antigen, or bispecific antibody specifically binding to*K*. *pneumoniae*O2 antigen and O1 antigen. In some embodiments, the kit comprises a) a composition comprising any one of the antibodies specifically binding to*K*. *pneumoniae*O2 antigen, antibodies specifically binding to*K*. *pneumoniae*O1 antigen, or bispecific antibodies specifically binding to*K. pneumoniae*O2 antigen and O1 antigen described herein, and b) instructions for administering the antibody specifically binding to*K*. *pneumoniae*O2 antigen, antibody specifically binding toK. *pneumoniae*O1 antigen, or bispecific antibody specifically binding to*K. pneumoniae*O2 antigen and O1 antigencomposition to an individual for treating a *K*. *pneumoniae* infection in an individual. In some embodiments, the kit comprises a) a composition comprising any one of theantibodies specifically binding to*K*. *pneumoniae*O2 antigen, antibodies specifically binding to*K. pneumoniae*O1 antigen, or bispecific antibodies specifically binding to*K*. *pneumoniae*O2 antigen and O1 antigen described herein, b) an effective amount of at least one other agent, wherein the other agent enhances the effect (e.g., treatment effect, detecting effect) of the antibody specifically binding to*K*. *pneumoniae*O2 antigen, antibody specifically binding to*K*. *pneumoniae*O1 antigen, or bispecific antibody specifically binding to*K*. *pneumoniae*O2 antigen and O1 antigen, and c) instructions for administering the antibody specifically binding to*K*. *pneumoniae*O2 antigen, antibody specifically binding to*K*. *pneumoniae*O1 antigen, or bispecific antibody specifically binding to*K. pneumoniae*O2 antigen and O1 antigencomposition and the other agent(s) to an individual for useful for treating a *K. pneumoniae* infection in an individual.The antibody specifically binding to*K*. *pneumoniae*O2 antigen, antibody specifically binding to*K*. *pneumoniae*O1 antigen, or bispecific antibody specifically binding to*K. pneumoniae*O2 antigen and O1 antigen and the other agent(s) can be present in separate containers or in a single container. For example, the kit may comprise one distinct composition or two or more compositions wherein one composition comprises an antibody specifically binding to*K*. *pneumoniae*O2 antigen, antibody specifically binding to*K. pneumoniae*O1 antigen, or bispecific antibody specifically binding toK. *pneumoniae*O2 antigen and O1 antigen and another composition comprises another agent.

In some embodiments, the kit comprises a nucleic acid (or set of nucleic acids) encoding an antibody specifically binding to*K*. *pneumoniae*O2 antigen, antibody specifically binding *toK. pneumoniae*O1 antigen, or bispecific antibody specifically binding to*K*. *pneumoniae*O2 antigen and O1 antigen (such as a full-length antibody specifically binding to*K*. *pneumoniae*O2 antigen, full-length antibody specifically binding to*K*. *pneumoniae*O1 antigen, or full-length bispecific antibody specifically binding to*K*. *pneumoniae*O2 antigen and O1 antigen). In some embodiments, the kit comprises a) a nucleic acid (or set of nucleic acids) encoding an antibody specifically binding to*K*. *pneumoniae*O2 antigen, antibody specifically binding to*K*. *pneumoniae*O1 antigen, or bispecific antibody specifically binding to*K*. *pneumoniae*O2 antigen and O1 antigen, and b) a host cell for expressing the nucleic acid (or set of nucleic acids). In some embodiments, the kit comprises a) a nucleic acid (or set of nucleic acids) encoding an antibody specifically binding to*K*. *pneumoniae*O2 antigen, antibody specifically binding toK. *pneumoniae*O1 antigen, or bispecific antibody specifically binding to*K*. *pneumoniae*O2 antigen and O1 antigenand b) instructions for i) expressing the antibody specifically binding to*K. pneumoniae*O2 antigen, antibody specifically binding toK. *pneumoniae*O1 antigen, or bispecific antibody specifically binding *toK. pneumoniae*O2 antigen and O1 antigenin a host cell, ii) preparing a composition comprising the antibody specifically binding toK. *pneumoniae*O2 antigen, antibody specifically binding toK. *pneumoniae*O1 antigen, or bispecific antibody specifically binding *toK. pneumoniae*O2 antigen and O1 antigen, and iii) administering the composition comprising the antibody specifically binding toK. *pneumoniae*O2 antigen, antibody specifically binding toK. *pneumoniae*O1 antigen, or bispecific antibody specifically binding *toK. pneumoniae*O2 antigen and O1 antigento an individual for treating or preventing a *K. pneumoniae* infection in an individual. In some embodiments, the kit comprises a) a nucleic acid (or set of nucleic acids) encoding an antibody specifically binding toK. *pneumoniae*O2 antigen, antibody specifically binding toK. *pneumoniae*O1 antigen, or bispecific antibody specifically binding *toK. pneumoniae*O2 antigen and O1 antigen, b) a host cell for expressing the nucleic acid (or set of nucleic acids), and c) instructions for i) expressing the antibody specifically binding toK. *pneumoniae*O2 antigen, antibody specifically binding *toK. pneumoniae*O1 antigen, or bispecific antibody specifically binding toK. *pneumoniae*O2 antigen and O1 antigenin the host cell, ii) preparing a composition comprising the antibody specifically binding toK. *pneumoniae*O2 antigen, antibody specifically binding *toK. pneumoniae*O1 antigen, or bispecific antibody specifically binding toK. *pneumoniae*O2 antigen and O1 antigenand iii) administering the composition comprising the antibody specifically binding toK. *pneumoniae*O2 antigen, antibody specifically binding toK. *pneumoniae*O1 antigen, or bispecific antibody specifically binding toK. *pneumoniae*O2 antigen and O1 antigento an individual for treating or preventing a *K. pneumoniae* infection in an individual.

The kits of the application are in suitable packaging. Suitable packaging includes, but is not limited to, vials, bottles, jars, flexible packaging (e.g., sealed Mylar or plastic bags), and the like. Kits may optionally provide additional components such as buffers and interpretative information. The present application thus also provides articles of manufacture, which include vials (such as sealed vials), bottles, jars, flexible packaging, and the like.

The instructions relating to the use of the antibody specifically binding toK. *pneumoniae*O2 antigen, antibody specifically binding toK. *pneumoniae*O1 antigen, or bispecific antibody specifically binding toK. *pneumoniae*O2 antigen and O1 antigen compositions generally include information as to dosage, dosing schedule, and route of administration for the intended treatment. The containers may be unit doses, bulk packages (e.g., multi-dose packages) or sub-unit doses. For example, kits may be provided that contain sufficient dosages of an antibody specifically binding toK. *pneumoniae*O2 antigen, antibody specifically binding toK. *pneumoniae*O1 antigen, or bispecific antibody specifically binding *toK. pneumoniae*O2 antigen and O1 antigen (such as a full-length antibody specifically binding toK. *pneumoniae*O2 antigen, full-length antibody specifically binding toK. *pneumoniae*O1 antigen, or full-length bispecific antibody specifically binding toK. *pneumoniae*O2 antigen and O1 antigen) as disclosed herein to provide effective treatment of an individual for an extended period, such as any of a week, 8 days, 9 days, 10 days, 11 days, 12 days, 13 days, 2 weeks, 3 weeks, 4 weeks, 6 weeks, 8 weeks, 3 months, 4 months, 5 months, 7 months, 8 months, 9 months, or more. Kits may also include multiple unit doses of the antibody specifically binding toK. *pneumoniae*O2 antigen, antibody specifically binding *toK. pneumoniae*O1 antigen, or bispecific antibody specifically binding toK. *pneumoniae*O2 antigen and O1 antigen and pharmaceutical compositions and instructions for use and packaged in quantities sufficient for storage and use in pharmacies, for example, hospital pharmacies and compounding pharmacies.

Those skilled in the art will recognize that several embodiments are possible within the scope and spirit of this application. The application will now be described in greater detail by reference to the following non-limiting examples. The following examples further illustrate the application but, of course, should not be construed as in any way limiting its scope.

### EXAMPLES

In the following disclosed embodiments, KP19173 represents O1 serotype of Klebsiella pneumoniae containing D-gal II and D-gal I repeating units in LPS(Fig.1); KP19213 represents O1 serotype of Klebsiella pneumoniae containing D-gal II and D-gal III repeating units in LPS(Fig.1); KP19180 represents O2 serotype of Klebsiella pneumoniae containing D-gal I repeating units in LPS(Fig. 2); KP19203 represents O2 serotype of *K. pneumoniae* containing D-gal III repeating units in LPS(Fig. 2). The anti-K. *pneumoniae*O antigen antibody G3-78 (binding to D-gal III repeating units) disclosed in publication CN107371365A, and/or anti-K. *pneumoniae*O1 antigen antibody MPG196 (binding to D-gal II repeating units) disclosed in publication WO2018/029356 A1 were used as control antibodies in the embodiments of the present invention.

### Example 1: Generation of lipopolysaccharide (LPS), O2 antigen and selection of anti-O2 single chain antibodies(scFv)

### Crud extraction of LPS

Klebsiella pneumoniae O2 strain single colony were inoculated into 2x YT medium for amplification. Bacteria were collected by centrifugation at 4000 rpm at room temperature and the supernatant was discarded. The bacteria precipitate was resuspended in sterile water, then frozen at -80°C, thawed three times and disruptedcell wall by ultrasonic. LPS was extracted by hot phenol-water method: same volume of 90%(w/v) phenol solution was added to bacterial suspension and stirred continuously under 68°C water bath conditions for 30min and then removed. The mixture was cooled down to room temperature and centrifuged at 4000 rpmfor 30 min. After centrifugation, the solution was separated into 3 layers. From top to bottom, there were aqueous layer, phenolic layer and the insoluble layer, respectively. The upper aqueous layer contained LPS, the middle layer contained denatured protein and the lower layer was pellet. The upper aqueous layer was carefully pipetted into a new tube and 6 volumes of 95% ethanol were added along with sodium acetate at a final concentration of 0.1% (sinopharm, 10018892). The mixture was at-20 °C overnight to precipitate LPS,then centrifuged at 4000 rpm for 30 min. The precipitation was KP19180 strain O2-type LPS crude extract.

### Purification of LPS

The KP19180 strain O2-type LPS crude extract was reconstituted with 100mM Tris-HCI (pH 8), DNaseI(Solarbio, D8071-25 mg) at a final concentration of 100µg/mLand RNaseA (Solarbio, R8020-25 mg) were added and digested overnight at 37 °C. The digested LPS solution was boiled in water for 10min, cooled to roomtemperature, and then Proteinase K (Roche, 14059723) at a final concentration of 100µg/mL was added,65°Cfor 2 h. Added water saturated phenol, mixed well and centrifuged at4000rpm for 30min, collected supernatant to dialysis bag, and dialyzed with distilled water at 4°C overnight. Placed the dialyzed solution in a freeze-drying machine for freeze-drying, weighed the dry weight, and stored at -20°C. Quality tests such as SDS-PAGE, DNA residue, RNA residue, and protein concentration on LPS pure was carried out, and LPS endotoxin activity tests was carried out to determine the endotoxin activity.

### Generation of KP19180O2 antigen

1g KP19180 strain O2-type LPS prepared from the above procedure was added to 200 mL of 1% acetic acid and put the mixture in a boiling water bath for 45 min. After cooling, the mixture was ultracentrifuged at 105000g, 4°C for 2 h. The pellet was lipid A and the supernatant was O2 antigen. The supernatant was taken, and the O2 antigenprepared above was concentrated by using vacuum centrifugation method and then dissolved in water for further use.

### Generation of biotinylated labeled O2 antigen

The appropriate amount of O2 antigen was added to a 3 kDa ultrafiltration tube and centrifuged at 4000 rpm for 20-30 min. 0.2M boric acid buffer (pH=9.0) was added to the ultrafiltration tube, centrifuged at 4000 rpm, and the step as a whole was repeated 3 times. The O2 antigen was dissolved in 0.2M boric acid buffer (pH=9.0), the concentration of polysaccharides in the O2 antigen solution were measured by phenol-sulfuric acid colorimetry, and the total amount of polysaccharides was calculated from the volume of the solution (Zhang qing, Zhang tianmin. Phenol-sulphuric acid colorimetric method to determine the content of polysaccharides [C]. Proceedings of the National Academic Conference on Biochemical New Drug Research and Clinical Applications of the Chinese Pharmaceutical Association. Qingdao: Chemical and Biotechnology Drug Professional Committee of the Chinese Pharmaceutical Association, 2004: 176-178.). The appropriate amount of Amine-PEG11-Biotin (Thermo, 26136) was added to the O2 antigen solution according to a mass ratio of Amine-PEG11-Biotin to O2 antigen of 1.35 :1. The mixture was incubated at 37°C for 4 days and 180µL ALD Coupling Buffer (Sterogene, 9704) was added to each milliliter of reaction solution at 1 h, 24 h, 48 h, 72 h, respectively. The solvent of biotinylated O2 antigen was replaced with PBS using a 3KDa ultrafiltration tube and the resulting solution was biotinylated O2 antigen (designated Bio-O2 antigen).

### Selection of single chain antibodies(scFv) specifically binding toK. pneumoniaeO2 antigen

Construction of scFv Antibody yeast display library: RNAs collected from 2000 human blood samples were reverse-transcribed into cDNA. V_{H} and V_{L} fragments were amplified using V_{H}-and V_{L}-specific primers. Upon gel extraction and purification, scFv was generated by linking V_{H} and V_{L}. These scFvs were cloned into the yeast display plasmid PYD1, which were then transduced into yeasts by electroporation to generate the scFv antibody yeast display library.

Selection of single chain antibodies(scFv) specifically binding toK. *pneumoniae*O2 antigen: After several rounds of panning, scFvs which specifically bound to *K. pneumoniae*O2 antigen were isolated from the yeast display library. Briefly, magnetic-activated cell sorting (MACS) was used to enrich yeast cells that bound to *K. pneumoniae*O2 antigen. Yeast cells were centrifuged at 2500g for 5min and cell pellets obtained were resuspended in 1L SGCAA medium at an initial concentration of OD600=1, and induced expression was conducted at 20 °C, 250 rpm for 40-48 hours. The cell culture medium was centrifugated and washed with PBSM solution, then the cell pellet was resuspended in 5-10 volumes of PBSM solution containing 1µM Bio-O2 antigen and incubated at 4 °C for 1 hour. After centrifugation and PBSM washing, unbound antigen was washed away with PBSM solution. Cells were mixed with magnetic beads and incubated on a rotary evaporator at 4 °C for 30 minutes. After centrifugation at 2500g for 5 minutes, the supernatant was discarded and the pellet was resuspended in 5-10 volumes of PBSM solution. 7 mL of cell suspension was added to the column each time until all of the cell suspension was passed through the column. The cells bound to the column was eluted and collected for culture and subsequent FACS sorting.

Single chain antibodies (scFv) specifically binding toK. *pneumoniae*O2 antigen were screened by FACS: Fluorescence-activated cell sorting (FACS) was performed on the enriched yeast after panning with MACS. Briefly, yeast cell pellets induced in SGCAA medium were washed with 1mL PBSM and centrifuged at 14000g for 30 sec. Yeast cells were resuspended in 100µL PBSM buffer containing KP19180 strain Bio-O2 antigen and incubated for 1 h at room temperature. After washing, cells were stained with Streptavidin-PE (BDBiosciences, 554061) and anti-V5 tag antibody [iFluor 488](GenScript, A01803-100), and the top 1% of double-positively stained cells were screened and sorted into culture medium for cell expansion. The Bio-O2 antigen obtained from strain KP19180 was applied for screeningfor 2-3 cycles. Single clones were detected by further FACS analysis. At the end of the screening, a series of positive scFv antibodies were obtained and ELISA binding assay was carried out.

### ELISA Binding Assay

ELISA binding assay was carried out with O2-type LPS prepared by KP19180 strain to identifying antibodies binding to KP19180 O2-type LPS. Briefly, LPS was dissolved in PBS (pH adjusted to 7.2, final concentration 0.2µg/mL) and coated on 96-well plates with 100µgL/well followed by incubation at 4 °C overnight. The 96-well plates were washed 5 times with 200µL/well PBST before antibodies were added. 200µL 10% BSA (Beyotime Biotechnology, ST023-200 g) was added to each well and incubated at 37 °C for 1h. The wells were washed 5 times with PBST. Firstly, each antibody sample was diluted to 1µg/mL, and then serially diluted at a ratio of 1:3. 50µL of serially diluted antibodies were added to each well and incubated for 1 h at 37°C. After washing with PBST for 5 times, 100µL of the mixture of primary antibody and secondary antibody (mouse anti-flag (1:2500) and anti-mouse FC-AP (1:20000)) was added to each well and incubated for 1h at 37°C. After washing with TBST for 3 times. PNPP was added to each well and incubated for 10-20 minutes at 37°C. 3M NaOHwas used to stop the reaction and plates were read for the absorbance at 450 nm. Binding curves were generated by Graphpad Prism and EC₅₀ values were calculated.

### Example 2: Generation and characterization of antibodiesspecifically binding toK. pneumoniaeO2 antigen

### Generation of full-length antibodiesspecifically binding toO2 antigen

The positive scFv antibodies were reformatted as full-length antibody molecules with a human IgG1 or IgG4 heavy chain constant domain, and a human kappa light chain constant domain or human lambdalight chain constant domain. V_{H} and V_{L} were amplified from the yeast expression vectors and introduced into eukaryotic expression vectors pTTS-L1 (containing kappa constant domain or containing lambdaconstant domain) and pTT5-H1 (containing IgG1 heavy chain constant domain), or pTT5-H4 (containing IgG4 heavy chain constant domain), respectively. Plasmids expressing the light or heavy chains were extracted and used to co-transfect 293F cells. After the cells were cultured at 37°C, 8% CO₂ and 120rpm for 5 days, the culture media was purified using Protein A affinity chromatography. Briefly, Protein A column was first equilibrated with 50mM PBS buffer (containing 0.15M NaCl, pH7.2) at a flow rate of 150cm/h. The supernatant of the culture media (pH was adjusted to 7.2) was passed through the column at 150cm/h. Upon further equilibration, 50mM sodium citrate (pH3.5) was used to wash the column and the elution was collected. The obtained full-length antibodies were then subjected to further biochemical and biological analysis.

### ELISA Binding Assay

The full-length antibodies prepared above were further detected by ELISA. The operation was same as Example 1. The difference was that the secondary antibody used was goat anti-human IgG- HRP (Beyotime Biotechnology, A0201) (1:10000)and incubated at 37°C for 1 hour, after adding the full-length antibodies to be tested. Washed by PBST for 5 times. TMB (southern biotech, 0410-01) was added with 100 µL/well and incubated for 10-20 minutes at 37°C. 2M H₂SO₄ was used to stop the reaction and plates were read for the absorbance at 450 nm. Binding curves were generated by Graphpad Prism and EC₅₀ values were calculated.

### LPS neutralization assay

The commercial reporter cell line HEK-Blue^{™} hTLR4(Invivogen, hkb-htlr4) stably expresses human TLR4, MD-2 and CD14 co-receptors, as well as NF-κB-induced secreted embryonic alkaline phosphatase (SEAP). Bacterial LPS can trigger Toll-like receptor 4 (TLR-4) signaling in this cell line, resulting in activation of downstream NF-κB transcription factors, thereby SEAP was secreted. QUANTI-Blue^{™} substrate(Cat # rep-qbs) can be used to detect and quantify SEAP activity. Briefly, according to the manufacture's protocol, HEK-Blue^{™}hTLR4 cells were maintained and seeded into 96 well culture plates with 2.5×10⁴ cells/well. 10µL KP19180LPS prepared in Example 1 (10µg/mL) and serial dilution of full-length antibodies specifically binding toK. *pneumoniae*O2 antigen (reconstitute as human IgG1 form) at an initial concentration of 1mg/ml were pre-mixed, added into the 96 well culture plates, and incubation at 37°C, 5% CO₂ for 6-16h. 40µL cell supernatant were mixed with 160µL pre-warmed QUANTI-Blue^{™} (Invivogen) solution, and incubated for 60-90 minutes. The absorbance at 620-655nm was read. In this experiment, the absorbance value of the wells stimulated with LPS only without antibody was used as the reference for the inhibition rate of 0%, and the absorbance value of the wells without LPS stimulation was used as the reference for the inhibition rate of 100%. According to the absorbance values of antibodies specifically binding toK. *pneumoniae*O2 antigen at different concentration, inhibition rates were calculated at different concentrations, inhibition curves were generated by Graphpad Prism, and IC₅₀ values were calculated.

### affinity maturation

According to the result of ELISA binding assay and the LPS neutralization assay of the full-length antibody molecules, K1 was selected as lead antibody, scFv phage display library containing CDR region mutation was prepared by using K1 scFv. The antibody clones specifically binding to *K. pneumoniae* O2 antigen with higher affinity and higher neutralization activity were selected through KP19180 LPS ELISA binding assay and LPS neutralization assay, and full-length antibodies were further constructed.Biochemical and biological analysis was performed on the lead antibodies and optimized antibodies.

Theprocedures for ELISA binding assay and KP19180 LPS neutralization assay were described above. The result of the binding ability and neutralizing activity of the lead antibodies and the optimized antibodies are shown in Table 11.

As shown in table 11, after affinity maturation, a series of optimized antibodies were able to bind to KP19180 O2-type LPS and retain neutralization activity against KP19180 O2-type LPS, and both binding ability and neutralization activity were improved compared to K1.

**Table 11**

| **Sample Name** | **Binding Ability EC50 (ng/mL)** | **Neutralizing Activity IC50 (µg/mL)** | **Sample Name** | **Binding AbilityEC50 (ng/mL) ng/mL)** | **Neutralizing ActivityIC50 (µg/mL )** |
|---|---|---|---|---|---|
| K1 | 126 | 1.778 | K4 | 108.8 | 0.6211 |
| K2 | 103.7 | 0.7188 | K5 | 83.66 | 1.421 |
| K3 | 105.6 | 0.3656 | | | |

### Example 3: Binding domain analysis for antibodies specifically binding to K. pneumoniae O2 antigen

*K. pneumoniae* is classified into different LPS serotypes according to its unique O antigenstructure. O1 and O2 serotype strains are currently common clinically prevalent strains.

Meanwhile, the O1 serotype also comprises 2 structures, which are represented by KP19173 andKP19213 respectively, the structure schematic diagram was shown in FIG. 1, and the O2 serotype alsocomprises 2 structures, which are represented by KP19180 and KP19203 respectively, and the structure schematic diagram was shown in FIG. 2.

Antibodies K2, K3 and K5 were selected for binding domain analysis experiments. The binding abilityofthe antibodies to the above four*K*. *pneumoniae* LPS with different O antigen structures was determined by ELISA, and the preparation method of the different *K*. *pneumoniae*LPS and the procedureof ELISA assay were described in Example 1.

ELISA binding assay confirmed that the antibodies K2, K3 and K5 specifically bindingto *K. pneumoniae* O2 antigen can specifically bind to KP19180 strain O2 LPS, as shown in FIG. 3A;the binding of an exemplary antibody specifically binding to *K. pneumoniae*O2 antigen to KP19203 strainO2 LPS was further determined, G3-78 was a control antibody in this experiment. As shown in FIG. 3B, theexemplary antibodyspecifically binding toK. *pneumoniae* O2 antigen did not bind to KP19203 strain O2LPS.

The binding of the antibodies to KP19173 strain O1 LPS and KP19213 strain O1LPS were further detected, as shown in FIG. 3C, the exemplary antibody specifically binding to *K. pneumoniae* O2 antigen canspecifically bind to KP19173 strain O1 LPS; as shown in FIG. 3D, MPG196 is a control antibody in thisexperiment, and the exemplary antibodyspecifically binding to *K. pneumoniae* O2 antigen did not bindto KP19213 strain O1 LPS.

According to the structural composition of different serotype strains described above, and the results of ELISAbinding assay, it was deduced that the antibodies specifically binding to *K. pneumoniae* O2 antigendescribed herein specifically bind to D-galactan I domain in *K. pneumoniae* Oantigen and did not bind to the D-galactan II and D-galactan III domains.

### Example 4: Broad binding spectrum of antibodies specifically binding to K. pneumoniae O2 antigen to O2 serotype K. pneumoniae

The binding abilityof antibodies specifically binding to *K. pneumoniae* O2 antigentoLPS from other clinically isolated *K. pneumoniae* O2 serotype strains (KP 19002, KP19003, KP19005 and KP19007 strain) were detected by the ELISA binding assay described in Example 1, the O2 serotype strains LPS described above all expressed D-galactan I structure.

As shown in FIG. 4A-4D, exemplary antibodiesK2, K3 and K5specifically binding to *K. pneumoniae* O2 antigencould bind to LPS from other O2serotype *K. pneumoniae*strains KP19002 (FIG. 4A), KP19003 (FIG. 4B), KP19005 (FIG. 4C), or KP19007 (FIG. 4D), which indicated that the binding of antibodies specifically binding to *K. pneumoniae* O2 antigen to *K. pneumoniae* O2 serotype strains expressed D-galactan I structure wasbroad spectrum.

### Example 5: Prophylactic protective effect of antibodies specifically binding to K. pneumoniae O2 antigen in mouse bacteremia model

C57BL6 mice were purchased from Charlesrivar and raised in an environment without specificpathogens. All animal experiments were conducted in accordance with the Institutional Animal Care and UseCommittee (IACUC) protocol and guidelines.

Mouse *K. pneumoniae* bacteremia model was prepared as described in the literature (seeCross-specificity of protective human antibodies against Klebsiella pneumoniae LPS O-antigen, Nat Immunol. 2018 Jun;19(6):617-624.).

Specifically, KP19180 strain suspension in logarithmic phase was centrifuged at 4000rpm for 10min at4°Cand the supernatant of the culture was carefully discarded, sterilized PBS was added, resuspended byvortexing, and the supernatant was discarded after centrifugation and repeated 2 times. Adding sterilized PBS forresuspension, and adjusting the concentration of bacterial suspension to 1×10⁸ CFU/100µL. Firstly, micewas injectedintraperitoneally with 100µLD-(+)-galactosamine hydrochloride(GalN, 200 mg/mL in pH=7.4 PBS, sigma,G1639-5G), then injected intravenously with 300 µl KP19180 bacterial suspension. The dosage was taken as the modeling dosage in the subsequent experiments.

To determine whether antibodies specifically binding to *K. pneumoniae* O2 antigen had a prophylactic protective effect, different concentrations of K2 or K5antibodies specifically binding to *K. pneumoniae* O2 antigen(reformatted as human IgG1), the dosages of which were 15 mpk or 30 mpk and negative reference antibody HIV referred as HIV-10E8 herein at a dosage of 30 mpk(Broad and potent neutralization of HIV-1 by a gp41-specific human antibody Nature 491 (7424), 406-412 (2012)) were injected intraperitoneally 24h prior to mouse modeling. After 24h, mouse *K. pneumoniae* bacteremia model was prepared as the method described above. Each group of mice was observed twice daily and the number of death, time of death and status of surviving mice were recorded for up to 8 days for each group. Mice mortality was determined using Graphpad Prism.

As shown in FIG. 5, in bacteremia model induced by KP19180 strain, compared with negative reference antibody HIV-10E8, exemplary antibodiesK2 and K5specifically binding to *K. pneumoniae* O2 antigen all could effectively improve the survival rate and/or prolong the survival time of mice no matter at a low dosage of 15mpk or a high dosage of 30mpk. When K5 antibody was administered at a dose of 30mpk, death did not occur until 72h, and survival rate of mice was maintained at around 70% until 144h at the end of the experiment. Thus, the antibodies specifically binding to *K. pneumoniae* O2 antigen had a prophylactic protective effect to *K. pneumoniae*bacteremia*.*

### Example 6: The combination therapeutic effect of antibodies specifically binding to K. pneumoniae O2 antigen and antibodies specifically binding to K. pneumoniae O1 antigen in mouse pneumonia model

Mouse pneumonia model was constructed, and the mousepneumonia infected by *K*. *pneumoniae*was treated by combination therapy of antibody specifically binding toK. *pneumoniae* O2 antigen andantibody specifically binding toK. *pneumoniae* O1 antigen. The mice of each group were treatedwith negative control antibody HIV-10E8, or with K5 antibody specifically binding to *K. pneumoniae* O2antigen in combination with G2 antibody specifically binding to *K. pneumoniae* O1 antigen, at thefollowing doses: K5+G2 (1.5mpk+1.5mpk), and K5+G2 (15mpk+15mpk), negative HIV-10E8 was applied at a doseof 30mpk.

Preparation of mouse pneumonia model: briefly, C57BL6 mice were purchased from Charlesrivar and raised in an environment without specificpathogens. All animal experiments were conducted in accordance with the Institutional Animal Care and UseCommittee (IACUC) protocol and guidelines. The experiment adopted a lungdelivery way to challengethe mice, and constructed a mice pneumonia model infected by *K. pneumoniae.* Specifically, the mice were firstly anesthetized and placed on an inclined plate, the mouth was opened,while the micethroat was illuminated by anesthetic laryngoscope held by the left hand, so that the throat was clearlyvisible, a fixed amount of bacterial fluid was aspirated withmicro-fluid intratracheal atomizer (KP 19173,od600=0.32), then the nozzle was gently inserted into the trachea, and the syringe was rapidly injected to spray the bacterial fluid into the trachea (Using Klebsiella pneumoniae to Model Acute Lung Inflammation in Mice, Methods Mol Biol. 2019;1960:169-180.). The bacterial fluid delivery dose was 25 µl/mouse. After the bacterial liquiddelivery was completed, the mice were put back into the cage, and after 1h, antibodies specifically binding toK. *pneumoniae* O2 antigen and antibodies specifically binding to *K. pneumoniae* O1 antigen were simultaneously injected through tail veins of the mice, and the mice in the negative control group were injected with HIV-10E8. After the antibody injection was completed, the mice were continuously observed to be awake. Each group of mice was observed twice daily and the number of deaths, time of death and status of surviving mice were recorded for up to 8 days for each group. Survival data for each group of mice was plotted in GraphPad Prism software to determine mice mortality.

As shown in FIG. 6, when the antibody specifically binding to *K. pneumoniae* O2 antigen was used in combination with the antibody specifically binding to *K. pneumoniae* O1 antigen, the antibody had a therapeutic effect on mice pneumonia caused by *K. pneumoniae* infection , and even when the antibody was used at a dose of 1.5mpk+1.5mpk, the mice survival rate was maintained at about 70% after pneumoniaeinfected 144h, and the result was statistically significant (p < 0.05) compared with the negative control.

### Example 7: Preparation of bispecific antibodies specifically binding toK. pneumoniae O2 antigen and O1 antigen of different formats

In the following sequence design: V_{H} And V_{L}represent the heavy chain variable domain and the lightchain variable domain of an antibody, respectively; C_{H}represents the antibody heavy chain constant domain, including C_{H}1, C_{H}2 and C_{H}3 domain; scFv is an antibody formed by linking V_{H} and V_{L} of the antibody through a peptide linker(Linker); IgGlFc represents the Fc region of an IgG1 subclass antibody, comprising C_{H}2 and C_{H}3 domain; C_{L}represents the light chain constant domain.

### 7.1 Constructionof DVD-Ig formatbispecific antibodies:

1) Sequence design: DVD-Ig (Dual-variable domain-Ig) bispecific antibody, the formatof which is connecting the V_{L} and V_{H} domains of another antibody respectivelyto the N-terminus of the V_{L} and V_{H} of a normal IgG antibody, formingan antigen-binding domain by the interaction between V_{H} and V_{L} of two antibodies, which can simultaneously bind to the corresponding antigen to achieve bispecific. DVD-Ig formatbispecificantibodies are described in literature Wu C, et al. Molecular construction and optimization of anti-human IL-1alpha/beta dual variable domain immunoglobulin (DVD-Ig) molecules. MAbs. 2009 Jul-Aug;1(4):339-47. The schematic diagramof the format was shown in FIG. 7A. Table 12-1 showed the composition designof DVD-Ig format bispecific antibodies used in the examples, and the amino acid sequences ofthe heavy chainsand light chains thereof were shown in Table 8-1. Kingsray Biotechnology was entrusted to synthesize the genes encoding the light chain (L chain) and heavy chain (H chain) of bispecific antibodies, respectively, and coding genes were optimized to be suitable for expression in 293F cells.
2) Expression and purification of bispecific antibodies: after cleavage by restriction enzyme, the genes encodinglight chains andheavy chains of bispecific antibodies were subcloned into pTTα1 vector, respectively.Recombinant plasmids expressing light chains andheavy chains of the antibodies were extracted and co-transfected into 293F cells. Cultured at37 °C,5% CO₂, 120rpm for 7 days, culture solution was purified by protein A affinity chromatography column. Briefly, the protein A column was first balanced with 6 columnvolumes of 50mM PBS buffer (pH 7.2) containing 0.15M NaCl at a flow rate of 150 cm/h. The pH of the culturesupernatant was adjusted to 7.2 and the sample was applied at a flow rate of 150 cm/h. After loading, the columnwas balanced and finally eluted with 50mM citrate-sodium citrate buffer (pH 3.5) and the eluate was collected. Concentrated with 30kDa ultrafiltration tube and replaced the buffer with PBS. After endotoxin removal by endotoxin removal kit (SA 031K, Changzhou Smart Lifesciences), protein concentration andendotoxin content were measured, respectively. The endotoxin-removed DVD-Igformat bispecific antibody samplesspecifically binding to *K. pneumoniae* O2 antigen and O1 antigen were subjected to reduced and non-reduced SDS-PAGE electrophoresis, respectively (results not shown), to ensure correct formation of structures.

**Table 12-1: Composition design of heavy chains and light chains of DVD-Ig formatbispecific antibodies specifically binding to K. pneumoniae O2 antigen and O1 antigen**

| **Bispecific antibody format** | **Antibody name** | **Heavy chain** | **Light chain** |
|---|---|---|---|
| **DVD-Ig** | G7-K5-Ig1 | G7V_{H}-K5V_{H}-IgGlC_{H} | G7V_{L}-K5V_{L}-C_{L} |
| | G2-K5-Ig1 | G2V_{H}-K5V_{H}-IgG1C_{H} | G2V_{L}-K5V_{L}-C_{L} |
| | K5-G2-Ig1 | K5V_{H}-G2V_{H}-IgG1C_{H} | K5V_{L}-G2V_{L}-C_{L} |
| | K5-G7-Ig1 | K5V_{H}-G7V_{H}-IgG1C_{H} | K5V_{L}-G7V_{L}-C_{L} |

### 7.2Constructionof Bs4Abformatbispecific antibodies:

1) Sequence design: Bs4Ab structure bispecific antibody contains a full-length IgG1 structure, the structure of which achieves bispecific by insertion of another binding unit scFv in its hinge region. Bs4Ab structure bispecificantibodies are described in literature Bezabeh B, et al. Insertion of scFv into the hinge domain of full-length IgG1 monoclonal antibody results in tetravalent bispecific molecule with robust properties. MAbs. 2017 Feb/Mar;9(2):240-256. The schematicdiagramof the format was shown in FIG. 7B. Table 12-2 showed the composition designof Bs4Ab structurebispecific antibodies used in the examples, and the amino acid sequences ofthe heavy chainsand light chains thereof were shown in Table 8-2. Kingsray Biotechnology was entrusted to synthesize the genes encoding the light chain (L chain) and heavy chain (H chain) of bispecific antibodies, and coding genes were optimized to be suitable for expression in 293F cells.
2) Expression and purification of bispecific antibodies: the specific procedurewas as described in 7.1.

**Table 12-2: Composition designof heavy chains and light chains of Bs4Abformat bispecific antibodies specifically binding to K. pneumoniae O2 antigen and O1 antigen**

| **Bispecific antibody form** | **Antibody name** | | **Heavy chain** | | **Light chain** |
|---|---|---|---|---|---|
| **Bs4Ab** | | G7-K5scFv-Ig1 | | G7V_{H}-C_{H}1-K5scFv-IgG1Fc | G7V_{L}-C_{L} |
| | | G2-K5scFv-Ig1 | | G2V_{H}-C_{H}1-K5scFv-IgG1Fc | G2V_{L}-C_{L} |
| | | KS-G2scFv-Ig1 | | K5V_{H}-C_{H}1-G2scFv-IgG1Fc | K5V_{L}-C_{L} |
| | | K5-G7scFv-Ig1 | | K5V_{H}-C_{H}1-G7scFv-IgG1Fc | K5V_{L}-C_{L} |

### 7.3Construction of Hetero H, CrossMabformatbispecific antibodies:

1) Sequence design: Hetero H, CrossMabformatbispecific antibody, i.e.,the knob-in-hole structure (KIH) isdesigned in the Fc region, introduces two Cys residue mutations that form stable disulfide bridges (S354C on the "knob" side and Y349C on the "hole" side). By KIHmethod, i.e., substitute threonine (T) at position 366 in antibody C_{H}3 domain with tryptophan (W) to form "knobs" structure, and substitute threonine (T) at position 366 with serine (S), leucine (L) at position 368 with alanine (A), tyrosine (Y) at position 407 with valine (V) in the C_{H}3 domain to form "holes" structure, which promotes dimerization of heterologous heavy chains by the decrease of steric hindrance effect after mutation and the formation of covalent disulfide bonds in the hinge region, wherein the numbering isin accordance with EU index ofKabat; the CrossMab technologyis also applied to ensure proper pairingbetween the light chainsand heavy chains of antibody. CrossMAb technology is based on the exchange of domains in one Fab arm of bispecific IgG antibodies, either as an exchange of intact Fab domains (CrossMAbFab), or as an exchange of only variable domains in the Fab (CrossMAbV_{H}-V_{L}) or constant region only(CrossMAb C_{H}1-C_{L}). Hetero H, CrossMabformatbispecificantibodies are described in literature Klein C, et al. The use of CrossMAb technology for the generation of bi- and multispecific antibodies. MAbs. 2016 Aug-Sep;8(6): 1010-20. The present application was specified in CrossMab C_{H}1-C_{L},format, the schematic diagramof the format was shown in FIG. 7C. Table 12-3 showed the composition designof Hetero H, CrossMabformat bispecific antibodies used in the examples, and the amino acid sequences ofthe heavy chainsand light chains thereof were shown in Table 8-3. Kingsray Biotechnology was entrusted to synthesize the genes encoding the light chain (L chain) and heavy chain (H chain) of bispecific antibodies, respectively, and coding genes were optimized to be suitable for expression in 293F cells.
2) Expression and purification of bispecific antibodies: the specific procedurewas as described in 7.1.

**Table 12-3: Composition designof heavy chains and light chains of Hetero H, CrossMabformatbispecific antibodies specifically binding to K. pneumoniae O2 antigen and O1 antigen**

| **Bispecific antibody form** | **Antibody name** | **Heavy chain** | **Light chain** |
|---|---|---|---|
| **Hetero H, CrossMab** | G7-K5-CrossMab-Ig1 | G7V_{H}-C_{L}-IgG1Fc-hole | G7V_{L}-C_{H}1 |
| | | K5V_{H}-IgG1C_{H}-knob | K5V_{L}-C_{L} |
| | G2-K5-Crossmab-Igl | G2V_{H}-C_{L}-IgG1Fc-hole | G2V_{L}-C_{H}1 |
| | | K5V_{H}-IgG1C_{H}-knob | K5V_{L}-C_{L} |

### 7.4Constructionof IgG-(scFv)₂formatbispecific antibodies:

1) Sequence design: **IgG-(scFv)₂formatbispecific** antibody, *i.e.,* bispecific is achieved by connecting the scFv fragment of another antibody to the Fc-terminus of two heavy chains of an IgG antibody.IgG-(scFv)₂formatbispecificantibodies are described in literature Coloma MJ, Morrison SL. Design and production of novel tetravalent bispecific antibodies. Nat Biotechnol. 1997 Feb;15(2):159-63.The schematicdiagram of the format was shown in FIG. 7D. Table 12-4 showed the composition designof IgG-(scFv)₂format bispecific antibodies used in the examples, and the specific amino acid sequences ofthe heavy chainsand light chains thereof were shown in Table 8-4. Kingsray Biotechnology was entrusted to synthesize the genes encoding the light chain (L chain) and heavy chain (H chain) of bispecific antibodies, and coding genes were optimized to be suitable for expression in 293F cells.
2) Expression and purification of bispecific antibodies: the specific entrustedwas as described in 7.1.

**Table 12-4: Composition designof heavy chains and light chains of IgG-(scFv)₂formatbispecific antibodies specifically binding to K. pneumoniae O2 antigen and O1 antigen**

| **Bispecific antibody form** | **Antibody name** | **Heavy chain** | | **Light chain** |
|---|---|---|---|---|
| **IgG-(scFv)₂** | G7-Ig1-K5scFv2 | | G7V_{H}-IgG1C_{H}-K5scFv | G7V_{L}-C_{L} |
| | G2-Ig1-K5scFv2 | | G2V_{H}-IgG1C_{H}-K5scFv | G2V_{L}-C_{L} |

### 7.5Constructionof scFv-Fab IgGformatbispecific antibodies:

1) Sequence design: scFv-Fab IgGformatbispecific antibody is heterodimeric antibody with IgG antibody structure, one Fab arm of which is replaced with scFv structure, wherein the first monomer contains scFv and Fc, scFv is connected to the N-terminus of Fc C_{H}2 domain by peptide linker, and the second monomer contains Fab and Fc.The schematic diagramof the format was shown in FIG. 7E. Table 12-5 showed the composition designof scFv-Fab IgG format bispecific antibodies used in the examples, and the amino acid sequences ofthe heavy chainsand light chains thereof were shown in Table 8-5. Kingsray Biotechnology was entrusted to synthesize the genes encoding the light chain (L chain) and heavy chain (H chain) of bispecific antibodies, and coding genes were optimized to be suitable for expression in 293F cells.
2) Expression and purification of bispecific antibodies: the specific procedurewas as described in 7.1.

**Table 12-5: Composition designof heavy chains and light chains of scFv-Fab IgGformat bispecific antibodies specifically binding to K. pneumoniae O2 antigen and O1 antigen**

| **Bispecific antibody form** | **Antibody name** | **Heavy chain** | **Light chain** |
|---|---|---|---|
| **scFv-Fab IgG** | G7-K5Xmab-Ig1 | G7V_{H}-IgG1C_{H} | G7V_{L}-C_{L} |
| | | K5scFv-IgG1Fc | |
| | G2-K5Xmab-Ig1 | G2V_{H}-IgG1C_{H} | G2V_{L}-C_{L} |
| | | K5scFv-IgG1Fc | |

### Example 8: EC50 values analysis of bispecific antibody binding to O1-typeLPS and O2-type LPS (D-galactan I)

ELISA binding assays were used to detect thebinding of bispecific antibodies to O1-type LPS (KP 19173strain O1-type LPS or KP19213 strain O1-type LPS) or O2-type LPS (KP 19180 O2-type LPS), respectively. Thepreparation method of *K. pneumoniae* LPS with different serotypes and specific procedure ofELISA binding assays were shown in Example 1. G2 and G7 antibody specifically binding to *K. pneumoniae* O1 antigen and K5 antibody specifically binding to *K. pneumoniae* O2 antigen were used ascontrol antibodies in the assay. Wherein K5 antibody did not bind to KP19213 strain O1-type LPS,and G2 and G7 antibody did not bind to KP19180 O2-type LPS. Briefly, 100µl LPS (0.2µl/mL, derivedfrom KP19173 strain, KP19213 strain or KP19180 strain, respectively) was added to each well of 96-well plate(Corning # 9018) and coated overnight at 4 °C. The next day, washed by PBST for 5 times (200µL/well), followedby 200µL 10% BSA (Beyotime Biotechnology, ST023-200 g) per well and incubated at 37°Cfor 1h. After washingby PBST 5 times (200. µL/well), Serially diluted bispecific antibody or control antibody was added to eachwell and incubated for 1h at 37 °C. After washingby PBST 5 times, 100µL diluted peroxidase labeled goat anti-human IgG (Beyotime Biotechnology, A0201) was added to each well and incubated at 37 °C for 1h. After washingby PBST 5 times (200. µL/well), TMB chromogenic substrate (Southern Biotech, 0410-01) was addedfor reacting 15min, the reaction was terminated with 2M H₂SO₄. The absorbance at 450nm (OD 450) wasmeasured with microplate reader (Spark 10M, Tecan). Binding curves were generated by GraphPad Prismand antibody binding EC50 values were calculated.

As shown in FIGS. 8A-8C, exemplary DVD-Ig format bispecific antibodieswere all able to bind to KP19173 strain O1-type LPS (FIG. 8A), KP19213 strain O1-type LPS (FIG. 8B), or KP19180 strain O2-type LPS (FIG. 8C).

As shown in FIGS. 8D-8F, exemplary Bs4Ab format bispecific antibodies were able to bind to KP19173strain O1-type LPS (FIG. 8D), KP19213 strain O1-type LPS (FIG. 8E), or KP19180 strain O2-type LPS (FIG. 8F).

As shown in FIGS. 8G-8I, exemplary Hetero H, CrossMabformat bispecific antibodies were able to bind toKP19173 strain O1-type LPS (FIG. 8G), KP19213 strain O1-type LPS (FIG. 8H), or KP19180 strain O2-type LPS (FIG.8I).

As shown in FIGS. 8J-8L, exemplary **IgG-(scFv)₂formatbispecific** antibodieswere able tobind to KP19173 strain O1-type LPS (FIG. 8J), KP19213 strain O1-type LPS (FIG. 8K), or KP19180 strain O2-type LPS (FIG. 8L).

As shown in FIGS. 8M-8O, exemplary scFv-Fab IgG format bispecific antibodies were able to bind toKP19173 strain O1-type LPS (FIG. 8M), KP19213 strain O1-type LPS (FIG. 8N), or KP19180 strain O2-type LPS (FIG.8O).

### Example 9: Neutralization activityassay of bispecific antibody againstK. pneumoniaeO1-type LPS and O2-type LPS (D-galactan I)

The neutralization activity of bispecific antibodies against O1 type LPS (KP 19173 strain O1 LPS orKP19213 strain O1 LPS) and O2 type LPS (KP 1918002 LPS) was detected using an LPSneutralization assay, respectively. See example 2 for specific procedures. Antibodies G2 and G7 that specifically bind toK. *pneumoniae* O1 antigen and antibody K5 that specifically bind toK. *pneumoniae* O2 antigenwere used as control antibodies inthis experiment. Wherein the antibody K5 has no neutralization activity toKP19213 strain O1 LPS and the antibodies G2 and G7 have no neutralization activity to KP19180 strain O2 LPS.Briefly, 180µL of HEK-Blue^{™} hTLR4cell was first added to each well in a 96-well plate (about 25,000 cells), then10µL LPS (10µg/mL, derived from KP19173 strain, KP19213 strain or KP19180 strain, respectively) andbispecific or control antibodies at different concentrations were added to each well, incubated at 37°Cfor 6-16h,and absorbance values at 620-655nm were measured using a microplate reader. The absorbance value of thewells without antibody added in this experiment was used as a reference with an inhibition rate of 0%, theabsorbance value of the wells without LPS added was used as a reference with an inhibition rate of 100%, theinhibition rates of bispecific antibodies at different concentrations were calculated, and the IC50 values werecalculated by Graphpad Prism software mapping.

As shown in FIG. 9A-9C, exemplary bispecific antibodies of DVD-Ig formatwere able to inhibit KP19173strain type O1 LPS (FIG. 9A), KP19213 strain type O1 LPS (FIG. 9B), or KP19180 strain type O2 LPS (FIG. 9C).

As shown in FIG. 9D-9F, exemplary bispecific antibodies of Bs4Ab formatwere able to inhibit KP19173strain O1-type LPS (FIG. 9D), KP19213 strain O1-type LPS (FIG. 9E), or KP19180 strain O2-type LPS (FIG. 9F).

As shown in FIG. 9G-9I, exemplary bispecific antibodies of the hereto H, crossMab formatwere able toinhibit KP19173 strain O1 LPS (FIG. 9G), KP19213 strain O1 LPS (FIG. 9H), or KP19180 strain O2 LPS (FIG. 9I).

As shown in FIGS. 9J-9L, exemplarybispecific antibodies of theIgG- (scFv)₂format were able toinhibitKP19173 strain O1 LPS (FIG. 9J), KP19213 strain O1 LPS (FIG. 9K), or KP19180 strain O2 LPS (FIG. 9L).

As shown in FIG. 9M-9O, exemplary bispecific antibodies of scFv-Fab IgG formatwere able to inhibitKP19173 strain O1-type LPS (FIG. 9M), KP19213 strain O1-type LPS (FIG. 9N), or KP19180 strain O2-type LPS (FIG.90).

### Example 10: Opsonophagocytic killing activity mediated by bispecific antibodies

Opsonophagocytic killing(OPK) activity refers to the death of cells (e.g., *K. pneumoniae*) that occurs as a result of phagocytosis of immune cells. Opsonophagocytic killingactivity wasmeasured using a bioluminescence assay according to the following experiment. This experiment was designed toevaluate bispecific antibody-mediated opsonophagocytickillingagainst *K. pneumoniae,* exemplifiedby the bispecific antibodies in DVD-Ig and Bs4Ab formats. The plasmid pUC18-mini-Tn7T-Gm-LUX expressingcomplete fluorescein was first electrotransformed into *K. pneumoniae* KP19173 to construct a luminescent*K*. *pneumoniae* strain KP19173-LUX, which is described in the literature (Choi K H, et al mini-Tn7insertionin bacteria with single attTn sites: example Pseudomonas aeruginosa [ J) ]Nature Protocols,2006,1 (1): 153-161).The OPK activity of the bispecific antibody was assessed by detecting changes in the Relative Light Units (RLU) ofthe luminous klebsiella strain by means of an enzyme-labelling instrument. OPK activity detection methods aredescribed in Wang Q, et al target-Agnostic Identification of Functional Monoclonal Antibodies Against K. pneumoniae Multimeric MrkA Fimbrial, subset.J. Effect Dis.2016Jun 1;213 1800-8. Briefly, the experiments wereperformed in 96-well plates, young rabbit serum was used as a complement source, and HL-60 cells differentiate into macrophages induced by DMF. Inoculating *K. pneumoniae* KP19173-LUX in logarithmic phase into96 plates, adding 5×10 differentiated macrophages, inactivated young rabbit serum (1: 10 Cedarlane),and bispecific antibody diluted at different concentration ratios per well, mixed well and incubated for 2h with shaking(250 rpm) at 37 °C. The Relative Light Units (RLU) were then measured using an enzyme-labeled instrument. The RLU value of the well without antibody was used as reference for opsonophagocytic killingactivity of 0%, the RLU value of the well to which the *K. pneumoniae* was added was used as a reference for opsonophagocytic killingactivity of 100%, theopsonophagocytic killingactivity (%) of the antibody at different concentrations was calculated from the RLUvalue of the well to which the bispecific antibody was added, and the IC50 value was calculated by GraphpadPrism software plotting.

As shown in FIGS. 10A-10B, the DVD-Ig format bispecific antibodies K5-G2-Ig1, K5-G7-Ig1 hadastrongactivity in promotingopsonophagocytickillingofKP19173 strain.

As shown in FIGS. 10C-10D, the Bs4Ab format bispecific antibodies K5-G2scFv-Ig1, K5-G7scFv-Iglantibodies had a strong activity in promotingopsonophagocytickilling of KP19173 strain.

The IC50 values of exemplary bispecific antibodies with different format in promotingopsonophagocytic killing of *K. pneumoniae* KP19173 wereshown in table 13.

**Table 13**

| Bispecific antibody | OPK IC50 (nM) | Bispecific antibody | OPK IC50 (nM) |
|---|---|---|---|
| K5-G2-Ig1 | 9.36E-03 | K5-G2scFv-Ig1 | 9.33E-05 |
| K5-G7-Ig1 | 1.21E-02 | K5-G7scFv-Ig1 | 7.78E-05 |

### Example 11: Serum bactericidal activitymediated by bispecific antibodies

Serum bactericidal activity (Serum Bactericidal Activity, SBA) experiments were designed to evaluatebispecific antibody-mediated complement-dependent serum bactericidal activity, exemplified by bispecificantibodies in the DVD-Ig and Bs4Ab formats. Firstly, a plasmid pUC 18 -mini -Tn7T-Gm -LUX expressing completefluorescein is electrotransferred into a *K. pneumoniae* strain KP19173 strain to construct a luminous*K*. *pneumoniae* strain KP19173-LUX. Bispecific antibody-mediated complement-dependent serumbactericidal activity was determined by detecting changes in the Relative Light Units (RLU) of *K. pneumoniae.* SBA detection method is disclosed in the literature anti-body-Mediated Killing of Carbapenemresistance ST258 *K. pneumoniae* by Human Neutrophils, mBio.2018Mar-Apr;9 (2) e 00297-18. Briefly, theexperiment was performed in 96-well plates, young rabbit serum was used as complement source, *K. pneumoniae* KP19173-LUX in log phase was pre-mixed with abispecific antibody gradient dilution with an initialconcentration of 1.3nM, and inoculated into 96-well plates, incubated at 37 °C with 5% CO₂for 15min. After adding 25µL of diluted young rabbit serum (1: 10 Cedarlane) and 35µL of PBS buffer per well, incubate for 3h at37°Cwith shaking (250 rpm). The Relative Light Units (RLU) were measured by a microplate reader. The RLUvalue of the wells without antibody was used as a reference for 0% of the serum bactericidal titer, the RLU value ofthe wells without *K*. *pneumoniae* was used as a reference for 100% of the serum bactericidal titer, theserum bactericidal titers of the antibodies at different concentrations were calculated from the RLU value of thewells with bispecific antibodies added, and the serum bactericidal activity IC50 values of the antibodies weredetermined by GraphPad Prism software plotting.

As shown in FIGS. 11A-11B, DVD-Ig format bispecific antibodies K5-G2-Ig1, K5-G7-Ig1 had antibody-mediated complement-dependent serum bactericidal activity against KP19173 strain.

As shown in FIGS. 11C-11D, Bs4Ab format bispecific antibodies K5-G2scFv-Ig1, K5-G7scFv-Ig1 hadantibody-mediated complement-dependent serum bactericidal activity against KP19173 strain.

The IC50 values of serum bactericidal activity of bispecific antibodies withdifferent formatagainst*K*. *pneumoniae* KP19173 were shown in table 14.

**Table 14**

| Bispecific antibody | SBA IC50 (nM) | Bispecific antibody | SBA IC50 (nM) |
|---|---|---|---|
| K5-G2-Ig1 | 9.78E-05 | K5-G2scFv-Ig1 | 1.39E-02 |
| K5-G7-Ig1 | 1.04E-04 | K5-G7scFv-Ig1 | 1.24E-02 |

### Example 12: Therapeutic effect of bispecific antibodies on K. pneumoniae infection (pneumonia)

C57BL6 mice were purchased from Vetong Liwa and raised in an environment without specific pathogens. All animal experiments were conducted in accordance with the Institutional Animal Care and Use Committee (IACUC) protocol and guidelines. Bispecific antibodies with DVD-Ig and Bs4Ab formats were exemplified. In order to detect whether the bispecific antibody had a therapeutic effect in a mouse *K. pneumoniae* infection pneumonia model, the experiment adopted a lung delivery route to challenge mouse, and a mouse *K*. *pneumoniae* infection pneumonia model was constructed. The specific preparation of the mouse model of pneumonia was described in Example 6. Wherein the bacterial liquid delivery dose was 25 µl KP19173 (od600=0.32)/mouse or 25 µl KP19180 (od600=0.5) bacteria solution/mouse. After the bacterial liquid delivery was completed, placing the mice back into a cage, and after 1 hour, injecting bispecific antibody K5-G2-Ig1 which specifically binds *K. pneumoniae* O2 antigen and O1 antigen through tail vein of the mice at a dose of 30mpk; or antibody K5 which specifically binds to *K. pneumoniae* O2 antigen at a dose of 30mpk; or an antibody G2 which specifically binds to *K. pneumoniae* O1 antigen at a dose of 30mpk; mice in the model group were injected with antibody Mab601 (30 mpk). Mab601 is an antibody that binds specifically to *K. pneumoniae* O3 mannose, described in literature Rollenske T, et al cross-specificity of protective human antibodies against K. pneumoniae LPS O-anti.nat immunol.2018jun;19 617-624, as described in (6). After the antibody injection was completed, the mice were continuously observed to be awake. Then observed 2 times per day until day 8, the number and time of death of each group of mice was recorded and the status of surviving mice was recorded. Survival data for each group of mice was plotted in GraphPad Prism software to determine the mortality of the mice.

As shown in FIG. 12A, in the *K. pneumoniae* KP19180 infection pneumonia model, compared with the model group, the bispecific antibody K5-G2-Ig1 significantly improved the survival rate of mice, and prolonged the survival time of mice.

As shown in FIG. 12B, bispecific antibody K5-G2-Ig1 provided 100% survival protection to mice in the *K. pneumoniae* KP19173 infection pneumonia model.

The results showed that the bispecific antibody of the invention had therapeutic effects on pneumonia caused by different *K. pneumoniae* strains.

### Example 13: Determination of affinity Kd value of antibodies specifically binding to K. pneumoniae O2 antigenand bispecific antibodies

The binding affinity of antibodies K4 and K5 (reconstituted into human IgG1 format) that specifically bound *K. pneumoniae* O2 antigen, bispecific antibodies K5-G2-Ig1,G7-K5-CrossMab-Ig1 and K5-G7scFv-Ig1 that specifically bound *K. pneumoniae* O2 antigen and O1antigen were tested with Biacore 3000 (GE). The method is a routine method well known to those skilled in the art, and experimental procedures areperformed according to the instructions for use. Briefly, the diluted O antigen of the biotinylated KP 19180 strain,the diluted O antigen of the KP 19173 strain or the diluted O antigen of the KP19213 strain were bound to the SAchip for 20s at a flow rate of 10µL/min, respectively. The affinity of the candidate antibodies was measured atdifferent concentrations, ranging from: 0M, 7.815E-10M, 1.563E-9M, 3.126E-9M, 6.252E-9M, 1.250E-8M, 2.501E-8M, 5.002E-8M. The binding and dissociation rates of the antibodies were measured using SPRtechniques and binding affinities were determined, and the Kon, koff, and Kd values of exemplary antibodies that specifically bind to *K. pneumoniae* O2 antigen and exemplary bispecific antibodies that specifically bind to *K. pneumoniae* O2antigen and O1 antigen bind to different *K. pneumoniae* strain O antigens werelisted in table 15 and table16, respectively.

**Table 15**

| **Ligand** | **K4antibody** | | |
|---|---|---|---|
| | Kon(1/Ms) | Koff(1/s) | Kd(M) |
| KP19173 Bio-O antigen | 5.84E+05 | 4.12E-03 | 7.058E-09 |
| KP19180 Bio-O antigen | 9.60E+05 | 2.13E-04 | 2.22E-10 |

| **Ligand** | **K5antibody** | | |
|---|---|---|---|
| | Kon(1/Ms) | Koff(1/s) | Kd(M) |
| KP19173 Bio-O antigen | 1.43E+06 | 5.22E-03 | 3.66E-09 |
| KP19180 Bio-O antigen | 9.43E+05 | 7.10E-04 | 7.53E-10 |

**Table 16**

| **Ligand** | **K5-G2-Ig1bispecific antibody** | | |
|---|---|---|---|
| | Kon(1/Ms) | Koff(1/s) | Kd(M) |
| KP19173 Bio-O antigen | 1.11E+06 | 1.51E-03 | 1.36E-09 |
| KP19180 Bio-O antigen | 9.55E+05 | 7.20E-04 | 7.54E-10 |
| KP19213 Bio-O antigen | 2.45E+06 | 8.85E-03 | 3.61E-09 |

| **Ligand** | **G7-K5-CrossMab-Ig1bispecific antibody** | | |
|---|---|---|---|
| | Kon(1/Ms) | Koff(1/s) | Kd(M) |
| KP19173 Bio-O antigen | 5.392E+5 | 1.160E-3 | 2.150E-9 |
| KP19180 Bio-O antigen | 3.431E+5 | 4.295E-4 | 1.252E-9 |
| KP19213 Bio-O antigen | 5.336E+5 | 2.023E-3 | 3.791E-9 |

| **Ligand** | **K5-G7scFv-Ig1bispecific antibody** | | |
|---|---|---|---|
| | Kon(1/Ms) | Koff(1/s) | Kd(M) |
| KP19173 Bio-O antigen | 5.212E+5 | 1.020E-3 | 1.96E-09 |
| KP19180 Bio-O antigen | 3.011E+5 | 4.015E-4 | 1.33E-09 |
| KP19213 Bio-O antigen | 5.216E+5 | 2.102E-3 | 4.03E-09 |

## Claims

1. An isolated antibody or antigen-binding fragment specifically binding to *Klebsiella pneumoniae* (*K. pneumoniae*) O2 antigen, comprising:
(i) a V_{H} comprising an HC-CDR1, an HC-CDR2, and an HC-CDR3 of a V_{H} comprising the amino acid sequence of SEQ ID NO: 17; and a V_{L} comprising an LC-CDR1, an LC-CDR2, and an LC-CDR3 of a V_{L} comprising the amino acid sequence of SEQ ID NO: 21;
(ii) a V_{H} comprising an HC-CDR1, an HC-CDR2, and an HC-CDR3 of a V_{H} comprising the amino acid sequence of SEQ ID NO: 18; and a V_{L} comprising an LC-CDR1, an LC-CDR2, and an LC-CDR3 of a V_{L} comprising the amino acid sequence of SEQ ID NO: 22;
(iii) a V_{H} comprising an HC-CDR1, an HC-CDR2, and an HC-CDR3 of a V_{H} comprising the amino acid sequence of SEQ ID NO: 19; and a V_{L} comprising an LC-CDR1, an LC-CDR2, and an LC-CDR3 of a V_{L} comprising the amino acid sequence of SEQ ID NO: 23;
(iv) a V_{H} comprising an HC-CDR1, an HC-CDR2, and an HC-CDR3 of a V_{H} comprising the amino acid sequence of SEQ ID NO: 19; and a V_{L} comprising an LC-CDR1, an LC-CDR2, and an LC-CDR3 of a V_{L} comprising the amino acid sequence of SEQ ID NO: 24; or
(v) a V_{H} comprising an HC-CDR1, an HC-CDR2, and an HC-CDR3 of a V_{H} comprising the amino acid sequence of SEQ ID NO: 20; and a V_{L} comprising an LC-CDR1, an LC-CDR2, and an LC-CDR3 of a V_{L} comprising the amino acid sequence of SEQ ID NO: 25.

2. The isolated antibody or antigen-binding fragment specifically binding to *K. pneumoniae* O2 antigen of claim 1, comprising:
(i) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 12, and an LC-CDR3 comprising the amino acid sequence of SEQ ID NO: 15, or a variant thereof comprising up to about 5 amino acid substitutions in the LC-CDRs;
(ii) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 9, an LC-CDR2 comprising the amino acid sequence of SEQ ID NO: 12, and an LC-CDR3 comprising the amino acid sequence of SEQ ID NO: 16, or a variant thereof comprising up to about 5 amino acid substitutions in the LC-CDRs;
(iii) a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 10, an LC-CDR2 comprising the amino acid sequence of SEQ ID NO: 13, and an LC-CDR3 comprising the amino acid sequence of SEQ ID NO: 16, or a variant thereof comprising up to about 5 amino acid substitutions in the LC-CDRs;
(iv) a V_{H} comprising an HC-CDR1 comprising the amino acid sequence of SEQ ID NO: 1, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16, or a variant thereof comprising up to about 5 amino acid substitutions in the LC-CDRs; or
(v) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 7, or a variant thereof comprising up to about 5 amino acid substitutions in the HC-CDRs; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15, or a variant thereof comprising up to about 5 amino acid substitutions in the LC-CDRs.

3. The isolated antibody or antigen-binding fragment specifically binding to *K. pneumoniae* O2 antigen of claims 1 or 2, comprising:
(i) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 21, or a variant thereof having at least about 80% sequence identity to the amino acid sequence of SEQ ID NO: 21;
(ii) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 22, or a variant thereof having at least about 80% sequence identity to the amino acid sequence of SEQ ID NO: 22;
(iii) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 23, or a variant thereof having at least about 80% sequence identity to the amino acid sequence of SEQ ID NO: 23;
(iv) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 24, or a variant thereof having at least about 80% sequence identity to the amino acid sequence of SEQ ID NO: 24; or
(v) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 25, or a variant thereof having at least about 80% sequence identity to the amino acid sequence of SEQ ID NO: 25.

4. An isolated antibody or antigen-binding fragment specifically binding to *K. pneumoniae* O2 antigen binds to *K. pneumoniae* with D-galactan I domain competitively with the isolated anti-O2 antigen antibody or antigen-binding fragment of any one of claims 1-3, or specifically binds to the same epitope as the isolated anti-O2 antigen antibody or antigen-binding fragment of any one of claims 1-3.

5. The isolated antibody or antigen-binding fragment specifically binding to *K. pneumoniae* O2 antigen according to any one of claims 1-4, wherein the antibody or antigen-binding fragment comprises an Fc fragment.

6. The isolated antibody or antigen-binding fragment specifically binding to *K. pneumoniae* O2 antigen of claim 5, wherein the antibody or antigen-binding fragment is a full-length IgA, IgD, IgE, IgG or IgM antibody.

7. The isolated antibody or antigen-binding fragment specifically binding to *K. pneumoniae* O2 antigen of claim 6, wherein the antibody or antigen-binding fragment is a full-length IgG1, IgG2, IgG3 or IgG4 antibody.

8. The isolated antibody or antigen-binding fragment specifically binding to *K. pneumoniae* O2 antigen of any one of claims 1-7, wherein the antibody or antigen-binding fragment is chimeric, human, or humanized.

9. The isolated antibody or antigen-binding fragment specifically binding to *K. pneumoniae* O2 antigen according to any one of claims 1-8, wherein the antigen-binding fragment is selected from the group consisting of Fab, Fab', F(ab)'2, Fab'-SH, single-chain Fv (scFv), Fv fragment, dAb, Fd, or diabody.

10. A bispecific antibody comprising a first antigen-binding domain specifically binding to *K. pneumoniae* O2 antigen, and a second antigen-binding domain specifically binding to *K. pneumoniae* O1 antigen, wherein the first antigen-binding domain comprises the antibody specifically binding to *K. pneumoniae* O2 antigen of any one of claims 1-9.

11. The bispecific antibody of claim 10, wherein the first antigen-binding domain comprises:
(a) a heavy chain variable domain (V_{H}) comprising a heavy chain complementarity determining region (HC-CDR) 1 comprising the amino acid sequence of SEQ ID NO: 1; an HC-CDR2 comprising the amino acid sequence of SEQ ID NO: 4; and an HC-CDR3 comprising the amino acid sequence of SEQ ID NO: 6; and a light chain variable domain (V_{L}) comprising a light chain complementarity determining region (LC-CDR) 1 comprising the amino acid sequence of SEQ ID NO: 11; an LC-CDR2 comprising the amino acid sequence of SEQ ID NO: 14; and an LC-CDR3 comprising the amino acid sequence of SEQ ID NO: 16; or
(b) a heavy chain variable domain (V_{H}) comprising a heavy chain complementarity determining region (HC-CDR) 1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 7; and a light chain variable domain (V_{L}) comprising a light chain complementarity determining region (LC-CDR) 1 comprising the amino acid sequence of SEQ ID NO: 10; an LC-CDR2 comprising the amino acid sequence of SEQ ID NO: 12; and an LC-CDR3 comprising the amino acid sequence of SEQ ID NO: 15.

12. The bispecific antibody of any one of claims 10-11, wherein the second antigen-binding domain comprises:
(a) a heavy chain variable domain (V_{H}) comprising a heavy chain complementarity determining region (HC-CDR) 1 comprising the amino acid sequence of SEQ ID NO: 35; an HC-CDR2 comprising the amino acid sequence of SEQ ID NO: 37; and an HC-CDR3 comprising the amino acid sequence of SEQ ID NO: 39; and a light chain variable domain (V_{L}) comprising a light chain complementarity determining region (LC-CDR) 1 comprising the amino acid sequence of SEQ ID NO: 41; an LC-CDR2 comprising the amino acid sequence of SEQ ID NO: 43; and an LC-CDR3 comprising the amino acid sequence of SEQ ID NO: 45; or
(b) a heavy chain variable domain (V_{H}) comprising a heavy chain complementarity determining region (HC-CDR) 1 comprising the amino acid sequence of SEQ ID NO: 36; an HC-CDR2 comprising the amino acid sequence of SEQ ID NO: 38; and an HC-CDR3 comprising the amino acid sequence of SEQ ID NO: 40; and a light chain variable domain (V_{L}) comprising a light chain complementarity determining region (LC-CDR) 1 comprising the amino acid sequence of SEQ ID NO: 42; an LC-CDR2 comprising the amino acid sequence of SEQ ID NO: 44; and an LC-CDR3 comprising the amino acid sequence of SEQ ID NO: 46.

13. The bispecific antibody of any one of claims 10-12, wherein:
(a) the first antigen-binding domain comprises a V_{H} comprising an HC-CDR1 comprising the amino acid sequence SEQ ID NO: 1, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16;
and wherein the second antigen-binding domain comprises:
a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45; or
(b) the first antigen-binding domain comprises a V_{H} comprising an HC-CDR1 comprising the amino acid sequence SEQ ID NO: 1, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16;
and wherein the second antigen-binding domain comprises: a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46; or
(c) the first antigen-binding domain comprises a V_{H} comprising an HC-CDR1 comprising the amino acid sequence SEQ ID NO: 1, an HC-CDR2 comprising the amino acid sequence of SEQ ID NO: 2, and an HC-CDR3 comprising the amino acid sequence of SEQ ID NO: 7; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15;
and wherein the second antigen-binding domain comprises:
a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45; or
(d) the first antigen-binding domain comprises a V_{H} comprising an HC-CDR1 comprising the amino acid sequence SEQ ID NO: 1, an HC-CDR2 comprising the amino acid sequence of SEQ ID NO: 2, and an HC-CDR3 comprising the amino acid sequence of SEQ ID NO: 7; and a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15;
and wherein the second antigen-binding domain comprises:
a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and a V_{L} comprising an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

14. The bispecific antibody of any one of claims 10-13, comprising a first antigen-binding domain specifically binding to *K. pneumoniae* O2 antigen, and a second antigen-binding domain specifically binding to *K. pneumoniae* O1 antigen, wherein the first antigen-binding domain comprises:
(a) a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; or a V_{H} comprising an HC-CDR1, an HC-CDR2 and an HC-CDR3 of the V_{H} comprising the amino acid sequence of SEQ ID NO: 19 or 28; and a V_{L} comprising an LC-CDR1, an LC-CDR2, and an LC-CDR3 of the V_{L} comprising the amino acid sequence of SEQ ID NO: 24 or 33; or
(b) a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; or a V_{H} comprising an HC-CDR1, an HC-CDR2 and an HC-CDR3 of the V_{H} comprising the amino acid sequence of SEQ ID NO: 20 or 29; and a V_{L} comprising an LC-CDR1, an LC-CDR2, and an LC-CDR3 of the V_{L} comprising the amino acid sequence of SEQ ID NO: 25 or 34.

15. The bispecific antibody of any one of claims 10-14, wherein the second antigen-binding domain comprises:
(a) a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53; or a V_{H} comprising an HC-CDR1, an HC-CDR2 and an HC-CDR3 of the V_{H} comprising the amino acid sequence of SEQ ID NO: 47 or 51; and a V_{L} comprising an LC-CDR1, an LC-CDR2, and an LC-CDR3 of the V_{L} comprising the amino acid sequence of SEQ ID NO: 49 or 53; or
(b) a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54; or a V_{H} comprising an HC-CDR1, an HC-CDR2 and an HC-CDR3 of the V_{H} comprising the amino acid sequence of SEQ ID NO: 48 or 52; and a V_{L} comprising an LC-CDR1, an LC-CDR2, and an LC-CDR3 of the V_{L} comprising the amino acid sequence of SEQ ID NO: 50 or 54.

16. The bispecific antibody of any one of claims 10-15, wherein:
(a) the first antigen-binding domain comprises: a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; and wherein
the second antigen-binding domain comprises: a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53; or
(b) the first antigen-binding domain comprises: a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; and wherein
the second antigen-binding domain comprises: a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54; or
(c) the first antigen-binding domain comprises: a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; and wherein
the second antigen-binding domain comprises: a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53; or
(d) the first antigen-binding domain comprises: a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; and wherein
the second antigen-binding domain comprises: a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54.

17. A bispecific antibody, comprising a first antigen-binding domain specifically binding to *Klebsiella pneumoniae* (*K. pneumoniae*) O2 antigen, and a second antigen-binding domain specifically binding to *Klebsiella pneumoniae* (*K. pneumoniae*) O1 antigen, wherein the second antigen-binding domain comprises:
(a) a heavy chain variable domain (V_{H}) comprising a heavy chain complementarity determining region (HC-CDR) 1 comprising the amino acid sequence of SEQ ID NO: 35; an HC-CDR2 comprising the amino acid sequence of SEQ ID NO: 37; and an HC-CDR3 comprising the amino acid sequence of SEQ ID NO: 39; and a light chain variable domain (V_{L}) comprising a light chain complementarity determining region (LC-CDR) 1 comprising the amino acid sequence of SEQ ID NO: 41; an LC-CDR2 comprising the amino acid sequence of SEQ ID NO: 43; and an LC-CDR3 comprising the amino acid sequence of SEQ ID NO: 45; or
(b) a heavy chain variable domain (V_{H}) comprising a heavy chain complementarity determining region (HC-CDR) 1 comprising the amino acid sequence of SEQ ID NO: 36; an HC-CDR2 comprising the amino acid sequence of SEQ ID NO: 38; and an HC-CDR3 comprising the amino acid sequence of SEQ ID NO: 40; and a light chain variable domain (V_{L}) comprising a light chain complementarity determining region (LC-CDR) 1 comprising the amino acid sequence of SEQ ID NO: 42; an LC-CDR2 comprising the amino acid sequence of SEQ ID NO: 44; and an LC-CDR3 comprising the amino acid sequence of SEQ ID NO: 46.

18. The bispecific antibody of claim 17, comprising a first antigen-binding domain specifically binding to *K. pneumoniae* O2 antigen, and a second antigen-binding domain specifically binding to *K. pneumoniae* O1 antigen, wherein the second antigen-binding domain comprises:
(a) a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53; or a V_{H} comprising an HC-CDR1, an HC-CDR2 and an HC-CDR3 of the V_{H} comprising the amino acid sequence of SEQ ID NO: 47 or 51; and a V_{L} comprising an LC-CDR1, an LC-CDR2, and an LC-CDR3 of the V_{L} comprising the amino acid sequence of SEQ ID NO: 49 or 53; or
(b) a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54; or a V_{H} comprising an HC-CDR1, an HC-CDR2 and an HC-CDR3 of the V_{H} comprising the amino acid sequence of SEQ ID NO: 48 or 52; and a V_{L} comprising an LC-CDR1, an LC-CDR2, and an LC-CDR3 of the V_{L} comprising the amino acid sequence of SEQ ID NO: 50 or 54.

19. The bispecific antibody of any one of claims 10-18, wherein the bispecific antibody has the formats selected from the group consisting of DVD-Ig, Bs4Ab, Hetero H, CrossMab, IgG-(scFv)₂ and scFv-Fab IgG.

20. The bispecific antibody of any one of claims 10-19, comprising four polypeptide chains:
wherein two of the polypeptide chains comprise V_{H}1-L-V_{H}2-C_{H}1 structure from N-terminus to C-terminus, wherein V_{H}1 is a heavy chain variable domain specifically binding to *K. pneumoniae* O2 antigen; V_{H}2 is a heavy chain variable domain specifically binding to *K. pneumoniae* O1 antigen; L is a peptide linker; C_{H}1 is a heavy chain constant domain 1; wherein the polypeptide chain further comprises an Fc comprising C_{H}2 and C_{H}3 domains; and
the other two of the polypeptide chains comprise V_{L}1-L-V_{L}2-C_{L} structure from N-terminus to C-terminus, wherein V_{L}1 is a light chain variable domain specifically binding to *K. pneumoniae* O2 antigen; V_{L}2 is a light chain variable domain specifically binding to *K. pneumoniae* O1 antigen; L is a peptide linker; C_{L} is a light chain constant domain.

21. The bispecific antibody of any one of claims 10-19, comprising four polypeptide chains:
wherein two of the polypeptide chains comprise V_{H}1-L-V_{H}2-C_{H}1 structure from N-terminus to C-terminus, wherein V_{H}1 is a heavy chain variable domain specifically binding to *K. pneumoniae* O1 antigen; V_{H}2 is a heavy chain variable domain specifically binding to *K. pneumoniae* O2 antigen; L is a peptide linker; C_{H}1 is a heavy chain constant domain 1; wherein the polypeptide chain further comprises an Fc comprising C_{H}2 and C_{H}3 domains; and
the other two of the polypeptide chains comprise V_{L}1-L-V_{L}2-C_{L} structure from N-terminus to C-terminus, wherein V_{L}1 is a light chain variable domain soecificallv binding to *K. pneumoniae* O1 antigen; V_{L}2 is a light chain variable domain specifically binding to *K. pneumoniae* O2 antigen; L is a peptide linker; C_{L} is a light chain constant domain.

22. The bispecific antibody of any one of claims 20-21, wherein the bispecific antibody comprises:
the amino acid sequence of SEQ ID NO: 61, or a variant thereof having at least about 80% sequence identity to the amino acid sequence of SEQ ID NO: 61; and/or
the amino acid sequence of SEQ ID NO: 62, or a variant thereof having at least about 80% sequence identity to the amino acid sequence of SEQ ID NO: 62.

23. The bispecific antibody of any one of claims 20-21, comprising:
the amino acid sequence of SEQ ID NO: 63, or a variant thereof having at least about 80% sequence identity to the amino acid sequence of SEQ ID NO: 63; and/or
the amino acid sequence of SEQ ID NO: 64, or a variant thereof having at least about 80% sequence identity to the amino acid sequence of SEQ ID NO: 64.

24. The bispecific antibody of any one of claims 20-21, comprising:
the amino acid sequence of SEQ ID NO: 65, or a variant thereof having at least about 80% sequence identity to the amino acid sequence of SEQ ID NO: 65; and/or
the amino acid sequence of SEQ ID NO: 66, or a variant thereof having at least about 80% sequence identity to the amino acid sequence of SEQ ID NO: 66.

25. The bispecific antibody of any one of claims 20-21, comprising:
the amino acid sequence of SEQ ID NO: 67, or a variant thereof having at least about 80% sequence identity to the amino acid sequence of SEQ ID NO: 67; and/or
the amino acid sequence of SEQ ID NO: 68, or a variant thereof having at least about 80% sequence identity to the amino acid sequence of SEQ ID NO: 68.

26. The bispecific antibody of any one of claims 20-21, wherein the bispecific antibody comprises:
the amino acid sequence of SEQ ID NO: 86, or a variant thereof having at least about 80% sequence identity to the amino acid sequence of SEQ ID NO: 86; and/or
the amino acid sequence of SEQ ID NO: 62, or a variant thereof having at least about 80% sequence identity to the amino acid sequence of SEQ ID NO: 62.

27. The bispecific antibody of any one of claims 20-21, comprising:
the amino acid sequence of SEQ ID NO: 87, or a variant thereof having at least about 80% sequence identity to the amino acid sequence of SEQ ID NO: 87; and/or
the amino acid sequence of SEQ ID NO: 64, or a variant thereof having at least about 80% sequence identity to the amino acid sequence of SEQ ID NO: 64.

28. The bispecific antibody of any one of claims 20-21, comprising:
the amino acid sequence of SEQ ID NO: 88, or a variant thereof having at least about 80% sequence identity to the amino acid sequence of SEQ ID NO: 88; and/or
the amino acid sequence of SEQ ID NO: 66, or a variant thereof having at least about 80% sequence identity to the amino acid sequence of SEQ ID NO: 66.

29. The bispecific antibody of any one of claims 20-21, comprising:
the amino acid sequence of SEQ ID NO: 89, or a variant thereof having at least about 80% sequence identity to the amino acid sequence of SEQ ID NO: 89; and/or
the amino acid sequence of SEQ ID NO: 68, or a variant thereof having at least about 80% sequence identity to the amino acid sequence of SEQ ID NO: 68.

30. The bispecific antibody of any one of claims 20-21, comprising:
the amino acid sequence of SEQ ID NO: 115, or a variant thereof having at least about 80% sequence identity to the amino acid sequence of SEQ ID NO: 115; and/or
the amino acid sequence of SEQ ID NO: 66, or a variant thereof having at least about 80% sequence identity to the amino acid sequence of SEQ ID NO: 66.

31. The bispecific antibody of any one of claims 10-19, comprising four polypeptide chains:
wherein two of the polypeptide chains comprise V_{H}1-C_{H}1-L1-V_{H}2-L3-V_{L}2 structure from N- terminus to C-terminus, wherein V_{H}1 is a heavy chain variable domain specifically binding to *K. pneumoniae* O2 antigen; V_{H}2 is a heavy chain variable domain specifically binding to *K. pneumoniae* O1 antigen; L1 and L3 are peptide linkers; C_{H}1 is a heavy chain constant domain 1; wherein the polypeptide chain further comprises an Fc comprising C_{H}2 and C_{H}3 domains; and
the other two of the polypeptide chains comprise V_{L}1-C_{L} structure from N- terminus to C-terminus, wherein V_{L}1 is a light chain variable domain specifically binding to *K. pneumoniae* O2 antigen, C_{L} is a light chain constant domain.

32. The bispecific antibody of any one of claims 10-19, comprising four polypeptide chains:
wherein two of the polypeptide chains comprise V_{H}1-C_{H}1-L1-V_{H}2-L3-V_{L}2 structure from N- terminus to C-terminus, wherein V_{H}1 is a heavy chain variable domain specifically binding to *K. pneumoniae* O1 antigen; V_{H}2 is a heavy chain variable domain specifically binding to *K. pneumoniae* O2 antigen; L1 and L3 are peptide linkers; C_{H}1 is a heavy chain constant domain 1; wherein the polypeptide chain further comprises an Fc comprising C_{H}2 and C_{H}3 domains; and
the other two of the polypeptide chains comprise V_{L}1-C_{L} structure from N- terminus to C-terminus, wherein V_{L}1 is a light chain variable domain specifically binding to *K. pneumoniae* O1 antigen, C_{L} is a light chain constant domain.

33. The bispecific antibody of any one of claims 31-32, wherein the bispecific antibody comprises:
the amino acid sequence of SEQ ID NO: 69, or a variant thereof having at least about 80% sequence identity to the amino acid sequence of SEQ ID NO: 69; and/or
the amino acid sequence of SEQ ID NO: 70, or a variant thereof having at least about 80% sequence identity to the amino acid sequence of SEQ ID NO: 70.

34. The bispecific antibody of any one of claims 31-32, comprising:
the amino acid sequence of SEQ ID NO: 71, or a variant thereof having at least about 80% sequence identity to the amino acid sequence of SEQ ID NO: 71; and/or
the amino acid sequence of SEQ ID NO: 72, or a variant thereof having at least about 80% sequence identity to the amino acid sequence of SEQ ID NO: 72.

35. The bispecific antibody of any one of claims 31-32, comprising:
the amino acid sequence of SEQ ID NO: 73, or a variant thereof having at least about 80% sequence identity to the amino acid sequence of SEQ ID NO: 73; and/or
the amino acid sequence of SEQ ID NO: 74, or a variant thereof having at least about 80% sequence identity to the amino acid sequence of SEQ ID NO: 74.

36. The bispecific antibody of any one of claims 31-32, comprising:
the amino acid sequence of SEQ ID NO: 75, or a variant thereof having at least about 80% sequence identity to the amino acid sequence of SEQ ID NO: 75; and/or
the amino acid sequence of SEQ ID NO: 74, or a variant thereof having at least about 80% sequence identity to the amino acid sequence of SEQ ID NO: 74.

37. The bispecific antibody of any one of claims 31-32, wherein the bispecific antibody comprises:
the amino acid sequence of SEQ ID NO: 90, or a variant thereof having at least about 80% sequence identity to the amino acid sequence of SEQ ID NO: 90; and/or
the amino acid sequence of SEQ ID NO: 70, or a variant thereof having at least about 80% sequence identity to the amino acid sequence of SEQ ID NO: 70.

38. The bispecific antibody of any one of claims 31-32, comprising:
the amino acid sequence of SEQ ID NO: 91, or a variant thereof having at least about 80% sequence identity to the amino acid sequence of SEQ ID NO: 91; and/or
the amino acid sequence of SEQ ID NO: 72, or a variant thereof having at least about 80% sequence identity to the amino acid sequence of SEQ ID NO: 72.

39. The bispecific antibody of any one of claims 31-32, comprising:
the amino acid sequence of SEQ ID NO: 92, or a variant thereof having at least about 80% sequence identity to the amino acid sequence of SEQ ID NO: 92; and/or
the amino acid sequence of SEQ ID NO: 74, or a variant thereof having at least about 80% sequence identity to the amino acid sequence of SEQ ID NO: 74.

40. The bispecific antibody of any one of claims 31-32, comprising:
the amino acid sequence of SEQ ID NO: 93, or a variant thereof having at least about 80% sequence identity to the amino acid sequence of SEQ ID NO: 93; and/or
the amino acid sequence of SEQ ID NO: 74, or a variant thereof having at least about 80% sequence identity to the amino acid sequence of SEQ ID NO: 74.

41. The bispecific antibody of any one of claims 10-19, comprising four polypeptide chains:
wherein one of the polypeptide chains comprises V_{H}1-C_{H}1 structure from N- terminus to C-terminus, wherein V_{H}1 is a heavy chain variable domain specifically binding to *K. pneumoniae* O2 antigen; C_{H}1 is a heavy chain constant domain 1; wherein the polypeptide chain further comprises an Fc comprising C_{H}2 and C_{H}3 domains;
one of the polypeptide chains comprises V_{L}1-C_{L} structure from N- terminus to C-terminus, wherein V_{L}1 is a light chain variable domain specifically binding to *K. pneumoniae* O2 antigen, C_{L} is a light chain constant domain;
one of the polypeptide chains comprises V_{H}2-C_{L} structure from N- terminus to C-terminus, wherein V_{H}2 is a heavy chain variable domain specifically binding to *K. pneumoniae* O1 antigen; C_{L} is a light chain constant domain; wherein the polypeptide chain further comprises an Fc comprising C_{H}2 and C_{H}3 domains; and
one of the polypeptide chains comprises V_{L}2-C_{H}1 structure from N- terminus to C-terminus, wherein V_{L}2 is a light chain variable domain specifically binding to *K. pneumoniae* O1 antigen, C_{H}1 is a heavy chain constant domain 1.

42. The bispecific antibody of any one of claims 10-19, comprising four polypeptide chains:
wherein one of the polypeptide chains comprises V_{H}1-C_{H}1 structure from N- terminus to C-terminus, wherein V_{H}1 is a heavy chain variable domain specifically binding to *K. pneumoniae* O1 antigen; C_{H}1 is a heavy chain constant domain 1; wherein the polypeptide chain further comprises an Fc comprising C_{H}2 and C_{H}3 domains;
one of the polypeptide chains comprises V_{L}1-C_{L} structure from N- terminus to C-terminus, wherein V_{L}1 is a light chain variable domain specifically binding to *K. pneumoniae* O1 antigen, C_{L} is a light chain constant domain;
one of the polypeptide chains comprises V_{H}2-C_{L} structure from N- terminus to C-terminus, wherein V_{H}2 is a heavy chain variable domain specifically binding to *K. pneumoniae* O2 antigen; C_{L} is a light chain constant domain; wherein the polypeptide chain further comprises an Fc comprising C_{H}2 and C_{H}3 domains; and
one of the polypeptide chains comprises V_{L}2-C_{H}1 structure from N- terminus to C-terminus, wherein V_{L}2 is a light chain variable domain specifically binding to *K. pneumoniae* O2 antigen, C_{H}1 is a heavy chain constant domain 1.

43. The bispecific antibody of any one of claims 41-42, comprising:
the amino acid sequence of SEQ ID NO: 76, or a variant thereof having at least about 80% sequence identity to the amino acid sequence of SEQ ID NO: 76; and/or
the amino acid sequence of SEQ ID NO: 77, or a variant thereof having at least about 80% sequence identity to the amino acid sequence of SEQ ID NO: 77; and/or
the amino acid sequence of SEQ ID NO: 78, or a variant thereof having at least about 80% sequence identity to the amino acid sequence of SEQ ID NO: 78; and/or
the amino acid sequence of SEQ ID NO: 74, or a variant thereof having at least about 80% sequence identity to the amino acid sequence of SEQ ID NO: 74.

44. The bispecific antibody of any one of claims 41-42, comprising:
the amino acid sequence of SEQ ID NO: 79, or a variant thereof having at least about 80% sequence identity to the amino acid sequence of SEQ ID NO: 79; and/or
the amino acid sequence of SEQ ID NO: 80, or a variant thereof having at least about 80% sequence identity to the amino acid sequence of SEQ ID NO: 80; and/or
the amino acid sequence of SEQ ID NO: 78, or a variant thereof having at least about 80% sequence identity to the amino acid sequence of SEQ ID NO: 78; and/or
the amino acid sequence of SEQ ID NO: 74, or a variant thereof having at least about 80% sequence identity to the amino acid sequence of SEQ ID NO: 74.

45. The bispecific antibody of any one of claims 41-42, comprising:
the amino acid sequence of SEQ ID NO: 94, or a variant thereof having at least about 80% sequence identity to the amino acid sequence of SEQ ID NO: 94; and/or
the amino acid sequence of SEQ ID NO: 77, or a variant thereof having at least about 80% sequence identity to the amino acid sequence of SEQ ID NO: 77; and/or
the amino acid sequence of SEQ ID NO: 95, or a variant thereof having at least about 80% sequence identity to the amino acid sequence of SEQ ID NO: 95; and/or
the amino acid sequence of SEQ ID NO: 74, or a variant thereof having at least about 80% sequence identity to the amino acid sequence of SEQ ID NO: 74.

46. The bispecific antibody of any one of claims 41-42, comprising:
the amino acid sequence of SEQ ID NO: 96, or a variant thereof having at least about 80% sequence identity to the amino acid sequence of SEQ ID NO: 96; and/or
the amino acid sequence of SEQ ID NO: 80, or a variant thereof having at least about 80% sequence identity to the amino acid sequence of SEQ ID NO: 80; and/or
the amino acid sequence of SEQ ID NO: 95, or a variant thereof having at least about 80% sequence identity to the amino acid sequence of SEQ ID NO: 95; and/or
the amino acid sequence of SEQ ID NO: 74, or a variant thereof having at least about 80% sequence identity to the amino acid sequence of SEQ ID NO: 74.

47. The bispecific antibody of any one of claims 10-19, comprising four polypeptide chains:
wherein two of the polypeptide chains comprise V_{H}1-C_{H}1-C_{H}2-C_{H}3-L-V_{H}2-L3-V_{L}2 structure from N- terminus to C-terminus, wherein V_{H}1 is a heavy chain variable domain specifically binding to *K. pneumoniae* O2 antigen; V_{H}2 is a heavy chain variable domain specifically binding to *K. pneumoniae* O1 antigen; V_{L}2 is a light chain variable domain specifically binding to *K. pneumoniae* O1 antigen; L and L3 are peptide linkers; C_{H}1 is a heavy chain constant domain 1; C_{H}2 is a heavy chain constant domain 2; C_{H}3 is a heavy chain constant domain 3; and
the other two of the polypeptide chains comprise V_{L}1-C_{L} structure from N- terminus to C-terminus, wherein V_{L}1 is a light chain variable domain specifically binding to *K. pneumoniae* O2 antigen, C_{L} is a light chain constant domain.

48. The bispecific antibody of any one of claims 10-19, comprising four polypeptide chains:
wherein two of the polypeptide chains comprise V_{H}1-C_{H}1-C_{H}2-C_{H}3-L-V_{H}2-L3-V_{L}2 structure from N- terminus to C-terminus, wherein V_{H}1 is a heavy chain variable domain specifically binding to *K. pneumoniae* O1 antigen; V_{H}2 is a heavy chain variable domain specifically binding to *K. pneumoniae* O2 antigen; V_{L}2 is a light chain variable domain specifically binding to *K. pneumoniae* O2 antigen; L and L3 are peptide linkers; C_{H}1 is a heavy chain constant domain 1; C_{H}2 is a heavy chain constant domain 2; C_{H}3 is a heavy chain constant domain 3; and
the other two of the polypeptide chains comprise V_{L}1-C_{L} structure from N- terminus to C-terminus, wherein V_{L}1 is a light chain variable domain specifically binding to *K. pneumoniae* O1 antigen, C_{L} is a light chain constant domain.

49. The bispecific antibody of any one of claims 47-48, wherein the bispecific antibody comprises:
the amino acid sequence of SEQ ID NO: 97, or a variant thereof having at least about 80% sequence identity to the amino acid sequence of SEQ ID NO: 97; and/or
the amino acid sequence of SEQ ID NO: 83, or a variant thereof having at least about 80% sequence identity to the amino acid sequence of SEQ ID NO: 83.

50. The bispecific antibody of any one of claims 47-48, wherein the bispecific antibody comprises:
the amino acid sequence of SEQ ID NO: 98, or a variant thereof having at least about 80% sequence identity to the amino acid sequence of SEQ ID NO: 98; and/or
the amino acid sequence of SEQ ID NO: 85, or a variant thereof having at least about 80% sequence identity to the amino acid sequence of SEQ ID NO: 85.

51. The bispecific antibody of any one of claims 10-19, comprising three polypeptide chains:
wherein one of the polypeptide chains comprises V_{H}1-C_{H}1 structure from N- terminus to C-terminus, wherein V_{H}1 is a heavy chain variable domain specifically binding to *K. pneumoniae* O2 antigen; C_{H}1 is a heavy chain constant domain 1; wherein the polypeptide chain further comprises an Fc comprising C_{H}2 and C_{H}3 domains;
one of the polypeptide chains comprises V_{L}1-C_{L} structure from N- terminus to C-terminus, wherein V_{L}1 is a light chain variable domain specifically binding to *K. pneumoniae* O2 antigen, C_{L} is a light chain constant domain; and
one of the polypeptide chains comprises V_{H}2-L3-V_{L}2 structure from N- terminus to C-terminus, wherein V_{H}2 is a heavy chain variable domain specifically binding to *K. pneumoniae* O1 antigen; V_{L}2 is a light chain variable domain specifically binding to *K. pneumoniae* O1 antigen; L3 is a peptide linker; wherein the polypeptide chain further comprises an Fc comprising C_{H}2 and C_{H}3 domains.

52. The bispecific antibody of any one of claims 10-19, comprising three polypeptide chains:
wherein one of the polypeptide chains comprises V_{H}1-C_{H}1 structure from N- terminus to C-terminus, wherein V_{H}1 is a heavy chain variable domain specifically binding to *K. pneumoniae* O1 antigen; C_{H}1 is a heavy chain constant domain 1; wherein the polypeptide chain further comprises an Fc comprising C_{H}2 and C_{H}3 domains; and
one of the polypeptide chains comprises V_{L}1-C_{L} structure from N- terminus to C-terminus, wherein V_{L}1 is a light chain variable domain specifically binding to *K. pneumoniae* O1 antigen, C_{L} is a light chain constant domain; and
one of the polypeptide chains comprises V_{H}2-L3-V_{L}2 structure from N- terminus to C-terminus, wherein V_{H}2 is a heavy chain variable domain specifically binding to *K. pneumoniae* O2 antigen; V_{L}2 is a light chain variable domain specifically binding to *K. pneumoniae* O2 antigen; L3 is a peptide linker; wherein the polypeptide chain further comprises an Fc comprising C_{H}2 and C_{H}3 domains.

53. The bispecific antibody of any one of claims 51-52, comprising:
the amino acid sequence of SEQ ID NO: 81, or a variant thereof having at least about 80% sequence identity to the amino acid sequence of SEQ ID NO: 81; and/or
the amino acid sequence of SEQ ID NO: 82, or a variant thereof having at least about 80% sequence identity to the amino acid sequence of SEQ ID NO: 82; and/or
the amino acid sequence of SEQ ID NO: 83, or a variant thereof having at least about 80% sequence identity to the amino acid sequence of SEQ ID NO: 83.

54. The bispecific antibody of any one of claims 51-52, comprising:
the amino acid sequence of SEQ ID NO: 84, or a variant thereof having at least about 80% sequence identity to the amino acid sequence of SEQ ID NO: 84; and/or
the amino acid sequence of SEQ ID NO: 82, or a variant thereof having at least about 80% sequence identity to the amino acid sequence of SEQ ID NO: 82; and/or
the amino acid sequence of SEQ ID NO: 85, or a variant thereof having at least about 80% sequence identity to the amino acid sequence of SEQ ID NO: 85.

55. The bispecific antibody of any one of claims 51-52, wherein the bispecific antibody comprises:
the amino acid sequence of SEQ ID NO: 99, or a variant thereof having at least about 80% sequence identity to the amino acid sequence of SEQ ID NO: 99; and/or
the amino acid sequence of SEQ ID NO: 100, or a variant thereof having at least about 80% sequence identity to the amino acid sequence of SEQ ID NO: 100; and/or
the amino acid sequence of SEQ ID NO: 83, or a variant thereof having at least about 80% sequence identity to the amino acid sequence of SEQ ID NO: 83.

56. The bispecific antibody of any one of claims 51-52, wherein the bispecific antibody comprises:
the amino acid sequence of SEQ ID NO: 101, or a variant thereof having at least about 80% sequence identity to the amino acid sequence of SEQ ID NO: 101; and/or
the amino acid sequence of SEQ ID NO: 100, or a variant thereof having at least about 80% sequence identity to the amino acid sequence of SEQ ID NO: 100; and/or
the amino acid sequence of SEQ ID NO: 85, or a variant thereof having at least about 80% sequence identity to the amino acid sequence of SEQ ID NO: 85.

57. A pharmaceutical composition, comprising: antibody or antigen-binding fragment specifically recognizing *K. pneumoniae* O2 antigen and antibody or antigen-binding fragment specifically recognizing *K. pneumoniae* O1 antigen; wherein the antibody or antigen-binding fragment specifically recognizing *K. pneumoniae* O2 antigen comprises:
(a) a heavy chain variable domain (V_{H}) comprising a heavy chain complementarity determining region (HC-CDR) 1 comprising the amino acid sequence of SEQ ID NO: 1; an HC-CDR2 comprising the amino acid sequence of SEQ ID NO: 4; and an HC-CDR3 comprising the amino acid sequence of SEQ ID NO: 6; and a light chain variable domain (V_{L}) comprising a light chain complementarity determining region (LC-CDR) 1 comprising the amino acid sequence of SEQ ID NO: 11; an LC-CDR2 comprising the amino acid sequence of SEQ ID NO: 14; and an LC-CDR3 comprising the amino acid sequence of SEQ ID NO: 16; or
(b) a heavy chain variable domain (V_{H}) comprising a heavy chain complementarity determining region (HC-CDR) 1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 7; and a light chain variable domain (V_{L}) comprising a light chain complementarity determining region (LC-CDR) 1 comprising the amino acid sequence of SEQ ID NO: 10; an LC-CDR2 comprising the amino acid sequence of SEQ ID NO: 12; and an LC-CDR3 comprising the amino acid sequence of SEQ ID NO: 15.

58. A method of treating and/or preventing a disease or condition in an individual in need thereof, comprising administering to the individual an effective amount of antibody or antigen-binding fragment specifically recognizing *Klebsiella pneumoniae (K. pneumoniae)* O2 antigen and antibody or antigen-binding fragment specifically recognizing *Klebsiella pneumoniae* (*K. pneumoniae*) O1 antigen; wherein the antibody or antigen-binding fragment specifically recognizing *Klebsiella pneumoniae (K. pneumoniae)* O2 antigen comprises:
(a) a heavy chain variable domain (V_{H}) comprising a heavy chain complementarity determining region (HC-CDR) 1 comprising the amino acid sequence of SEQ ID NO: 1; an HC-CDR2 comprising the amino acid sequence of SEQ ID NO: 4; and an HC-CDR3 comprising the amino acid sequence of SEQ ID NO: 6; and a light chain variable domain (V_{L}) comprising a light chain complementarity determining region (LC-CDR) 1 comprising the amino acid sequence of SEQ ID NO: 11; an LC-CDR2 comprising the amino acid sequence of SEQ ID NO: 14; and an LC-CDR3 comprising the amino acid sequence of SEQ ID NO: 16; or
(b) a heavy chain variable domain (V_{H}) comprising a heavy chain complementarity determining region (HC-CDR) 1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 7; and a light chain variable domain (V_{L}) comprising a light chain complementarity determining region (LC-CDR) 1 comprising the amino acid sequence of SEQ ID NO: 10; an LC-CDR2 comprising the amino acid sequence of SEQ ID NO: 12; and an LC-CDR3 comprising the amino acid sequence of SEQ ID NO: 15.

59. The pharmaceutical composition of claim 57 or the method of claim 58, wherein the antibody or antigen-binding fragment specifically recognizing *K. pneumoniae* O1 antigen comprises:
(a) a heavy chain variable domain (V_{H}) comprising a heavy chain complementarity determining region (HC-CDR) 1 comprising the amino acid sequence of SEQ ID NO: 35; an HC-CDR2 comprising the amino acid sequence of SEQ ID NO: 37; and an HC-CDR3 comprising the amino acid sequence of SEQ ID NO: 39; and a light chain variable domain (V_{L}) comprising a light chain complementarity determining region (LC-CDR) 1 comprising the amino acid sequence of SEQ ID NO: 41; an LC-CDR2 comprising the amino acid sequence of SEQ ID NO: 43; and an LC-CDR3 comprising the amino acid sequence of SEQ ID NO: 45; or
(b) a heavy chain variable domain (V_{H}) comprising a heavy chain complementarity determining region (HC-CDR) 1 comprising the amino acid sequence of SEQ ID NO: 36; an HC-CDR2 comprising the amino acid sequence of SEQ ID NO: 38; and an HC-CDR3 comprising the amino acid sequence of SEQ ID NO: 40; and a light chain variable domain (V_{L}) comprising a light chain complementarity determining region (LC-CDR) 1 comprising the amino acid sequence of SEQ ID NO: 42; an LC-CDR2 comprising the amino acid sequence of SEQ ID NO: 44; and an LC-CDR3 comprising the amino acid sequence of SEQ ID NO: 46.

60. The pharmaceutical composition of any one of claims 57 or 59, or the method of any one of claims 58-59,
(a) wherein the antibody or antigen-binding fragment specifically binding to *K. pneumoniae* O2 antigen comprises:
a V_{H} comprising an HC-CDR1 comprising the amino acid sequence SEQ ID NO: 1, an HC-CDR2 comprising the amino acid sequence of SEQ ID NO: 2, and an HC-CDR3 comprising the amino acid sequence of SEQ ID NO: 7; and
a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15;
and wherein the antibody or antigen-binding fragment specifically binding to *K. pneumoniae* O1 antigen comprises:
a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and
a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45; or
(b) wherein the antibody or antigen-binding fragment specifically binding to *K. pneumoniae* O2 antigen comprises:
a V_{H} comprising an HC-CDR1 comprising the amino acid sequence SEQ ID NO: 1, an HC-CDR2 comprising the amino acid sequence of SEQ ID NO: 2, and an HC-CDR3 comprising the amino acid sequence of SEQ ID NO: 7; and
a V_{L} comprising an LC-CDR1 comprising the amino acid sequence of SEQ ID NO: 10, an LC-CDR2 comprising the amino acid sequence of SEQ ID NO: 12, and an LC-CDR3 comprising the amino acid sequence of SEQ ID NO: 15;
and wherein the antibody or antigen-binding fragment specifically binding to *K. pneumoniae* O1 antigen comprises:
a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and
a V_{L} comprising an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46; or
(c) wherein the antibody or antigen-binding fragment specifically binding to *K. pneumoniae* O2 antigen comprises:
a V_{H} comprising an HC-CDR1 comprising the amino acid sequence SEQ ID NO: 1, an HC-CDR2 comprising the amino acid sequence of SEQ ID NO: 4, and an HC-CDR3 comprising the amino acid sequence of SEQ ID NO: 6; and
a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16;
and wherein the antibody or antigen-binding fragment specifically binding to *K. pneumoniae* O1 antigen comprises:
a V_{H} comprising an HC-CDR1 comprising the amino acid sequence of SEQ ID NO: 35, an HC-CDR2 comprising the amino acid sequence of SEQ ID NO: 37, and an HC-CDR3 comprising the amino acid sequence of SEQ ID NO: 39; and
a V_{L} comprising an LC-CDR1 comprising the amino acid sequence of SEQ ID NO: 41, an LC-CDR2 comprising the amino acid sequence of SEQ ID NO: 43, and an LC-CDR3 comprising the amino acid sequence of SEQ ID NO: 45; or
(d) wherein the antibody or antigen-binding fragment specifically binding to *K. pneumoniae* O2 antigen comprises:
a V_{H} comprising an HC-CDR1 comprising the amino acid sequence SEQ ID NO: 1, an HC-CDR2 comprising the amino acid sequence of SEQ ID NO: 4, and an HC-CDR3 comprising the amino acid sequence of SEQ ID NO: 6; and
a V_{L} comprising an LC-CDR1 comprising the amino acid sequence of SEQ ID NO: 11, an LC-CDR2 comprising the amino acid sequence of SEQ ID NO: 14, and an LC-CDR3 comprising the amino acid sequence of SEQ ID NO: 16;
and wherein the antibody or antigen-binding fragment specifically binding to *K. pneumoniae* O1 antigen comprises:
a V_{H} comprising an HC-CDR1 comprising the amino acid sequence of SEQ ID NO: 36, an HC-CDR2 comprising the amino acid sequence of SEQ ID NO: 38, and an HC-CDR3 comprising the amino acid sequence of SEQ ID NO: 40; and
a V_{L} comprising: an LC-CDR1 comprising the amino acid sequence of SEQ ID NO: 42, an LC-CDR2 comprising the amino acid sequence of SEQ ID NO: 44, and an LC-CDR3 comprising the amino acid sequence of SEQ ID NO: 46.

61. The pharmaceutical composition of any one of claims 57, 59-60, or the method of any one of claims 58-60, wherein the antibody or antigen-binding fragment specifically recognizing *K. pneumoniae* O2 antigen comprises:
(a) a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33; or a V_{H} comprising an HC-CDR1, an HC-CDR2 and an HC-CDR3 of the V_{H} comprising the amino acid sequence of SEQ ID NO: 19 or 28; and a V_{L} comprising an LC-CDR1, an LC-CDR2, and an LC-CDR3 of the V_{L} comprising the amino acid sequence of SEQ ID NO: 24 or 33; or
(b) a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34; or a V_{H} comprising an HC-CDR1, an HC-CDR2 and an HC-CDR3 of the V_{H} comprising the amino acid sequence of SEQ ID NO: 20 or 29; and a V_{L} comprising an LC-CDR1, an LC-CDR2, and an LC-CDR3 of the V_{L} comprising the amino acid sequence of SEQ ID NO: 25 or 34.

62. The pharmaceutical composition of any one of claims 57, 59-61, or the method of any one of claims 58-61, wherein the antibody or antigen-binding fragment specifically recognizing *K. pneumoniae* O1 antigen comprises:
(a) a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53; or a V_{H} comprising an HC-CDR1, an HC-CDR2 and an HC-CDR3 of the V_{H} comprising the amino acid sequence of SEQ ID NO: 47 or 51; and a V_{L} comprising an LC-CDR1, an LC-CDR2, and an LC-CDR3 of the V_{L} comprising the amino acid sequence of SEQ ID NO: 49 or 53; or
(b) a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54; or a V_{H} comprising an HC-CDR1, an HC-CDR2 and an HC-CDR3 of the V_{H} comprising the amino acid sequence of SEQ ID NO: 48 or 52; and a V_{L} comprising an LC-CDR1, an LC-CDR2, and an LC-CDR3 of the V_{L} comprising the amino acid sequence of SEQ ID NO: 50 or 54.

63. The pharmaceutical composition of any one of claims 57, 59-62, or the method of any one of claims 58-62,
(a) wherein the antibody or antigen-binding fragment specifically binding to *K. pneumoniae* O2 antigen comprises:
a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34;
and wherein the antibody or antigen-binding fragment specifically binding to *K. pneumoniae* O1 antigen comprises:
a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53;
(b) wherein the antibody or antigen-binding fragment specifically binding to *K. pneumoniae* O2 antigen comprises:
a V_{H} comprising the amino acid sequence of SEQ ID NO: 20 or 29, or a variant thereof having at least about 80% sequence identity to the amino acid sequence of SEQ ID NO: 20 or 29; and a V_{L} comprising the amino acid sequence of SEQ ID NO: 25 or 34, or a variant thereof having at least about 80% sequence identity to the amino acid sequence of SEQ ID NO: 25 or 34;
and wherein the antibody or antigen-binding fragment specifically binding to *K. pneumoniae* O1 antigen comprises:
a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54;
(c) wherein the antibody or antigen-binding fragment specifically binding to *K. pneumoniae* O2 antigen comprises:
a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO: 24 or 33;
and wherein the antibody or antigen-binding fragment specifically binding to *K. pneumoniae* O1 antigen comprises:
a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53; or
(d) wherein the antibody or antigen-binding fragment specifically binding to *K. pneumoniae* O2 antigen comprises:
a V_{H} comprising the amino acid sequence of SEQ ID NO: 19 or 28, or a variant thereof having at least about 80% sequence identity to the amino acid sequence of SEQ ID NO: 19 or 28; and a V_{L} comprising the amino acid sequence of SEQ ID NO: 24 or 33, or a variant thereof having at least about 80% sequence identity to the amino acid sequence of SEQ ID NO:24 or 33;
and wherein the antibody or antigen-binding fragment specifically binding to *K. pneumoniae* O1 antigen comprises:
a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54.

64. A pharmaceutical composition, comprising: antibody or antigen-binding fragment specifically recognizing *Klebsiella pneumoniae* (*K. pneumoniae*) O2 antigen and antibody or antigen-binding fragment specifically recognizing *Klebsiella pneumoniae* (*K. pneumoniae*) O1 antigen; wherein the antibody or antigen-binding fragment specifically recognizing *Klebsiella pneumoniae* (*K. pneumoniae*) O1 antigen comprises:
(a) a heavy chain variable domain (V_{H}) comprising a heavy chain complementarity determining region (HC-CDR) 1 comprising the amino acid sequence of SEQ ID NO: 35; an HC-CDR2 comprising the amino acid sequence of SEQ ID NO: 37; and an HC-CDR3 comprising the amino acid sequence of SEQ ID NO: 39; and a light chain variable domain (V_{L}) comprising a light chain complementarity determining region (LC-CDR) 1 comprising the amino acid sequence of SEQ ID NO: 41; an LC-CDR2 comprising the amino acid sequence of SEQ ID NO: 43; and an LC-CDR3 comprising the amino acid sequence of SEQ ID NO: 45; or
(b) a heavy chain variable domain (V_{H}) comprising a heavy chain complementarity determining region (HC-CDR) 1 comprising the amino acid sequence of SEQ ID NO: 36; an HC-CDR2 comprising the amino acid sequence of SEQ ID NO: 38; and an HC-CDR3 comprising the amino acid sequence of SEQ ID NO: 40; and a light chain variable domain (V_{L}) comprising a light chain complementarity determining region (LC-CDR) 1 comprising the amino acid sequence of SEQ ID NO: 42; an LC-CDR2 comprising the amino acid sequence of SEQ ID NO: 44; and an LC-CDR3 comprising the amino acid sequence of SEQ ID NO: 46.

65. A method of treating and/or preventing a disease or condition in an individual in need thereof, comprising administering to the individual an effective amount of antibody or antigen-binding fragment specifically recognizing *Klebsiella pneumoniae* (*K. pneumoniae*) O2 antigen and antibody or antigen-binding fragment specifically recognizing *Klebsiella pneumoniae* (*K. pneumoniae*) O1 antigen; wherein the antibody or antigen-binding fragment specifically recognizing *Klebsiella pneumoniae* (*K. pneumoniae*) O1 antigen comprises:
(a) a heavy chain variable domain (V_{H}) comprising a heavy chain complementarity determining region (HC-CDR) 1 comprising the amino acid sequence of SEQ ID NO: 35; an HC-CDR2 comprising the amino acid sequence of SEQ ID NO: 37; and an HC-CDR3 comprising the amino acid sequence of SEQ ID NO: 39; and a light chain variable domain (V_{L}) comprising a light chain complementarity determining region (LC-CDR) 1 comprising the amino acid sequence of SEQ ID NO: 41; an LC-CDR2 comprising the amino acid sequence of SEQ ID NO: 43; and an LC-CDR3 comprising the amino acid sequence of SEQ ID NO: 45; or
(b) a heavy chain variable domain (V_{H}) comprising a heavy chain complementarity determining region (HC-CDR) 1 comprising the amino acid sequence of SEQ ID NO: 36; an HC-CDR2 comprising the amino acid sequence of SEQ ID NO: 38; and an HC-CDR3 comprising the amino acid sequence of SEQ ID NO: 40; and a light chain variable domain (V_{L}) comprising a light chain complementarity determining region (LC-CDR) 1 comprising the amino acid sequence of SEQ ID NO: 42; an LC-CDR2 comprising the amino acid sequence of SEQ ID NO: 44; and an LC-CDR3 comprising the amino acid sequence of SEQ ID NO: 46.

66. The pharmaceutical composition of claim 64, or the method of claim 65, wherein the antibody or antigen-binding fragment specifically recognizing *K. pneumoniae* O1 antigen comprises:
(a) a V_{H} comprising the amino acid sequence of SEQ ID NO: 47 or 51, or a variant thereof having at least about 80% sequence identity to the amino acid sequence of SEQ ID NO: 47 or 51; and a V_{L} comprising the amino acid sequence of SEQ ID NO: 49 or 53, or a variant thereof having at least about 80% sequence identity to the amino acid sequence of SEQ ID NO: 49 or 53; or a V_{H} comprising an HC-CDR1, an HC-CDR2 and an HC-CDR3 of the V_{H} comprising the amino acid sequence of SEQ ID NO: 47 or 51; and a V_{L} comprising an LC-CDR1, an LC-CDR2, and an LC-CDR3 of the V_{L} comprising the amino acid sequence of SEQ ID NO: 49 or 53; or
(b) a V_{H} comprising the amino acid sequence of SEQ ID NO: 48 or 52, or a variant thereof having at least about 80% sequence identity to the amino acid sequence of SEQ ID NO: 48 or 52; and a V_{L} comprising the amino acid sequence of SEQ ID NO: 50 or 54, or a variant thereof having at least about 80% sequence identity to the amino acid sequence of SEQ ID NO: 50 or 54; or a V_{H} comprising an HC-CDR1, an HC-CDR2 and an HC-CDR3 of the V_{H} comprising the amino acid sequence of SEQ ID NO: 48 or 52; and a V_{L} comprising an LC-CDR1, an LC-CDR2, and an LC-CDR3 of the V_{L} comprising the amino acid sequence of SEQ ID NO: 50 or 54.

67. The method of any one of claims 58-63, and 65-66, wherein the antibody or antigen-binding fragment specifically recognizing *K. pneumoniae* O2 antigen and the antibody or antigen-binding fragment specifically recognizing *K. pneumoniae* O1 antigen are administered to the individual concurrently or administered to the individual consecutively.

68. An isolated nucleic acid molecule that encodes the antibody or antigen-binding fragment specifically binding to *K. pneumoniae* O2 antigen according to any one of claims 1-9 or the bispecific antibody according to any one of claims 10-56.

69. A vector comprising the nucleic acid molecule of claim 65.

70. An isolated host cell comprising the antibody or antigen-binding fragment specifically binding to *K. pneumoniae* O2 antigen according to any one of claims 1-9, or the bispecific antibody according to any one of claims 10-56, the nucleic acid molecule of claim 68, or the vector of claim 69.

71. A method of producing the antibody or antigen-binding fragment specifically binding to *K. pneumoniae* O2 antigen according to any one of claims 1-9 or the bispecific antibody according to any one of claims 10-56, comprising:
a) culturing the host cell of claim 70 under conditions effective to express antibody; and
b) obtaining the expressed antibody from the host cell.

72. A pharmaceutical composition comprising the antibody or antigen-binding fragment specifically binding to *K. pneumoniae* O2 antigen according to any one of claims 1-9, or the bispecific antibody specifically binding to *K. pneumoniae* O2 antigen and O1 antigen according to any one of claims 10-56, the nucleic acid molecule of claim 68, or the vector of claim 69, or the isolated host cell of claim 70 and a pharmaceutically acceptable carrier or adjunct.

73. A method of treating and/or preventing a disease or condition in an individual in need thereof, comprising administering to the individual an effective amount of the pharmaceutical composition according to any one of claims 57, 59-64, 66 or 72, the bispecific antibody specifically binding to *K. pneumoniae* O1 antigen and O2 antigen according to any one of claims 10-56 or the antibody or antigen-binding fragment specifically binding to *K. pneumoniae* O2 antigen according to any one of claims 1-9.

74. The method any one of claims 58-63, 65-67, wherein the disease or condition comprises one or more symptoms caused by *Klebsiella* infection.

75. The method of claim 74, wherein the *Klebsiella* is *Klebsiella pneumoniae.*

76. The method of claim 75, wherein the disease or condition comprises pneumonia, urinary tract infection, septicaemia/bacteremia/sepsis, neonatal septicaemia/bacteremia/sepsis, diarrhea, soft tissue infection, infection after organ transplantation, surgical infection, wound infection, lung infection, purulent liver abscess, lung abscess, cellulitis, necrotizing myositis, myositis, endophthalmitis, peritonitis, meningitis, necrotizing meningitis, ankylosing spondylitis or spinal joint disease.
